# EUROPEAN PATENT APPLICATION

(11) **EP 2 415 756 A1**
(43) Date of publication of application: **08.02.2012**
(21) Application number: 10758619.0
(22) Date of filing: 29.03.2010
(51) Int. Cl.: C07C 275/28, A61K 31/44, A61K 31/4965, A61K 31/505, A61P 25/28, A61P 43/00, C07C 335/12, C07C 335/16, C07C 335/18, C07D 213/81, C07D 213/85, C07D 239/42, C07D 241/24, C07D 401/12, C07D 405/12

(54) **ISOTHIOUREA DERIVATIVES OR ISOUREA DERIVATIVES HAVING BACE1 INHIBITORY ACTIVITY**

(30) Priority: 31.03.2009 JP 2009084120; 25.12.2009 JP 2009293885
(71) Applicant: Shionogi & Co., Ltd., Osaka 541-0045 (JP)
(72) Inventor: TAMURA, Yuusuke, Osaka-shi Osaka 553-0002 (JP); SUZUKI, Shinji, Osaka-shi Osaka 553-0002 (JP); MATSUMOTO, Sae, Osaka-shi Osaka 553-0002 (JP); HORI, Akihiro, Osaka-shi Osaka 553-0002 (JP); KOORIYAMA, Yuuji, Osaka-shi Osaka 541-0045 (JP)
(74) Representative: Vossius & Partner
(86) International application number: PCT/JP2010/055528
(87) International publication number: WO 2010/113848

(57) **Abstract**

The present invention provides, for example, a compound of the following formula (I): 1 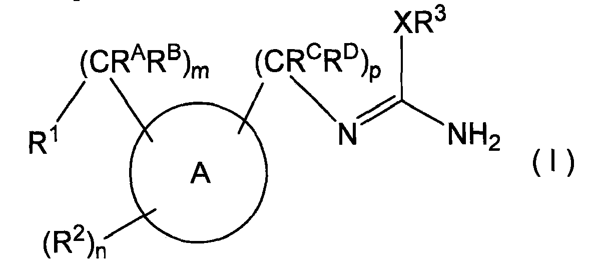 wherein R¹ is substituted amino and the like,
R² is halogen and the like,
R³ is substituted or unsubstituted lower alkyl and the like,
R^{A} and R^{B} are each independently hydrogen, substituted or unsubstituted lower alkyl and the like,
R^{C} and R^{D} are each independently hydrogen, substituted or unsubstituted lower alkyl, or R^{C} and R^{D} together with the carbon atom to which they are attached may form a substituted or unsubstituted carbocycle, and
ring A is a carbocycle or a heterocycle,
its pharmaceutically acceptable salt, or a solvate thereof as a therapeutic agent for diseases induced by production, secretion and/or deposition of amyloid-β proteins.

## Description

### [Techinical Field]

The present invention relates to a compound having an effect of inhibiting amyloid-β production and is useful as a therapeutic agent for diseases induced by production, secretion and/or deposition of amyloid-β proteins.

### [Background Art]

In the brains of patients with Alzheimer's disease, peptides each consisting of approximately 40 amino acids, called amyloid-β proteins, which widely accumulate outside neurons to form insoluble plaques (senile plaques) are observed. These senile plaques are thought to kill neurons and cause the onset of Alzheimer's disease. As therapeutic agents for Alzheimer's disease, agents promoting degradation of amyloid-β proteins and amyloid-β vaccines have been studied.
Secretases are enzymes which cleave a protein called amyloid-β precursor protein (APP) within a cell and generate an amyloid-β protein. An enzyme which produces N-terminals of amyloid-β proteins is called as BACE1 (beta-site APP-cleaving enzyme 1, β-secretase). It is considered that production of amyloid-β proteins may be suppressed by inhibiting this enzyme, and thus a substance with such an effect can serve as a therapeutic agent for Alzheimer's disease.
Patent Documents 1 to 16 etc. disclose BACE1 inhibitors, but each of these compounds has a structure different from those of the compounds of the present invention.
Patent Documents 17 to 19, and Non-Patent Document 1 disclose compounds having a structure similar to those of the compounds of the present invention. Each of these document discloses each of these compound is useful as a therapeutic agent for neurological diseases or NOS inhibitor and the like.

### [Prior Art]

### [Patent Document]

Patent Document 1: WO2007/049532 pamphlet
Patent Document 2: WO2008/133274 pamphlet
Patent Document 3: WO2008/133273 pamphlet
Patent Document 4: WO2009/151098 pamphlet
Patent Document 5: US2006/0183790
Patent Document 6: US2006/0183792
Patent Document 7: US2006/0183943

Patent Document 8: WO02/96897 pamphlet
Patent Document 9: WO04/043916 pamphlet
Patent Document 10: WO2005/05831 pamphlet
Patent Document 11: WO2005/097767 pamphlet
Patent Document 12: WO2006/041404 pamphlet
Patent Document 13: WO2006/041405 pamphlet
Patent Document 14: US2007/0004786
Patent Document 15: US2007/0004730
Patent Document 16: US2007/27199
Patent Document 17: WO95/09619 pamphlet
Patent Document 18: WO94/12165 pamphlet
Patent Document 19: WO96/18608 pamphlet

### [Non-Patent Document]

Non-Patent Document 1: Bioorg. Med. Chem.4189,11,(2003)

### [Summary of the invention]

### [The problems to be solved by the invention]

The present invention providers a compound which has an effect of inhibiting amyloid-β production, in particular a BACE1 inhibitory effect, and is useful as a therapeutic agent for diseases induced by production, secretion or deposition of amyloid-β proteins.

### [Means to solve the problems]

The present invention provides:
1) A compound of formula (I): 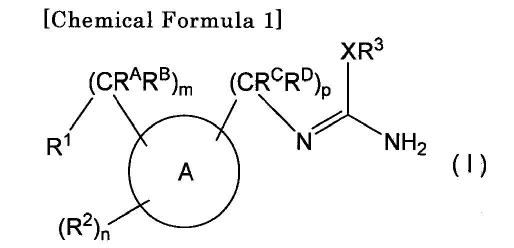 wherein X is an oxygen atom or a sulfur atom,
   ring A is a carbocycle or a heterocycle,
   (1) when X is an oxygen atom,
   R¹ is substituted amino, provided that substituted amino is not imino(phenoxy)methylamino or imino(lower alkoxy)methylamino; substituted or unsubstituted mercapto; substituted or unsubstituted carbocyclyl; or substituted or unsubstituted heterocyclyl;
   and R¹ may be hydrogen when ring A is naphthalene,
(2) when X is a sulfur atom,
   R¹ is substituted or unsubstituted acylamino, provided that "substituted or unsubstituted acylamino" is not unsubstituted acetylamino or unsubstituted benzoylamino; substituted or unsubstituted halogenosulfonylamino; substituted or unsubstituted lower alkylsulfonylamino; substituted or unsubstituted lower alkenylsulfonylamino; substituted or unsubstituted lower alkynylsulfonylamino; substituted or unsubstituted acylsulfonylamino; substituted or unsubstituted carbocyclylsulfonylamino; substituted or unsubstituted heterocyclylsulfonylamino; substituted or unsubstituted mercapto; substituted or unsubstituted carbocyclyl; or substituted or unsubstituted heterocyclyl, provided that "substituted or unsubstituted heterocyclyl" is not thiazolyl substituted with unsubstituted acetylamino;
   and R¹ may be hydrogen when ring A is naphthalene,
   R² is halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted acyl, substituted or unsubstituted carbocyclyl, or substituted or unsubstituted heterocyclyl,
   R³ is substituted or unsubstituted lower alkyl, substituted or unsubstituted lower alkenyl, substituted or unsubstituted lower alkynyl, substituted or unsubstituted carbocyclyl, or substituted or unsubstituted heterocyclyl,
   R^{A} and R^{B} are each independently hydrogen, halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted acyl, substituted or unsubstituted carbocyclyl, or substituted or unsubstituted heterocyclyl, or R^{A} and R^{B} taken together may form oxo, or R^{A} and R^{B} together with the carbon atom to which they are attached may form a substituted or unsubstituted carbocycle or a substituted or unsubstituted heterocycle,
   R^{C} and R^{D} are each independently hydrogen, halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted acyl, substituted or unsubstituted carbocyclyl, or substituted or unsubstituted heterocyclyl, or R^{C} and R^{D} together with the carbon atom to which they are attached may form a substituted or unsubstituted carbocycle or a substituted or unsubstituted heterocycle, m is an integer of 0 to 2,
   n is an integer of 0 to 4, and
   p is an integer of 0 to 2,
   its pharmaceutically acceptable salt, or a solvate thereof.
   1') A compound of fomula (I): 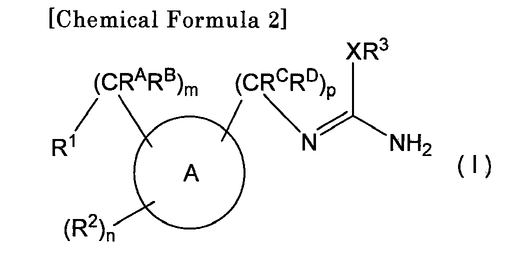 wherein
   X is a sulfur atom or an oxgen atom,
   ring A is a heterocycle or a carbocycle,
   R¹ is substituted amino, provided that (i) when X is a sulfur atom, then the substituent is not unsubstituted lower alkoxycarbonyl, imino(substituted mercapto)methyl or substituted 1,3,5-triazin-2-yl, and (ii) when X is an oxgen atom, then the substituent is not imino(phenoxy)methyl, imino(methoxy)methy or imino(ethoxy)methyl;
   substituted or unsubstituted mercapto; substituted or unsubstituted cycloalkyl substituted or unsubstituted aryl; or substituted or unsubstituted heterocyclyl, provided that when X is a sulfur atom, then substituted or unsubstituted heterocyclyl is not thiazolyl substituted with acetylamino, and
   R¹ may be hydrogen when ring A is naphthalene,
   R² is halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted cycloalkyl, substituted or unsubstituted cycloalkenyl, substituted or unsubstituted acyl, substituted or unsubstituted aryl or substituted or unsubstituted heterocyclyl,
   R³ is substituted or unsubstituted lower alkyl, substituted or unsubstituted lower alkenyl, substituted or unsubstituted lower alkynyl, substituted or unsubstituted cycloalkyl, substituted or unsubstituted cycloalkenyl, substituted or unsubstituted aryl, or substituted or unsubstituted heterocyclyl,
   R^{A} and R^{B} are each independently hydrogen, halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted cycloalkyl, substituted or unsubstituted cycloalkenyl, substituted or unsubstituted acyl, substituted or unsubstituted aryl or substituted or unsubstituted heterocyclyl, or R^{A} and R^{B} taken together may form oxo, or
   R^{A} and R^{B} together with the carbon atom to which they are attached may form a substituted or unsubstituted carbocycle,
   R^{C} and R^{D} are each independently hydrogen, halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted cycloalkyl, substituted or unsubstituted cycloalkenyl, substituted or unsubstituted acyl, substituted or unsubstituted aryl, or substituted or unsubstituted heterocyclyl, or R^{C} and R^{D} together with the carbon atom to which they are attached may form a substituted or unsubstituted carbocycle,
   m is an integer of 0 to 2,
   n is an integer of 0 to 4, and
   p is an integer of 0 to 2,
   its pharmaceutically acceptable salt, or a solvate thereof.
   1") The compound of formula (I): 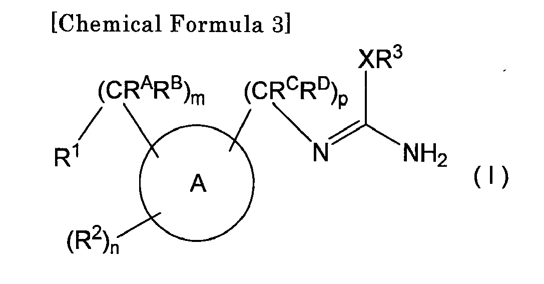 wherein R¹ is substituted amino, provided that the substituent of "amino" is not unsubstituted lower alkoxycarbonyl, imino(substituted mercapto)methyl or substituted 1,3,5-triazine-2-yl; substituted or unsubstituted mercapto; substituted or unsubstituted cycloalkyl; substituted or unsubstituted aryl; or substituted or unsubstituted heterocyclyl , provided that "substituted or unsubstituted heterocyclyl" is not thiazolyl substituted with acetylamino; and
   R² is halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted cycloalkyl, substituted or unsubstituted cycloalkenyl, substituted or unsubstituted acyl, substituted or unsubstituted aryl or substituted or unsubstituted heterocyclyl,
   R³ is substituted or unsubstituted lower alkyl, substituted or unsubstituted lower alkenyl, substituted or unsubstituted lower alkynyl, substituted or unsubstituted cycloalkyl, substituted or unsubstituted cycloalkenyl, substituted or unsubstituted aryl, or substituted or unsubstituted heterocyclyl,
   R^{A} and R^{B} are each independently hydrogen, halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted cycloalkyl, substituted or unsubstituted cycloalkenyl, substituted or unsubstituted acyl, substituted or unsubstituted aryl, or substituted or unsubstituted heterocyclyl, or R^{A} and R^{B} taken together may form oxo, or
   R^{A} and R^{B} together with the carbon atom to which they are attached may form a substituted or unsubstituted carbocycle,
   R^{C} and R^{D} are each independently hydrogen, halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted cycloalkyl, substituted or unsubstituted cycloalkenyl, substituted or unsubstituted acyl, substituted or unsubstituted aryl, or substituted or unsubstituted heterocyclyl, or R^{C} and R^{D} together with the carbon atom to which they are attached may form a substituted or unsubstituted carbocycle,
   ring A is a heterocycle or a carbocycle,
   X is a sulfur atom or an oxygen atom,
   m is an integer of 0 to 2,
   n is an integer of 0 to 4, and
   p is an integer of 0 to 2, provided that when ring A is a monocycle, then p is not 0,
   its pharmaceutically acceptable salt, or a solvate thereof.

2) The compound according to any one of the above 1), 1') or 1") wherein
   (1) when X is an oxygen atom, R¹ is substituted amino, provided that "substituted amino" is not imino(phenoxy)methylamino or imino(lower alkoxy)methylamino; substituted or unsubstituted mercapto; substituted or unsubstituted cycloalkyl; substituted or unsubstituted aryl; or substituted or unsubstituted heterocyclyl; and
   (2) when X is a sulfur atom, R¹ is substituted or unsubstituted acylamino, provided that "substituted or unsubstituted acylamino" is not unsubstituted acetylamino or unsubstituted benzoylamino; substituted or unsubstituted halogenosulfonylamino; substituted or unsubstituted lower alkylsulfonylamino; substituted or unsubstituted lower alkenylsulfonylamino; substituted or unsubstituted lower alkynylsulfonylamino; substituted or unsubstituted acylsulfonylamino; substituted or unsubstituted carbocyclylsulfonylamino; substituted or unsubstituted heterocyclylsulfonylamino; substituted or unsubstituted mercapto; substituted or unsubstituted cycloalkyl; substituted or unsubstituted aryl; or substituted or unsubstituted heterocyclyl, provided that "substituted or unsubstituted heterocyclyl" is not thiazolyl substituted with unsubstituted acetylamino;
      its pharmaceutically acceptable salt, or a solvate thereof
      2') The compound according to any one of the above 1), 1') or 1") wherein
      R¹ is substituted amino, provided that (i) when X is a sulfur atom, then the substituent is not unsubstituted lower alkoxycarbonyl, imino(substituted mercapto)methyl or substituted 1,3,5-triazin-2-yl; and (ii) when X is an oxygen atom, then the substituent is not imino(phenoxy)methyl, imino(methoxy)methyl or imino(ethoxy)methyl);
      substituted or unsubstituted mercapto; substituted or unsubstituted cycloalkyl; substituted or unsubstituted aryl; or substituted or unsubstituted heterocyclyl, provided that when X is a sulfur atom, then "substituted or unsubstituted heterocyclyl" is not thiazolyl substituted with acetylamino;
      its pharmaceutically acceptable salt, or a solvate thereof.
3) The compound according to any one of the above 1), 1'), 1"), 2) or 2") wherein
   ring A is an aromatic carbocycle or an aromatic heterocycle,
   m is an integer of 0,
   R¹ is substituted or unsubstituted acylamino, provided that when X is a sulfur atom, then "substituted or unsubstituted" acylamino is not unsubstituted acetylamino or unsubstituted benzoylamino; and
   R² is halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted acyl, substituted or unsubstituted carbocyclyl, or substituted or unsubstituted heterocyclyl,
   its pharmaceutically acceptable salt or a solvate thereof
4) The compound according to any one of the above 1), 1'), 1"), 2), 2') or 3), wherein X is an oxygen atom, its pharmaceutically acceptable salt or a solvate thereof.
5) The compound according to any one of the above 1), 1'), 1"), 2), 2') or 3),wherein X is a sulfur atom, its pharmaceutically acceptable salt or a solvate thereof.
6) The compound according to any one of the above 1), 1'), 1"), 2), 2') or 3)to 5) wherein (i) ring A is a monocycle and p is 1 or 2, or (ii) ring A is a fused ring and p is 0, its pharmaceutically acceptable salt or a solvate thereof.
7) The compound according to any one of the above 1), 1'), 1"), 2), 2') or 3)to 6) wherein ring A is a benzene ring or a benzene ring fused with another ring, its pharmaceutically acceptable salt or a solvate thereof.
7') The compound according to any one of the above 1), 1'), 1"), 2), 2') or 3) to 6) wherein the formula: 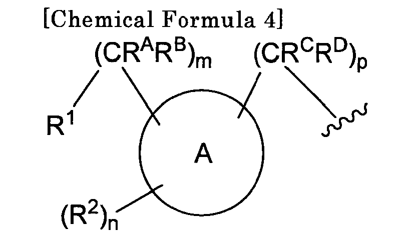 is 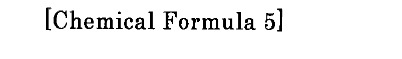 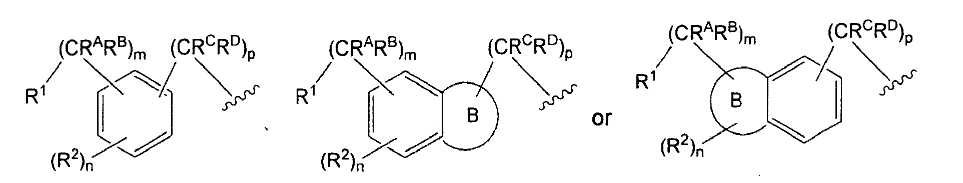 wherein R¹, R², R^{A}, R^{B}, R^{C}, R^{D}, m, n and p is the same as defined in the above 1), and ring B is a carbocycle.
8) The compound according to any one of the above 1), 1'), 1"), 2), 2') or 3) to 7) wherein R¹ is substituted or unsubstituted acylamino, provided that when X is a sulfur atom, then "substituted or unsubstituted acylamino" is not unsubstituted acetylamino or unsubstituted benzoylamino; substituted or unsubstituted halogenosulfonylamino; substituted or unsubstituted lower alkylsulfonylamino; substituted or unsubstituted lower alkenylsulfonylamino; substituted or unsubstituted lower alkynylsulfonylamino; substituted or unsubstituted acylsulfonylamino; substituted or unsubstituted carbocyclylsulfonylamino; substituted or unsubstituted heterocyclylsulfonylamino; or substituted or unsubstituted aryl; its pharmaceutically acceptable salt or a solvate thereof.
9) The compound according to any one of the above 1), 1'), 1"), 2), 2') or 3) to 8) wherein R¹ is substituted or unsubstituted acylamino, provided that when X is a sulfur atom, then "substituted or unsubstituted acylamino" is not unsubstituted acetylamino or unsubstituted benzoylamino; or substituted or unsubstituted lower alkylsulfonylamino; its pharmaceutically acceptable salt or a solvate thereof.
10) The compound according to any one of the above 1), 1'), 1"), 2), 2') or 3) to 9) wherein m is an integer of 0, its pharmaceutically acceptable salt or a solvate thereof.
11) The compound according to any one of the above 1), 1'), 1"), 2), 2') or 3) to 10) wherein R² is halogen and n is an integer of 0 or 1, its pharmaceutically acceptable salt or a solvate thereof.
12) The compound according to any one of the above the above 1), 1'), 1"), 2), 2') or 3) to 11) wherein R³ is substituted or unsubstituted lower alkyl or substituted or unsubstituted lower alkenyl, its pharmaceutically acceptable salt or a solvate thereof.
13) The compound according to any one of the above 1), 1'), 1"), 2), 2') or 3) to 12) wherein R^{C} and R^{D} are each independently hydrogen, substituted or unsubstituted lower alkyl, substituted or unsubstituted aryl or substituted or unsubstituted heterocyclyl, or R^{C} and R^{D} together with the carbon atom to which they are attached may form a substituted or unsubstituted carbocycle, and p is an integer of 1, its pharmaceutically acceptable salt or a solvate thereof.
13') The compound according to the above 4) wherein m is an integer of 0, R¹ is amino substituted with substituted or unsubstituted acyl, its pharmaceutically acceptable salt or a solvate thereof.
13") The compound according to the above 5) or 6) wherein ring A is a benzene ring, R¹ is substituted amino, substituted or unsubstituted mercapto, substituted or unsubstituted aryl, or substituted or unsubstituted heterocyclyl, and p is an integer of 1 or 2, its pharmaceutically acceptable salt or a solvate thereof.
14) A compound of formula (II): 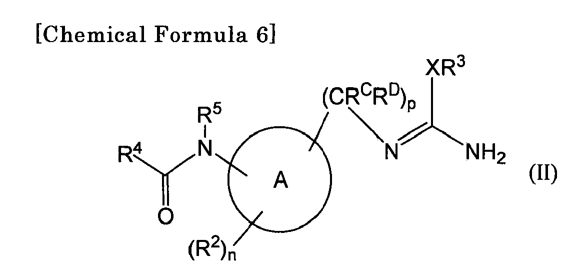 wherein ring A is a carbocycle or a heterocycle,
   R⁵ is hydrogen, substituted or unsubstituted lower alkyl, substituted or unsubstituted lower alkenyl, substituted or unsubstituted lower alkynyl or substituted or unsubstituted acyl,
   R⁴ is substituted or unsubstituted lower alkyl, provided that when X is a sulfur atom, then "substituted or unsubstituted lower alkyl" is not unsubstituted methyl; substituted or unsubstituted lower alkenyl; a substituted or unsubstituted carbocycle, provided that when X is a sulfur atom, then "a substituted or unsubstituted carbocycle" is not unsubstituted phenyl; or a substituted or unsubstituted heterocycle;
   R² is halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted acyl, substituted or unsubstituted carbocyclyl or substituted or unsubstituted heterocyclyl,
   R³ is substituted or unsubstituted lower alkyl, substituted or unsubstituted lower alkenyl, substituted or unsubstituted lower alkynyl, substituted or unsubstituted carbocyclyl or substituted or unsubstituted heterocyclyl,
   R^{C} and R^{D} are each independently hydrogen, halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted acyl, substituted or unsubstituted carbocyclyl, or substituted or unsubstituted heterocyclyl, or R^{C} and R^{D} together with the carbon atom to which they are attached may form a substituted or unsubstituted carbocycle or a substituted or unsubstituted heterocycle,
   n is an integer of 0 to 4, and
   p is an integer of 0 to 2,
   its pharmaceutically acceptable salt or a solvate thereof.
15) The compound according to the above 14) wherein R⁴ is a substituted or unsubstituted aromatic heterocycle or a substituted or unsubstituted aromatic carbocycle, its pharmaceutically acceptable salt or a solvate thereof.
16) The compound according to the above 14) or 15) wherein ring A is a monocycle, and R⁴ is a substituted or unsubstituted nitrogen-containing heterocycle, its pharmaceutically acceptable salt or a solvate thereof.
17) A pharmaceutical composition comprising the compound according to any one of the above 1), 1'), 1"), 2), 2'), or 3) to 16), its pharmaceutically acceptable salt or a solvate thereof.
18) A pharmaceutical composition having BACE1 inhibitory activity comprising the compound according to any one of the above 1), 1'), 1"), 2), 2'), or 3) to 16), its pharmaceutically acceptable salt or a solvate thereof.
19) A pharmaceutical composition having amyloid β production inhibitory activity comprising the compound according to any one of the above 1), 1'), 1"), 2), 2'), or 3) to 16), its pharmaceutically acceptable salt or a solvate thereof.
20) A method for inhibiting BACE1 activity comprising administering the compound according to any one of he above 1), 1'), 1"), 2), 2'), or 3) to 16), its pharmaceutically acceptable salt or a solvate thereof.
21) A compound according to any one of he above 1), 1'), 1"), 2), 2'), or 3) to 16), its pharmaceutically acceptable salt or a solvate thereof for use in a method for inhibiting BACE1 activity.
22) A method for inhibiting amyloid β production comprising administering the compound according to any one of the above 1), 1'), 1"), 2), 2'), or 3) to 16), its pharmaceutically acceptable salt or a solvate thereof.
23) A compound according to any one of the above 1), 1'), 1"), 2), 2'), or 3) to 16), its pharmaceutically acceptable salt or a solvate thereof for use in a method for inhibiting amyloid β production.
23') Use of the compound according to any one of the above 1), 1'), 1"), 2), 2'), or 3) to 16), its pharmaceutically acceptable salt or a solvate thereof in the manufacture of a medicament for inhibiting amyloid β production.
24) A method for treating diseases induced by production, secretion or deposition of amyloid-β proteins comprising administering the compound according to any one of the above 1), 1'), 1"), 2), 2'), or 3) to 16) or a pharmaceutically acceptable salt or a solvate thereof.
25) A compound according to any one of the above 1), 1'), 1"), 2), 2'), or 3) to 16) or a pharmaceutically acceptable salt or a solvate thereof for use in a method for treating diseases induced by production, secretion or deposition of amyloid-β proteins.
25') Use of the compound according to any one of the above 1), 1'), 1"), 2), 2'), or 3) to 16) or a pharmaceutically acceptable salt or a solvate thereof in the manufacture of a medicament for treating diseases induced by production, secretion or deposition of amyloid-β proteins.
26) A method for treating Alzheimer's disease comprising administering the compound according to any one of the above 1), 1'), 1"), 2), 2'), or 3) to 16) or a pharmaceutically acceptable salt or a solvate thereof.
27) A compound according to any one of the above 1), 1'), 1"), 2), 2'), or 3) to 16) or a pharmaceutically acceptable salt or a solvate thereof for use in a method for treating Alzheimer's disease.
27') Use of the compound according to any one of the above 1), 1'), 1"), 2), 2'), or 3) to 16) or a pharmaceutically acceptable salt or a solvate thereof in the manufacture of a medicament for treating Alzheimer's disease.

### [Effect of Invention]

The compound fo the present invention is useful as a therapeutic agent for diseases induced by production, secretion or deposition of amyloid-β proteins (e.g. Alzheimer's disease).

### [Modes for Carrying out the Invention]

The term "halogen" as used herein includes fluorine, chlorine, bromine and iodine.
The halogen portions in "halogenosulfonyl" and "halogenosulfonylamino" are as defined above for the "halogen."
The term "lower alkyl" as used herein includes C1-C15, preferably C1-C 10, more preferably C1-C6, and further preferably C1-C3 linear or branched alkyl. Examples thereof include methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl, n-decyl, n-undecyl, n-dodecyl, n-tridecyl, n-tetradecyl and n-pentadecyl.

The lower alkyl portions in "lower alkoxy", "hydroxy lower alkoxy", "lower alkoxy lower alkoxy", "lower alkoxy acylamino", "lower alkoxycarbonyl", "lower alkyl alcohol", "lower alkylsulfonyl", "lower alkylthio", "lower alkylamino", "lower alkylsulfonyl amino", "lower alkoxy imino", "lower alkylthio", "lower alkylcarbamoyl", "hydroxy lower alkylcarbamoyl", "lower alkysulfamoyl", "lower alkylsulfinyl "and "imino lower alkoxymethyl" are as defined above for the "lower alkyl."
Examples of the substituent of the "substituted or unsubstituted lower alkyl" are the substituent selected from the following Subsituent Group α. The term "substituted alkyl" includes alkyl substituted with one or more selected from the Substituent Group α.

The Substituent Group α herein consists of halogen, carboxy, lower alkoxycarbonyl, hydroxy optionally substituted with one or more selected from the Substituent Group β, amino optionally substituted with one or more selected from the Substituent Group β, acylamino optionally substituted with one or more selected from the Substituent Group γ, imino optionally substituted with one or more selected from the Substituent Group β, mercapto optionally substituted with one or more selected from the Substituent Group β, sulfamoyl, lower alkylsulfamoyl, lower alkylsulfinyl, lower alkylsulfonyl, lower alkenylsulfamoyl, loweralkenylsulfinyl, lower alkenylsulfonyl, lower alkynylsulfamoyl, lower alkynylsulfinyl, lower alkynylsulfonyl, acyl, cyano, nitro, oxo, carbocyclyl optionally substituted with one or more selected from the Substituent Group γ, and heterocyclyl optionally substituted with one or more selected from the Substituent Group γ.
The Substituent Group β herein consists of halogen, lower alkyl, lower alkenyl, lower alkynyl, cyano, carbocyclyl optionally substituted with one or more selected from the Substituent Group γ, and heterocyclyloptionally substituted with one or more selected from the Substituent Group γ.
The Substituent Group γ herein consists of halogen, hydroxy, lower alkyl, lower alkenyl, lower alkynyl, lower alkoxy, hydroxy lower alkoxy, lower alkoxy lower alkoxy, lower alkenyloxy, lower alkynyloxy, acyl, acyloxy, carboxy, lower alkoxycarbonyl, amino, acylamino, lower alkylamino, imino, hydroxyimino, lower alkoxyimino, lower alkylthio, carbamoyl, lower alkylcarbamoyl, hydroxy lower alkylcarbamoyl, sulfamoyl, lower alkylsulfamoyl, lower alkylsulfinyl, lower alkylsulfonyl, cyano, nitro, carbocyclyl and heterocyclyl.
The substituents of "substituted or unsubstituted lower alkylsulfonylamino" are the same as those of "substituted or unsubstituted lower alkyl."

The term "lower alkenyl" as used herein includes linear or branched C2-C15, preferably C2-C10, more preferably C2-C6, more preferably C2-C4 alkenyl having one or more double bonds at optional positions. Examples include vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl and pentadecenyl.
The term "lower alkynyl" as used herein includes straight or branched C2-C10, preferably C2-C8, more preferably C3-C6 alkynyl having one or more triple bonds at optional positions. Exampless include ethynyl, propynyl, butynyl, pentynyl, hexynyl, heptynyl, octynyl, nonyl and decynyl. These may further a double bond at an optional position.
Examples of the substituent of "substituted or unsubstituted lower alkenyl" and "substituted or unsubstituted lower alkynyl" include one or more selected from the above Substituent Group α. The terms "substituted alkenyl" and "substituted alkynyl" as used herein include alkenyl and alkynyl, each of which is substituted with one or more selected from Substituent Group α.
The lower alkenyl portions in "lower alkenylsulfonyl", "lower alkenyloxy", "lower alkenyloxy acylamino", "lower alkenylamino", "lower alkenylsulfonyl amino", "lower alkenylsulfamoyl" and "lower alkenylsulfinyl" are as defined above for the "lower alkenyl."
The lower alkynyl portions in "lower alkynyloxy", "lower alkynyloxy acyl amino", "lower alkynylamino", "lower alkynylsulfonyl", "lower alkynylsulfonylamino", "lower alkynylsulfamoyl" and "lower alkynylsulfinyl" are as defined above for the "lower alkynyl."
The substituents of "substituted or unsubstituted lower alkenylsulfonylamino" and "substituted or unsubstituted lower alkynylsulfonylamino" are the same as those of "substituted or unsubstituted lower alkenyl" and "substituted or unsubstituted lower alkynyl."

Examples of substituents of "substituted or unsubstituted hydroxy", "substituted mercapto", "substituted or unsubstituted mercapto", "substituted amino" and "substituted or unsubstituted amino" include substituted or unsubstituted lower alkyl, substituted or unsubstituted lower alkenyl, substituted or unsubstituted lower alkynyl, substituted or unsubstituted acyl, substituted or unsubstituted lower alkoxycarbonyl, substituted or unsubstituted halogenosulfonyl, substituted or unsubstituted lower alkylsulfonyl, substituted or unsubstituted lower alkenyl sulfonyl, substituted or unsubstituted lower alkynylsulfonyl, substituted or unsubstituted cycloalkylsulfonyl, substituted or unsubstituted cycloalkenylsulfonyl, substituted or unsubstituted acylsulfonyl, substituted or unsubstituted arylsulfonyl, substituted or unsubstituted heterocyclylsulfonyl, substituted or unsubstituted carbocyclyl and substituted or unsubstituted heterocyclyl. "Substituted or unsubstituted hydroxy", "substituted mercapto", "substituted or unsubstituted mercapto", "substituted amino " and "substituted or unsubstituted amino" can be optionally substituted with 1 to 2 groups selected from the above.
The amino portions in "substituted or unsubstituted acylamino", "substituted or unsubstituted halogenosulfonylamino", "substituted or unsubstituted lower alkylsulfonylamino", "substituted or unsubstituted lower alkenylsulfonylamino", "substituted or unsubstituted lower alkynylsulfonylamino", "substituted or unsubstituted cycloalkyl sulfonylamino", "substituted or unsubstituted cycloalkenyl sulfonylamino", "substituted or unsubstituted acylsulfonylamino", "substituted or unsubstituted arylsulfonylamino "and "substituted or unsubstituted heterocyclylsulfonylamino" may be further substituted with a group selected from the following groups.
Lower alkyl optionally substituted with one or more selected from Substituent Group α, lower alkenyl optionally substituted with one or more selected from Substituent Group α, lower alkynyl optionally substituted with one or more selected from Substituent Group α, acyl optionally substituted with one or more selected from Substituent Group α, carboxy, lower alkoxycarbonyl, hydroxy optionally substituted with one or more selected from Substituent Group β, amino optionally substituted with one or more selected from Substituent Group β, acylamino optionally substituted with one or more selected from Substituent Group γ, mercapto optionally substituted with one or more selected from Substituent Group β, sulfamoyl, lower alkylsulfamoyl, lower alkylsulfinyl, lower alkylsulfonyl, lower alkenylsulfamoyl, lower alkenylsulfinyl, lower alkenylsulfonyl, lower alkynylsulfamoyl, lower alkynylsulfinyl, lower alkynylsulfonyl, acyl, cyano, carbocyclyl optionally substituted with one or more selected from Substituent Group γ, and heterocyclyl optionally substituted with one or more selected from Substituent Group γ.
The term "acyl" as used herein includes C1-C10 aliphatic acyl, carbocyclylcarbonyl and heterocyclylcarbonyl. Examples include formyl, acetyl, propionyl, butyryl, isobutyryl, valeryl, pivaloyl, hexanoyl, acryloyl, propioloyl, methacryloyl, crotonoyl, benzoyl, cyclohexanecarbonyl, pyridinecarbonyl, furancarbonyl, thiophenecarbonyl, benzothiazolecarbonyl, pyrazinecarbonyl, piperidinecarbonyl and thiomorpholino.
The acyl portions in "acylamino", "lower alkoxy acylamino", "lower alkenyloxy acylamino", "lower alkynyloxy acylamino", "acylsulfonyl "and "acylsulfonylamino" are as defined above.
Examples of the substituent of the "substituted or unsubstituted acyl " include the substituent selected from the above Subsitutent Group α. The term "substituted acyl" include acyl substituted with one or more selected from Substituent Group α. The ring portions in carbocyclylcarbonyl and heterocyclylcarbonyl may be substituted with one or more selected from the group of (i) lower alkyl optionally substituted with one or more selected from Substituent Group α, (ii) lower alkenyl optionally substituted with one or more selected from Substituent Group α, (iii) lower alkynyl optionally substituted with one or more selected from Substituent Group α, and (iv) Substituent Group α.
The substituents of "substituted or unsubstituted acylamino "and "substituted or unsubstituted acylsulfonylamino" are the same as those of "substituted or unsubstituted acyl"

The term "carbocycle" as used herein includes C3-C10 cycloalkane, C3-C10 cycloalkene, aromatic carbocycle and non-aromatic fused carbocycle. The carbocycle portions in "carbocyclylcarbonyl" and "carbocyclylsulfonylamino" are the same as the above "carbocycle."
The term "carbocyclyl" as used herein includes cycloalkyl, cycloalkenyl, aryl and non-aromatic fused carbocyclyl.
The term "cycloalkane" includes C3-C10, preferably C3-C8, and more preferably C4-C8 carbocycle. Examples are cyclopropane, cyclobutane, cyclopentane, cyclohexane, cycloheptane, cyclooctane, cyclononane and cyclodecane.
The term "cycloalkyl" as used herein includes C3-C10, preferably C3-C8, and more preferably C4-C8 carbocyclyl. Examples are cyclopropyl, cyclobutyl, cyclopentyl, cyclohexyl,cycloheptyl, cyclooctyl, cyclononyl and cyclodecyl.
The cycloalkyl portions in "cycloalkylsulfonyl "and "cycloalkylsulfonylamino" are as defined for the "cycloalkyl."
The term "cycloalkene" as used herein includes a ring of the above "cycloalkane" having one or more double bonds at optional positions such as cyclopropene, cyclobutene, cyclopentene, cyclohexene, cycloheptene, cyclooctene and cyclohexadiene.
The term "cycloalkenyl" as used herein includes a ring of the above "cycloalkyl" having one or more double bonds at optional positions such as cyclopropenyl, cyclobutenyl, cyclopentenyl, cyclohexenyl, cycloheptenyl, cyclooctenyl and cyclohexadienyl.
The cycloalkenyl portion in "cycloalkenylsulfonyl" and "cycloalkenylsulfonylamino" are as defined above for the "cycloalkenyl."
The term "aromatic carbocycle" as used herein includes C6-C14, preferably C6-C10, more preferably C6 carbocycle and examples are a benzene ring, a naphtharene ring, an anthracene ring, and a phenanthrene ring. Specific example is a benzene ring.
The term "aryl" as used herein includes phenyl, naphthyl, anthryl and phenanthryl. Specific example is phenyl.
The aryl portions in "arylsulfonyl" and "arylsulfonylamino " are as defined for the "aryl."
The term as used herein "non-aromatic fused carbocycle " includes fused rings comprising two or more rings, each of ring is selected from the group of "cycloalkane", "cycloalkene" and "aromatic carbocycle", and at least one ring is "cycloalkane" or "cycloalkene." Examples are indane, indene and fluorine.
The term as used herein "non-aromatic fused carbocyclyl" include a monovalent group derived by eliminating a hydrogen atom from the ring of the "non-aromatic fused carbocycle" such as indenyl, indenyl and fluorenyl.
The phrase as used herein "together with the carbon atom to which they are attached may form a carbocycle" includes the "carbocycle" which is formed by two substituents and the carbon atom to which the substituents are attached such as 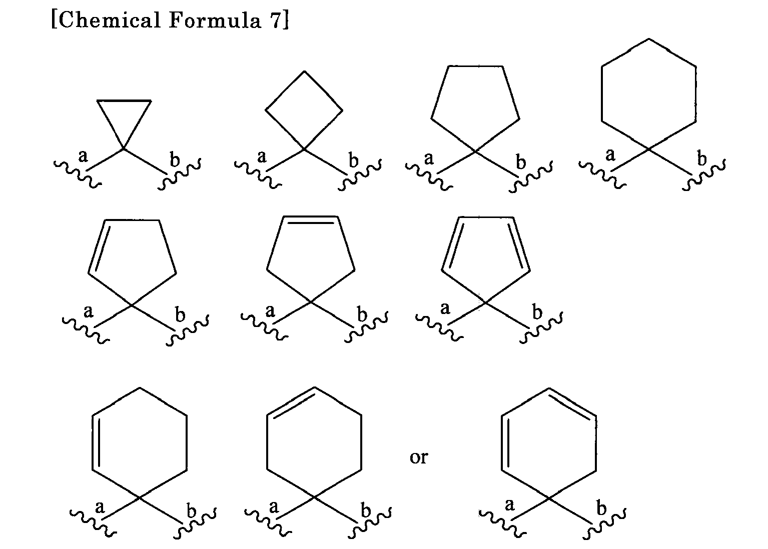 wherein either one of bonds indicated by a and b bonds to ring A,
The phrase as used herein "together with the carbon atom to which they are attached may form a substituted or unsubstituted carbocycle" includes the carbocycle optionally substituted with the after-mentioned substituents for "substituted or unsubstituted carbocycle."

The term as used herein "heterocyclyl" includes heterocyclyl having one or more hetero atoms arbitrarily selected from O, S and N in the ring. Specific examples thereof include 5 to 6- membered monocyclic heteroaryl such as pyrrolyl, imidazolyl, pyrazolyl, pyridyl, pyridazinyl, pyrimidinyl, pyrazinyl, triazolyl, triazinyl, tetrazolyl, furyl, thienyl, isoxazolyl, oxazolyl, oxadiazolyl, isothiazolyl, thiazolyl, and thiadiazolyl; 5- to 6- membered monocyclic non-aromatic heterocyclyl such as dioxanyl, thiiranyl, oxyranyl, oxetanyl, oxathiolanyl, azetidinyl, thianyl, thiazolidinyl, pyrrolidinyl, pyrrolinyl, imidazolidinyl, imidazolinyl, pyrazolidinyl, pyrazolinyl, piperidyl, piperazinyl, morpholinyl, morpholino, thiomorpholinyl, thiomorpholino, dihydropyridyl, tetrahydropyridyl, tetrahydrofuryl, tetrahydropyranyl, dihydrothiazolyl, tetrahydrothiazolyl, tetrahydroisothiazolyl, dihydrooxazinyl, hexahydroazepinyl, tetrahydroazepinyl, and tetrahydropyridazinyl;
fused bicyclic heterocyclic groups such as indolyl, isoindolyl, indazolyl, indolizinyl, indolinyl, isoindolinyl, quinolyl, isoquinolyl, cinnolinyl, phthalazinyl, quinazolinyl, naphthyridinyl, quinoxalinyl, purinyl, pteridinyl, benzopyranyl, benzimidazolyl, benzotriazolyl, benzisooxazolyl, benzoxazolyl, benzoxadiazolyl, benzoisothiazolyl, benzothiazolyl, benzothiadiazolyl, benzofuryl, isobenzofuryl, benzothienyl, benzotriazolyl, thienopyridyl, thienopyrrolyl, thienopyrazolyl, thienopyrazinyl, furopyrrolyl, thienothienyl, imidazopyridyl, pyrazolopyridyl, thiazolopyridyl, pyrazolopyrimidinyl, pyrazolotriazinyl, pyridazolopyridyl, triazolopyridyl, imidazothiazolyl, pyrazinopyridazinyl, quinazolinyl, quinolyl, isoquinolyl, naphthyridinyl, dihydrothiazolopyrimidinyl, tetrahydroquinolyl,tetrahydroisoquinolyl, dihydrobenzofuryl, dihydrobenzoxazinyl, dihydrobenzimidazolyl, tetrahydrobenzothienyl, tetrahydrobenzofuryl, benzodioxolyl, benzodioxonyl, chromanyl, chromenyl, octahydrochromenyl, dihydrobenzodioxynyl, dihydrobenzooxezinyl, dihydrobenzodioxepinyl and dihydrothienodioxynyl; fused tricyclic heterocyclyl such as carbazolyl, acridinyl, xanthenyl, phenothiazinyl, p henoxathiinyl, phenoxazinyl, dibenzofuryl, imidazoquinolyl and tetrahydrocarbazolyl. Preferable examples are 5 to 6-membered monocyclic heteroaryl or non-aromatic het erocyclyl.

The term as used herein "heterocycle "is a cyclic structure which is a constituent of the "heterocyclyl."
The heterocyclyl portions in "heterocyclylsulfonyl", "heterocyclylsulfonylamino "and "heterocyclylcarbonyl" are as defined above for the "heterocyclyl."
The term as used herein "aromatic heterocycle" includes aromatic rings in a cyclic structure which is a constituent of the "heterocyclyl." Examples are pyrrole, imidazole, pyrazole, pyridine, pyridazine, pyrimidine, pyrazine, triazole, triazine, tetrazole, furan, thiophen, isoxazole, oxazole, oxadiazole, isothiazole, thiazole, thiadiazole, indole, isoindole, indazole, indolizine, indoline, isoindoline, quinoline, isoquinoline, cinnoline, phthalazine, quinazoline, naphthyridine, quinoxa;ome, purine, pteridine, benzimidazole, benzotriazole, benzisooxazole, benzoxazole, benzoxadiazole, benzoisothiazole, benzothiazole, benzothiadiazole, benzofuran, isobenzofuran, benzothiophen, benzotriazole, thienopyridine, thienopyrrole, thienopyrazole, thienopyrazine, furopyrrole, thienothiophen, imidazopyridine, pyrazolopyridine, thiazolopyridine, pyrazolopyrimidine, pyrazolotriazine, pyridazolopyridine, triazolopyridine, imidazothiazole and pyrazinopyridazine.
The term as used herein "nitrogen-containing heterocycle" includes rings which contain one or more nitrogen atoms in the above "heterocycle" such as pyrrole, imidazole, pyrazole, pyridine, pyridazine, pyrimidine, pyrazine, triazole, triazine, tetrazole, isoxazole, oxazole, oxadiazole, isothiazole, thiazole, thiadiazole, pyrrolidine, pyrroline, imidazolidine, imidazone, pyrazolidine, pyrazoline, piperidine, piperazine, morpholine, thiomorpholine, dihydropyridine and tetrahydropyridine. Specific examples are pyrrole, imidazole, pyrazole, pyridine, pyridazine, pyrimidine, pyrazine, triazole, triazine and tetrazole.
The term as used herein "nitrogen-containing heterocyclyl" includes a monovalent group derived from the "nitrogen-containing heterocycle."
The phrase as used herein "together with the carbon atom to which they are attached may form a heterocycle" includes the "heterocycle" which is formed by two substituent and the carbon atom to which the substituents are attached such as 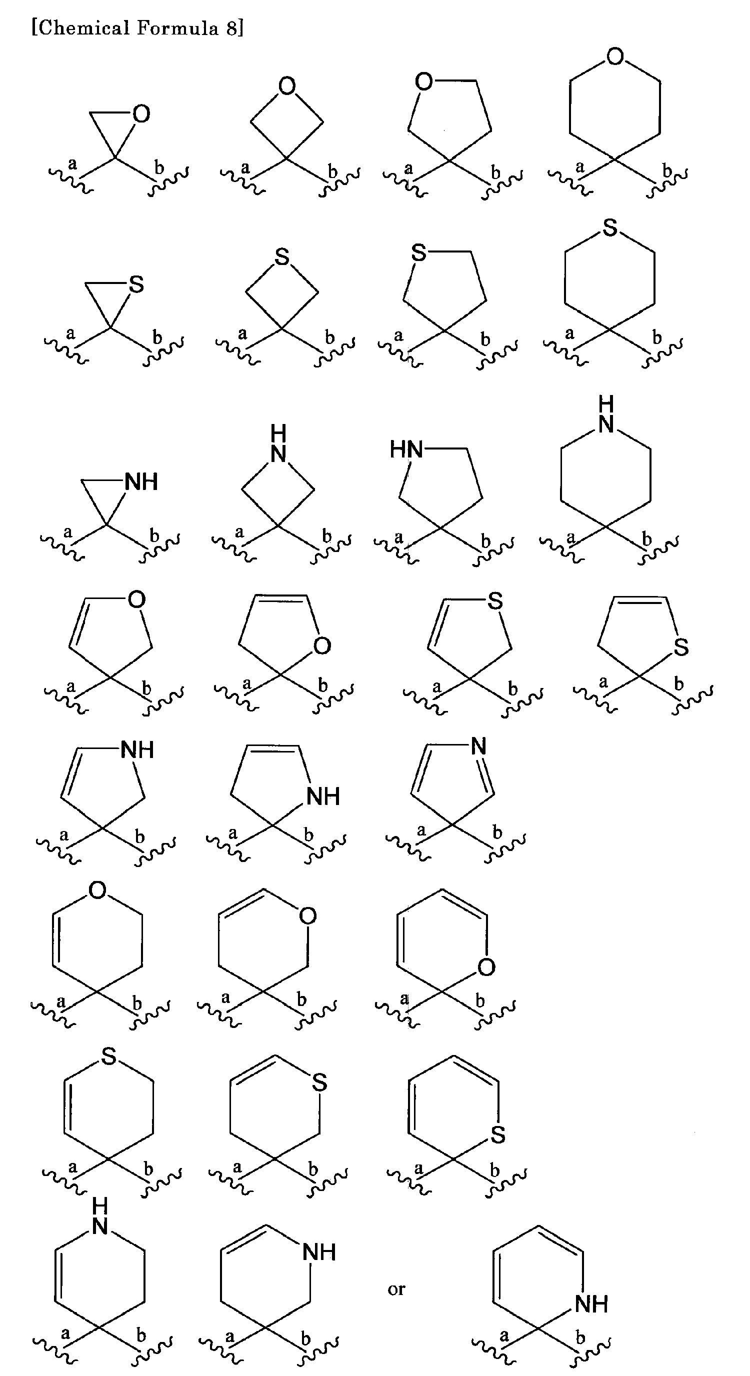 wherein either one of bonds indicated by a and b bond to ring A, The phrase as used herein "together with the carbon atom to which they are attached may form substituted or unsubstituted heterocycle" includes the heterocycle optionally substituted with the substituents of the after-mentioned "substituted or unsubstituted heterocycle."

Examples of the substituent of "substituted or unsubstituted carbocycle", "substituted or unsubstituted aromatic carbocycle", "substituted or unsubstituted carbocyclyl", "substituted or unsubstituted cycloalkyl", "substituted or unsubstituted cycloalkyl sulfonylamino", "substituted or unsubstituted cycloalkenyl", "substituted or unsubstituted cycloalkenyl sulfonylamino", "substituted or unsubstituted aryl", "substituted or unsubstituted arylsulfonylamino", "substituted or unsubstituted heterocycle", "substituted or unsubstituted aromatic heterocycle", "substituted or unsubstituted nitrogen-containing heterocycle", "substituted or unsubstituted heterocyclyl" and "substituted or unsubstituted heterocyclylsulfonylamino" are one or more selected from the group of lower alkyl optionally substituted with one or more selected from the Substituent Group α, lower alkenyl optionally substituted with one or more selected from the Substituent Group α, lower alkynyl optionally substituted with one or more selected from the Substituent Group α, lower alkoxyl optionally substituted with one or more selected from the Substituent Group α, and one or more selected from the Substituent Group α.
The term "heteroaryl" as used herein includes aromatic heterocyclyl in the "heterocyclyl."
The phrase "a benzene ring fused with another ring" as used herein includes groups formed by fusion of a benzene rng and another cyclic group selected from the above "carbocycle "or the above "heterocycle", and the examples are naphthalene, indane, indene, indole, isoindole, indazole, chromen, chroman, quinoline, isoquinoline, cinnoline, quinazoline, quinoxaline, benzofuran, benzimidazole, benzisoxazole, benzopyrazole, benzothiophen, benzothiazole. Examples of "ring A is a benzene ring fused with another ring" includes 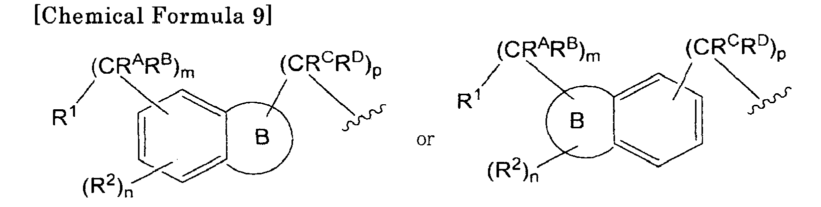 wherein R¹, R², R^{A}, R^{B}, R^{C}, R^{D}, m, n, and p are as defined above, ring B is a carbocycle or a heterocycle.
Examples of ring B are benzene, cycloalkane and pyridine.

The term "solvate" as used herein includes, for example, solvates with organic solvents and hydrates. In the case that a hydrate is formed, the compound or salt may be coordinated with any number of water molecules.
The compound of formula (I) include pharmaceutically acceptable salts thereof. Examples thereof include salts with alkaline metals (e.g. lithium, sodium and potassium), alkaline earth metals (e.g. magnesium and calcium), ammonium, organic bases and amino acids, and salts with inorganic acids (e.g. hydrochloric acid, sulfuric acid, nitric acid, hydrobromic acid, phosphoric acid and hydroiodic acid) and organic acids (e.g. formic acid, acetic acid, trifluoroacetic acid, citric acid, lactic acid, tartaric acid, oxalic acid, maleic acid, fumaric acid, mandelic acid, glutaric acid, malic acid, benzoic acid, phthalic acid, benzenesulfonic acid, p-toluenesulfonic acid, methanesulfonic acid and ethanesulfonic acid). Specifically preferable are hydrochloric acid, formic acid, hydroiodic acid and trifluoroacetic acid. These salts may be formed by a routine method.

The compound of formula (I) is not limited to a specific isomer, and include any possible isomers (e.g. keto-enol isomers, imine-enamine isomers, diastereoisomers, optical isomers and rotamers) and racemic mixtures. For example, a compound (I) incoudes the following tautomers: 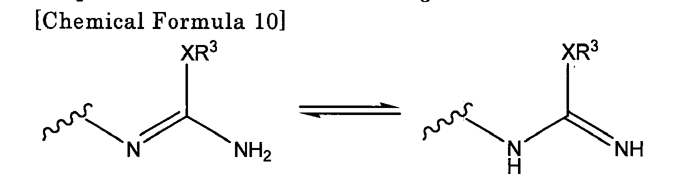 In addition, one or more hydrogen, carbon or other atoms of a compound of formula (I) can be replaced by an isotope of the hydrogen, carbon or other atoms. Compounds of formula (I) include all radiolabeled forms of compounds of formula (I). The "radiolabeled," "radiolabeled form", and the like of a compound of formula (I) are encompassed by the invention and useful as a research and/or diagnostic tool in metabolism pharmacokinetic studies and in binding assays.
Examples of isotopes that can be incorporated into a compound of formula (I) of the invention include isotopes of hydrogen, carbon, nitrogen, oxygen, phosphorous, sulfur, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively. Radiolabeled compounds of the invention can be prepared by methods known in the art. For example, tritiated compounds of formula (I) can be prepared by introducing tritium into the particular compound of formula (I), for example, by catalytic dehalogenation with tritium. This method may include reacting a suitably halogen substituted precursor of a compound of formula (I) with tritium gas in the presence of a suitable catalyst such as Pd/C, in the presence or absence of a base. Other suitable methods for preparing tritiated compounds can be found in Filer, "The Preparation and Characterization of Tritiated Neurochemicals," Chapter 6, pp. 155-192 in Isotopes in the Physical and Biomedical Sciences, Vol. 1, Labeled Compounds (Part A) (1987). ¹⁴C-labeled compounds can be prepared by employing starting materials having a ¹⁴C carbon.

For example, the compound of formula (I) wherein X is a sulfur atom can be prepared by the following procedures for synthesis of Compound d. 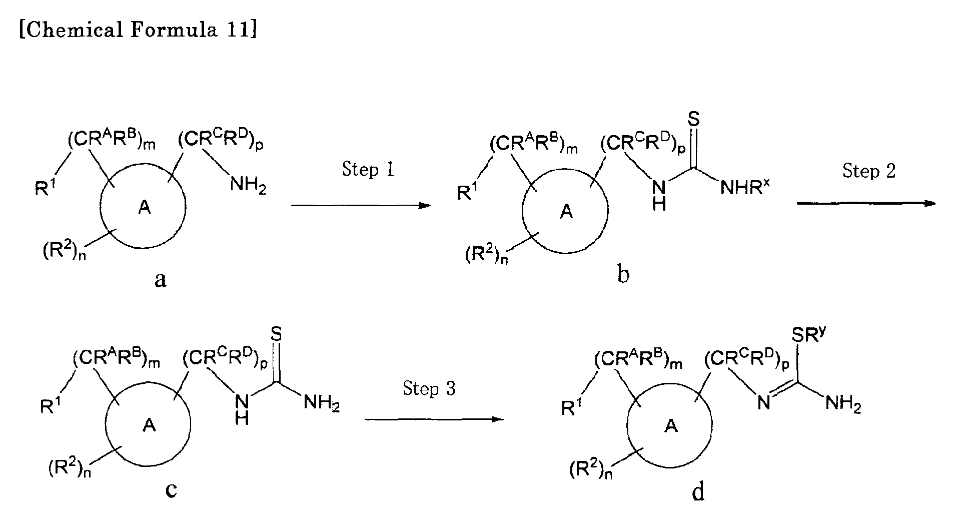 wherein each symbol is as defined above.

### Step 1

To a solution of Compound a in a solvent such as dichloromethane, dioxane, tetrahydrofuran, toluene or acetone, or a mixed solvent thereof is added an isothiocyanate having a protecting group which is commercially available such as benzoylisothiocyanate or which can be prepared by known methods. The mixture is allowed to treat at a temperature between -30°C to 100°C, preferably between -20°C to 70°C for 0.1 to 12 hours, preferably 0.1 to 4 hours to afford Compound b.

### Step 2

To a solution of Compound b in a solvent such as methanol, ethanol or isopropyl alcohol, or a mixed solvent thereof is added a base such as an aqueous solution of sodium hydroxide, an aqueous solution of potassium hydroxide or an aqueous solution of lithium hydroxide. The mixture is allowed to treat at a temperature between -20°C to 120°C, preferably between 10°C to 100°C for 0.1 to 6 hours, preferably 0.5 to 3 hours to affored Compund c.

### Step 3

To a solution of Compound c in a solvent such as dioxane, tetrahydrofuran, acetonitrile, dimethylformamide, dimethylacetoamide, N-methyl pyrrolidone or acetone, or a mixed solvent thereof is added an alkylating agent such as alkyl iodide, alkyl bromide or alkyl sulfate. The mixture is allowed to treat at a tempreture between -20°C to 120°C, preferably between 10°C to 100°C for 0.1 to 12 hours, preferably 0.5 to 6 hours to afford Compound d.

Compound of the formula (I) can also be prepared from Compound b by the following procedures for synthesis of Compound d 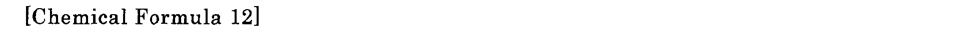 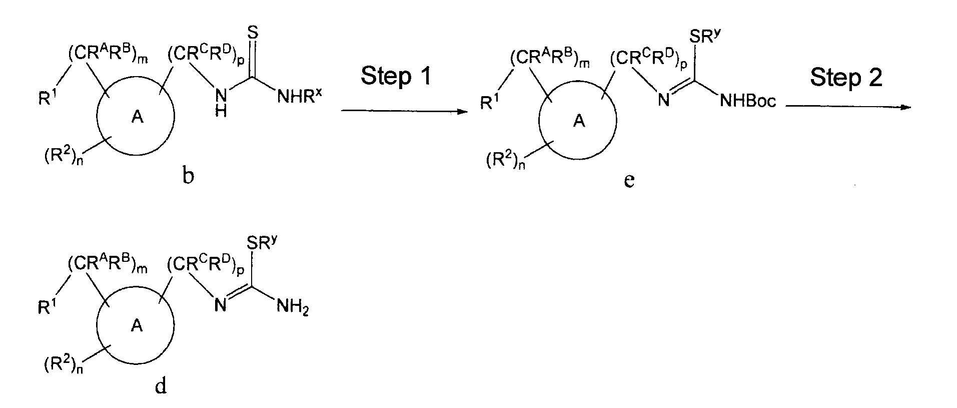 wherein each symbol is as defined above.

### Step 1

To a solution of Compound b in a solvent such as methanol, ethanol or isopropyl alcohol, or a mixed solvent thereof is added a base such as an aqueous solution of sodium hydroxide, an aqueous solution of potassium hydroxide or an aqueous solution of lithium hydroxide. The mixture is allowed to treat at a temperature between -20°C to 120°C, preferably between 10°C to 100°C for 0.1 to 6 hours, preferably 0.5 to 3 hours.
Then the mixture is extracted and the extract is dissolved in a solvent such as dioxane, tetrahydrofuran, acetonitrile, dimethylformamide, dimethylacetoamide, N-methylpyrrolidone or acetone, or a mixed solvent thereof. An alkylating agent such as alkyl iodide, alkyl bromide or alkyl sulfate is added to the mixture and the mixture is allowed to treat at a temperature between -20°C to 120°C, preferably between 10°C to 100°C for 0.1 to 12 hours, preferably 0.5 to 6 hours.
The mixture is concentrated and replaced a solvent such as methanol, ethanol, isopropylalcohol, dimethylformamide or tetrahydrofuran, or a mixed solvent thereof. A base such as triethylamine, diisopropylamine, diisopropylethyl and a Boc agent such as t-butyl dicarbonate are added to the mixture and the mixture is allowed to treat at a temperature between -20°C to 120°C, preferably between 10°C to 100°C or 0.1 to 4 hours, preferably, 0.5 to 2 hours to afford Compound e.

### Step 2

To a solution of Compound e in a solvent such as dichloromethan, chloroform, dioxane, tetrahydrofuran, acetonitrile or acetone, or a mixed solvent thereof is added an acid such as trifluoroacetic acid, hydrochloric acid or hydrobromic acid. The mixture is allowed to treat at a temperature between -20°C to 100°C, preferably between 10°C to 80°C for 0.1 to 4 hours, preferably 0.5 to 2 hours to afford Compound d.

For example, the compound of formula (I) wherein X is an oxygen atom can be prepared by the following procedures for synthesis of Compound g. 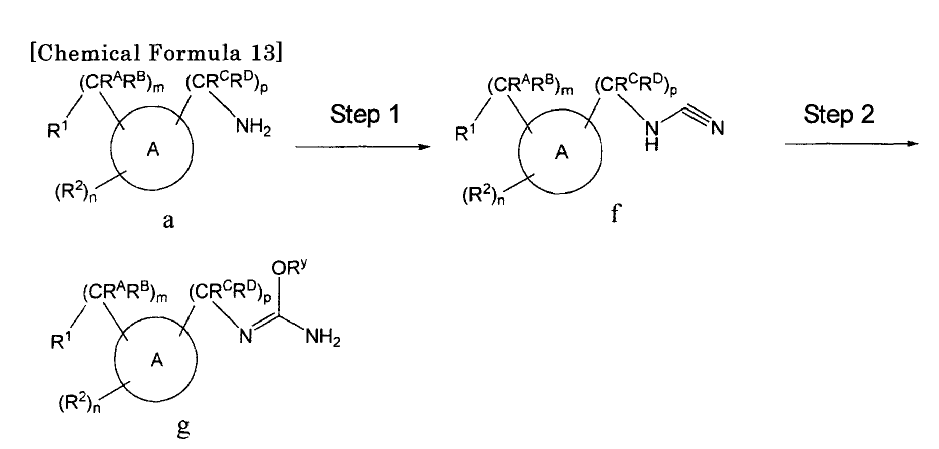 wherein each symbol is as defined above.

### Step 1

To a solution of Compound a in a solvent such as dichloromethane, chloroform, dioxane, tetrahydrofuran, toluene, acetone or methanol, or a mixed solvent thereof are added cyanogen bromide and, if necessary, a base such as triethylamine, sodium carbonate or sodium hydrogen carbonate. The mixture is allowed to treat at a temperature between -30°C to 100°C, preferably between -10°C to 80°C for 0.1 to 6 hours, preferably 0.5 to 4 hours to afford Compound f.

### Step 2

To a solution of Compound f in lower alkyl alcohol such as methanol is added an acid such as hydrogen chloride, sulfuric acid or perchloric acid. The mixture is allowed to treat at a temperature between -30°C to 100°C, preferably between -10°C to 50°C for 0.5 to 6 hours, preferably 1 to 4 hours to afford Compound g.

In the case that a substituent which inhibits a reaction (e.g. hydroxy, mercapto, amino, formyl, carbonyl and carboxy) exists in any of the above steps, the substituent may be preliminarily protected by, for example, the method described in "Protective Groups in Organic Synthesis, Theodora W Green (John Wiley & Sons)", and the protecting group may be removed at a desired step.
Further, during all the above-mentioned steps, the order of the steps to be performed may be appropriately changed. In each step, an intermediate may be isolated and then used in the next step.

The following embodiments are exemplified for the compound of the present invention.
The compound of formula (I): 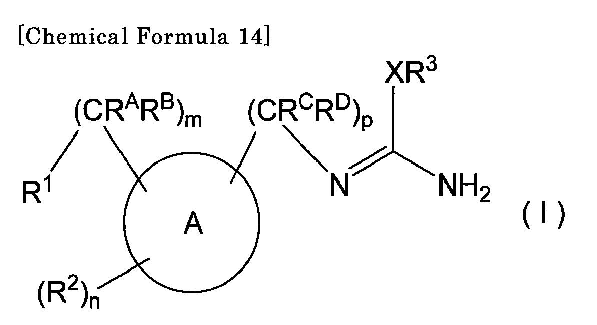 wherein X is an oxygen atom or sulfur atom, m is an integer of 0, and
1) ring A is benzene (hereinafter referred to as "ring A is A1"), ring A is a benzene ring fused with another ring (hereinafter referred to as "ring A is A2"), or
   ring A is naphthalene, indane, quinoline or chromane (hereinafter referred to as "ring A is A3"),
2) R¹ is substituted or unsubstituted acylamino (hereinafter referred to as "R¹ is R11"), R¹ is substituted or unsubstituted acylamino wherein the substituent is one or more selected from the group of halogen, hydroxy, cyano, lower alkyl, lower alkenyl, lower alkynyl, lower alkoxy, lower alkenyloxy and lower alkynyloxy (hereinafter referred to as "R¹ is R12"),
   R¹ is substituted or unsubstituted acyl amino wherein acyl is carbocyclylcarbonyl or heterocyclylcarbonyl and the substituent is one or more selected from the group of halogen, hydroxy, cyano, lower alkyl, lower alkenyl, lower alkynyl, lower alkoxy, lower alkenyloxy, lower alkynyl oxy, carboxy, lower alkoxycarbonyl, amino, alkylamino, acylamino, imino, mercapto, lower alkylthio, lower alkenylthio, lower alkynylthio, sulfamoyl, lower alkylsulfamoyl, lower alkylsulfinyl, lower alkylsulfonyl, lower alkenylsulfamoyl, lower alkenylsulfinyl, lower alkenylsulfonyl, lower alkynylsulfamoyl, lower alkynylsulfinyl, lower alkynylsulfonyl, acyl, nitro, oxo, carbocyclyl optionally substituted with one or more selected from Substituent Group γ and heterocyclyl optionally substituted with one or more selected from Substituent Group γ (hereinafter referred to as "R¹ is R13"),
   R¹ is substituted or unsubstituted acylamino wherein acyl is carbocyclylcarbonyl or heterocyclylcarbonyl and the substituent is one or more selected from the group of halogen, hydroxy, cyano, lower alkyl, lower alkenyl, lower alkynyl, lower alkoxy, lower alkenyloxy and lower alkynyoxy (hereinafter referred to as "R¹ is R14"),
   R¹ is substituted or unsubstituted halogenosulfonylamino, substituted or unsubstituted lower alkylsulfonylamino, substituted or unsubstituted lower alkenylsulfonyl amino, substituted or unsubstituted lower alkynylsulfonylamino, substituted or unsubstituted cycloalkylsulfonylamino, substituted or unsubstituted cycloalkenylsulfonylamino, substituted or unsubstituted acylsulfonylamino, substituted or unsubstituted arylsulfonylamino or substituted or unsubstituted heterocyclylsulfonylamino (hereinafter referred to as "R¹ is R15"),
   R¹ is unsubstituted lower alkylsulfonylamino (hereinafter referred to as "R¹ is R16"),
   R¹ is substituted or unsubstituted aryl (hereinafter referred to as "R¹ is R17"),
   R¹ is aryl wherein the substituent is one or more selected from the group of halogen, hydroxy, lower alkyl, lower alkenyl, lower alkynyl, lower alkoxy, lower alkenyloxy, lower alkynyloxy, amino, acylamino, lower alkoxy acylamino, lower alkenyloxy acylamino and lower alkynyloxy acylamino (hereinafter referred to as "R¹ is R18"), or
   R¹ is substituted or unsubstituted heterocyclyl whrein the substituent is one or more selected from the group of halogen, hydroxy, lower alkyl, lower alkenyl, lower alkynyl, lower alkoxy, lower alkenyloxy, lower alkynyloxy, amino, acylamino, lower alkoxy acylamino, lower alkenyloxy acylamino and lower alkynyloxy acylamino (hereinafter referred to as "R¹ is R19"),
3) n is an integer of 0 (hereinafter referred to as "R² is R21"),
   n is an integer of 1, and R² is halogen (hereinafter referred to as "R² is R22"),
   n is an integer of 1, and R² is substituted or unsubstituted lower alkyl (hereinafter referred to as "R² is R23"),
   n is an integer of 1, and R² is unsubstituted lower alkyl (hereinafter referred to as "R² is R24"),
   n is an integer of 1, and R² is substituted or unsubstituted amino wherein the substituent is one or more selected from the group of lower alkyl, lower alkenyl, lower alkynyl, acyl, lower alkoxycarbonyl, halogenosulfonyl, lower alkylsulfonyl, lower alkenylsulfonyl, lower alkynylsulfonyl, cycloalkylsulfonyl, cycloalkenylsulfonyl, acylsulfonyl, arylsulfonyl, heterocyclylsulfonyl, carbocyclyl optionally substituted with one or more selected from Substituent Group γ and heterocyclyl optionally substituted with one or more selected from Substituent Group γ (hereinafter referred to as "R² is R25"), or
   n is an integer of 1, and R² is substituted or unsubstituted hydroxy wheiren the substituent is one or more selected from the group of lower alkyl, lower alkenyl, lower alkynyl, acyl, lower alkoxycarbonyl, halogenosulfonyl, lower alkylsulfonyl, lower alkenylsulfonyl, lower alkynylsulfonyl, cycloalkylsulfonyl, cycloalkenylsulfonyl, acylsulfonyl, arylsulfonyl, heterocyclylsulfonyl, aryl lower alkyl optionally substituted with one or more selected from Substituent Group γ,carbocyclyl optionally substituted with one or more selected from Substituent Group γ and heterocyclyl optionally substituted with one or more selected from Substituent Group γ (hereinafter referred to as "R² is R26"),
4) R³ is substituted or unsubstituted lower alkyl (hereinafter referred to as "R³ is R31"), R³ is substituted or unsubstituted lower alkyl wherein the substituent is one or more selected from the group of halogen, hydroxy, lower alkoxy, lower alkenyloxy, lower alkynyloxy, amino, lower alkylamino, lower alkenylamino, lower alkynylamino, acylamino, carbocyclyl and heterocyclyl (hereinafter referred to as "R³ is R32"),
   R³ is unsubstituted lower alkyl (hereinafter referred to as "R³ is R33"),
   R³ is substituted or unsubstituted lower alkenyl (hereinafter referred to as "R³ is R34"), or
   R³ is unsubstituted lower alkenyl wherein the substituent is one or more selected from the group of halogen, hydroxy, lower alkoxy, lower alkenyloxy, lower alkynyloxy, amino, lower alkylamino, lower alkenylamino, lower alkynylamino, acylamino, carbocyclyl and heterocyclyl (hereinafter referred to as "R³ is R35"),
   or
5) p is an integer of 0 (hereinafter referred to as "R^{C} and R^{D} are CD1"),
   p is an intger of 1 or 2, and R^{C} and R^{D} are all hydrogen (hereinafter referred to as "R^{C} and R^{D} are CD2"),
   p is an integer of 1 or 2, and R^{C} and R^{D} are each independently hydrogen, substituted or unsubstituted lower alkyl, substituted or unsubstituted aryl or substituted or unsubstituted heterocyclyl (hereinafter referred to as "R^{C} and R^{D} are CD3"),
   R^{C} and R^{D} are each independently hydrogen, substituted or unsubstituted lower alkyl, substituted or unsubstituted aryl or substituted or unsubstituted heterocyclyl wherein the substituent is one or more selected from the group of halogen, hydroxy, lower alkoxy, lower alkenyloxy, lower alkynyloxy, lower alkoxycarbonyl, carbocyclyl lower alkoxy or heterocyclyl lower alkoxy (hereinafter referred to as "R^{C} and R^{D} are CD4"),
   p is an integer of 1 or 2, and R^{C} and R^{D} are each independently hydrogen, unsubstituted lower alkyl, halogenolower alkyl, substituted or unsubstituted phenyl or substituted or unsubstituted nitrogen-containingheterocyclyl wherein the substituent is one or more selected from the group of halogen, lower alkoxy, lower alkoxycarbonyl and aryl lower alkoxy (hereinafter referred to as "R^{C} and R^{D} are CD5),
   p is an integer of 1, and R^{C} and R^{D} together with the carbon atom to which they are attached may form a substituted or unsubstituted carbocycle (hereinafter referred to as "R^{C} and R^{D} are CD6"), or
   p is an integer of 1, and R^{C} and R^{D} together with the carbon atom to which they are attached may form an unsubstituted cycloalkane ring (hereinafter referred to as "R^{C} and R^{D} are CD7").

A compound of formula (I) wherein X is an oxygen atom or a sulfur atom, m is an integer of 0, and the combination of ring A, R¹, n and R², R³, R^{C} and R^{D}, and p is as follows:
(A1,R11,R21,R31,CD1),(A1,R11,R21,R31,CD2),(A1,R11,R21,R31,CD3),(A1,R11,R21,R31,C D4),(A1,R11,R21,R31,CD5),(A1,R11,R21,R31,CD6),(A1,R11,R21,R31,CD7),(A1,R11,R21,R 32,CD1),(A1,R11,R21,R32,CD2),(A1,R11,R21,R32,CD3),(A1,R11,R21,R32,CD4),(A1,R11,R 21,R32,CD5),(A1,R11,R21,R32,CD6),(A1,R11,R21,R32,CD7),(A1,R11,R21,R33,CD1),(A1,R 11,R21,R33,CD2),(A1,R11,R21,R33,CD3),(A1,R11,R21,R33,CD4),(A1,R11,R21,R33,CD5),( A1,R11,R21,R33,CD6),(A1,R11,R21,R33,CD7),(A1,R11,R21,R34,CD1),(A1,R11,R21,R34,C D2),(A1,R11,R21,R34,CD3),(A1,R11,R21,R34,CD4),(A1,R11,R21,R34,CD5),(A1,R11,R21,R 34,CD6),(A1,R11,R21,R34,CD7),(A1,R11,R21,R35,CD1),(A1,R11,R21,R35,CD2),(A1,R11,R 21,R35,CD3),(A1,R11,R21,R35,CD4),(A1,R11,R21,R35,CD5),(A1,R11,R21,R35,CD6),(A1,R 11,R21,R35,CD7),(A1,R11,R22,R31,CD1),(A1,R11,R22,R31,CD2),(A1,R11,R22,R31,CD3),( A1,R11,R22,R31,CD4),(A1,R11,R22,R31,CD5),(A1,R11,R22,R31,CD6),(A1,R11,R22,R31,C D7),(A1,R11,R22,R32,CD1),(A1,R11,R22,R32,CD2),(A1,R11,R22,R32,CD3),(A1,R11,R22,R 32,CD4),(A1,R11,R22,R32,CD5),(A1,R11,R22,R32,CD6),(A1,R11,R22,R32,CD7),(A1,R11,R 22,R33,CD1),(A1,R11,R22,R33,CD2),(A1,R11,R22,R33,CD3),(A1,R11,R22,R33,CD4),(A1,R 11,R22,R33,CD5),(A1,R11,R22,R33,CD6),(A1,R11,R22,R33,CD7),(A1,R11,R22,R34,CD1),( A1,R11,R22,R34,CD2),(A1,R11,R22,R34,CD3),(A1,R11,R22,R34,CD4),(A1,R11,R22,R34,C D5),(A1,R11,R22,R34,CD6),(A1,R11,R22,R34,CD7),(A1,R11,R22,R35,CD1),(A1,R11,R22,R 35,CD2),(A1,R11,R22,R35,CD3),(A1,R11,R22,R35,CD4),(A1,R11,R22,R35,CD5),(A1,R11,R 22,R35,CD6),(A1,R11,R22,R35,CD7),(A1,R11,R23,R31,CD1),(A1,R11,R23,R31,CD2),(A1,R 11,R23,R31,CD3),(A1,R11,R23,R31,CD4),(A1,R11,R23,R31,CD5),(A1,R11,R23,R31,CD6),( A1,R11,R23,R31,CD7),(A1,R11,R23,R32,CD1),(A1,R11,R23,R32,CD2),(A1,R11,R23,R32,C D3),(A1,R11,R23,R32,CD4),(A1,R11,R23,R32,CD5),(A1,R11,R23,R32,CD6),(A1,R11,R23,R 32,CD7),(A1,R11,R23,R33,CD1),(A1,R11,R23,R33,CD2),(A1,R11,R23,R33,CD3),(A1,R11,R 23,R33,CD4),(A1,R11,R23,R33,CD5),(A1,R11,R23,R33,CD6),(A1,R11,R23,R33,CD7),(A1,R 11,R23,R34,CD1),(A1,R11,R23,R34,CD2),(A1,R11,R23,R34,CD3),(A1,R11,R23,R34,CD4),( A1,R11,R23,R34,CD5),(A1,R11,R23,R34,CD6),(A1,R11,R23,R34,CD7),(A1,R11,R23,R35,C D1),(A1,R11,R23,R35,CD2),(A1,R11,R23,R35,CD3),(A1,R11,R23,R35,CD4),(A1,R11,R23,R 35,CD5),(A1,R11,R23,R35,CD6),(A1,R11,R23,R35,CD7),(A1,R11,R24,R31,CD1),(A1,R11,R 24,R31,CD2),(A1,R11,R24,R31,CD3),(A1,R11,R24,R31,CD4),(A1,R11,R24,R31,CD5),(A1,R 11,R24,R31,CD6),(A1,R11,R24,R31,CD7),(A1,R11,R24,R32,CD1),(A1,R11,R24,R32,CD2),( A1,R11,R24,R32,CD3),(A1,R11,R24,R32,CD4),(A1,R11,R24,R32,CD5),(A1,R11,R24,R32,C D6),(A1,R11,R24,R32,CD7),(A1,R11,R24,R33,CD1),(A1,R11,R24,R33,CD2),(A1,R11,R24,R 33,CD3),(A1,R11,R24,R33,CD4),(A1,R11,R24,R33,CD5),(A1,R11,R24,R33,CD6),(A1,R11,R 24,R33,CD7),(A1,R11,R24,R34,CD1),(A1,R11,R24,R34,CD2),(A1,R11,R24,R34,CD3),(A1,R 11,R24,R34,CD4),(A1,R11,R24,R34,CD5),(A1,R11,R24,R34,CD6),(A1,R11,R24,R34,CD7),( A1,R11,R24,R35,CD1),(A1,R11,R24,R35,CD2),(A1,R11,R24,R35,CD3),(A1,R11,R24,R35,C D4),(A1,R11,R24,R35,CD5),(A1,R11,R24,R35,CD6),(A1,R11,R24,R35,CD7),(A1,R11,R25,R 31,CD1),(A1,R11,R25,R31,CD2),(A1,R11,R25,R31,CD3),(A1,R11,R25,R31,CD4),(A1,R11,R 25,R31,CD5),(A1,R11,R25,R31,CD6),(A1,R11,R25,R31,CD7),(A1,R11,R25,R32,CD1),(A1,R 11,R25,R32,CD2),(A1,R11,R25,R32,CD3),(A1,R11,R25,R32,CD4),(A1,R11,R25,R32,CD5),( A1,R11,R25,R32,CD6),(A1,R11,R25,R32,CD7),(A1,R11,R25,R33,CD1),(A1,R11,R25,R33,C D2),(A1,R11,R25,R33,CD3),(A1,R11,R25,R33,CD4),(A1,R11,R25,R33,CD5),(A1,R11,R25,R 33,CD6),(A1,R11,R25,R33,CD7),(A1,R11,R25,R34,CD1),(A1,R11,R25,R34,CD2),(A1,R11,R 25,R34,CD3),(A1,R11,R25,R34,CD4),(A1,R11,R25,R34,CD5),(A1,R11,R25,R34,CD6),(A1,R 11,R25,R34,CD7),(A1,R11,R25,R35,CD1),(A1,R11,R25,R35,CD2),(A1,R11,R25,R35,CD3),( A1,R11,R25,R35,CD4),(A1,R11,R25,R35,CD5),(A1,R11,R25,R35,CD6),(A1,R11,R25,R35,C D7),(A1,R11,R26,R31,CD1),(A1,R11,R26,R31,CD2),(A1,R11,R26,R31,CD3),(A1,R11,R26,R 31,CD4),(A1,R11,R26,R31,CD5),(A1,R11,R26,R31,CD6),(A1,R11,R26,R31,CD7),(A1,R11,R 26,R32,CD1),(A1,R11,R26,R32,CD2),(A1,R11,R26,R32,CD3),(A1,R11,R26,R32,CD4),(A1,R 11,R26,R32,CD5),(A1,R11,R26,R32,CD6),(A1,R11,R26,R32,CD7),(A1,R11,R26,R33,CD1),( A1,R11,R26,R33,CD2),(A1,R11,R26,R33,CD3),(A1,R11,R26,R33,CD4),(A1,R11,R26,R33,C D5),(A1,R11,R26,R33,CD6),(A1,R11,R26,R33,CD7),(A1,R11,R26,R34,CD1),(A1,R11,R26,R 34,CD2),(A1,R11,R26,R34,CD3),(A1,R11,R26,R34,CD4),(A1,R11,R26,R34,CD5),(A1,R11,R 26,R34,CD6),(A1,R11,R26,R34,CD7),(A1,R11,R26,R35,CD1),(A1,R11,R26,R35,CD2),(A1,R 11,R26,R35,CD3),(A1,R11,R26,R35,CD4),(A1,R11,R26,R35,CD5),(A1,R11,R26,R35,CD6),( A1,R11,R26,R35,CD7),(A1,R12,R21,R31,CD1),(A1,R12,R21,R31,CD2),(A1,R12,R21,R31,C D3),(A1,R12,R21,R31,CD4),(A1,R12,R21,R31,CD5),(A1,R12,R21,R31,CD6),(A1,R12,R21,R 31,CD7),(A1,R12,R21,R32,CD1),(A1,R12,R21,R32,CD2),(A1,R12,R21,R32,CD3),(A1,R12,R 21,R32,CD4),(A1,R12,R21,R32,CD5),(A1,R12,R21,R32,CD6),(A1,R12,R21,R32,CD7),(A1,R 12,R21,R33,CD1),(A1,R12,R21,R33,CD2),(A1,R12,R21,R33,CD3),(A1,R12,R21,R33,CD4),( A1,R12,R21,R33,CD5),(A1,R12,R21,R33,CD6),(A1,R12,R21,R33,CD7),(A1,R12,R21,R34,C D1),(A1,R12,R21,R34,CD2),(A1,R12,R21,R34,CD3),(A1,R12,R21,R34,CD4),(A1,R12,R21,R 34,CD5),(A1,R12,R21,R34,CD6),(A1,R12,R21,R34,CD7),(A1,R12,R21,R35,CD1),(A1,R12,R 21,R35,CD2),(A1,R12,R21,R35,CD3),(A1,R12,R21,R35,CD4),(A1,R12,R21,R35,CD5),(A1,R 12,R21,R35,CD6),(A1,R12,R21,R35,CD7),(A1,R12,R22,R31,CD1),(A1,R12,R22,R31,CD2),( A1,R12,R22,R31,CD3),(A1,R12,R22,R31,CD4),(A1,R12,R22,R31,CD5),(A1,R12,R22,R31,C D6),(A1,R12,R22,R31,CD7),(A1,R12,R22,R32,CD1),(A1,R12,R22,R32,CD2),(A1,R12,R22,R 32,CD3),(A1,R12,R22,R32,CD4),(A1,R12,R22,R32,CD5),(A1,R12,R22,R32,CD6),(A1,R12,R 22,R32,CD7),(A1,R12,R22,R33,CD1),(A1,R12,R22,R33,CD2),(A1,R12,R22,R33,CD3),(A1,R 12,R22,R33,CD4),(A1,R12,R22,R33,CD5),(A1,R12,R22,R33,CD6),(A1,R12,R22,R33,CD7),( A1,R12,R22,R34,CD1),(A1,R12,R22,R34,CD2),(A1,R12,R22,R34,CD3),(A1,R12,R22,R34,C D4),(A1,R12,R22,R34,CD5),(A1,R12,R22,R34,CD6),(A1,R12,R22,R34,CD7),(A1,R12,R22,R 35,CD1),(A1,R12,R22,R35,CD2),(A1,R12,R22,R35,CD3),(A1,R12,R22,R35,CD4),(A1,R12,R 22,R35,CD5),(A1,R12,R22,R35,CD6),(A1,R12,R22,R35,CD7),(A1,R12,R23,R31,CD1),(A1,R 12,R23,R31,CD2),(A1,R12,R23,R31,CD3),(A1,R12,R23,R31,CD4),(A1,R12,R23,R31,CD5),( A1,R12,R23,R31,CD6),(A1,R12,R23,R31,CD7),(A1,R12,R23,R32,CD1),(A1,R12,R23,R32,C D2),(A1,R12,R23,R32,CD3),(A1,R12,R23,R32,CD4),(A1,R12,R23,R32,CD5),(A1,R12,R23,R 32,CD6),(A1,R12,R23,R32,CD7),(A1,R12,R23,R33,CD1),(A1,R12,R23,R33,CD2),(A1,R12,R 23,R33,CD3),(A1,R12,R23,R33,CD4),(A1,R12,R23,R33,CD5),(A1,R12,R23,R33,CD6),(A1,R 12,R23,R33,CD7),(A1,R12,R23,R34,CD1),(A1,R12,R23,R34,CD2),(A1,R12,R23,R34,CD3),( A1,R12,R23,R34,CD4),(A1,R12,R23,R34,CD5),(A1,R12,R23,R34,CD6),(A1,R12,R23,R34,C D7),(A1,R12,R23,R35,CD1),(A1,R12,R23,R35,CD2),(A1,R12,R23,R35,CD3),(A1,R12,R23,R 35,CD4),(A1,R12,R23,R35,CD5),(A1,R12,R23,R35,CD6),(A1,R12,R23,R35,CD7),(A1,R12,R 24,R31,CD1),(A1,R12,R24,R31,CD2),(A1,R12,R24,R31,CD3),(A1,R12,R24,R31,CD4),(A1,R 12,R24,R31,CD5),(A1,R12,R24,R31,CD6),(A1,R12,R24,R31,CD7),(A1,R12,R24,R32,CD1),( A1,R12,R24,R32,CD2),(A1,R12,R24,R32,CD3),(A1,R12,R24,R32,CD4),(A1,R12,R24,R32,C D5),(A1,R12,R24,R32,CD6),(A1,R12,R24,R32,CD7),(A1,R12,R24,R33,CD1),(A1,R12,R24,R 33,CD2),(A1,R12,R24,R33,CD3),(A1,R12,R24,R33,CD4),(A1,R12,R24,R33,CD5),(A1,R12,R 24,R33,CD6),(A1,R12,R24,R33,CD7),(A1,R12,R24,R34,CD1),(A1,R12,R24,R34,CD2),(A1,R 12,R24,R34,CD3),(A1,R12,R24,R34,CD4),(A1,R12,R24,R34,CD5),(A1,R12,R24,R34,CD6),( A1,R12,R24,R34,CD7),(A1,R12,R24,R35,CD1),(A1,R12,R24,R35,CD2),(A1,R12,R24,R35,C D3),(A1,R12,R24,R35,CD4),(A1,R12,R24,R35,CD5),(A1,R12,R24,R35,CD6),(A1,R12,R24,R 35,CD7),(A1,R12,R25,R31,CD1),(A1,R12,R25,R31,CD2),(A1,R12,R25,R31,CD3),(A1,R12,R 25,R31,CD4),(A1,R12,R25,R31,CD5),GA1,R12,R25,R31,CD6),(A1,R12,R25,R31,CD7),(A1,R 12,R25,R32,CD1),(A1,R12,R25,R32,CD2),(A1,R12,R25,R32,CD3),(A1,R12,R25,R32,CD4),( A1,R12,R25,R32,CD5),(A1,R12,R25,R32,CD6),(A1,R12,R25,R32,CD7),(A1,R12,R25,R33,C D1),(A1,R12,R25,R33,CD2),(A1,R12,R25,R33,CD3),(A1,R12,R25,R33,CD4),(A1,R12,R25,R 33,CD5),(A1,R12,R25,R33,CD6),(A1,R12,R25,R33,CD7),(A1,R12,R25,R34,CD1),(A1,R12,R 25,R34,CD2),(A1,R12,R25,R34,CD3),(A1,R12,R25,R34,CD4),(A1,R12,R25,R34,CD5),(A1,R 12,R25,R34,CD6),(A1,R12,R25,R34,CD7),(A1,R12,R25,R35,CD1),(A1,R12,R25,R35,CD2),( A1,R12,R25,R35,CD3),(A1,R12,R25,R35,CD4),(A1,R12,R25,R35,CD5),(A1,R12,R25,R35,C D6),(A1,R12,R25,R35,CD7),(A1,R12,R26,R31,CD1),(A1,R12,R26,R31,CD2),(A1,R12,R26,R 31,CD3),(A1,R12,R26,R31,CD4),(A1,R12,R26,R31,CD5),(A1,R12,R26,R31,CD6),(A1,R12,R 26,R31,CD7),(A1,R12,R26,R32,CD1),(A1,R12,R26,R32,CD2),(A1,R12,R26,R32,CD3),(A1,R 12,R26,R32,CD4),(A1,R12,R26,R32,CD5),(A1,R12,R26,R32,CD6),(A1,R12,R26,R32,CD7),( A1,R12,R26,R33,CD1),(A1,R12,R26,R33,CD2),(A1,R12,R26,R33,CD3),(A1,R12,R26,R33,C D4),(A1,R12,R26,R33,CD5),(A1,R12,R26,R33,CD6),(A1,R12,R26,R33,CD7),(A1,R12,R26,R 34,CD1),(A1,R12,R26,R34,CD2),(A1,R12,R26,R34,CD3),(A1,R12,R26,R34,CD4),(A1,R12,R 26,R34,CD5),(A1,R12,R26,R34,CD6),(A1,R12,R26,R34,CD7),(A1,R12,R26,R35,CD1),(A1,R 12,R26,R35,CD2),(A1,R12,R26,R35,CD3),(A1,R12,R26,R35,CD4),(A1,R12,R26,R35,CD5),( A1,R12,R26,R35,CD6),(A1,R12,R26,R35,CD7),(A1,R13,R21,R31,CD1),(A1,R13,R21,R31,C D2),(A1,R13,R21,R31,CD3),(A1,R13,R21,R31,CD4),(A1,R13,R21,R31,CD5),(A1,R13,R21,R 31,CD6),(A1,R13,R21,R31,CD7),(A1,R13,R21,R32,CD1),(A1,R13,R21,R32,CD2),(A1,R13,R 21,R32,CD3),(A1,R13,R21,R32,CD4),(A1,R13,R21,R32,CD5),(A1,R13,R21,R32,CD6),(A1,R 13,R21,R32,CD7),(A1,R13,R21,R33,CD1),(A1,R13,R21,R33,CD2),(A1,R13,R21,R33,CD3),( A1,R13,R21,R33,CD4),(A1,R13,R21,R33,CD5),(A1,R13,R21,R33,CD6),(A1,R13,R21,R33,C D7),(A1,R13,R21,R34,CD1),(A1,R13,R21,R34,CD2),(A1,R13,R21,R34,CD3),(A1,R13,R21,R 34,CD4),(A1,R13,R21,R34,CD5),(A1,R13,R21,R34,CD6),(A1,R13,R21,R34,CD7),(A1,R13,R 21,R35,CD1),(A1,R13,R21,R35,CD2),(A1,R13,R21,R35,CD3),(A1,R13,R21,R35,CD4),(A1,R 13,R21,R35,CD5),(A1,R13,R21,R35,CD6),(A1,R13,R21,R35,CD7),(A1,R13,R22,R31,CD1),( A1,R13,R22,R31,CD2),(A1,R13,R22,R31,CD3),(A1,R13,R22,R31,CD4),(A1,R13,R22,R31,C D5),(A1,R13,R22,R31,CD6),(A1,R13,R22,R31,CD7),(A1,R13,R22,R32,CD1),(A1,R13,R22,R 32,CD2),(A1,R13,R22,R32,CD3),(A1,R13,R22,R32,CD4),(A1,R13,R22,R32,CD5),(A1,R13,R 22,R32,CD6),(A1,R13,R22,R32,CD7),(A1,R13,R22,R33,CD1),(A1,R13,R22,R33,CD2),(A1,R 13,R22,R33,CD3),(A1,R13,R22,R33,CD4),(A1,R13,R22,R33,CD5),(A1,R13,R22,R33,CD6),( A1,R13,R22,R33,CD7),(A1,R13,R22,R34,CD1),(A1,R13,R22,R34,CD2),(A1,R13,R22,R34,C D3),(A1,R13,R22,R34,CD4),(A1,R13,R22,R34,CD5),(A1,R13,R22,R34,CD6),(A1,R13,R22,R 34,CD7),(A1,R13,R22,R35,CD1),(A1,R13,R22,R35,CD2),(A1,R13,R22,R35,CD3),(A1,R13,R 22,R35,CD4),(A1,R13,R22,R35,CD5),(A1,R13,R22,R35,CD6),(A1,R13,R22,R35,CD7),(A1,R 13,R23,R31,CD1),(A1,R13,R23,R31,CD2),(A1,R13,R23,R31,CD3),(A1,R13,R23,R31,CD4),( A1,R13,R23,R31,CD5),(A1,R13,R23,R31,CD6),(A1,R13,R23,R31,CD7),(A1,R13,R23,R32,C D1),(A1,R13,R23,R32,CD2),(A1,R13,R23,R32,CD3),(A1,R13,R23,R32,CD4),(A1,R13,R23,R 32,CD5),(A1,R13,R23,R32,CD6),(A1,R13,R23,R32,CD7),(A1,R13,R23,R33,CD1),(A1,R13,R 23,R33,CD2),(A1,R13,R23,R33,CD3),(A1,R13,R23,R33,CD4),(A1,R13,R23,R33,CD5),(A1,R 13,R23,R33,CD6),(A1,R13,R23,R33,CD7),(A1,R13,R23,R34,CD1),(A1,R13,R23,R34,CD2),( A1,R13,R23,R34,CD3),(A1,R13,R23,R34,CD4),(A1,R13,R23,R34,CD5),(A1,R13,R23,R34,C D6),(A1,R13,R23,R34,CD7),(A1,R13,R23,R35,CD1),(A1,R13,R23,R35,CD2),(A1,R13,R23,R 35,CD3),(A1,R13,R23,R35,CD4),(A1,R13,R23,R35,CD5),(A1,R13,R23,R35,CD6),(A1,R13,R 23,R35,CD7),(A1,R13,R24,R31,CD1),(A1,R13,R24,R31,CD2),(A1,R13,R24,R31,CD3),(A1,R 13,R24,R31,CD4),(A1,R13,R24,R31,CD5),(A1,R13,R24,R31,CD6),(A1,R13,R24,R31,CD7),( A1,R13,R24,R32,CD1),(A1,R13,R24,R32,CD2),(A1,R13,R24,R32,CD3),(A1,R13,R24,R32,C D4),(A1,R13,R24,R32,CD5),(A1,R13,R24,R32,CD6),(A1,R13,R24,R32,CD7),(A1,R13,R24,R 33,CD1),(A1,R13,R24,R33,CD2),(A1,R13,R24,R33,CD3),(A1,R13,R24,R33,CD4),(A1,R13,R 24,R33,CD5),(A1,R13,R24,R33,CD6),(A1,R13,R24,R33,CD7),(A1,R13,R24,R34,CD1),(A1,R 13,R24,R34,CD2),(A1,R13,R24,R34,CD3),(A1,R13,R24,R34,CD4),(A1,R13,R24,R34,CD5),( A1,R13,R24,R34,CD6),(A1,R13,R24,R34,CD7),(A1,R13,R24,R35,CD1),(A1,R13,R24,R35,C D2),(A1,R13,R24,R35,CD3),(A1,R13,R24,R35,CD4),(A1,R13,R24,R35,CD5),(A1,R13,R24,R 35,CD6),(A1,R13,R24,R35,CD7),(A1,R13,R25,R31,CD1),(A1,R13,R25,R31,CD2),(A1,R13,R 25,R31,CD3),(A1,R13,R25,R31,CD4),(A1,R13,R25,R31,CD5),(A1,R13,R25,R31,CD6),(A1,R 13,R25,R31,CD7),(A1,R13,R25,R32,CD1),(A1,R13,R25,R32,CD2),(A1,R13,R25,R32,CD3),( A1,R13,R25,R32,CD4),(A1,R13,R25,R32,CD5),(A1,R13,R25,R32,CD6),(A1,R13,R25,R32,C D7),(A1,R13,R25,R33,CD1),(A1,R13,R25,R33,CD2),(A1,R13,R25,R33,CD3),(A1,R13,R25,R 33,CD4),(A1,R13,R25,R33,CD5),(A1,R13,R25,R33,CD6),(A1,R13,R25,R33,CD7),(A1,R13,R 25,R34,CD1),(A1,R13,R25,R34,CD2),(A1,R13,R25,R34,CD3),(A1,R13,R25,R34,CD4),(A1,R 13,R25,R34,CD5),(A1,R13,R25,R34,CD6),(A1,R13,R25,R34,CD7),(A1,R13,R25,R35,CD1),( A1,R13,R25,R35,CD2),(A1,R13,R25,R35,CD3),(A1,R13,R25,R35,CD4),(A1,R13,R25,R35,C D5),(A1,R13,R25,R35,CD6),(A1,R13,R25,R35,CD7),(A1,R13,R26,R31,CD1),(A1,R13,R26,R 31,CD2),(A1,R13,R26,R31,CD3),(A1,R13,R26,R31,CD4),(A1,R13,R26,R31,CD5),(A1,R13,R 26,R31,CD6),(A1,R13,R26,R31,CD7),(A1,R13,R26,R32,CD1),(A1,R13,R26,R32,CD2),(A1,R 13,R26,R32,CD3),(A1,R13,R26,R32,CD4),(A1,R13,R26,R32,CD5),(A1,R13,R26,R32,CD6),( A1,R13,R26,R32,CD7),(A1,R13,R26,R33,CD1),(A1,R13,R26,R33,CD2),(A1,R13,R26,R33,C D3),(A1,R13,R26,R33,CD4),(A1,R13,R26,R33,CD5),(A1,R13,R26,R33,CD6),(A1,R13,R26,R 33,CD7),(A1,R13,R26,R34,CD1),(A1,R13,R26,R34,CD2),(A1,R13,R26,R34,CD3),(A1,R13,R 26,R34,CD4),(A1,R13,R26,R34,CD5),(A1,R13,R26,R34,CD6),(A1,R13,R26,R34,CD7),(A1,R 13,R26,R35,CD1),(A1,R13,R26,R35,CD2),(A1,R13,R26,R35,CD3),(A1,R13,R26,R35,CD4),( A1,R13,R26,R35,CD5),(A1,R13,R26,R35,CD6),(A1,R13,R26,R35,CD7),(A1,R14,R21,R31,C D1),(A1,R14,R21,R31,CD2),(A1,R14,R21,R31,CD3),(A1,R14,R21,R31,CD4),(A1,R14,R21,R 31,CD5),(A1,R14,R21,R31,CD6),(A1,R14,R21,R31,CD7),(A1,R14,R21,R32,CD1),(A1,R14,R 21,R32,CD2),(A1,R14,R21,R32,CD3),(A1,R14,R21,R32,CD4),(A1,R14,R21,R32,CD5),(A1,R 14,R21,R32,CD6),(A1,R14,R21,R32,CD7),(A1,R14,R21,R33,CD1),(A1,R14,R21,R33,CD2),( A1,R14,R21,R33,CD3),(A1,R14,R21,R33,CD4),(A1,R14,R21,R33,CD5),(A1,R14,R21,R33,C D6),(A1,R14,R21,R33,CD7),(A1,R14,R21,R34,CD1),(A1,R14,R21,R34,CD2),(A1,R14,R21,R 34,CD3),(A1,R14,R21,R34,CD4),(A1,R14,R21,R34,CD5),(A1,R14,R21,R34,CD6),(A1,R14,R 21,R34,CD7),(A1,R14,R21,R35,CD1),(A1,R14,R21,R35,CD2),(A1,R14,R21,R35,CD3),(A1,R 14,R21,R35,CD4),(A1,R14,R21,R35,CD5),(A1,R14,R21,R35,CD6),(A1,R14,R21,R35,CD7),( A1,R14,R22,R31,CD1),(A1,R14,R22,R31,CD2),(A1,R14,R22,R31,CD3),(A1,R14,R22,R31,C D4),(A1,R14,R22,R31,CD5),(A1,R14,R22,R31,CD6),(A1,R14,R22,R31,CD7),(A1,R14,R22,R 32,CD1),(A1,R14,R22,R32,CD2),(A1,R14,R22,R32,CD3),(A1,R14,R22,R32,CD4),(A1,R14,R 22,R32,CD5),(A1,R14,R22,R32,CD6),(A1,R14,R22,R32,CD7),(A1,R14,R22,R33,CD1),(A1,R 14,R22,R33,CD2),(A1,R14,R22,R33,CD3),(A1,R14,R22,R33,CD4),(A1,R14,R22,R33,CD5),( A1,R14,R22,R33,CD6),(A1,R14,R22,R33,CD7),(A1,R14,R22,R34,CD1),(A1,R14,R22,R34,C D2),(A1,R14,R22,R34,CD3),(A1,R14,R22,R34,CD4),(A1,R14,R22,R34,CD5),(A1,R14,R22,R 34,CD6),(A1,R14,R22,R34,CD7),(A1,R14,R22,R35,CD1),(A1,R14,R22,R35,CD2),(A1,R14,R 22,R35,CD3),(A1,R14,R22,R35,CD4),(A1,R14,R22,R35,CD5),(A1,R14,R22,R35,CD6),(A1,R 14,R22,R35,CD7),(A1,R14,R23,R31,CD1),(A1,R14,R23,R31,CD2),(A1,R14,R23,R31,CD3),( A1,R14,R23,R31,CD4),(A1,R14,R23,R31,CD5),(A1,R14,R23,R31,CD6),(A1,R14,R23,R31,C D7),(A1,R14,R23,R32,CD1),(A1,R14,R23,R32,CD2),(A1,R14,R23,R32,CD3),(A1,R14,R23,R 32,CD4),(A1,R14,R23,R32,CD5),(A1,R14,R23,R32,CD6),(A1,R14,R23,R32,CD7),(A1,R14,R 23,R33,CD1),(A1,R14,R23,R33,CD2),(A1,R14,R23,R33,CD3),(A1,R14,R23,R33,CD4),(A1,R 14,R23,R33,CD5),(A1,R14,R23,R33,C1)6),(A1,R14,R23,R33,CD7),(A1,R14,R23,R34,CD1),( A1,R14,R23,R34,CD2),(A1,R14,R23,R34,CD3),(A1,R14,R23,R34,CD4),(A1,R14,R23,R34,C D5),(A1,R14,R23,R34,CD6),(A1,R14,R23,R34,CD7),(A1,R14,R23,R35,CD1),(A1,R14,R23,R 35,CD2),(A1,R14,R23,R35,CD3),(A1,R14,R23,R35,CD4),(A1,R14,R23,R35,CD5),(A1,R14,R 23,R35,CD6),(A1,R14,R23,R35,CD7),(A1,R14,R24,R31,CD1),(A1,R14,R24,R31,CD2),(A1,R 14,R24,R31,CD3),(A1,R14,R24,R31,CD4),(A1,R14,R24,R31,CD5),(A1,R14,R24,R31,CD6),( A1,R14,R24,R31,CD7),(A1,R14,R24,R32,CD1),(A1,R14,R24,R32,CD2),(A1,R14,R24,R32,C D3),(A1,R14,R24,R32,CD4),(A1,R14,R24,R32,CD5),(A1,R14,R24,R32,CD6),(A1,R14,R24,R 32,CD7),(A1,R14,R24,R33,CD1),(A1,R14,R24,R33,CD2),(A1,R14,R24,R33,CD3),(A1,R14,R 24,R33,CD4),(A1,R14,R24,R33,CD5),(A1,R14,R24,R33,CD6),(A1,R14,R24,R33,CD7),(A1,R 14,R24,R34,CD1),(A1,R14,R24,R34,CD2),(A1,R14,R24,R34,CD3),(A1,R14,R24,R34,CD4),( A1,R14,R24,R34,CD5),(A1,R14,R24,R34,CD6),(A1,R14,R24,R34,CD7),(A1,R14,R24,R35,C D1),(A1,R14,R24,R35,CD2),(A1,R14,R24,R35,CD3),(A1,R14,R24,R35,CD4),(A1,R14,R24,R 35,CDS),(A1,R14,R24,R35,CD6),(A1,R14,R24,R35,CD7),(A1,R14,R25,R31,CD1),(A1,R14,R 25,R31,CD2),(A1,R14,R25,R31,CD3),(A1,R14,R25,R31,CD4),(A1,R14,R25,R31,CD5),(A1,R 14,R25,R31,CD6),(A1,R14,R25,R31,CD7),(A1,R14,R25,R32,CD1),(A1,R14,R25,R32,CD2),( A1,R14,R25,R32,CD3),(A1,R14,R25,R32,CD4),(A1,R14,R25,R32,CD5),(A1,R14,R25,R32,C D6),(A1,R14,R25,R32,CD7),(A1,R14,R25,R33,CD1),(A1,R14,R25,R33,CD2),(A1,R14,R25,R 33,CD3),(A1,R14,R25,R33,CD4),(A1,R14,R25,R33,CD5),(A1,R14,R25,R33,CD6),(A1,R14,R 25,R33,CD7),(A1,R14,R25,R34,CD1),(A1,R14,R25,R34,CD2),(A1,R14,R25,R34,CD3),(A1,R 14,R25,R34,CD4),(A1,R14,R25,R34,CD5),(A1,R14,R25,R34,CD6),(A1,R14,R25,R34,CD7),( A1,R14,R25,R35,CD1),(A1,R14,R25,R35,CD2),(A1,R14,R25,R35,CD3),(A1,R14,R25,R35,C D4),(A1,R14,R25,R35,CD5),(A1,R14,R25,R35,CD6),(A1,R14,R25,R35,CD7),(A1,R14,R26,R 31,CD1),(A1,R14,R26,R31,CD2),(A1,R14,R26,R31,CD3),(A1,R14,R26,R31,CD4),(A1,R14,R 26,R31,CD5),(A1,R14,R26,R31,CD6),(A1,R14,R26,R31,CD7),(A1,R14,R26,R32,CD1),(A1,R 14,R26,R32,CD2),(A1,R14,R26,R32,CD3),(A1,R14,R26,R32,CD4),(A1,R14,R26,R32,CD5),( A1,R14,R26,R32,CD6),(A1,R14,R26,R32,CD7),(A1,R14,R26,R33,CD1),(A1,R14,R26,R33,C D2),(A1,R14,R26,R33,CD3),(A1,R14,R26,R33,CD4),(A1,R14,R26,R33,CD5),(A1,R14,R26,R 33,CD6),(A1,R14,R26,R33,CD7),(A1,R14,R26,R34,CD1),(A1,R14,R26,R34,CD2),(A1,R14,R 26,R34,CD3),(A1,R14,R26,R34,CD4),(A1,R14,R26,R34,CD5),(A1,R14,R26,R34,CD6),(A1,R 14,R26,R34,CD7),(A1,R14,R26,R35,CD1),(A1,R14,R26,R35,CD2),(A1,R14,R26,R35,CD3),( A1,R14,R26,R35,CD4),(A1,R14,R26,R35,CD5),(A1,R14,R26,R35,CD6),(A1,R14,R26,R35,C D7),(A1,R15,R21,R31,CD1),(A1,R15,R21,R31,CD2),(A1,R15,R21,R31,CD3),(A1,R15,R21,R 31,CD4),(A1,R15,R21,R31,CD5),(A1,R15,R21,R31,CD6),(A1,R15,R21,R31,CD7),(A1,R15,R 21,R32,CD1),(A1,R15,R21,R32,CD2),(A1,R15,R21,R32,CD3),(A1,R15,R21,R32,CD4),(A1,R 15,R21,R32,CD5),(A1,R15,R21,R32,CD6),(A1,R15,R21,R32,CD7),(A1,R15,R21,R33,CD1),( A1,R15,R21,R33,CD2),(A1,R15,R21,R33,CD3),(A1,R15,R21,R33,CD4),(A1,R15,R21,R33,C D5),(A1,R15,R21,R33,CD6),(A1,R15,R21,R33,CD7),(A1,R15,R21,R34,CD1),(A1,R15,R21,R 34,CD2),(A1,R15,R21,R34,CD3),(A1,R15,R21,R34,CD4),(A1,R15,R21,R34,CD5),(A1,R15,R 21,R34,CD6),(A1,R15,R21,R34,CD7),(A1,R15,R21,R35,CD1),(A1,R15,R21,R35,CD2),(A1,R 15,R21,R35,CD3),(A1,R15,R21,R35,CD4),(A1,R15,R21,R35,CD5),(A1,R15,R21,R35,CD6),( A1,R15,R21,R35,CD7),(A1,R15,R22,R31,CD1),(A1,R15,R22,R31,CD2),(A1,R15,R22,R31,C D3),(A1,R15,R22,R31,CD4),(A1,R15,R22,R31,CD5),(A1,R15,R22,R31,CD6),(A1,R15,R22,R 31,CD7),(A1,R15,R22,R32,CD1),(A1,R15,R22,R32,CD2),(A1,R15,R22,R32,CD3),(A1,R15,R 22,R32,CD4),(A1,R15,R22,R32,CD5),(A1,R15,R22,R32,CD6),(A1,R15,R22,R32,CD7),(A1,R 15,R22,R33,CD1),(A1,R15,R22,R33,CD2),(A1,R15,R22,R33,CD3),(A1,R15,R22,R33,CD4),( A1,R15,R22,R33,CD5),(A1,R15,R22,R33,CD6),(A1,R15,R22,R33,CD7),(A1,R15,R22,R34,C D1),(A1,R15,R22,R34,CD2),(A1,R15,R22,R34,CD3),(A1,R15,R22,R34,CD4),(A1,R15,R22,R 34,CD5),(A1,R15,R22,R34,CD6),(A1,R15,R22,R34,CD7),(A1,R15,R22,R35,CD1),(A1,R15,R 22,R35,CD2),(A1,R15,R22,R35,CD3),(A1,R15,R22,R35,CD4),(A1,R15,R22,R35,CD5),(A1,R 15,R22,R35,CD6),(A1,R15,R22,R35,CD7),(A1,R15,R23,R31,CD1),(A1,R15,R23,R31,CD2),( A1,R15,R23,R31,CD3),(A1,R15,R23,R31,CD4),(A1,R15,R23,R31,CD5),(A1,R15,R23,R31,C D6),(A1,R15,R23,R31,CD7),(A1,R15,R23,R32,CD1),(A1,R15,R23,R32,CD2),(A1,R15,R23,R 32,CD3),(A1,R15,R23,R32,CD4),(A1,R15,R23,R32,CD5),(A1,R15,R23,R32,CD6),(A1,R15,R 23,R32,CD7),(A1,R15,R23,R33,CD1),(A1,R15,R23,R33,CD2),(A1,R15,R23,R33,CD3),(A1,R 15,R23,R33,CD4),(A1,R15,R23,R33,CD5),(A1,R15,R23,R33,CD6),(A1,R15,R23,R33,CD7),( A1,R15,R23,R34,CD1),(A1,R15,R23,R34,CD2),(A1,R15,R23,R34,CD3),(A1,R15,R23,R34,C D4),(A1,R15,R23,R34,CD5),(A1,R15,R23,R34,CD6),(A1,R15,R23,R34,CD7),(A1,R15,R23,R 35,CD1),(A1,R15,R23,R35,CD2),(A1,R15,R23,R35,CD3),(A1,R15,R23,R35,CD4),(A1,R15,R 23,R35,CD5),(A1,R15,R23,R35,CD6),(A1,R15,R23,R35,CD7),(A1,R15,R24,R31,CD1),(A1,R 15,R24,R31,CD2),(A1,R15,R24,R31,CD3),(A1,R15,R24,R31,CD4),(A1,R15,R24,R31,CD5),( A1,R15,R24,R31,CD6),(A1,R15,R24,R31,CD7),(A1,R15,R24,R32,CD1),(A1,R15,R24,R32,C D2),(A1,R15,R24,R32,CD3),(A1,R15,R24,R32,CD4),(A1,R15,R24,R32,CD5),(A1,R15,R24,R 32,CD6),(A1,R15,R24,R32,CD7),(A1,R15,R24,R33,CD1),(A1,R15,R24,R33,CD2),(A1,R15,R 24,R33,CD3),(A1,R15,R24,R33,CD4),(A1,R15,R24,R33,CD5),(A1,R15,R24,R33,CD6),(A1,R 15,R24,R33,CD7),(A1,R15,R24,R34,CD1),(A1,R15,R24,R34,CD2),(A1,R15,R24,R34,CD3),( A1,R15,R24,R34,CD4),(A1,R15,R24,R34,CD5),(A1,R15,R24,R34,CD6),(A1,R15,R24,R34,C D7),(A1,R15,R24,R35,CD1),(A1,R15,R24,R35,CD2),(A1,R15,R24,R35,CD3),(A1,R15,R24,R 35,CD4),(A1,R15,R24,R35,CD5),(A1,R15,R24,R35,CD6),(A1,R15,R24,R35,CD7),(A1,R15,R 25,R31,CD1),(A1,R15,R25,R31,CD2),(A1,R15,R25,R31,CD3),(A1,R15,R25,R31,CD4),(A1,R 15,R25,R31,CD5),(A1,R15,R25,R31,CD6),(A1,R15,R25,R31,CD7),(A1,R15,R25,R32,CD1),( A1,R15,R25,R32,CD2),(A1,R15,R25,R32,CD3),(A1,R15,R25,R32,CD4),(A1,R15,R25,R32,C D5),(A1,R15,R25,R32,CD6),(A1,R15,R25,R32,CD7),(A1,R15,R25,R33,CD1),(A1,R15,R25,R 33,CD2),(A1,R15,R25,R33,CD3),(A1,R15,R25,R33,CD4),(A1,R15,R25,R33,CD5),(A1,R15,R 25,R33,CD6),(A1,R15,R25,R33,CD7),(A1,R15,R25,R34,CD1),(A1,R15,R25,R34,CD2),(A1,R 15,R25,R34,CD3),(A1,R15,R25,R34,CD4),(A1,R15,R25,R34,CD5),(A1,R15,R25,R34,CD6),( A1,R15,R25,R34,CD7),(A1,R15,R25,R35,CD1),(A1,R15,R25,R35,CD2),(A1,R15,R25,R35,C D3),(A1,R15,R25,R35,CD4),(A1,R15,R25,R35,CD5),(A1,R15,R25,R35,CD6),(A1,R15,R25,R 35,CD7),(A1,R15,R26,R31,CD1),(A1,R15,R26,R31,CD2),(A1,R15,R26,R31,CD3),(A1,R15,R 26,R31,CD4),(A1,R15,R26,R31,CD5),(A1,R15,R26,R31,CD6),(A1,R15,R26,R31,CD7),(A1,R 15,R26,R32,CD1),(A1,R15,R26,R32,CD2),(A1,R15,R26,R32,CD3),(A1,R15,R26,R32,CD4),( A1,R15,R26,R32,CD5),(A1,R15,R26,R32,CD6),(A1,R15,R26,R32,CD7),(A1,R15,R26,R33,C D1),(A1,R15,R26,R33,CD2),(A1,R15,R26,R33,CD3),(A1,R15,R26,R33,CD4),(A1,R15,R26,R 33,CD5),(A1,R15,R26,R33,CD6),(A1,R15,R26,R33,CD7),(A1,R15,R26,R34,CD1),(A1,R15,R 26,R34,CD2),(A1,R15,R26,R34,CD3),(A1,R15,R26,R34,CD4),(A1,R15,R26,R34,CD5),(A1,R 15,R26,R34,CD6),(A1,R15,R26,R34,CD7),(A1,R15,R26,R35,CD1),(A1,R15,R26,R35,CD2),( A1,R15,R26,R35,CD3),(A1,R15,R26,R35,CD4),(A1,R15,R26,R35,CD5),(A1,R15,R26,R35,C D6),(A1,R15,R26,R35,CD7),(A1,R16,R21,R31,CD1),(A1,R16,R21,R31,CD2),(A1,R16,R21,R 31,CD3),(A1,R16,R21,R31,CD4),(A1,R16,R21,R31,CD5),(A1,R16,R21,R31,CD6),(A1,R16,R 21,R31,CD7),(A1,R16,R21,R32,CD1),(A1,R16,R21,R32,CD2),(A1,R16,R21,R32,CD3),(A1,R 16,R21,R32,CD4),(A1,R16,R21,R32,CD5),(A1,R16,R21,R32,CD6),(A1,R16,R21,R32,CD7),( A1,R16,R21,R33,CD1),(A1,R16,R21,R33,CD2),(A1,R16,R21,R33,CD3),(A1,R16,R21,R33,C D4),(A1,R16,R21,R33,CD5),(A1,R16,R21,R33,CD6),(A1,R16,R21,R33,CD7),(A1,R16,R21,R 34,CD1),(A1,R16,R21,R34,CD2),(A1,R16,R21,R34,CD3),(A1,R16,R21,R34,CD4),(A1,R16,R 21,R34,CD5),(A1,R16,R21,R34,CD6),(A1,R16,R21,R34,CD7),(A1,R16,R21,R35,CD1),(A1,R 16,R21,R35,CD2),(A1,R16,R21,R35,CD3),(A1,R16,R21,R35,CD4),(A1,R16,R21,R35,CD5),( A1,R16,R21,R35,CD6),(A1,R16,R21,R35,CD7),(A1,R16,R22,R31,CD1),(A1,R16,R22,R31,C D2),(A1,R16,R22,R31,CD3),(A1,R16,R22,R31,CD4),(A1,R16,R22,R31,CD5),(A1,R16,R22,R 31,CD6),(A1,R16,R22,R31,CD7),(A1,R16,R22,R32,CD1),(A1,R16,R22,R32,CD2),(A1,R16,R 22,R32,CD3),(A1,R16,R22,R32,CD4),(A1,R16,R22,R32,CD5),(A1,R16,R22,R32,CD6),(A1,R 16,R22,R32,CD7),(A1,R16,R22,R33,CD1),(A1,R16,R22,R33,CD2),(A1,R16,R22,R33,CD3),( A1,R16,R22,R33,CD4),(A1,R16,R22,R33,CD5),(A1,R16,R22,R33,CD6),(A1,R16,R22,R33,C D7),(A1,R16,R22,R34,CD1),(A1,R16,R22,R34,CD2),(A1,R16,R22,R34,CD3),(A1,R16,R22,R 34,CD4),(A1,R16,R22,R34,CD5),(A1,R16,R22,R34,CD6),(A1,R16,R22,R34,CD7),(A1,R16,R 22,R35,CD1),(A1,R16,R22,R35,CD2),(A1,R16,R22,R35,CD3),(A1,R16,R22,R35,CD4),(A1,R 16,R22,R35,CD5),(A1,R16,R22,R35,CD6),(A1,R16,R22,R35,CD7),(A1,R16,R23,R31,CD1),( A1,R16,R23,R31,CD2),(A1,R16,R23,R31,CD3),(A1,R16,R23,R31,CD4),(A1,R16,R23,R31,C D5),(A1,R16,R23,R31,CD6),(A1,R16,R23,R31,CD7),(A1,R16,R23,R32,CD1),(A1,R16,R23,R 32,CD2),(A1,R16,R23,R32,CD3),(A1,R16,R23,R32,CD4),(A1,R16,R23,R32,CD5),(A1,R16,R 23,R32,CD6),(A1,R16,R23,R32,CD7),(A1,R16,R23,R33,CD1),(A1,R16,R23,R33,CD2),(A1,R 16,R23,R33,CD3),(A1,R16,R23,R33,CD4),(A1,R16,R23,R33,CD5),(A1,R16,R23,R33,CD6),( A1,R16,R23,R33,CD7),(A1,R16,R23,R34,CD1),(A1,R16,R23,R34,CD2),(A1,R16,R23,R34,C D3),(A1,R16,R23,R34,CD4),(A1,R16,R23,R34,CD5),(A1,R16,R23,R34,CD6),(A1,R16,R23,R 34,CD7),(A1,R16,R23,R35,CD1),(A1,R16,R23,R35,CD2),(A1,R16,R23,R35,CD3),(A1,R16,R 23,R35,CD4),(A1,R16,R23,R35,CD5),(A1,R16,R23,R35,CD6),(A1,R16,R23,R35,CD7),(A1,R 16,R24,R31,CD1),(A1,R16,R24,R31,CD2),(A1,R16,R24,R31,CD3),(A1,R16,R24,R31,CD4),( A1,R16,R24,R31,CD5),(A1,R16,R24,R31,CD6),(A1,R16,R24,R31,CD7),(A1,R16,R24,R32,C D1),(A1,R16,R24,R32,CD2),(A1,R16,R24,R32,CD3),(A1,R16,R24,R32,CD4),(A1,R16,R24,R 32,CD5),(A1,R16,R24,R32,CD6),(A1,R16,R24,R32,CD7),(A1,R16,R24,R33,CD1),(A1,R16,R 24,R33,CD2),(A1,R16,R24,R33,CD3),(A1,R16,R24,R33,CD4),(A1,R16,R24,R33,CD5),(A1,R 16,R24,R33,CD6),(A1,R16,R24,R33,CD7),(A1,R16,R24,R34,CD1),(A1,R16,R24,R34,CD2),( A1,R16,R24,R34,CD3),(A1,R16,R24,R34,CD4),(A1,R16,R24,R34,CD5),(A1,R16,R24,R34,C D6),(A1,R16,R24,R34,CD7),(A1,R16,R24,R35,CD1),(A1,R16,R24,R35,CD2),(A1,R16,R24,R 35,CD3),(A1,R16,R24,R35,CD4),(A1,R16,R24,R35,CD5),(A1,R16,R24,R35,CD6),(A1,R16,R 24,R35,CD7),(A1,R16,R25,R31,CD1),(A1,R16,R25,R31,CD2),(A1,R16,R25,R31,CD3),(A1,R 16,R25,R31,CD4),(A1,R16,R25,R31,CD5),(A1,R16,R25,R31,CD6),(A1,R16,R25,R31,CD7),( A1,R16,R25,R32,CD1),(A1,R16,R25,R32,CD2),(A1,R16,R25,R32,CD3),(A1,R16,R25,R32,C D4),(A1,R16,R25,R32,CD5),(A1,R16,R25,R32,CD6),(A1,R16,R25,R32,CD7),(A1,R16,R25,R 33,CD1),(A1,R16,R25,R33,CD2),(A1,R16,R25,R33,CD3),(A1,R16,R25,R33,CD4),(A1,R16,R 25,R33,CD5),(A1,R16,R25,R33,CD6),(A1,R16,R25,R33,CD7),(A1,R16,R25,R34,CD1),(A1,R 16,R25,R34,CD2),(A1,R16,R25,R34,CD3),(A1,R16,R25,R34,CD4),(A1,R16,R25,R34,CD5),( A1,R16,R25,R34,CD6),(A1,R16,R25,R34,CD7),(A1,R16,R25,R35,CD1),(A1,R16,R25,R35,C D2),(A1,R16,R25,R35,CD3),(A1,R16,R25,R35,CD4),(A1,R16,R25,R35,CD5),(A1,R16,R25,R 35,CD6),(A1,R16,R25,R35,CD7),(A1,R16,R26,R31,CD1),(A1,R16,R26,R31,CD2),(A1,R16,R 26,R31,CD3),(A1,R16,R26,R31,CD4),(A1,R16,R26,R31,CD5),(A1,R16,R26,R31,CD6),(A1,R 16,R26,R31,CD7),(A1,R16,R26,R32,CD1),(A1,R16,R26,R32,CD2),(A1,R16,R26,R32,CD3),( A1,R16,R26,R32,CD4),(A1,R16,R26,R32,CD5),(A1,R16,R26,R32,CD6),(A1,R16,R26,R32,C D7),(A1,R16,R26,R33,CD1),(A1,R16,R26,R33,CD2),(A1,R16,R26,R33,CD3),(A1,R16,R26,R 33,CD4),(A1,R16,R26,R33,CD5),(A1,R16,R26,R33,CD6),(A1,R16,R26,R33,CD7),(A1,R16,R 26,R34,CD1),(A1,R16,R26,R34,CD2),(A1,R16,R26,R34,CD3),(A1,R16,R26,R34,CD4),(A1,R 16,R26,R34,CD5),(A1,R16,R26,R34,CD6),(A1,R16,R26,R34,CD7),(A1,R16,R26,R35,CD1),( A1,R16,R26,R35,CD2),(A1,R16,R26,R315,CD3),(A1,R16,R26,R35,CD4),(A1,R16,R26,R35,C D5),(A1,R16,R26,R35,CD6),(A1,R16,R26,R35,CD7),(A1,R17,R21,R31,CD1),(A1,R17,R21,R 31,CD2),(A1,R17,R21,R31,CD3),(A1,R17,R21,R31,CD4),(A1,R17,R21,R31,CD5),(A1,R17,R 21,R31,CD6),(A1,R17,R21,R31,CD7),(A1,R17,R21,R32,CD1),(A1,R17,R21,R32,CD2),(A1,R 17,R21,R32,CD3),(A1,R17,R21,R32,C174),(A1,R17,R21,R32,CD5),(A1,R17,R21,R32,CD6),( A1,R17,R21,R32,CD7),(A1,R17,R21,R33,CD1),(A1,R17,R21,R33,CD2),(A1,R17,R21,R33,C D3),(A1,R17,R21,R33,CD4),(A1,R17,R21,R33,CD5),(A1,R17,R21,R33,CD6),(A1,R17,R21,R 33,CD7),(A1,R17,R21,R34,CD1),(A1,R17,R21,R34,CD2),(A1,R17,R21,R34,CD3),(A1,R17,R 21,R34,CD4),(A1,R17,R21,R34,CD5),(A1,R17,R21,R34,CD6),(A1,R17,R21,R34,CD7),(A1,R 17,R21,R35,CD1),(A1,R17,R21,R35,CD2),(A1,R17,R21,R35,CD3),(A1,R17,R21,R35,CD4),( A1,R17,R21,R35,CD5),(A1,R17,R21,R35,CD6),(A1,R17,R21,R35,CD7),(A1,R17,R22,R31,C D1),(A1,R17,R22,R31,CD2),(A1,R17,R22,R31,CD3),(A1,R17,R22,R31,CD4),(A1,R17,R22,R 31,CD5),(A1,R17,R22,R31,CD6),(A1,R17,R22,R31,CD7),(A1,R17,R22,R32,CD1),(A1,R17,R 22,R32,CD2),(A1,R17,R22,R32,CD3),(A1,R17,R22,R32,CD4),(A1,R17,R22,R32,CD5),(A1,R 17,R22,R32,CD6),(A1,R17,R22,R32,CD7),(A1,R17,R22,R33,CD1),(A1,R17,R22,R33,CD2),( A1,R17,R22,R33,CD3),(A1,R17,R22,R33,CD4),(A1,R17,R22,R33,CD5),(A1,R17,R22,R33,C D6),(A1,R17,R22,R33,CD7),(A1,R17,R22,R34,CD1),(A1,R17,R22,R34,CD2),(A1,R17,R22,R 34,CD3),(A1,R17,R22,R34,CD4),(A1,R17,R22,R34,CD5),(A1,R17,R22,R34,CD6),(A1,R17,R 22,R34,CD7),(A1,R17,R22,R35,CD1),(A1,R17,R22,R35,CD2),(A1,R17,R22,R35,CD3),(A1,R 17,R22,R35,CD4),(A1,R17,R22,R35,CD5),(A1,R17,R22,R35,CD6),(A1,R17,R22,R35,CD7),( A1,R17,R23,R31,CD1),(A1,R17,R23,R31,CD2),(A1,R17,R23,R31,CD3),(A1,R17,R23,R31,C D4),(A1,R17,R23,R31,CD5),(A1,R17,R23,R31,CD6),(A1,R17,R23,R31,CD7),(A1,R17,R23,R 32,CD1),(A1,R17,R23,R32,CD2),(A1,R17,R23,R32,CD3),(A1,R17,R23,R32,CD4),(A1,R17,R 23,R32,CD5),(A1,R17,R23,R32,CD6),(A1,R17,R23,R32,CD7),(A1,R17,R23,R33,CD1),(A1,R 17,R23,R33,CD2),(A1,R17,R23,R33,CD3),(A1,R17,R23,R33,CD4),(A1,R17,R23,R33,CD5),( A1,R17,R23,R33,CD6),(A1,R17,R23,R33,CD7),(A1,R17,R23,R34,CD1),(A1,R17,R23,R34,C D2),(A1,R17,R23,R34,CD3),(A1,R17,R23,R34,CD4),(A1,R17,R23,R34,CD5),(A1,R17,R23,R 34,CD6),(A1,R17,R23,R34,CD7),(A1,R17,R23,R35,CD1),(A1,R17,R23,R35,CD2),(A1,R17,R 23,R35,CD3),(A1,R17,R23,R35,CD4),(A1,R17,R23,R35,CD5),(A1,R17,R23,R35,CD6),(A1,R 17,R23,R35,CD7),(A1,R17,R24,R31,CD1),(A1,R17,R24,R31,CD2),(A1,R17,R24,R31,CD3),( A1,R17,R24,R31,CD4),(A1,R17,R24,R31,CD5),(A1,R17,R24,R31,CD6),(A1,R17,R24,R31,C D7),(A1,R17,R24,R32,CD1),(A1,R17,R24,R32,CD2),(A1,R17,R24,R32,CD3),(A1,R17,R24,R 32,CD4),(A1,R17,R24,R32,CD5),(A1,R17,R24,R32,CD6),(A1,R17,R24,R32,CD7),(A1,R17,R 24,R33,CD1),(A1,R17,R24,R33,CD2),(A1,R17,R24,R33,CD3),(A1,R17,R24,R33,CD4),(A1,R 17,R24,R33,CD5),(A1,R17,R24,R33,CD6),(A1,R17,R24,R33,CD7),(A1,R17,R24,R34,CD1),( A1,R17,R24,R34,CD2),(A1,R17,R24,R34,CD3),(A1,R17,R24,R34,CD4),(A1,R17,R24,R34,C D5),(A1,R17,R24,R34,CD6),(A1,R17,R24,R34,CD7),(A1,R17,R24,R35,CD1),(A1,R17,R24,R 35,CD2),(A1,R17,R24,R35,CD3),(A1,R17,R24,R35,CD4),(A1,R17,R24,R35,CD5),(A1,R17,R 24,R35,CD6),(A1,R17,R24,R35,CD7),(A1,R17,R25,R31,CD1),(A1,R17,R25,R31,CD2),(A1,R 17,R25,R31,CD3),(A1,R17,R25,R31,CD4),(A1,R17,R25,R31,CD5),(A1,R17,R25,R31,CD6),( A1,R17,R25,R31,CD7),(A1,R17,R25,R32,CD1),(A1,R17,R25,R32,CD2),(A1,R17,R25,R32,C D3),(A1,R17,R25,R32,CD4),(A1,R17,R25,R32,CD5),(A1,R17,R25,R32,CD6),(A1,R17,R25,R 32,CD7),(A1,R17,R25,R33,CD1),(A1,R17,R25,R33,CD2),(A1,R17,R25,R33,CD3),(A1,R17,R 25,R33,CD4),(A1,R17,R25,R33,CD5),(A1,R17,R25,R33,CD6),(A1,R17,R25,R33,CD7),(A1,R 17,R25,R34,CD1),(A1,R17,R25,R34,CD2),(A1,R17,R25,R34,CD3),(A1,R17,R25,R34,CD4),( A1,R17,R25,R34,CD5),(A1,R17,R25,R34,CD6),(A1,R17,R25,R34,CD7),(A1,R17,R25,R35,C D1),(A1,R17,R25,R35,CD2),(A1,R17,R25,R35,CD3),(A1,R17,R25,R35,CD4),(A1,R17,R25,R 35,CD5),(A1,R17,R25,R35,CD6),(A1,R17,R25,R35,CD7),(A1,R17,R26,R31,CD1),(A1,R17,R 26,R31,CD2),(A1,R17,R26,R31,CD3),(A1,R17,R26,R31,CD4),(A1,R17,R26,R31,CD5),(A1,R 17,R26,R31,CD6),(A1,R17,R26,R31,CD7),(A1,R17,R26,R32,CD1),(A1,R17,R26,R32,CD2),( A1,R17,R26,R32,CD3),(A1,R17,R26,R32,CD4),(A1,R17,R26,R32,CD5),(A1,R17,R26,R32,C D6),(A1,R17,R26,R32,CD7),(A1,R17,R26,R33,CD1),(A1,R17,R26,R33,CD2),(A1,R17,R26,R 33,CD3),(A1,R17,R26,R33,CD4),(A1,R17,R26,R33,CD5),(A1,R17,R26,R33,CD6),(A1,R17,R 26,R33,CD7),(A1,R17,R26,R34,CD1),(A1,R17,R26,R34,CD2),(A1,R17,R26,R34,CD3),(A1,R 17,R26,R34,CD4),(A1,R17,R26,R34,CD5),(A1,R17,R26,R34,CD6),(A1,R17,R26,R34,CD7),( A1,R17,R26,R35,CD1),(A1,R17,R26,R35,CD2),(A1,R17,R26,R35,CD3),(A1,R17,R26,R35,C D4),(A1,R17,R26,R35,CD5),(A1,R17,R26,R35,CD6),(A1,R17,R26,R35,CD7),(A1,R18,R21,R 31,CD1),(A1,R18,R21,R31,CD2),(A1,R18,R21,R31,CD3),(A1,R18,R21,R31,CD4),(A1,R18,R 21,R31,CD5),(A1,R18,R21,R31,CD6),(A1,R18,R21,R31,CD7),(A1,R18,R21,R32,CD1),(A1,R 18,R21,R32,CD2),(A1,R18,R21,R32,CD3),(A1,R18,R21,R32,CD4),(A1,R18,R21,R32,CD5),( A1,R18,R21,R32,CD6),(A1,R18,R21,R32,CD7),(A1,R18,R21,R33,CD1),(A1,R18,R21,R33,C D2),(A1,R18,R21,R33,CD3),(A1,R18,R21,R33,CD4),(A1,R18,R21,R33,CD5),(A1,R18,R21,R 33,CD6),(A1,R18,R21,R33,CD7),(A1,R18,R21,R34,CD1),(A1,R18,R21,R34,CD2),(A1,R18,R 21,R34,CD3),(A1,R18,R21,R34,CD4),(A1,R18,R21,R34,CD5),(A1,R18,R21,R34,CD6),(A1,R 18,R21,R34,CD7),(A1,R18,R21,R35,CD1),(A1,R18,R21,R35,CD2),(A1,R18,R21,R35,CD3),( A1,R18,R21,R35,CD4),(A1,R18,R21,R35,CD5),(A1,R18,R21,R35,CD6),(A1,R18,R21,R35,C D7),(A1,R18,R22,R31,CD1),(A1,R18,R22,R31,CD2),(A1,R18,R22,R31,CD3),(A1,R18,R22,R 31,CD4),(A1,R18,R22,R31,CD5),(A1,R18,R22,R31,CD6),(A1,R18,R22,R31,CD7),(A1,R18,R 22,R32,CD1),(A1,R18,R22,R32,CD2),(A1,R18,R22,R32,CD3),(A1,R18,R22,R32,CD4),(A1,R 18,R22,R32,CD5),(A1,R18,R22,R32,CD6),(A1,R18,R22,R32,CD7),(A1,R18,R22,R33,CD1),( A1,R18,R22,R33,CD2),(A1,R18,R22,R33,CD3),(A1,R18,R22,R33,CD4),(A1,R18,R22,R33,C D5),(A1,R18,R22,R33,CD6),(A1,R18,R22,R33,CD7),(A1,R18,R22,R34,CD1),(A1,R18,R22,R 34,CD2),(A1,R18,R22,R34,CD3),(A1,R18,R22,R34,CD4),(A1,R18,R22,R34,CD5),(A1,R18,R 22,R34,CD6),(A1,R18,R22,R34,CD7),(A1,R18,R22,R35,CD1),(A1,R18,R22,R35,CD2),(A1,R 18,R22,R35,CD3),(A1,R18,R22,R35,CD4),(A1,R18,R22,R35,CD5),(A1,R18,R22,R35,CD6),( A1,R18,R22,R35,CD7),(A1,R18,R23,R31,CD1),(A1,R18,R23,R31,CD2),(A1,R18,R23,R31,C D3),(A1,R18,R23,R31,CD4),(A1,R18,R23,R31,CD5),(A1,R18,R23,R31,CD6),(A1,R18,R23,R 31,CD7),(A1,R18,R23,R32,CD1),(A1,R18,R23,R32,CD2),(A1,R18,R23,R32,CD3),(A1,R18,R 23,R32,CD4),(A1,R18,R23,R32,CD5),(A1,R18,R23,R32,CD6),(A1,R18,R23,R32,CD7),(A1,R 18,R23,R33,CD1),(A1,R18,R23,R33,CD2),(A1,R18,R23,R33,CD3),(A1,R18,R23,R33,CD4),( A1,R18,R23,R33,CD5),(A1,R18,R23,R33,CD6),(A1,R18,R23,R33,CD7),(A1,R18,R23,R34,C D1),(A1,R18,R23,R34,CD2),(A1,R18,R23,R34,CD3),(A1,R18,R23,R34,CD4),(A1,R18,R23,R 34,CD5),(A1,R18,R23,R34,CD6),(A1,R18,R23,R34,CD7),(A1,R18,R23,R35,CD1),(A1,R18,R 23,R35,CD2),(A1,R18,R23,R35,CD3),(A1,R18,R23,R35,CD4),(A1,R18,R23,R35,CD5),(A1,R 18,R23,R35,CD6),(A1,R18,R23,R35,CD7),(A1,R18,R24,R31,CD1),(A1,R18,R24,R31,CD2),( A1,R18,R24,R31,CD3),(A1,R18,R24,R31,CD4),(A1,R18,R24,R31,CD5),(A1,R18,R24,R31,C D6),(A1,R18,R24,R31,CD7),(A1,R18,R24,R32,CD1),(A1,R18,R24,R32,CD2),(A1,R18,R24,R 32,CD3),(A1,R18,R24,R32,CD4),(A1,R18,R24,R32,CD5),(A1,R18,R24,R32,CD6),(A1,R18,R 24,R32,CD7),(A1,R18,R24,R33,CD1),(A1,R18,R24,R33,CD2),(A1,R18,R24,R33,CD3),(A1,R 18,R24,R33,CD4),(A1,R18,R24,R33,CD5),(A1,R18,R24,R33,CD6),(A1,R18,R24,R33,CD7),( A1,R18,R24,R34,CD1),(A1,R18,R24,R34,CD2),(A1,R18,R24,R34,CD3),(A1,R18,R24,R34,C D4),(A1,R18,R24,R34,CD5),(A1,R18,R24,R34,CD6),(A1,R18,R24,R34,CD7),(A1,R18,R24,R 35,CD1),(A1,R18,R24,R35,CD2),(A1,R18,R24,R35,CD3),(A1,R18,R24,R35,CD4),(A1,R18,R 24,R35,CD5),(A1,R18,R24,R35,CD6),(A1,R18,R24,R35,CD7),(A1,R18,R25,R31,CD1),(A1,R 18,R25,R31,CD2),(A1,R18,R25,R31,CD3),(A1,R18,R25,R31,CD4),(A1,R18,R25,R31,CD5),( A1,R18,R25,R31,CD6),(A1,R18,R25,R31,CD7),(A1,R18,R25,R32,CD1),(A1,R18,R25,R32,C D2),(A1,R18,R25,R32,CD3),(A1,R18,R25,R32,CD4),(A1,R18,R25,R32,CD5),(A1,R18,R25,R 32,CD6),(A1,R18,R25,R32,CD7),(A1,R18,R25,R33,CD1),(A1,R18,R25,R33,CD2),(A1,R18,R 25,R33,CD3),(A1,R18,R25,R33,CD4),(A1,R18,R25,R33,CD5),(A1,R18,R25,R33,CD6),(A1,R 18,R25,R33,CD7),(A1,R18,R25,R34,CD1),(A1,R18,R25,R34,CD2),(A1,R18,R25,R34,CD3),( A1,R18,R25,R34,CD4),(A1,R18,R25,R34,CD5),(A1,R18,R25,R34,CD6),(A1,R18,R25,R34,C D7),(A1,R18,R25,R35,CD1),(A1,R18,R25,R35,CD2),(A1,R18,R25,R35,CD3),(A1,R18,R25,R 35,CD4),(A1,R18,R25,R35,CD5),(A1,R18,R25,R35,CD6),(A1,R18,R25,R35,CD7),(A1,R18,R 26,R31,CD1),(A1,R18,R26,R31,CD2),(A1,R18,R26,R31,CD3),(A1,R18,R26,R31,CD4),(A1,R 18,R26,R31,CD5),(A1,R18,R26,R31,CD6),(A1,R18,R26,R31,CD7),(A1,R18,R26,R32,CD1),( A1,R18,R26,R32,CD2),(A1,R18,R26,R32,CD3),(A1,R18,R26,R32,CD4),(A1,R18,R26,R32,C D5),(A1,R18,R26,R32,CD6),(A1,R18,R26,R32,CD7),(A1,R18,R26,R33,CD1),(A1,R18,R26,R 33,CD2),(A1,R18,R26,R33,CD3),(A1,R18,R26,R33,CD4),(A1,R18,R26,R33,CD5),(A1,R18,R 26,R33,CD6),(A1,R18,R26,R33,CD7),(A1,R18,R26,R34,CD1),(A1,R18,R26,R34,CD2),(A1,R 18,R26,R34,CD3),(A1,R18,R26,R34,CD4),(A1,R18,R26,R34,CD5),(A1,R18,R26,R34,CD6),( A1,R18,R26,R34,CD7),(A1,R18,R26,R35,CD1),(A1,R18,R26,R35,CD2),(A1,R18,R26,R35,C D3),(A1,R18,R26,R35,CD4),(A1,R18,R26,R35,CD5),(A1,R18,R26,R35,CD6),(A1,R18,R26,R 35,CD7),(A1,R19,R21,R31,CD1),(A1,R19,R21,R31,CD2),(A1,R19,R21,R31,CD3),(A1,R19,R 21,R31,CD4),(A1,R19,R21,R31,CD5),(A1,R19,R21,R31,CD6),(A1,R19,R21,R31,CD7),(A1,R 19,R21,R32,CD1),(A1,R19,R21,R32,CD2),(A1,R19,R21,R32,CD3),(A1,R19,R21,R32,CD4),( A1,R19,R21,R32,CD5),(A1,R19,R21,R32,CD6),(A1,R19,R21,R32,CD7),(A1,R19,R21,R33,C D1),(A1,R19,R21,R33,CD2),(A1,R19,R21,R33,CD3),(A1,R19,R21,R33,CD4),(A1,R19,R21,R 33,CD5),(A1,R19,R21,R33,CD6),(A1,R19,R21,R33,CD7),(A1,R19,R21,R34,CD1),(A1,R19,R 21,R34,CD2),(A1,R19,R21,R34,CD3),(A1,R19,R21,R34,CD4),(A1,R19,R21,R34,CD5),(A1,R 19,R21,R34,CD6),(A1,R19,R21,R34,CD7),(A1,R19,R21,R35,CD1),(A1,R19,R21,R35,CD2),( A1,R19,R21,R35,CD3),(A1,R19,R21,R35,CD4),(A1,R19,R21,R35,CD5),(A1,R19,R21,R35,C D6),(A1,R19,R21,R35,CD7),(A1,R19,R22,R31,CD1),(A1,R19,R22,R31,CD2),(A1,R19,R22,R 31,CD3),(A1,R19,R22,R31,CD4),(A1,R19,R22,R31,CD5),(A1,R19,R22,R31,CD6),(A1,R19,R 22,R31,CD7),(A1,R19,R22,R32,CD1),(A1,R19,R22,R32,CD2),(A1,R19,R22,R32,CD3),(A1,R 19,R22,R32,CD4),(A1,R19,R22,R32,CD5),(A1,R19,R22,R32,CD6),(A1,R19,R22,R32,CD7),( A1,R19,R22,R33,CD1),(A1,R19,R22,R33,CD2),(A1,R19,R22,R33,CD3),(A1,R19,R22,R33,C D4),(A1,R19,R22,R33,CD5),(A1,R19,R22,R33,CD6),(A1,R19,R22,R33,CD7),(A1,R19,R22,R 34,CD1),(A1,R19,R22,R34,CD2),(A1,R19,R22,R34,CD3),(A1,R19,R22,R34,CD4),(A1,R19,R 22,R34,CD5),(A1,R19,R22,R34,CD6),(A1,R19,R22,R34,CD7),(A1,R19,R22,R35,CD1),(A1,R 19,R22,R35,CD2),(A1,R19,R22,R35,CD3),(A1,R19,R22,R35,CD4),(A1,R19,R22,R35,CD5),( A1,R19,R22,R35,CD6),(A1,R19,R22,R35,CD7),(A1,R19,R23,R31,CD1),(A1,R19,R23,R31,C D2),(A1,R19,R23,R31,CD3),(A1,R19,R23,R31,CD4),(A1,R19,R23,R31,CD5),(A1,R19,R23,R 31,CD6),(A1,R19,R23,R31,CD7),(A1,R19,R23,R32,CD1),(A1,R19,R23,R32,CD2),(A1,R19,R 23,R32,CD3),(A1,R19,R23,R32,CD4),(A1,R19,R23,R32,CD5),(A1,R19,R23,R32,CD6),(A1,R 19,R23,R32,CD7),(A1,R19,R23,R33,CD1),(A1,R19,R23,R33,CD2),(A1,R19,R23,R33,CD3),( A1,R19,R23,R33,CD4),(A1,R19,R23,R33,CD5),(A1,R19,R23,R33,CD6),(A1,R19,R23,R33,C D7),(A1,R19,R23,R34,CD1),(A1,R19,R23,R34,CD2),(A1,R19,R23,R34,CD3),(A1,R19,R23,R 34,CD4),(A1,R19,R23,R34,CD5),(A1,R19,R23,R34,CD6),(A1,R19,R23,R34,CD7),(A1,R19,R 23,R35,CD1),(A1,R19,R23,R35,CD2),(A1,R19,R23,R35,CD3),(A1,R19,R23,R35,CD4),(A1,R 19,R23,R35,CD5),(A1,R19,R23,R35,CD6),(A1,R19,R23,R35,CD7),(A1,R19,R24,R31,CD1),( A1,R19,R24,R31,CD2),(A1,R19,R24,R31,CD3),(A1,R19,R24,R31,CD4),(A1,R19,R24,R31,C D5),(A1,R19,R24,R31,CD6),(A1,R19,R24,R31,CD7),(A1,R19,R24,R32,CD1),(A1,R19,R24,R 32,CD2),(A1,R19,R24,R32,CD3),(A1,R19,R24,R32,CD4),(A1,R19,R24,R32,CD5),(A1,R19,R 24,R32,CD6),(A1,R19,R24,R32,CD7),(A1,R19,R24,R33,CD1),(A1,R19,R24,R33,CD2),(A1,R 19,R24,R33,CD3),(A1,R19,R24,R33,CD4),(A1,R19,R24,R33,CD5),(A1,R19,R24,R33,CD6),( A1,R19,R24,R33,CD7),(A1,R19,R24,R34,CD1),(A1,R19,R24,R34,CD2),(A1,R19,R24,R34,C D3),(A1,R19,R24,R34,CD4),(A1,R19,R24,R34,CD5),(A1,R19,R24,R34,CD6),(A1,R19,R24,R 34,CD7),(A1,R19,R24,R35,CD1),(A1,R19,R24,R35,CD2),(A1,R19,R24,R35,CD3),(A1,R19,R 24,R35,CD4),(A1,R19,R24,R35,CD5),(A1,R19,R24,R35,CD6),(A1,R19,R24,R35,CD7),(A1,R 19,R25,R31,CD1),(A1,R19,R25,R31,CD2),(A1,R19,R25,R31,CD3),(A1,R19,R25,R31,CD4),( A1,R19,R25,R31,CD5),(A1,R19,R25,R31,CD6),(A1,R19,R25,R31,CD7),(A1,R19,R25,R32,C D1),(A1,R19,R25,R32,CD2),(A1,R19,R25,R32,CD3),(A1,R19,R25,R32,CD4),(A1,R19,R25,R 32,CD5),(A1,R19,R25,R32,CD6),(A1,R19,R25,R32,CD7),(A1,R19,R25,R33,CD1),(A1,R19,R 25,R33,CD2),(A1,R19,R25,R33,CD3),(A1,R19,R25,R33,CD4),(A1,R19,R25,R33,CD5),(A1,R 19,R25,R33,CD6),(A1,R19,R25,R33,CD7),(A1,R19,R25,R34,CD1),(A1,R19,R25,R34,CD2),( A1,R19,R25,R34,CD3),(A1,R19,R25,R34,CD4),(A1,R19,R25,R34,CD5),(A1,R19,R25,R34,C D6),(A1,R19,R25,R34,CD7),(A1,R19,R25,R35,CD1),(A1,R19,R25,R35,CD2),(A1,R19,R25,R 35,CD3),(A1,R19,R25,R35,CD4),(A1,R19,R25,R35,CD5),(A1,R19,R25,R35,CD6),(A1,R19,R 25,R35,CD7),(A1,R19,R26,R31,CD1),(A1,R19,R26,R31,CD2),(A1,R19,R26,R31,CD3),(A1,R 19,R26,R31,CD4),(A1,R19,R26,R31,CD5),(A1,R19,R26,R31,CD6),(A1,R19,R26,R31,CD7),( A1,R19,R26,R32,CD1),(A1,R19,R26,R32,CD2),(A1,R19,R26,R32,CD3),(A1,R19,R26,R32,C D4),(A1,R19,R26,R32,CD5),(A1,R19,R26,R32,CD6),(A1,R19,R26,R32,CD7),(A1,R19,R26,R 33,CD1),(A1,R19,R26,R33,CD2),(A1,R19,R26,R33,CD3),(A1,R19,R26,R33,CD4),(A1,R19,R 26,R33,CD5),(A1,R19,R26,R33,CD6),(A1,R19,R26,R33,CD7),(A1,R19,R26,R34,CD1),(A1,R 19,R26,R34,CD2),(A1,R19,R26,R34,CD3),(A1,R19,R26,R34,CD4),(A1,R19,R26,R34,CD5),( A1,R19,R26,R34,CD6),(A1,R19,R26,R34,CD7),(A1,R19,R26,R35,CD1),(A1,R19,R26,R35,C D2),(A1,R19,R26,R35,CD3),(A1,R19,R26,R35,CD4),(A1,R19,R26,R35,CD5),(A1,R19,R26,R 35,CD6),(A1,R19,R26,R35,CD7),

(A2,R11,R21,R31,CD1),(A2,R11,R21,R31,CD2),(A2,R11,R21,R31,CD3),(A2,R11,R21,R31,C D4),(A2,R11,R21,R31,CD5),(A2,R11,R21,R31,CD6),(A2,R11,R21,R31,CD7),(A2,R11,R21,R 32,CD1),(A2,R11,R21,R32,CD2),(A2,R11,R21,R32,CD3),(A2,R11,R21,R32,CD4),(A2,R11,R 21,R32,CD5),(A2,R11,R21,R32,CD6),(A2,R11,R21,R32,CD7),(A2,R11,R21,R33,CD1),(A2,R 11,R21,R33,CD2),(A2,R11,R21,R33,CD3),(A2,R11,R21,R33,CD4),(A2,R11,R21,R33,CD5),( A2,R11,R21,R33,CD6),(A2,R11,R21,R33,CD7),(A2,R11,R21,R34,CD1),(A2,R11,R21,R34,C D2),(A2,R11,R21,R34,CD3),(A2,R11,R21,R34,CD4),(A2,R11,R21,R34,CD5),(A2,R11,R21,R 34,CD6),(A2,R11,R21,R34,CD7),(A2,R11,R21,R35,CD1),(A2,R11,R21,R35,CD2),(A2,R11,R 21,R35,CD3),(A2,R11,R21,R35,CD4),(A2,R11,R21,R35,CD5),(A2,R11,R21,R35,CD6),(A2,R 11,R21,R35,CD7),(A2,R11,R22,R31,CD1),(A2,R11,R22,R31,CD2),(A2,R11,R22,R31,CD3),( A2,R11,R22,R31,CD4),(A2,R11,R22,R31,CD5),(A2,R11,R22,R31,CD6),(A2,R11,R22,R31,C D7),(A2,R11,R22,R32,CD1),(A2,R11,R22,R32,CD2),(A2,R11,R22,R32,CD3),(A2,R11,R22,R 32,CD4),(A2,R11,R22,R32,CD5),(A2,R11,R22,R32,CD6),(A2,R11,R22,R32,CD7),(A2,R11,R 22,R33,CD1),(A2,R11,R22,R33,CD2),(A2,R11,R22,R33,CD3),(A2,R11,R22,R33,CD4),(A2,R 11,R22,R33,CD5),(A2,R11,R22,R33,CD6),(A2,R11,R22,R33,CD7),(A2,R11,R22,R34,CD1),( A2,R11,R22,R34,CD2),(A2,R11,R22,R34,CD3),(A2,R11,R22,R34,CD4),(A2,R11,R22,R34,C D5),(A2,R11,R22,R34,CD6),(A2,R11,R22,R34,CD7),(A2,R11,R22,R35,CD1),(A2,R11,R22,R 35,CD2),(A2,R11,R22,R35,CD3),(A2,R11,R22,R35,CD4),(A2,R11,R22,R35,CD5),(A2,R11,R 22,R35,CD6),(A2,R11,R22,R35,CD7),(A2,R11,R23,R31,CD1),(A2,R11,R23,R31,CD2),(A2,R 11,R23,R31,CD3),(A2,R11,R23,R31,CD4),(A2,R11,R23,R31,CD5),(A2,R11,R23,R31,CD6),( A2,R11,R23,R31,CD7),(A2,R11,R23,R32,CD1),(A2,R11,R23,R32,CD2),(A2,R11,R23,R32,C D3),(A2,R11,R23,R32,CD4),(A2,R11,R23,R32,CD5),(A2,R11,R23,R32,CD6),(A2,R11,R23,R 32,CD7),(A2,R11,R23,R33,CD1),(A2,R11,R23,R33,CD2),(A2,R11,R23,R33,CD3),(A2,R11,R 23,R33,CD4),(A2,R11,R23,R33,CD5),(A2,R11,R23,R33,CD6),(A2,R11,R23,R33,CD7),(A2,R 11,R23,R34,CD1),(A2,R11,R23,R34,CD2),(A2,R11,R23,R34,CD3),(A2,R11,R23,R34,CD4),( A2,R11,R23,R34,CD5),(A2,R11,R23,R34,CD6),(A2,R11,R23,R34,CD7),(A2,R11,R23,R35,C D1),(A2,R11,R23,R35,CD2),(A2,R11,R23,R35,CD3),(A2,R11,R23,R35,CD4),(A2,R11,R23,R 35,CD5),(A2,R11,R23,R35,CD6),(A2,R11,R23,R35,CD7),(A2,R11,R24,R31,CD1),(A2,R11,R 24,R31,CD2),(A2,R11,R24,R31,CD3),(A2,R11,R24,R31,CD4),(A2,R11,R24,R31,CD5),(A2,R 11,R24,R31,CD6),(A2,R11,R24,R31,CD7),(A2,R11,R24,R32,CD1),(A2,R11,R24,R32,CD2),( A2,R11,R24,R32,CD3),(A2,R11,R24,R32,CD4),(A2,R11,R24,R32,CD5),(A2,R11,R24,R32,C D6),(A2,R11,R24,R32,CD7),(A2,R11,R24,R33,CD1),(A2,R11,R24,R33,CD2),(A2,R11,R24,R 33,CD3),(A2,R11,R24,R33,CD4),(A2,R11,R24,R33,CD5),(A2,R11,R24,R33,CD6),(A2,R11,R 24,R33,CD7),(A2,R11,R24,R34,CD1),(A2,R11,R24,R34,CD2),(A2,R11,R24,R34,CD3),(A2,R 11,R24,R34,CD4),(A2,R11,R24,R34,CD5),(A2,R11,R24,R34,CD6),(A2,R11,R24,R34,CD7),( A2,R11,R24,R35,CD1),(A2,R11,R24,R35,CD2),(A2,R11,R24,R35,CD3),(A2,R11,R24,R35,C D4),(A2,R11,R24,R35,CD5),(A2,R11,R24,R35,CD6),(A2,R11,R24,R35,CD7),(A2,R11,R25,R 31,CD1),(A2,R11,R25,R31,CD2),(A2,R11,R25,R31,CD3),(A2,R11,R25,R31,CD4),(A2,R11,R 25,R31,CD5),(A2,R11,R25,R31,CD6),(A2,R11,R25,R31,CD7),(A2,R11,R25,R32,CD1),(A2,R 11,R25,R32,CD2),(A2,R11,R25,R32,CD3),(A2,R11,R25,R32,CD4),(A2,R11,R25,R32,CD5),( A2,R11,R25,R32,CD6),(A2,R11,R25,R32,CD7),(A2,R11,R25,R33,CD1),(A2,R11,R25,R33,C D2),(A2,R11,R25,R33,CD3),(A2,R11,R25,R33,CD4),(A2,R11,R25,R33,CD5),(A2,R11,R25,R 33,CD6),(A2,R11,R25,R33,CD7),(A2,R11,R25,R34,CD1),(A2,R11,R25,R34,CD2),(A2,R11,R 25,R34,CD3),(A2,R11,R25,R34,CD4),(A2,R11,R25,R34,CD5),(A2,R11,R25,R34,CD6),(A2,R 11,R25,R34,CD7),(A2,R11,R25,R35,CD1),(A2,R11,R25,R35,CD2),(A2,R11,R25,R35,CD3),( A2,R11,R25,R35,CD4),(A2,R11,R25,R35,CD5),(A2,R11,R25,R35,CD6),(A2,R11,R25,R35,C D7),(A2,R11,R26,R31,CD1),(A2,R11,R26,R31,CD2),(A2,R11,R26,R31,CD3),(A2,R11,R26,R 31,CD4),(A2,R11,R26,R31,CD5),(A2,R11,R26,R31,CD6),(A2,R11,R26,R31,CD7),(A2,R11,R 26,R32,CD1),(A2,R11,R26,R32,CD2),(A2,R11,R26,R32,CD3),(A2,R11,R26,R32,CD4),(A2,R 11,R26,R32,CD5),(A2,R11,R26,R32,CD6),(A2,R11,R26,R32,CD7),(A2,R11,R26,R33,CD1),( A2,R11,R26,R33,CD2),(A2,R11,R26,R33,CD3),(A2,R11,R26,R33,CD4),(A2,R11,R26,R33,C D5),(A2,R11,R26,R33,CD6),(A2,R11,R26,R33,CD7),(A2,R11,R26,R34,CD1),(A2,R11,R26,R 34,CD2),(A2,R11,R26,R34,CD3),(A2,R11,R26,R34,CD4),(A2,R11,R26,R34,CD5),(A2,R11,R 26,R34,CD6),(A2,R11,R26,R34,CD7),(A2,R11,R26,R35,CD1),(A2,R11,R26,R35,CD2),(A2,R 11,R26,R35,CD3),(A2,R11,R26,R35,CD4),(A2,R11,R26,R35,CD5),(A2,R11,R26,R35,CD6),( A2,R11,R26,R35,CD7),(A2,R12,R21,R31,CD1),(A2,R12,R21,R31,CD2),(A2,R12,R21,R31,C D3),(A2,R12,R21,R31,CD4),(A2,R12,R21,R31,CD5),(A2,R12,R21,R31,CD6),(A2,R12,R21,R 31,CD7),(A2,R12,R21,R32,CD1),(A2,R12,R21,R32,CD2),(A2,R12,R21,R32,CD3),(A2,R12,R 21,R32,CD4),(A2,R12,R21,R32,CD5),(A2,R12,R21,R32,CD6),(A2,R12,R21,R32,CD7),(A2,R 12,R21,R33,CD1),(A2,R12,R21,R33,CD2),(A2,R12,R21,R33,CD3),(A2,R12,R21,R33,CD4),( A2,R12,R21,R33,CD5),(A2,R12,R21,R33,CD6),(A2,R12,R21,R33,CD7),(A2,R12,R21,R34,C D1),(A2,R12,R21,R34,CD2),(A2,R12,R21,R34,CD3),(A2,R12,R21,R34,CD4),(A2,R12,R21,R 34,CD5),(A2,R12,R21,R34,CD6),(A2,R12,R21,R34,CD7),(A2,R12,R21,R35,CD1),(A2,R12,R 21,R35,CD2),(A2,R12,R21,R35,CD3),(A2,R12,R21,R35,CD4),(A2,R12,R21,R35,CD5),(A2,R 12,R21,R35,CD6),(A2,R12,R21,R35,CD7),(A2,R12,R22,R31,CD1),(A2,R12,R22,R31,CD2),( A2,R12,R22,R31,CD3),(A2,R12,R22,R31,CD4),(A2,R12,R22,R31,CD5),(A2,R12,R22,R31,C D6),(A2,R12,R22,R31,CD7),(A2,R12,R22,R32,CD1),(A2,R12,R22,R32,CD2),(A2,R12,R22,R 32,CD3),(A2,R12,R22,R32,CD4),(A2,R12,R22,R32,CD5),(A2,R12,R22,R32,CD6),(A2,R12,R 22,R32,CD7),(A2,R12,R22,R33,CD1),(A2,R12,R22,R33,CD2),(A2,R12,R22,R33,CD3),(A2,R 12,R22,R33,CD4),(A2,R12,R22,R33,CD5),(A2,R12,R22,R33,CD6),(A2,R12,R22,R33,CD7),( A2,R12,R22,R34,CD1),(A2,R12,R22,R34,CD2),(A2,R12,R22,R34,CD3),(A2,R12,R22,R34,C D4),(A2,R12,R22,R34,CD5),(A2,R12,R22,R34,CD6),(A2,R12,R22,R34,CD7),(A2,R12,R22,R 35,CD1),(A2,R12,R22,R35,CD2),(A2,R12,R22,R35,CD3),(A2,R12,R22,R35,CD4),(A2,R12,R 22,R35,CD5),(A2,R12,R22,R35,CD6),(A2,R12,R22,R35,CD7),(A2,R12,R23,R31,CD1),(A2,R 12,R23,R31,CD2),(A2,R12,R23,R31,CD3),(A2,R12,R23,R31,CD4),(A2,R12,R23,R31,CD5),( A2,R12,R23,R31,CD6),(A2,R12,R23,R31,CD7),(A2,R12,R23,R32,CD1),(A2,R12,R23,R32,C D2),(A2,R12,R23,R32,CD3),(A2,R12,R23,R32,CD4),(A2,R12,R23,R32,CD5),(A2,R12,R23,R 32,CD6),(A2,R12,R23,R32,CD7),(A2,R12,R23,R33,CD1),(A2,R12,R23,R33,CD2),(A2,R12,R 23,R33,CD3),(A2,R12,R23,R33,CD4),(A2,R12,R23,R33,CD5),(A2,R12,R23,R33,CD6),(A2,R 12,R23,R33,CD7),(A2,R12,R23,R34,CD1),(A2,R12,R23,R34,CD2),(A2,R12,R23,R34,CD3),( A2,R12,R23,R34,CD4),(A2,R12,R23,R34,CD5),(A2,R12,R23,R34,CD6),(A2,R12,R23,R34,C D7),(A2,R12,R23,R35,CD1),(A2,R12,R23,R35,CD2),(A2,R12,R23,R35,CD3),(A2,R12,R23,R 35,CD4),(A2,R12,R23,R35,CD5),(A2,R12,R23,R35,CD6),(A2,R12,R23,R35,CD7),(A2,R12,R 24,R31,CD1),(A2,R12,R24,R31,CD2),(A2,R12,R24,R31,CD3),(A2,R12,R24,R31,CD4),(A2,R 12,R24,R31,CD5),(A2,R12,R24,R31,CD6),(A2,R12,R24,R31,CD7),(A2,R12,R24,R32,CD1),( A2,R12,R24,R32,CD2),(A2,R12,R24,R32,CD3),(A2,R12,R24,R32,CD4),(A2,R12,R24,R32,C D5),(A2,R12,R24,R32,CD6),(A2,R12,R24,R32,CD7),(A2,R12,R24,R33,CD1),(A2,R12,R24,R 33,CD2),(A2,R12,R24,R33,CD3),(A2,R12,R24,R33,CD4),(A2,R12,R24,R33,CD5),(A2,R12,R 24,R33,CD6),(A2,R12,R24,R33,CD7),(A2,R12,R24,R34,CD1),(A2,R12,R24,R34,CD2),(A2,R 12,R24,R34,CD3),(A2,R12,R24,R34,CD4),(A2,R12,R24,R34,CD5),(A2,R12,R24,R34,CD6),( A2,R12,R24,R34,CD7),(A2,R12,R24,R35,CD1),(A2,R12,R24,R35,CD2),(A2,R12,R24,R35,C D3),(A2,R12,R24,R35,CD4),(A2,R12,R24,R35,CD5),(A2,R12,R24,R35,CD6),(A2,R12,R24,R 35,CD7),(A2,R12,R25,R31,CD1),(A2,R12,R25,R31,CD2),(A2,R12,R25,R31,CD3),(A2,R12,R 25,R31,CD4),(A2,R12,R25,R31,CD5),(A2,R12,R25,R31,CD6),(A2,R12,R25,R31,CD7),(A2,R 12,R25,R32,CD1),(A2,R12,R25,R32,CD2),(A2,R12,R25,R32,CD3),(A2,R12,R25,R32,CD4),( A2,R12,R25,R32,CD5),(A2,R12,R25,R32,CD6),(A2,R12,R25,R32,CD7),(A2,R12,R25,R33,C D1),(A2,R12,R25,R33,CD2),(A2,R12,R25,R33,CD3),(A2,R12,R25,R33,CD4),(A2,R12,R25,R 33,CD5),(A2,R12,R25,R33,CD6),(A2,R12,R25,R33,CD7),(A2,R12,R25,R34,CD1),(A2,R12,R 25,R34,CD2),(A2,R12,R25,R34,CD3),(A2,R12,R25,R34,CD4),(A2,R12,R25,R34,CD5),(A2,R 12,R25,R34,CD6),(A2,R12,R25,R34,CD7),(A2,R12,R25,R35,CD1),(A2,R12,R25,R35,CD2),( A2,R12,R25,R35,CD3),(A2,R12,R25,R35,CD4),(A2,R12,R25,R35,CD5),(A2,R12,R25,R35,C D6),(A2,R12,R25,R35,CD7),(A2,R12,R26,R31,CD1),(A2,R12,R26,R31,CD2),(A2,R12,R26,R 31,CD3),(A2,R12,R26,R31,CD4),(A2,R12,R26,R31,CD5),(A2,R12,R26,R31,CD6),(A2,R12,R 26,R31,CD7),(A2,R12,R26,R32,CD1),(A2,R12,R26,R32,CD2),(A2,R12,R26,R32,CD3),(A2,R 12,R26,R32,CD4),(A2,R12,R26,R32,CD5),(A2,R12,R26,R32,CD6),(A2,R12,R26,R32,CD7),( A2,R12,R26,R33,CD1),(A2,R12,R26,R33,CD2),(A2,R12,R26,R33,CD3),(A2,R12,R26,R33,C D4),(A2,R12,R26,R33,CD5),(A2,R12,R26,R33,CD6),(A2,R12,R26,R33,CD7),(A2,R12,R26,R 34,CD1),(A2,R12,R26,R34,CD2),(A2,R12,R26,R34,CD3),(A2,R12,R26,R34,CD4),(A2,R12,R 26,R34,CD5),(A2,R12,R26,R34,CD6),(A2,R12,R26,R34,CD7),(A2,R12,R26,R35,CD1),(A2,R 12,R26,R35,CD2),(A2,R12,R26,R35,CD3),(A2,R12,R26,R35,CD4),(A2,R12,R26,R35,CD5),( A2,R12,R26,R35,CD6),(A2,R12,R26,R35,CD7),(A2,R13,R21,R31,CD1),(A2,R13,R21,R31,C D2),(A2,R13,R21,R31,CD3),(A2,R13,R21,R31,CD4),(A2,R13,R21,R31,CD5),(A2,R13,R21,R 31,CD6),(A2,R13,R21,R31,CD7),(A2,R13,R21,R32,CD1),(A2,R13,R21,R32,CD2),(A2,R13,R 21,R32,CD3),(A2,R13,R21,R32,CD4),(A2,R13,R21,R32,CD5),(A2,R13,R21,R32,CD6),(A2,R 13,R21,R32,CD7),(A2,R13,R21,R33,CD1),(A2,R13,R21,R33,CD2),(A2,R13,R21,R33,CD3),( A2,R13,R21,R33,CD4),(A2,R13,R21,R33,CD5),(A2,R13,R21,R33,CD6),(A2,R13,R21,R33,C D7),(A2,R13,R21,R34,CD1),(A2,R13,R21,R34,CD2),(A2,R13,R21,R34,CD3),(A2,R13,R21,R 34,CD4),(A2,R13,R21,R34,CD5),(A2,R13,R21,R34,CD6),(A2,R13,R21,R34,CD7),(A2,R13,R 21,R35,CD1),(A2,R13,R21,R35,CD2),(A2,R13,R21,R35,CD3),(A2,R13,R21,R35,CD4),(A2,R 13,R21,R35,CD5),(A2,R13,R21,R35,CD6),(A2,R13,R21,R35,CD7),(A2,R13,R22,R31,CD1),( A2,R13,R22,R31,CD2),(A2,R13,R22,R31,CD3),(A2,R13,R22,R31,CD4),(A2,R13,R22,R31,C D5),(A2,R13,R22,R31,CD6),(A2,R13,R22,R31,CD7),(A2,R13,R22,R32,CD1),(A2,R13,R22,R 32,CD2),(A2,R13,R22,R32,CD3),(A2,R13,R22,R32,CD4),(A2,R13,R22,R32,CD5),(A2,R13,R 22,R32,CD6),(A2,R13,R22,R32,CD7),(A2,R13,R22,R33,CD1),(A2,R13,R22,R33,CD2),(A2,R 13,R22,R33,CD3),(A2,R13,R22,R33,CD4),(A2,R13,R22,R33,CD5),(A2,R13,R22,R33,CD6),( A2,R13,R22,R33,CD7),(A2,R13,R22,R34,CD1),(A2,R13,R22,R34,CD2),(A2,R13,R22,R34,C D3),(A2,R13,R22,R34,CD4),(A2,R13,R22,R34,CD5),(A2,R13,R22,R34,CD6),(A2,R13,R22,R 34,CD7),(A2,R13,R22,R35,CD1),(A2,R13,R22,R35,CD2),(A2,R13,R22,R35,CD3),(A2,R13,R 22,R35,CD4),(A2,R13,R22,R35,CD5),(A2,R13,R22,R35,CD6),(A2,R13,R22,R35,CD7),(A2,R 13,R23,R31,CD1),(A2,R13,R23,R31,CD2),(A2,R13,R23,R31,CD3),(A2,R13,R23,R31,CD4),( A2,R13,R23,R31,CD5),(A2,R13,R23,R31,CD6),(A2,R13,R23,R31,CD7),(A2,R13,R23,R32,C D1),(A2,R13,R23,R32,CD2),(A2,R13,R23,R32,CD3),(A2,R13,R23,R32,CD4),(A2,R13,R23,R 32,CD5),(A2,R13,R23,R32,CD6),(A2,R13,R23,R32,CD7),(A2,R13,R23,R33,CD1),(A2,R13,R 23,R33,CD2),(A2,R13,R23,R33,CD3),(A2,R13,R23,R33,CD4),(A2,R13,R23,R33,CD5),(A2,R 13,R23,R33,CD6),(A2,R13,R23,R33,CD7),(A2,R13,R23,R34,CD1),(A2,R13,R23,R34,CD2),( A2,R13,R23,R34,CD3),(A2,R13,R23,R34,CD4),(A2,R13,R23,R34,CD5),(A2,R13,R23,R34,C D6),(A2,R13,R23,R34,CD7),(A2,R13,R23,R35,CD1),(A2,R13,R23,R35,CD2),(A2,R13,R23,R 35,CD3),(A2,R13,R23,R35,CD4),(A2,R13,R23,R35,CD5),(A2,R13,R23,R35,CD6),(A2,R13,R 23,R35,CD7),(A2,R13,R24,R31,CD1),(A2,R13,R24,R31,CD2),(A2,R13,R24,R31,CD3),(A2,R 13,R24,R31,CD4),(A2,R13,R24,R31,CD5),(A2,R13,R24,R31,CD6),(A2,R13,R24,R31,CD7),( A2,R13,R24,R32,CD1),(A2,R13,R24,R32,CD2),(A2,R13,R24,R32,CD3),(A2,R13,R24,R32,C D4),(A2,R13,R24,R32,CD5),(A2,R13,R24,R32,CD6),(A2,R13,R24,R32,CD7),(A2,R13,R24,R 33,CD1),(A2,R13,R24,R33,CD2),(A2,R13,R24,R33,CD3),(A2,R13,R24,R33,CD4),(A2,R13,R 24,R33,CD5),(A2,R13,R24,R33,CD6),(A2,R13,R24,R33,CD7),(A2,R13,R24,R34,CD1),(A2,R 13,R24,R34,CD2),(A2,R13,R24,R34,CD3),(A2,R13,R24,R34,CD4),(A2,R13,R24,R34,CD5),( A2,R13,R24,R34,CD6),(A2,R13,R24,R34,CD7),(A2,R13,R24,R35,CD1),(A2,R13,R24,R35,C D2),(A2,R13,R24,R35,CD3),(A2,R13,R24,R35,CD4),(A2,R13,R24,R35,CD5),(A2,R13,R24,R 35,CD6),(A2,R13,R24,R35,CD7),(A2,R13,R25,R31,CD1),(A2,R13,R25,R31,CD2),(A2,R13,R 25,R31,CD3),(A2,R13,R25,R31,CD4),(A2,R13,R25,R31,CD5),(A2,R13,R25,R31,CD6),(A2,R 13,R25,R31,CD7),(A2,R13,R25,R32,CD1),(A2,R13,R25,R32,CD2),(A2,R13,R25,R32,CD3),( A2,R13,R25,R32,CD4),(A2,R13,R25,R32,CD5),(A2,R13,R25,R32,CD6),(A2,R13,R25,R32,C D7),(A2,R13,R25,R33,CD1),(A2,R13,R25,R33,CD2),(A2,R13,R25,R33,CD3),(A2,R13,R25,R 33,CD4),(A2,R13,R25,R33,CD5),(A2,R13,R25,R33,CD6),(A2,R13,R25,R33,CD7),(A2,R13,R 25,R34,CD1),(A2,R13,R25,R34,CD2),(A2,R13,R25,R34,CD3),(A2,R13,R25,R34,CD4),(A2,R 13,R25,R34,CD5),(A2,R13,R25,R34,CD6),(A2,R13,R25,R34,CD7),(A2,R13,R25,R35,CD1),( A2,R13,R25,R35,CD2),(A2,R13,R25,R35,CD3),(A2,R13,R25,R35,CD4),(A2,R13,R25,R35,C D5),(A2,R13,R25,R35,CD6),(A2,R13,R25,R35,CD7),(A2,R13,R26,R31,CD1),(A2,R13,R26,R 31,CD2),(A2,R13,R26,R31,CD3),(A2,R13,R26,R31,CD4),(A2,R13,R26,R31,CD5),(A2,R13,R 26,R31,CD6),(A2,R13,R26,R31,CD7),(A2,R13,R26,R32,CD1),(A2,R13,R26,R32,CD2),(A2,R 13,R26,R32,CD3),(A2,R13,R26,R32,CD4),(A2,R13,R26,R32,CD5),(A2,R13,R26,R32,CD6),( A2,R13,R26,R32,CD7),(A2,R13,R26,R33,CD1),(A2,R13,R26,R33,CD2),(A2,R13,R26,R33,C D3),(A2,R13,R26,R33,CD4),(A2,R13,R26,R33,CD5),(A2,R13,R26,R33,CD6),(A2,R13,R26,R 33,CD7),(A2,R13,R26,R34,CD1),(A2,R13,R26,R34,CD2),(A2,R13,R26,R34,CD3),(A2,R13,R 26,R34,CD4),(A2,R13,R26,R34,CD5),(A2,R13,R26,R34,CD6),(A2,R13,R26,R34,CD7),(A2,R 13,R26,R35,CD1),(A2,R13,R26,R35,CD2),(A2,R13,R26,R35,CD3),(A2,R13,R26,R35,CD4),( A2,R13,R26,R35,CD5),(A2,R13,R26,R35,CD6),(A2,R13,R26,R35,CD7),(A2,R14,R21,R31,C D1),(A2,R14,R21,R31,CD2),(A2,R14,R21,R31,CD3),(A2,R14,R21,R31,CD4),(A2,R14,R21,R 31,CD5),(A2,R14,R21,R31,CD6),(A2,R14,R21,R31,CD7),(A2,R14,R21,R32,CD1),(A2,R14,R 21,R32,CD2),(A2,R14,R21,R32,CD3),(A2,R14,R21,R32,CD4),(A2,R14,R21,R32,CD5),(A2,R 14,R21,R32,CD6),(A2,R14,R21,R32,CD7),(A2,R14,R21,R33,CD1),(A2,R14,R21,R33,CD2),( A2,R14,R21,R33,CD3),(A2,R14,R21,R33,CD4),(A2,R14,R21,R33,CD5),(A2,R14,R21,R33,C D6),(A2,R14,R21,R33,CD7),(A2,R14,R21,R34,CD1),(A2,R14,R21,R34,CD2),(A2,R14,R21,R 34,CD3),(A2,R14,R21,R34,CD4),(A2,R14,R21,R34,CD5),(A2,R14,R21,R34,CD6),(A2,R14,R 21,R34,CD7),(A2,R14,R21,R35,CD1),(A2,R14,R21,R35,CD2),(A2,R14,R21,R35,CD3),(A2,R 14,R21,R35,CD4),(A2,R14,R21,R35,CD5),(A2,R14,R21,R35,CD6),(A2,R14,R21,R35,CD7),( A2,R14,R22,R31,CD1),(A2,R14,R22,R31,CD2),(A2,R14,R22,R31,CD3),(A2,R14,R22,R31,C D4),(A2,R14,R22,R31,CD5),(A2,R14,R22,R31,CD6),(A2,R14,R22,R31,CD7),(A2,R14,R22,R 32,CD1),(A2,R14,R22,R32,CD2),(A2,R14,R22,R32,CD3),(A2,R14,R22,R32,CD4),(A2,R14,R 22,R32,CD5),(A2,R14,R22,R32,CD6),(A2,R14,R22,R32,CD7),(A2,R14,R22,R33,CD1),(A2,R 14,R22,R33,CD2),(A2,R14,R22,R33,CD3),(A2,R14,R22,R33,CD4),(A2,R14,R22,R33,CD5),( A2,R14,R22,R33,CD6),(A2,R14,R22,R33,CD7),(A2,R14,R22,R34,CD1),(A2,R14,R22,R34,C D2),(A2,R14,R22,R34,CD3),(A2,R14,R22,R34,CD4),(A2,R14,R22,R34,CD5),(A2,R14,R22,R 34,CD6),(A2,R14,R22,R34,CD7),(A2,R14,R22,R35,CD1),(A2,R14,R22,R35,CD2),(A2,R14,R 22,R35,CD3),(A2,R14,R22,R35,CD4),(A2,R14,R22,R35,CD5),(A2,R14,R22,R35,CD6),(A2,R 14,R22,R35,CD7),(A2,R14,R23,R31,CD1),(A2,R14,R23,R31,CD2),(A2,R14,R23,R31,CD3),( A2,R14,R23,R31,CD4),(A2,R14,R23,R31,CD5),(A2,R14,R23,R31,CD6),(A2,R14,R23,R31,C D7),(A2,R14,R23,R32,CD1),(A2,R14,R23,R32,CD2),(A2,R14,R23,R32,CD3),(A2,R14,R23,R 32,CD4),(A2,R14,R23,R32,CD5),(A2,R14,R23,R32,CD6),(A2,R14,R23,R32,CD7),(A2,R14,R 23,R33,CD1),(A2,R14,R23,R33,CD2),(A2,R14,R23,R33,CD3),(A2,R14,R23,R33,CD4),(A2,R 14,R23,R33,CD5),(A2,R14,R23,R33,CD6),(A2,R14,R23,R33,CD7),(A2,R14,R23,R34,CD1),( A2,R14,R23,R34,CD2),(A2,R14,R23,R34,CD3),(A2,R14,R23,R34,CD4),(A2,R14,R23,R34,C D5),(A2,R14,R23,R34,CD6),(A2,R14,R23,R34,CD7),(A2,R14,R23,R35,CD1),(A2,R14,R23,R 35,CD2),(A2,R14,R23,R35,CD3),(A2,R14,R23,R35,CD4),(A2,R14,R23,R35,CD5),(A2,R14,R 23,R35,CD6),(A2,R14,R23,R35,CD7),(A2,R14,R24,R31,CD1),(A2,R14,R24,R31,CD2),(A2,R 14,R24,R31,CD3),(A2,R14,R24,R31,CD4),(A2,R14,R24,R31,CD5),(A2,R14,R24,R31,CD6),( A2,R14,R24,R31,CD7),(A2,R14,R24,R32,CD1),(A2,R14,R24,R32,CD2),(A2,R14,R24,R32,C D3),(A2,R14,R24,R32,CD4),(A2,R14,R24,R32,CD5),(A2,R14,R24,R32,CD6),(A2,R14,R24,R 32,CD7),(A2,R14,R24,R33,CD1),(A2,R14,R24,R33,CD2),(A2,R14,R24,R33,CD3),(A2,R14,R 24,R33,CD4),(A2,R14,R24,R33,CD5),(A2,R14,R24,R33,CD6),(A2,R14,R24,R33,CD7),(A2,R 14,R24,R34,CD1),(A2,R14,R24,R34,CD2),(A2,R14,R24,R34,CD3),(A2,R14,R24,R34,CD4),( A2,R14,R24,R34,CD5),(A2,R14,R24,R34,CD6),(A2,R14,R24,R34,CD7),(A2,R14,R24,R35,C D1),(A2,R14,R24,R35,CD2),(A2,R14,R24,R35,CD3),(A2,R14,R24,R35,CD4),(A2,R14,R24,R 35,CD5),(A2,R14,R24,R35,CD6),(A2,R14,R24,R35,CD7),(A2,R14,R25,R31,CD1),(A2,R14,R 25,R31,CD2),(A2,R14,R25,R31,CD3),(A2,R14,R25,R31,CD4),(A2,R14,R25,R31,CD5),(A2,R 14,R25,R31,CD6),(A2,R14,R25,R31,CD7),(A2,R14,R25,R32,CD1),(A2,R14,R25,R32,CD2),( A2,R14,R25,R32,CD3),(A2,R14,R25,R32,CD4),(A2,R14,R25,R32,CD5),(A2,R14,R25,R32,C D6),(A2,R14,R25,R32,CD7),(A2,R14,R25,R33,CD1),(A2,R14,R25,R33,CD2),(A2,R14,R25,R 33,CD3),(A2,R14,R25,R33,CD4),(A2,R14,R25,R33,CD5),(A2,R14,R25,R33,CD6),(A2,R14,R 25,R33,CD7),(A2,R14,R25,R34,CD1),(A2,R14,R25,R34,CD2),(A2,R14,R25,R34,CD3),(A2,R 14,R25,R34,CD4),(A2,R14,R25,R34,CD5),(A2,R14,R25,R34,CD6),(A2,R14,R25,R34,CD7),( A2,R14,R25,R35,CD1),(A2,R14,R25,R35,CD2),(A2,R14,R25,R35,CD3),(A2,R14,R25,R35,C D4),(A2,R14,R25,R35,CD5),(A2,R14,R25,R35,CD6),(A2,R14,R25,R35,CD7),(A2,R14,R26,R 31,CD1),(A2,R14,R26,R31,CD2),(A2,R14,R26,R31,CD3),(A2,R14,R26,R31,CD4),(A2,R14,R 26,R31,CD5),(A2,R14,R26,R31,CD6),(A2,R14,R26,R31,CD7),(A2,R14,R26,R32,CD1),(A2,R 14,R26,R32,CD2),(A2,R14,R26,R32,CD3),(A2,R14,R26,R32,CD4),(A2,R14,R26,R32,CD5),( A2,R14,R26,R32,CD6),(A2,R14,R26,R32,CD7),(A2,R14,R26,R33,CD1),(A2,R14,R26,R33,C D2),(A2,R14,R26,R33,CD3),(A2,R14,R26,R33,CD4),(A2,R14,R26,R33,CD5),(A2,R14,R26,R 33,CD6),(A2,R14,R26,R33,CD7),(A2,R14,R26,R34,CD1),(A2,R14,R26,R34,CD2),(A2,R14,R 26,R34,CD3),(A2,R14,R26,R34,CD4),(A2,R14,R26,R34,CD5),(A2,R14,R26,R34,CD6),(A2,R 14,R26,R34,CD7),(A2,R14,R26,R35,CD1),(A2,R14,R26,R35,CD2),(A2,R14,R26,R35,CD3),( A2,R14,R26,R35,CD4),(A2,R14,R26,R35,CD5),(A2,R14,R26,R35,CD6),(A2,R14,R26,R35,C D7),(A2,R15,R21,R31,CD1),(A2,R15,R21,R31,CD2),(A2,R15,R21,R31,CD3),(A2,R15,R21,R 31,CD4),(A2,R15,R21,R31,CD5),(A2,R15,R21,R31,CD6),(A2,R15,R21,R31,CD7),(A2,R15,R 21,R32,CD1),(A2,R15,R21,R32,CD2),(A2,R15,R21,R32,CD3),(A2,R15,R21,R32,CD4),(A2,R 15,R21,R32,CD5),(A2,R15,R21,R32,CD6),(A2,R15,R21,R32,CD7),(A2,R15,R21,R33,CD1),( A2,R15,R21,R33,CD2),(A2,R15,R21,R33,CD3),(A2,R15,R21,R33,CD4),(A2,R15,R21,R33,C D5),(A2,R15,R21,R33,CD6),(A2,R15,R21,R33,CD7),(A2,R15,R21,R34,CD1),(A2,R15,R21,R 34,CD2),(A2,R15,R21,R34,CD3),(A2,R15,R21,R34,CD4),(A2,R15,R21,R34,CD5),(A2,R15,R 21,R34,CD6),(A2,R15,R21,R34,CD7),(A2,R15,R21,R35,CD1),(A2,R15,R21,R35,CD2),(A2,R 15,R21,R35,CD3),(A2,R15,R21,R35,CD4),(A2,R15,R21,R35,CD5),(A2,R15,R21,R35,CD6),( A2,R15,R21,R35,CD7),(A2,R15,R22,R311,CD1),(A2,R15,R22,R31,CD2),(A2,R15,R22,R31,C D3),(A2,R15,R22,R31,CD4),(A2,R15,R22,R31,CD5),(A2,R15,R22,R31,CD6),(A2,R15,R22,R 31,CD7),(A2,R15,R22,R32,CD1),(A2,R15,R22,R32,CD2),(A2,R15,R22,R32,CD3),(A2,R15,R 22,R32,CD4),(A2,R15,R22,R32,CD5),(A2,R15,R22,R32,CD6),(A2,R15,R22,R32,CD7),(A2,R 15,R22,R33,CD1),(A2,R15,R22,R33,CD2),(A2,R15,R22,R33,CD3),(A2,R15,R22,R33,CD4),( A2,R15,R22,R33,CD5),(A2,R15,R22,R33,CD6),(A2,R15,R22,R33,CD7),(A2,R15,R22,R34,C D1),(A2,R15,R22,R34,CD2),(A2,R15,R22,R34,CD3),(A2,R15,R22,R34,CD4),(A2,R15,R22,R 34,CD5),(A2,R15,R22,R34,CD6),(A2,R15,R22,R34,CD7),(A2,R15,R22,R35,CD1),(A2,R15,R 22,R35,CD2),(A2,R15,R22,R35,CD3),(A2,R15,R22,R35,CD4),(A2,R15,R22,R35,CD5),(A2,R 15,R22,R35,CD6),(A2,R15,R22,R35,CD7),(A2,R15,R23,R31,CD1),(A2,R15,R23,R31,CD2),( A2,R15,R23,R31,CD3),(A2,R15,R23,R31,CD4),(A2,R15,R23,R31,CD5),(A2,R15,R23,R31,C D6),(A2,R15,R23,R31,CD7),(A2,R15,R23,R32,CD1),(A2,R15,R23,R32,CD2),(A2,R15,R23,R 32,CD3),(A2,R15,R23,R32,CD4),(A2,R15,R23,R32,CD5),(A2,R15,R23,R32,CD6),(A2,R15,R 23,R32,CD7),(A2,R15,R23,R33,CD1),(A2,R15,R23,R33,CD2),(A2,R15,R23,R33,CD3),(A2,R 15,R23,R33,CD4),(A2,R15,R23,R33,CD5),(A2,R15,R23,R33,CD6),(A2,R15,R23,R33,CD7),( A2,R15,R23,R34,CD1),(A2,R15,R23,R34,CD2),(A2,R15,R23,R34,CD3),(A2,R15,R23,R34,C D4),(A2,R15,R23,R34,CD5),(A2,R15,R23,R34,CD6),(A2,R15,R23,R34,CD7),(A2,R15,R23,R 35,CD1),(A2,R15,R23,R35,CD2),(A2,R15,R23,R35,CD3),(A2,R15,R23,R35,CD4),(A2,R15,R 23,R35,CD5),(A2,R15,R23,R35,CD6),(A2,R15,R23,R35,CD7),(A2,R15,R24,R31,CD1),(A2,R 15,R24,R31,CD2),(A2,R15,R24,R31,CD3),(A2,R15,R24,R31,CD4),(A2,R15,R24,R31,CD5),( A2,R15,R24,R31,CD6),(A2,R15,R24,R31,CD7),(A2,R15,R24,R32,CD1),(A2,R15,R24,R32,C D2),(A2,R15,R24,R32,CD3),(A2,R15,R24,R32,CD4),(A2,R15,R24,R32,CD5),(A2,R15,R24,R 32,CD6),(A2,R15,R24,R32,CD7),(A2,R15,R24,R33,CD1),(A2,R15,R24,R33,CD2),(A2,R15,R 24,R33,CD3),(A2,R15,R24,R33,CD4),(A2,R15,R24,R33,CD5),(A2,R15,R24,R33,CD6),(A2,R 15,R24,R33,CD7),(A2,R15,R24,R34,CD1),(A2,R15,R24,R34,CD2),(A2,R15,R24,R34,CD3),( A2,R15,R24,R34,CD4),(A2,R15,R24,R34,CD5),(A2,R15,R24,R34,CD6),(A2,R15,R24,R34,C D7),(A2,R15,R24,R35,CD1),(A2,R15,R24,R35,CD2),(A2,R15,R24,R35,CD3),(A2,R15,R24,R 35,CD4),(A2,R15,R24,R35,CD5),(A2,R15,R24,R35,CD6),(A2,R15,R24,R35,CD7),(A2,R15,R 25,R31,CD1),(A2,R15,R25,R31,CD2),(A2,R15,R25,R31,CD3),(A2,R15,R25,R31,CD4),(A2,R 15,R25,R31,CD5),(A2,R15,R25,R31,CD6),(A2,R15,R25,R31,CD7),(A2,R15,R25,R32,CD1),( A2,R15,R25,R32,CD2),(A2,R15,R25,R32,CD3),(A2,R15,R25,R32,CD4),(A2,R15,R25,R32,C D5),(A2,R15,R25,R32,CD6),(A2,R15,R25,R32,CD7),(A2,R15,R25,R33,CD1),(A2,R15,R25,R 33,CD2),(A2,R15,R25,R33,CD3),(A2,R15,R25,R33,CD4),(A2,R15,R25,R33,CD5),(A2,R15,R 25,R33,CD6),(A2,R15,R25,R33,CD7),(A2,R15,R25,R34,CD1),(A2,R15,R25,R34,CD2),(A2,R 15,R25,R34,CD3),(A2,R15,R25,R34,CD4),(A2,R15,R25,R34,CD5),(A2,R15,R25,R34,CD6),( A2,R15,R25,R34,CD7),(A2,R15,R25,R35,CD1),(A2,R15,R25,R35,CD2),(A2,R15,R25,R35,C D3),(A2,R15,R25,R35,CD4),(A2,R15,R25,R35,CD5),(A2,R15,R25,R35,CD6),(A2,R15,R25,R 35,CD7),(A2,R15,R26,R31,CD1),(A2,R15,R26,R31,CD2),(A2,R15,R26,R31,CD3),(A2,R15,R 26,R31,CD4),(A2,R15,R26,R31,CD5),(A2,R15,R26,R31,CD6),(A2,R15,R26,R31,CD7),(A2,R 15,R26,R32,CD1),(A2,R15,R26,R32,CD2),(A2,R15,R26,R32,CD3),(A2,R15,R26,R32,CD4),( A2,R15,R26,R32,CD5),(A2,R15,R26,R32,CD6),(A2,R15,R26,R32,CD7),(A2,R15,R26,R33,C D1),(A2,R15,R26,R33,CD2),(A2,R15,R26,R33,CD3),(A2,R15,R26,R33,CD4),(A2,R15,R26,R 33,CD5),(A2,R15,R26,R33,CD6),(A2,R15,R26,R33,CD7),(A2,R15,R26,R34,CD1),(A2,R15,R 26,R34,CD2),(A2,R15,R26,R34,CD3),(A2,R15,R26,R34,CD4),(A2,R15,R26,R34,CD5),(A2,R 15,R26,R34,CD6),(A2,R15,R26,R34,CD7),(A2,R15,R26,R35,CD1),(A2,R15,R26,R35,CD2),( A2,R15,R26,R35,CD3),(A2,R15,R26,R35,CD4),(A2,R15,R26,R35,CD5),(A2,R15,R26,R35,C D6),(A2,R15,R26,R35,CD7),(A2,R16,R21,R31,CD1),(A2,R16,R21,R31,CD2),(A2,R16,R21,R 31,CD3),(A2,R16,R21,R31,CD4),(A2,R16,R21,R31,CD5),(A2,R16,R21,R31,CD6),(A2,R16,R 21,R31,CD7),(A2,R16,R21,R32,CD1),(A2,R16,R21,R32,CD2),(A2,R16,R21,R32,CD3),(A2,R 16,R21,R32,CD4),(A2,R16,R21,R32,CD5),(A2,R16,R21,R32,CD6),(A2,R16,R21,R32,CD7),( A2,R16,R21,R33,CD1),(A2,R16,R21,R33,CD2),(A2,R16,R21,R33,CD3),(A2,R16,R21,R33,C D4),(A2,R16,R21,R33,CD5),(A2,R16,R21,R33,CD6),(A2,R16,R21,R33,CD7),(A2,R16,R21,R 34,CD1),(A2,R16,R21,R34,CD2),(A2,R16,R21,R34,CD3),(A2,R16,R21,R34,CD4),(A2,R16,R 21,R34,CD5),(A2,R16,R21,R34,CD6),(A2,R16,R21,R34,CD7),(A2,R16,R21,R35,CD1),(A2,R 16,R21,R35,CD2),(A2,R16,R21,R35,CD3),(A2,R16,R21,R35,CD4),(A2,R16,R21,R35,CD5),( A2,R16,R21,R35,CD6),(A2,R16,R21,R35,CD7),(A2,R16,R22,R31,CD1),(A2,R16,R22,R31,C D2),(A2,R16,R22,R31,CD3),(A2,R16,R22,R31,CD4),(A2,R16,R22,R31,CD5),(A2,R16,R22,R 31,CD6),(A2,R16,R22,R31,CD7),(A2,R16,R22,R32,CD1),(A2,R16,R22,R32,CD2),(A2,R16,R 22,R32,CD3),(A2,R16,R22,R32,CD4),(A2,R16,R22,R32,CD5),(A2,R16,R22,R32,CD6),(A2,R 16,R22,R32,CD7),(A2,R16,R22,R33,CD1),(A2,R16,R22,R33,CD2),(A2,R16,R22,R33,CD3),( A2,R16,R22,R33,CD4),(A2,R16,R22,R33,CD5),(A2,R16,R22,R33,CD6),(A2,R16,R22,R33,C D7),(A2,R16,R22,R34,CD1),(A2,R16,R22,R34,CD2),(A2,R16,R22,R34,CD3),(A2,R16,R22,R 34,CD4),(A2,R16,R22,R34,CD5),(A2,R16,R22,R34,CD6),(A2,R16,R22,R34,CD7),(A2,R16,R 22,R35,CD1),(A2,R16,R22,R35,CD2),(A2,R16,R22,R35,CD3),(A2,R16,R22,R35,CD4),(A2,R 16,R22,R35,CD5),(A2,R16,R22,R35,CD6),(A2,R16,R22,R35,CD7),(A2,R16,R23,R31,CD1),( A2,R16,R23,R31,CD2),(A2,R16,R23,R31,CD3),(A2,R16,R23,R31,CD4),(A2,R16,R23,R31,C D5),(A2,R16,R23,R31,CD6),(A2,R16,R23,R31,CD7),(A2,R16,R23,R32,CD1),(A2,R16,R23,R 32,CD2),(A2,R16,R23,R32,CD3),(A2,R16,R23,R32,CD4),(A2,R16,R23,R32,CD5),(A2,R16,R 23,R32,CD6),(A2,R16,R23,R32,CD7),(A2,R16,R23,R33,CD1),(A2,R16,R23,R33,CD2),(A2,R 16,R23,R33,CD3),(A2,R16,R23,R33,CD4),(A2,R16,R23,R33,CD5),(A2,R16,R23,R33,CD6),( A2,R16,R23,R33,CD7),(A2,R16,R23,R34,CD1),(A2,R16,R23,R34,CD2),(A2,R16,R23,R34,C D3),(A2,R16,R23,R34,CD4),(A2,R16,R23,R34,CD5),(A2,R16,R23,R34,CD6),(A2,R16,R23,R 34,CD7),(A2,R16,R23,R35,CD1),(A2,R16,R23,R35,CD2),(A2,R16,R23,R35,CD3),(A2,R16,R 23,R35,CD4),(A2,R16,R23,R35,CD5),(A2,R16,R23,R35,CD6),(A2,R16,R23,R35,CD7),(A2,R 16,R24,R31,CD1),(A2,R16,R24,R31,CD2),(A2,R16,R24,R31,CD3),(A2,R16,R24,R31,CD4),( A2,R16,R24,R31,CD5),(A2,R16,R24,R31,CD6),(A2,R16,R24,R31,CD7),(A2,R16,R24,R32,C D1),(A2,R16,R24,R32,CD2),(A2,R16,R24,R32,CD3),(A2,R16,R24,R32,CD4),(A2,R16,R24,R 32,CD5),(A2,R16,R24,R32,CD6),(A2,R16,R24,R32,CD7),(A2,R16,R24,R33,CD1),(A2,R16,R 24,R33,CD2),(A2,R16,R24,R33,CD3),(A2,R16,R24,R33,CD4),(A2,R16,R24,R33,CD5),(A2,R 16,R24,R33,CD6),(A2,R16,R24,R33,CD7),(A2,R16,R24,R34,CD1),(A2,R16,R24,R34,CD2),( A2,R16,R24,R34,CD3),(A2,R16,R24,R34,CD4),(A2,R16,R24,R34,CD5),(A2,R16,R24,R34,C D6),(A2,R16,R24,R34,CD7),(A2,R16,R24,R35,CD1),(A2,R16,R24,R35,CD2),(A2,R16,R24,R 35,CD3),(A2,R16,R24,R35,CD4),(A2,R16,R24,R35,CD5),(A2,R16,R24,R35,CD6),(A2,R16,R 24,R35,CD7),(A2,R16,R25,R31,CD1),(A2,R16,R25,R31,CD2),(A2,R16,R25,R31,CD3),(A2,R 16,R25,R31,CD4),(A2,R16,R25,R31,CD5),(A2,R16,R25,R31,CD6),(A2,R16,R25,R31,CD7),( A2,R16,R25,R32,CD1),(A2,R16,R25,R32,CD2),(A2,R16,R25,R32,CD3),(A2,R16,R25,R32,C D4),(A2,R16,R25,R32,CD5),(A2,R16,R25,R32,CD6),(A2,R16,R25,R32,CD7),(A2,R16,R25,R 33,CD1),(A2,R16,R25,R33,CD2),(A2,R16,R25,R33,CD3),(A2,R16,R25,R33,CD4),(A2,R16,R 25,R33,CD5),(A2,R16,R25,R33,CD6),(A2,R16,R25,R33,CD7),(A2,R16,R25,R34,CD1),(A2,R 16,R25,R34,CD2),(A2,R16,R25,R34,CD3),(A2,R16,R25,R34,CD4),(A2,R16,R25,R34,CD5),( A2,R16,R25,R34,CD6),(A2,R16,R25,R34,CD7),(A2,R16,R25,R35,CD1),(A2,R16,R25,R35,C D2),(A2,R16,R25,R35,CD3),(A2,R16,R25,R35,CD4),(A2,R16,R25,R35,CD5),(A2,R16,R25,R 35,CD6),(A2,R16,R25,R35,CD7),(A2,R16,R26,R31,CD1),(A2,R16,R26,R31,CD2),(A2,R16,R 26,R31,CD3),(A2,R16,R26,R31,CD4),(A2,R16,R26,R31,CD5),(A2,R16,R26,R31,CD6),(A2,R 16,R26,R31,CD7),(A2,R16,R26,R32,CD1),(A2,R16,R26,R32,CD2),(A2,R16,R26,R32,CD3),( A2,R16,R26,R32,CD4),(A2,R16,R26,R32,CD5),(A2,R16,R26,R32,CD6),(A2,R16,R26,R32,C D7),(A2,R16,R26,R33,CD1),(A2,R16,R26,R33,CD2),(A2,R16,R26,R33,CD3),(A2,R16,R26,R 33,CD4),(A2,R16,R26,R33,CD5),(A2,R16,R26,R33,CD6),(A2,R16,R26,R33,CD7),(A2,R16,R 26,R34,CD1),(A2,R16,R26,R34,CD2),(A2,R16,R26,R34,CD3),(A2,R16,R26,R34,CD4),(A2,R 16,R26,R34,CD5),(A2,R16,R26,R34,CD6),(A2,R16,R26,R34,CD7),(A2,R16,R26,R35,CD1),( A2,R16,R26,R35,CD2),(A2,R16,R26,R35,CD3),(A2,R16,R26,R35,CD4),(A2,R16,R26,R35,C D5),(A2,R16,R26,R35,CD6),(A2,R16,R26,R35,CD7),(A2,R17,R21,R31,CD1),(A2,R17,R21,R 31,CD2),(A2,R17,R21,R31,CD3),(A2,R17,R21,R31,CD4),(A2,R17,R21,R31,CD5),(A2,R17,R 21,R31,CD6),(A2,R17,R21,R31,CD7),(A2,R17,R21,R32,CD1),(A2,R17,R21,R32,CD2),(A2,R 17,R21,R32,CD3),(A2,R17,R21,R32,CD4),(A2,R17,R21,R32,CD5),(A2,R17,R21,R32,CD6),( A2,R17,R21,R32,CD7),(A2,R17,R21,R33,CD1),(A2,R17,R21,R33,CD2),(A2,R17,R21,R33,C D3),(A2,R17,R21,R33,CD4),(A2,R17,R21,R33,CD5),(A2,R17,R21,R33,CD6),(A2,R17,R21,R 33,CD7),(A2,R17,R21,R34,CD1),(A2,R17,R21,R34,CD2),(A2,R17,R21,R34,CD3),(A2,R17,R 21,R34,CD4),(A2,R17,R21,R34,CD5),(A2,R17,R21,R34,CD6),(A2,R17,R21,R34,CD7),(A2,R 17,R21,R35,CD1),(A2,R17,R21,R35,CD2),(A2,R17,R21,R35,CD3),(A2,R17,R21,R35,CD4),( A2,R17,R21,R35,CD5),(A2,R17,R21,R35,CD6),(A2,R17,R21,R35,CD7),(A2,R17,R22,R31,C D1),(A2,R17,R22,R31,CD2),(A2,R17,R22,R31,CD3),(A2,R17,R22,R31,CD4),(A2,R17,R22,R 31,CD5),(A2,R17,R22,R31,CD6),(A2,R17,R22,R31,CD7),(A2,R17,R22,R32,CD1),(A2,R17,R 22,R32,CD2),(A2,R17,R22,R32,CD3),(A2,R17,R22,R32,CD4),(A2,R17,R22,R32,CD5),(A2,R 17,R22,R32,CD6),(A2,R17,R22,R32,CD7),(A2,R17,R22,R33,CD1),(A2,R17,R22,R33,CD2),( A2,R17,R22,R33,CD3),(A2,R17,R22,R33,CD4),(A2,R17,R22,R33,CD5),(A2,R17,R22,R33,C D6),(A2,R17,R22,R33,CD7),(A2,R17,R22,R34,CD1),(A2,R17,R22,R34,CD2),(A2,R17,R22,R 34,CD3),(A2,R17,R22,R34,CD4),(A2,R17,R22,R34,CD5),(A2,R17,R22,R34,CD6),(A2,R17,R 22,R34,CD7),(A2,R17,R22,R35,CD1),(A2,R17,R22,R35,CD2),(A2,R17,R22,R35,CD3),(A2,R 17,R22,R35,CD4),(A2,R17,R22,R35,CD5),(A2,R17,R22,R35,CD6),(A2,R17,R22,R35,CD7),( A2,R17,R23,R31,CD1),(A2,R17,R23,R31,CD2),(A2,R17,R23,R31,CD3),(A2,R17,R23,R31,C D4),(A2,R17,R23,R31,CD5),(A2,R17,R23,R31,CD6),(A2,R17,R23,R31,CD7),(A2,R17,R23,R 32,CD1),(A2,R17,R23,R32,CD2),(A2,R17,R23,R32,CD3),(A2,R17,R23,R32,CD4),(A2,R17,R 23,R32,CD5),(A2,R17,R23,R32,CD6),(A2,R17,R23,R32,CD7),(A2,R17,R23,R33,CD1),(A2,R 17,R23,R33,CD2),(A2,R17,R23,R33,CD3),(A2,R17,R23,R33,CD4),(A2,R17,R23,R33,CD5),( A2,R17,R23,R33,CD6),(A2,R17,R23,R33,CD7),(A2,R17,R23,R34,CD1),(A2,R17,R23,R34,C D2),(A2,R17,R23,R34,CD3),(A2,R17,R23,R34,CD4),(A2,R17,R23,R34,CD5),(A2,R17,R23,R 34,CD6),(A2,R17,R23,R34,CD7),(A2,R17,R23,R35,CD1),(A2,R17,R23,R35,CD2),(A2,R17,R 23,R35,CD3),(A2,R17,R23,R35,CD4),(A2,R17,R23,R35,CD5),(A2,R17,R23,R35,CD6),(A2,R 17,R23,R35,CD7),(A2,R17,R24,R31,CD1),(A2,R17,R24,R31,CD2),(A2,R17,R24,R31,CD3),( A2,R17,R24,R31,CD4),(A2,R17,R24,R31,CD5),(A2,R17,R24,R31,CD6),(A2,R17,R24,R31,C D7),(A2,R17,R24,R32,CD1),(A2,R17,R24,R32,CD2),(A2,R17,R24,R32,CD3),(A2,R17,R24,R 32,CD4),(A2,R17,R24,R32,CD5),(A2,R17,R24,R32,CD6),(A2,R17,R24,R32,CD7),(A2,R17,R 24,R33,CD1),(A2,R17,R24,R33,CD2),(A2,R17,R24,R33,CD3),(A2,R17,R24,R33,CD4),(A2,R 17,R24,R33,CD5),(A2,R17,R24,R33,CD6),(A2,R17,R24,R33,CD7),(A2,R17,R24,R34,CD1),( A2,R17,R24,R34,CD2),(A2,R17,R24,R34,CD3),(A2,R17,R24,R34,CD4),(A2,R17,R24,R34,C D5),(A2,R17,R24,R34,CD6),(A2,R17,R24,R34,CD7),(A2,R17,R24,R35,CD1),(A2,R17,R24,R 35,CD2),(A2,R17,R24,R35,CD3),(A2,R17,R24,R35,CD4),(A2,R17,R24,R35,CD5),(A2,R17,R 24,R35,CD6),(A2,R17,R24,R35,CD7),(A2,R17,R25,R31,CD1),(A2,R17,R25,R31,CD2),(A2,R 17,R25,R31,CD3),(A2,R17,R25,R31,CD4),(A2,R17,R25,R31,CD5),(A2,R17,R25,R31,CD6),( A2,R17,R25,R31,CD7),(A2,R17,R25,R32,CD1),(A2,R17,R25,R32,CD2),(A2,R17,R25,R32,C D3),(A2,R17,R25,R32,CD4),(A2,R17,R25,R32,CD5),(A2,R17,R25,R32,CD6),(A2,R17,R25,R 32,CD7),(A2,R17,R25,R33,CD1),(A2,R17,R25,R33,CD2),(A2,R17,R25,R33,CD3),(A2,R17,R 25,R33,CD4),(A2,R17,R25,R33,CD5),(A2,R17,R25,R33,CD6),(A2,R17,R25,R33,CD7),(A2,R 17,R25,R34,CD1),(A2,R17,R25,R34,CD2),(A2,R17,R25,R34,CD3),(A2,R17,R25,R34,CD4),( A2,R17,R25,R34,CD5),(A2,R17,R25,R34,CD6),(A2,R17,R25,R34,CD7),(A2,R17,R25,R35,C D1),(A2,R17,R25,R35,CD2),(A2,R17,R25,R35,CD3),(A2,R17,R25,R35,CD4),(A2,R17,R25,R 35,CD5),(A2,R17,R25,R35,CD6),(A2,R17,R25,R35,CD7),(A2,R17,R26,R31,CD1),(A2,R17,R 26,R31,CD2),(A2,R17,R26,R31,CD3),(A2,R17,R26,R31,CD4),(A2,R17,R26,R31,CD5),(A2,R 17,R26,R31,CD6),(A2,R17,R26,R31,CD7),(A2,R17,R26,R32,CD1),(A2,R17,R26,R32,CD2),( A2,R17,R26,R32,CD3),(A2,R17,R26,R32,CD4),(A2,R17,R26,R32,CD5),(A2,R17,R26,R32,C D6),(A2,R17,R26,R32,CD7),(A2,R17,R26,R33,CD1),(A2,R17,R26,R33,CD2),(A2,R17,R26,R 33,CD3),(A2,R17,R26,R33,CD4),(A2,R17,R26,R33,CD5),(A2,R17,R26,R33,CD6),(A2,R17,R 26,R33,CD7),(A2,R17,R26,R34,CD1),(A2,R17,R26,R34,CD2),(A2,R17,R26,R34,CD3),(A2,R 17,R26,R34,CD4),(A2,R17,R26,R34,CD5),(A2,R17,R26,R34,CD6),(A2,R17,R26,R34,CD7),( A2,R17,R26,R35,CD1),(A2,R17,R26,R35,CD2),(A2,R17,R26,R35,CD3),(A2,R17,R26,R35,C D4),(A2,R17,R26,R35,CD5),(A2,R17,R26,R35,CD6),(A2,R17,R26,R35,CD7),(A2,R18,R21,R 31,CD1),(A2,R18,R21,R31,CD2),(A2,R18,R21,R31,CD3),(A2,R18,R21,R31,CD4),(A2,R18,R 21,R31,CD5),(A2,R18,R21,R31,CD6),(A2,R18,R21,R31,CD7),(A2,R18,R21,R32,CD1),(A2,R 18,R21,R32,CD2),(A2,R18,R21,R32,CD3),(A2,R18,R21,R32,CD4),(A2,R18,R21,R32,CD5),( A2,R18,R21,R32,CD6),(A2,R18,R21,R32,CD7),(A2,R18,R21,R33,CD1),(A2,R18,R21,R33,C D2),(A2,R18,R21,R33,CD3),(A2,R18,R21,R33,CD4),(A2,R18,R21,R33,CD5),(A2,R18,R21,R 33,CD6),(A2,R18,R21,R33,CD7),(A2,R18,R21,R34,CD1),(A2,R18,R21,R34,CD2),(A2,R18,R 21,R34,CD3),(A2,R18,R21,R34,CD4),(A2,R18,R21,R34,CD5),(A2,R18,R21,R34,CD6),(A2,R 18,R21,R34,CD7),(A2,R18,R21,R35,CD1),(A2,R18,R21,R35,CD2),(A2,R18,R21,R35,CD3),( A2,R18,R21,R35,CD4),(A2,R18,R21,R35,CD5),(A2,R18,R21,R35,CD6),(A2,R18,R21,R35,C D7),(A2,R18,R22,R31,CD1),(A2,R18,R22,R31,CD2),(A2,R18,R22,R31,CD3),(A2,R18,R22,R 31,CD4),(A2,R18,R22,R31,CD5),(A2,R18,R22,R31,CD6),(A2,R18,R22,R31,CD7),(A2,R18,R 22,R32,CD1),(A2,R18,R22,R32,CD2),(A2,R18,R22,R32,CD3),(A2,R18,R22,R32,CD4),(A2,R 18,R22,R32,CD5),(A2,R18,R22,R32,CD6),(A2,R18,R22,R32,CD7),(A2,R18,R22,R33,CD1),( A2,R18,R22,R33,CD2),(A2,R18,R22,R33,CD3),(A2,R18,R22,R33,CD4),(A2,R18,R22,R33,C D5),(A2,R18,R22,R33,CD6),(A2,R18,R22,R33,CD7),(A2,R18,R22,R34,CD1),(A2,R18,R22,R 34,CD2),(A2,R18,R22,R34,CD3),(A2,R18,R22,R34,CD4),(A2,R18,R22,R34,CD5),(A2,R18,R 22,R34,CD6),(A2,R18,R22,R34,CD7),(A2,R18,R22,R35,CD1),(A2,R18,R22,R35,CD2),(A2,R 18,R22,R35,CD3),(A2,R18,R22,R35,CD4),(A2,R18,R22,R35,CD5),(A2,R18,R22,R35,CD6),( A2,R18,R22,R35,CD7),(A2,R18,R23,R31,CD1),(A2,R18,R23,R31,CD2),(A2,R18,R23,R31,C D3),(A2,R18,R23,R31,CD4),(A2,R18,R23,R31,CD5),(A2,R18,R23,R31,CD6),(A2,R18,R23,R 31,CD7),(A2,R18,R23,R32,CD1),(A2,R18,R23,R32,CD2),(A2,R18,R23,R32,CD3),(A2,R18,R 23,R32,CD4),(A2,R18,R23,R32,CD5),(A2,R18,R23,R32,CD6),(A2,R18,R23,R32,CD7),(A2,R 18,R23,R33,CD1),(A2,R18,R23,R33,CD2),(A2,R18,R23,R33,CD3),(A2,R18,R23,R33,CD4),( A2,R18,R23,R33,CD5),(A2,R18,R23,R33,CD6),(A2,R18,R23,R33,CD7),(A2,R18,R23,R34,C D1),(A2,R18,R23,R34,CD2),(A2,R18,R23,R34,CD3),(A2,R18,R23,R34,CD4),(A2,R18,R23,R 34,CD5),(A2,R18,R23,R34,CD6),(A2,R18,R23,R34,CD7),(A2,R18,R23,R35,CD1),(A2,R18,R 23,R35,CD2),(A2,R18,R23,R35,CD3),(A2,R18,R23,R35,CD4),(A2,R18,R23,R35,CD5),(A2,R 18,R23,R35,CD6),(A2,R18,R23,R35,CD7),(A2,R18,R24,R31,CD1),(A2,R18,R24,R31,CD2),( A2,R18,R24,R31,CD3),(A2,R18,R24,R31,CD4),(A2,R18,R24,R31,CD5),(A2,R18,R24,R31,C D6),(A2,R18,R24,R31,CD7),(A2,R18,R24,R32,CD1),(A2,R18,R24,R32,CD2),(A2,R18,R24,R 32,CD3),(A2,R18,R24,R32,CD4),(A2,R18,R24,R32,CD5),(A2,R18,R24,R32,CD6),(A2,R18,R 24,R32,CD7),(A2,R18,R24,R33,CD1),(A2,R18,R24,R33,CD2),(A2,R18,R24,R33,CD3),(A2,R 18,R24,R33,CD4),(A2,R18,R24,R33,CD5),(A2,R18,R24,R33,CD6),(A2,R18,R24,R33,CD7),( A2,R18,R24,R34,CD1),(A2,R18,R24,R34,CD2),(A2,R18,R24,R34,CD3),(A2,R18,R24,R34,C D4),(A2,R18,R24,R34,CD5),(A2,R18,R24,R34,CD6),(A2,R18,R24,R34,CD7),(A2,R18,R24,R 35,CD1),(A2,R18,R24,R35,CD2),(A2,R18,R24,R35,CD3),(A2,R18,R24,R35,CD4),(A2,R18,R 24,R35,CD5),(A2,R18,R24,R35,CD6),(A2,R18,R24,R35,CD7),(A2,R18,R25,R31,CD1),(A2,R 18,R25,R31,CD2),(A2,R18,R25,R31,CD3),(A2,R18,R25,R31,CD4),(A2,R18,R25,R31,CD5),( A2,R18,R25,R31,CD6),(A2,R18,R25,R31,CD7),(A2,R18,R25,R32,CD1),(A2,R18,R25,R32,C D2),(A2,R18,R25,R32,CD3),(A2,R18,R25,R32,CD4),(A2,R18,R25,R32,CD5),(A2,R18,R25,R 32,CD6),(A2,R18,R25,R32,CD7),(A2,R18,R25,R33,CD1),(A2,R18,R25,R33,CD2),(A2,R18,R 25,R33,CD3),(A2,R18,R25,R33,CD4),(A2,R18,R25,R33,CD5),(A2,R18,R25,R33,CD6),(A2,R 18,R25,R33,CD7),(A2,R18,R25,R34,CD1),(A2,R18,R25,R34,CD2),(A2,R18,R25,R34,CD3),( A2,R18,R25,R34,CD4),(A2,R18,R25,R34,CD5),(A2,R18,R25,R34,CD6),(A2,R18,R25,R34,C D7),(A2,R18,R25,R35,CD1),(A2,R18,R25,R35,CD2),(A2,R18,R25,R35,CD3),(A2,R18,R25,R 35,CD4),(A2,R18,R25,R35,CD5),(A2,R18,R25,R35,CD6),(A2,R18,R25,R35,CD7),(A2,R18,R 26,R31,CD1),(A2,R18,R26,R31,CD2),(A2,R18,R26,R31,CD3),(A2,R18,R26,R31,CD4),(A2,R 18,R26,R31,CD5),(A2,R18,R26,R31,CD6),(A2,R18,R26,R31,CD7),(A2,R18,R26,R32,CD1),( A2,R18,R26,R32,CD2),(A2,R18,R26,R32,CD3),(A2,R18,R26,R32,CD4),(A2,R18,R26,R32,C D5),(A2,R18,R26,R32,CD6),(A2,R18,R26,R32,CD7),(A2,R18,R26,R33,CD1),(A2,R18,R26,R 33,CD2),(A2,R18,R26,R33,CD3),(A2,R18,R26,R33,CD4),(A2,R18,R26,R33,CD5),(A2,R18,R 26,R33,CD6),(A2,R18,R26,R33,CD7),(A2,R18,R26,R34,CD1),(A2,R18,R26,R34,CD2),(A2,R 18,R26,R34,CD3),(A2,R18,R26,R34,CD4),(A2,R18,R26,R34,CD5),(A2,R18,R26,R34,CD6),( A2,R18,R26,R34,CD7),(A2,R18,R26,R35,CD1),(A2,R18,R26,R35,CD2),(A2,R18,R26,R35,C D3),(A2,R18,R26,R35,CD4),(A2,R18,R26,R35,CD5),(A2,R18,R26,R35,CD6),(A2,R18,R26,R 35,CD7),(A2,R19,R21,R31,CD1),(A2,R19,R21,R31,CD2),(A2,R19,R21,R31,CD3),(A2,R19,R 21,R31,CD4),(A2,R19,R21,R31,CD5),(A2,R19,R21,R31,CD6),(A2,R19,R21,R31,CD7),(A2,R 19,R21,R32,CD1),(A2,R19,R21,R32,CD2),(A2,R19,R21,R32,CD3),(A2,R19,R21,R32,CD4),( A2,R19,R21,R32,CD5),(A2,R19,R21,R32,CD6),(A2,R19,R21,R32,CD7),(A2,R19,R21,R33,C D1),(A2,R19,R21,R33,CD2),(A2,R19,R21,R33,CD3),(A2,R19,R21,R33,CD4),(A2,R19,R21,R 33,CD5),(A2,R19,R21,R33,CD6),(A2,R19,R21,R33,CD7),(A2,R19,R21,R34,CD1),(A2,R19,R 21,R34,CD2),(A2,R19,R21,R34,CD3),(A2,R19,R21,R34,CD4),(A2,R19,R21,R34,CD5),(A2,R 19,R21,R34,CD6),(A2,R19,R21,R34,CD7),(A2,R19,R21,R35,CD1),(A2,R19,R21,R35,CD2),( A2,R19,R21,R35,CD3),(A2,R19,R21,R35,CD4),(A2,R19,R21,R35,CD5),(A2,R19,R21,R35,C D6),(A2,R19,R21,R35,CD7),(A2,R19,R22,R31,CD1),(A2,R19,R22,R31,CD2),(A2,R19,R22,R 31,CD3),(A2,R19,R22,R31,CD4),(A2,R19,R22,R31,CD5),(A2,R19,R22,R31,CD6),(A2,R19,R 22,R31,CD7),(A2,R19,R22,R32,CD1),(A2,R19,R22,R32,CD2),(A2,R19,R22,R32,CD3),(A2,R 19,R22,R32,CD4),(A2,R19,R22,R32,CD5),(A2,R19,R22,R32,CD6),(A2,R19,R22,R32,CD7),( A2,R19,R22,R33,CD1),(A2,R19,R22,R33,CD2),(A2,R19,R22,R33,CD3),(A2,R19,R22,R33,C D4),(A2,R19,R22,R33,CD5),(A2,R19,R22,R33,CD6),(A2,R19,R22,R33,CD7),(A2,R19,R22,R 34,CD1),(A2,R19,R22,R34,CD2),(A2,R19,R22,R34,CD3),(A2,R19,R22,R34,CD4),(A2,R19,R 22,R34,CD5),(A2,R19,R22,R34,CD6),(A2,R19,R22,R34,CD7),(A2,R19,R22,R35,CD1),(A2,R 19,R22,R35,CD2),(A2,R19,R22,R35,CD3),(A2,R19,R22,R35,CD4),(A2,R19,R22,R35,CD5),( A2,R19,R22,R35,CD6),(A2,R19,R22,R35,CD7),(A2,R19,R23,R31,CD1),(A2,R19,R23,R31,C D2),(A2,R19,R23,R31,CD3),(A2,R19,R23,R31,CD4),(A2,R19,R23,R31,CD5),(A2,R19,R23,R 31,CD6),(A2,R19,R23,R31,CD7),(A2,R19,R23,R32,CD1),(A2,R19,R23,R32,CD2),(A2,R19,R 23,R32,CD3),(A2,R19,R23,R32,CD4),(A2,R19,R23,R32,CD5),(A2,R19,R23,R32,CD6),(A2,R 19,R23,R32,CD7),(A2,R19,R23,R33,CD1),(A2,R19,R23,R33,CD2),(A2,R19,R23,R33,CD3),( A2,R19,R23,R33,CD4),(A2,R19,R23,R33,CD5),(A2,R19,R23,R33,CD6),(A2,R19,R23,R33,C D7),(A2,R19,R23,R34,CD1),(A2,R19,R23,R34,CD2),(A2,R19,R23,R34,CD3),(A2,R19,R23,R 34,CD4),(A2,R19,R23,R34,CD5),(A2,R19,R23,R34,CD6),(A2,R19,R23,R34,CD7),(A2,R19,R 23,R35,CD1),(A2,R19,R23,R35,CD2),(A2,R19,R23,R35,CD3),(A2,R19,R23,R35,CD4),(A2,R 19,R23,R35,CD5),(A2,R19,R23,R35,CD6),(A2,R19,R23,R35,CD7),(A2,R19,R24,R31,CD1),( A2,R19,R24,R31,CD2),(A2,R19,R24,R31,CD3),(A2,R19,R24,R31,CD4),(A2,R19,R24,R31,C D5),(A2,R19,R24,R31,CD6),(A2,R19,R24,R31,CD7),(A2,R19,R24,R32,CD1),(A2,R19,R24,R 32,CD2),(A2,R19,R24,R32,CD3),(A2,R19,R24,R32,CD4),(A2,R19,R24,R32,CD5),(A2,R19,R 24,R32,CD6),(A2,R19,R24,R32,CD7),(A2,R19,R24,R33,CD1),(A2,R19,R24,R33,CD2),(A2,R 19,R24,R33,CD3),(A2,R19,R24,R33,CD4),(A2,R19,R24,R33,CD5),(A2,R19,R24,R33,CD6),( A2,R19,R24,R33,CD7),(A2,R19,R24,R34,CD1),(A2,R19,R24,R34,CD2),(A2,R19,R24,R34,C D3),(A2,R19,R24,R34,CD4),(A2,R19,R24,R34,CD5),(A2,R19,R24,R34,CD6),(A2,R19,R24,R 34,CD7),(A2,R19,R24,R35,CD1),(A2,R19,R24,R35,CD2),(A2,R19,R24,R35,CD3),(A2,R19,R 24,R35,CD4),(A2,R19,R24,R35,CD5),(A2,R19,R24,R35,CD6),(A2,R19,R24,R35,CD7),(A2,R 19,R25,R31,CD1),(A2,R19,R25,R31,CD2),(A2,R19,R25,R31,CD3),(A2,R19,R25,R31,CD4),( A2,R19,R25,R31,CD5),(A2,R19,R25,R31,CD6),(A2,R19,R25,R31,CD7),(A2,R19,R25,R32,C D1),(A2,R19,R25,R32,CD2),(A2,R19,R25,R32,CD3),(A2,R19,R25,R32,CD4),(A2,R19,R25,R 32,CD5),(A2,R19,R25,R32,CD6),(A2,R19,R25,R32,CD7),(A2,R19,R25,R33,CD1),(A2,R19,R 25,R33,CD2),(A2,R19,R25,R33,CD3),(A2,R19,R25,R33,CD4),(A2,R19,R25,R33,CD5),(A2,R 19,R25,R33,CD6),(A2,R19,R25,R33,CD7),(A2,R19,R25,R34,CD1),(A2,R19,R25,R34,CD2),( A2,R19,R25,R34,CD3),(A2,R19,R25,R34,CD4),(A2,R19,R25,R34,CD5),(A2,R19,R25,R34,C D6),(A2,R19,R25,R34,CD7),(A2,R19,R25,R35,CD1),(A2,R19,R25,R35,CD2),(A2,R19,R25,R 35,CD3),(A2,R19,R25,R35,CD4),(A2,R19,R25,R35,CD5),(A2,R19,R25,R35,CD6),(A2,R19,R 25,R35,CD7),(A2,R19,R26,R31,CD1),(A2,R19,R26,R31,CD2),(A2,R19,R26,R31,CD3),(A2,R 19,R26,R31,CD4),(A2,R19,R26,R31,CD5),(A2,R19,R26,R31,CD6),(A2,R19,R26,R31,CD7),( A2,R19,R26,R32,CD1),(A2,R19,R26,R32,CD2),(A2,R19,R26,R32,CD3),(A2,R19,R26,R32,C D4),(A2,R19,R26,R32,CD5),(A2,R19,R26,R32,CD6),(A2,R19,R26,R32,CD7),(A2,R19,R26,R 33,CD1),(A2,R19,R26,R33,CD2),(A2,R19,R26,R33,CD3),(A2,R19,R26,R33,CD4),(A2,R19,R 26,R33,CD5),(A2,R19,R26,R33,CD6),(A2,R19,R26,R33,CD7),(A2,R19,R26,R34,CD1),(A2,R 19,R26,R34,CD2),(A2,R19,R26,R34,CD3),(A2,R19,R26,R34,CD4),(A2,R19,R26,R34,CD5),( A2,R19,R26,R34,CD6),(A2,R19,R26,R34,CD7),(A2,R19,R26,R35,CD1),(A2,R19,R26,R35,C D2),(A2,R19,R26,R35,CD3),(A2,R19,R26,R35,CD4),(A2,R19,R26,R35,CD5),(A2,R19,R26,R 35,CD6),(A2,R19,R26,R35,CD7),

(A3,R11,R21,R31,CD1),(A3,R11,R21,R31,CD2),(A3,R11,R21,R31,CD3),(A3,R11,R21,R31,C D4),(A3,R11,R21,R31,CD5),(A3,R11,R21,R31,CD6),(A3,R11,R21,R31,CD7),(A3,R11,R21,R 32,CD1),(A3,R11,R21,R32,CD2),(A3,R11,R21,R32,CD3),(A3,R11,R21,R32,CD4),(A3,R11,R 21,R32,CD5),(A3,R11,R21,R32,CD6),(A3,R11,R21,R32,CD7),(A3,R11,R21,R33,CD1),(A3,R 11,R21,R33,CD2),(A3,R11,R21,R33,CD3),(A3,R11,R21,R33,CD4),(A3,R11,R21,R33,CD5),( A3,R11,R21,R33,CD6),(A3,R11,R21,R33,CD7),(A3,R11,R21,R34,CD1),(A3,R11,R21,R34,C D2),(A3,R11,R21,R34,CD3),(A3,R11,R21,R34,CD4),(A3,R11,R21,R34,CD5),(A3,R11,R21,R 34,CD6),(A3,R11,R21,R34,CD7),(A3,R11,R21,R35,CD1),(A3,R11,R21,R35,CD2),(A3,R11,R 21,R35,CD3),(A3,R11,R21,R35,CD4),(A3,R11,R21,R35,CD5),(A3,R11,R21,R35,CD6),(A3,R 11,R21,R35,CD7),(A3,R11,R22,R31,CD1),(A3,R11,R22,R31,CD2),(A3,R11,R22,R31,CD3),( A3,R11,R22,R31,CD4),(A3,R11,R22,R31,CD5),(A3,R11,R22,R31,CD6),(A3,R11,R22,R31,C D7),(A3,R11,R22,R32,CD1),(A3,R11,R22,R32,CD2),(A3,R11,R22,R32,CD3),(A3,R11,R22,R 32,CD4),(A3,R11,R22,R32,CD5),(A3,R11,R22,R32,CD6),(A3,R11,R22,R32,CD7),(A3,R11,R 22,R33,CD1),(A3,R11,R22,R33,CD2),(A3,R11,R22,R33,CD3),(A3,R11,R22,R33,CD4),(A3,R 11,R22,R33,CD5),(A3,R11,R22,R33,CD6),(A3,R11,R22,R33,CD7),(A3,R11,R22,R34,CD1),( A3,R11,R22,R34,CD2),(A3,R11,R22,R34,CD3),(A3,R11,R22,R34,CD4),(A3,R11,R22,R34,C D5),(A3,R11,R22,R34,CD6),(A3,R11,R22,R34,CD7),(A3,R11,R22,R35,CD1),(A3,R11,R22,R 35,CD2),(A3,R11,R22,R35,CD3),(A3,R11,R22,R35,CD4),(A3,R11,R22,R35,CD5),(A3,R11,R 22,R35,CD6),(A3,R11,R22,R35,CD7),(A3,R11,R23,R31,CD1),(A3,R11,R23,R31,CD2),(A3,R 11,R23,R31,CD3),(A3,R11,R23,R31,CD4),(A3,R11,R23,R31,CD5),(A3,R11,R23,R31,CD6),( A3,R11,R23,R31,CD7),(A3,R11,R23,R32,CD1),(A3,R11,R23,R32,CD2),(A3,R11,R23,R32,C D3),(A3,R11,R23,R32,CD4),(A3,R11,R23,R32,CD5),(A3,R11,R23,R32,CD6),(A3,R11,R23,R 32,CD7),(A3,R11,R23,R33,CD1),(A3,R11,R23,R33,CD2),(A3,R11,R23,R33,CD3),(A3,R11,R 23,R33,CD4),(A3,R11,R23,R33,CD5),(A3,R11,R23,R33,CD6),(A3,R11,R23,R33,CD7),(A3,R 11,R23,R34,CD1),(A3,R11,R23,R34,CD2),(A3,R11,R23,R34,CD3),(A3,R11,R23,R34,CD4),( A3,R11,R23,R34,CD5),(A3,R11,R23,R314,CD6),(A3,R11,R23,R34,CD7),(A3,R11,R23,R35,C D1),(A3,R11,R23,R35,CD2),(A3,R11,R23,R35,CD3),(A3,R11,R23,R35,CD4),(A3,R11,R23,R 35,CD5),(A3,R11,R23,R35,CD6),(A3,R11,R23,R35,CD7),(A3,R11,R24,R31,CD1),(A3,R11,R 24,R31,CD2),(A3,R11,R24,R31,CD3),(A3,R11,R24,R31,CD4),(A3,R11,R24,R31,CD5),(A3,R 11,R24,R31,CD6),(A3,R11,R24,R31,CD7),(A3,R11,R24,R32,CD1),(A3,R11,R24,R32,CD2),( A3,R11,R24,R32,CD3),(A3,R11,R24,R32,CD4),(A3,R11,R24,R32,CD5),(A3,R11,R24,R32,C D6),(A3,R11,R24,R32,CD7),(A3,R11,R24,R33,CD1),(A3,R11,R24,R33,CD2),(A3,R11,R24,R 33,CD3),(A3,R11,R24,R33,CD4),(A3,R11,R24,R33,CD5),(A3,R11,R24,R33,CD6),(A3,R11,R 24,R33,CD7),(A3,R11,R24,R34,CD1),(A3,R11,R24,R34,CD2),(A3,R11,R24,R34,CD3),(A3,R 11,R24,R34,CD4),(A3,R11,R24,R34,CD5),(A3,R11,R24,R34,CD6),(A3,R11,R24,R34,CD7),( A3,R11,R24,R35,CD1),(A3,R11,R24,R35,CD2),(A3,R11,R24,R35,CD3),(A3,R11,R24,R35,C D4),(A3,R11,R24,R35,CD5),(A3,R11,R24,R35,CD6),(A3,R11,R24,R35,CD7),(A3,R11,R25,R 31,CD1),(A3,R11,R25,R31,CD2),(A3,R11,R25,R31,CD3),(A3,R11,R25,R31,CD4),(A3,R11,R 25,R31,CD5),(A3,R11,R25,R31,CD6),(A3,R11,R25,R31,CD7),(A3,R11,R25,R32,CD1),(A3,R 11,R25,R32,CD2),(A3,R11,R25,R32,CD3),(A3,R11,R25,R32,CD4),(A3,R11,R25,R32,CD5),( A3,R11,R25,R32,CD6),(A3,R11,R25,R32,CD7),(A3,R11,R25,R33,CD1),(A3,R11,R25,R33,C D2),(A3,R11,R25,R33,CD3),(A3,R11,R25,R33,CD4),(A3,R11,R25,R33,CD5),(A3,R11,R25,R 33,CD6),(A3,R11,R25,R33,CD7),(A3,R11,R25,R34,CD1),(A3,R11,R25,R34,CD2),(A3,R11,R 25,R34,CD3),(A3,R11,R25,R34,CD4),(A3,R11,R25,R34,CD5),(A3,R11,R25,R34,CD6),(A3,R 11,R25,R34,CD7),(A3,R11,R25,R35,CD1),(A3,R11,R25,R35,CD2),(A3,R11,R25,R35,CD3),( A3,R11,R25,R35,CD4),(A3,R11,R25,R35,CD5),(A3,R11,R25,R35,CD6),(A3,R11,R25,R35,C D7),(A3,R11,R26,R31,CD1),(A3,R11,R26,R31,CD2),(A3,R11,R26,R31,CD3),(A3,R11,R26,R 31,CD4),(A3,R11,R26,R31,CD5),(A3,R11,R26,R31,CD6),(A3,R11,R26,R31,CD7),(A3,R11,R 26,R32,CD1),(A3,R11,R26,R32,CD2),(A3,R11,R26,R32,CD3),(A3,R11,R26,R32,CD4),(A3,R 11,R26,R32,CD5),(A3,R11,R26,R32,CD6),(A3,R11,R26,R32,CD7),(A3,R11,R26,R33,CD1),( A3,R11,R26,R33,CD2),(A3,R11,R26,R33,CD3),(A3,R11,R26,R33,CD4),(A3,R11,R26,R33,C D5),(A3,R11,R26,R33,CD6),(A3,R11,R26,R33,CD7),(A3,R11,R26,R34,CD1),(A3,R11,R26,R 34,CD2),(A3,R11,R26,R34,CD3),(A3,R11,R26,R34,CD4),(A3,R11,R26,R34,CD5),(A3,R11,R 26,R34,CD6),(A3,R11,R26,R34,CD7),(A3,R11,R26,R35,CD1),(A3,R11,R26,R35,CD2),(A3,R 11,R26,R35,CD3),(A3,R11,R26,R35,CD4),(A3,R11,R26,R35,CD5),(A3,R11,R26,R35,CD6),( A3,R11,R26,R35,CD7),(A3,R12,R21,R31,CD1),(A3,R12,R21,R31,CD2),(A3,R12,R21,R31,C D3),(A3,R12,R21,R31,CD4),(A3,R12,R21,R31,CD5),(A3,R12,R21,R31,CD6),(A3,R12,R21,R 31,CD7),(A3,R12,R21,R32,CD1),(A3,R12,R21,R32,CD2),(A3,R12,R21,R32,CD3),(A3,R12,R 21,R32,CD4),(A3,R12,R21,R32,CD5),(A3,R12,R21,R32,CD6),(A3,R12,R21,R32,CD7),(A3,R 12,R21,R33,CD1),(A3,R12,R21,R33,CD2),(A3,R12,R21,R33,CD3),(A3,R12,R21,R33,CD4),( A3,R12,R21,R33,CD5),(A3,R12,R21,R33,CD6),(A3,R12,R21,R33,CD7),(A3,R12,R21,R34,C D1),(A3,R12,R21,R34,CD2),(A3,R12,R21,R34,CD3),(A3,R12,R21,R34,CD4),(A3,R12,R21,R 34,CD5),(A3,R12,R21,R34,CD6),(A3,R12,R21,R34,CD7),(A3,R12,R21,R35,CD1),(A3,R12,R 21,R35,CD2),(A3,R12,R21,R35,CD3),(A3,R12,R21,R35,CD4),(A3,R12,R21,R35,CD5),(A3,R 12,R21,R35,CD6),(A3,R12,R21,R35,CD7),(A3,R12,R22,R31,CD1),(A3,R12,R22,R31,CD2),( A3,R12,R22,R31,CD3),(A3,R12,R22,R31,CD4),(A3,R12,R22,R31,CD5),(A3,R12,R22,R31,C D6),(A3,R12,R22,R31,CD7),(A3,R12,R22,R32,CD1),(A3,R12,R22,R32,CD2),(A3,R12,R22,R 32,CD3),(A3,R12,R22,R32,CD4),(A3,R12,R22,R32,CD5),(A3,R12,R22,R32,CD6),(A3,R12,R 22,R32,CD7),(A3,R12,R22,R33,CD1),(A3,R12,R22,R33,CD2),(A3,R12,R22,R33,CD3),(A3,R 12,R22,R33,CD4),(A3,R12,R22,R33,CD5),(A3,R12,R22,R33,CD6),(A3,R12,R22,R33,CD7),( A3,R12,R22,R34,CD1),(A3,R12,R22,R34,CD2),(A3,R12,R22,R34,CD3),(A3,R12,R22,R34,C D4),(A3,R12,R22,R34,CD5),(A3,R12,R22,R34,CD6),(A3,R12,R22,R34,CD7),(A3,R12,R22,R 35,CD1),(A3,R12,R22,R35,CD2),(A3,R12,R22,R35,CD3),(A3,R12,R22,R35,CD4),(A3,R12,R 22,R35,CD5),(A3,R12,R22,R35,CD6),(A3,R12,R22,R35,CD7),(A3,R12,R23,R31,CD1),(A3,R 12,R23,R31,CD2),(A3,R12,R23,R31,CD3),(A3,R12,R23,R31,CD4),(A3,R12,R23,R31,CD5),( A3,R12,R23,R31,CD6),(A3,R12,R23,R31,CD7),(A3,R12,R23,R32,CD1),(A3,R12,R23,R32,C D2),(A3,R12,R23,R32,CD3),(A3,R12,R23,R32,CD4),(A3,R12,R23,R32,CD5),(A3,R12,R23,R 32,CD6),(A3,R12,R23,R32,CD7),(A3,R12,R23,R33,CD1),(A3,R12,R23,R33,CD2),(A3,R12,R 23,R33,CD3),(A3,R12,R23,R33,CD4),(A3,R12,R23,R33,CD5),(A3,R12,R23,R33,CD6),(A3,R 12,R23,R33,CD7),(A3,R12,R23,R34,CD1),(A3,R12,R23,R34,CD2),(A3,R12,R23,R34,CD3),( A3,R12,R23,R34,CD4),(A3,R12,R23,R34,CD5),(A3,R12,R23,R34,CD6),(A3,R12,R23,R34,C D7),(A3,R12,R23,R35,CD1),(A3,R12,R23,R35,CD2),(A3,R12,R23,R35,CD3),(A3,R12,R23,R 35,CD4),(A3,R12,R23,R35,CD5),(A3,R12,R23,R35,CD6),(A3,R12,R23,R35,CD7),(A3,R12,R 24,R31,CD1),(A3,R12,R24,R31,CD2),(A3,R12,R24,R31,CD3),(A3,R12,R24,R31,CD4),(A3,R 12,R24,R31,CD5),(A3,R12,R24,R31,CD6),(A3,R12,R24,R31,CD7),(A3,R12,R24,R32,CD1),( A3,R12,R24,R32,CD2),(A3,R12,R24,R32,CD3),(A3,R12,R24,R32,CD4),(A3,R12,R24,R32,C D5),(A3,R12,R24,R32,CD6),(A3,R12,R24,R32,CD7),(A3,R12,R24,R33,CD1),(A3,R12,R24,R 33,CD2),(A3,R12,R24,R33,CD3),(A3,R12,R24,R33,CD4),(A3,R12,R24,R33,CD5),(A3,R12,R 24,R33,CD6),(A3,R12,R24,R33,CD7),(A3,R12,R24,R34,CD1),(A3,R12,R24,R34,CD2),(A3,R 12,R24,R34,CD3),(A3,R12,R24,R34,CD4),(A3,R12,R24,R34,CD5),(A3,R12,R24,R34,CD6),( A3,R12,R24,R34,CD7),(A3,R12,R24,R35,CD1),(A3,R12,R24,R35,CD2),(A3,R12,R24,R35,C D3),(A3,R12,R24,R35,CD4),(A3,R12,R24,R35,CD5),(A3,R12,R24,R35,CD6),(A3,R12,R24,R 35,CD7),(A3,R12,R25,R31,CD1),(A3,R12,R25,R31,CD2),(A3,R12,R25,R31,CD3),(A3,R12,R 25,R31,CD4),(A3,R12,R25,R31,CD5),(A3,R12,R25,R31,CD6),(A3,R12,R25,R31,CD7),(A3,R 12,R25,R32,CD1),(A3,R12,R25,R32,CD2),(A3,R12,R25,R32,CD3),(A3,R12,R25,R32,CD4),( A3,R12,R25,R32,CD5),(A3,R12,R25,R32,CD6),(A3,R12,R25,R32,CD7),(A3,R12,R25,R33,C D1),(A3,R12,R25,R33,CD2),(A3,R12,R25,R33,CD3),(A3,R12,R25,R33,CD4),(A3,R12,R25,R 33,CD5),(A3,R12,R25,R33,CD6),(A3,R12,R25,R33,CD7),(A3,R12,R25,R34,CD1),(A3,R12,R 25,R34,CD2),(A3,R12,R25,R34,CD3),(A3,R12,R25,R34,CD4),(A3,R12,R25,R34,CD5),(A3,R 12,R25,R34,CD6),(A3,R12,R25,R34,CD7),(A3,R12,R25,R35,CD1),(A3,R12,R25,R35,CD2),( A3,R12,R25,R35,CD3),(A3,R12,R25,R35,CD4),(A3,R12,R25,R35,CD5),(A3,R12,R25,R35,C D6),(A3,R12,R25,R35,CD7),(A3,R12,R26,R31,CD1),(A3,R12,R26,R31,CD2),(A3,R12,R26,R 31,CD3),(A3,R12,R26,R31,CD4),(A3,R12,R26,R31,CD5),(A3,R12,R26,R31,CD6),(A3,R12,R 26,R31,CD7),(A3,R12,R26,R32,CD1),(A3,R12,R26,R32,CD2),(A3,R12,R26,R32,CD3),(A3,R 12,R26,R32,CD4),(A3,R12,R26,R32,CD5),(A3,R12,R26,R32,CD6),(A3,R12,R26,R32,CD7),( A3,R12,R26,R33,CD1),(A3,R12,R26,R33,CD2),(A3,R12,R26,R33,CD3),(A3,R12,R26,R33,C D4),(A3,R12,R26,R33,CD5),(A3,R12,R26,R33,CD6),(A3,R12,R26,R33,CD7),(A3,R12,R26,R 34,CD1),(A3,R12,R26,R34,CD2),(A3,R12,R26,R34,CD3),(A3,R12,R26,R34,CD4),(A3,R12,R 26,R34,CD5),(A3,R12,R26,R34,CD6),(A3,R12,R26,R34,CD7),(A3,R12,R26,R35,CD1),(A3,R 12,R26,R35,CD2),(A3,R12,R26,R35,CD3),(A3,R12,R26,R35,CD4),(A3,R12,R26,R35,CD5),( A3,R12,R26,R35,CD6),(A3,R12,R26,R35,CD7),(A3,R13,R21,R31,CD1),(A3,R13,R21,R31,C D2),(A3,R13,R21,R31,CD3),(A3,R13,R21,R31,CD4),(A3,R13,R21,R31,CD5),(A3,R13,R21,R 31,CD6),(A3,R13,R21,R31,CD7),(A3,R13,R21,R32,CD1),(A3,R13,R21,R32,CD2),(A3,R13,R 21,R32,CD3),(A3,R13,R21,R32,CD4),(A3,R13,R21,R32,CD5),(A3,R13,R21,R32,CD6),(A3,R 13,R21,R32,CD7),(A3,R13,R21,R33,CD1),(A3,R13,R21,R33,CD2),(A3,R13,R21,R33,CD3),( A3,R13,R21,R33,CD4),(A3,R13,R21,R33,CD5),(A3,R13,R21,R33,CD6),(A3,R13,R21,R33,C D7),(A3,R13,R21,R34,CD1),(A3,R13,R21,R34,CD2),(A3,R13,R21,R34,CD3),(A3,R13,R21,R 34,CD4),(A3,R13,R21,R34,CD5),(A3,R13,R21,R34,CD6),(A3,R13,R21,R34,CD7),(A3,R13,R 21,R35,CD1),(A3,R13,R21,R35,CD2),(A3,R13,R21,R35,CD3),(A3,R13,R21,R35,CD4),(A3,R 13,R21,R35,CD5),(A3,R13,R21,R35,CD6),(A3,R13,R21,R35,CD7),(A3,R13,R22,R31,CD1),( A3,R13,R22,R31,CD2),(A3,R13,R22,R31,CD3),(A3,R13,R22,R31,CD4),(A3,R13,R22,R31,C D5),(A3,R13,R22,R31,CD6),(A3,R13,R22,R31,CD7),(A3,R13,R22,R32,CD1),(A3,R13,R22,R 32,CD2),(A3,R13,R22,R32,CD3),(A3,R13,R22,R32,CD4),(A3,R13,R22,R32,CD5),(A3,R13,R 22,R32,CD6),(A3,R13,R22,R32,CD7),(A3,R13,R22,R33,CD1),(A3,R13,R22,R33,CD2),(A3,R 13,R22,R33,CD3),(A3,R13,R22,R33,CD4),(A3,R13,R22,R33,CD5),(A3,R13,R22,R33,CD6),( A3,R13,R22,R33,CD7),(A3,R13,R22,R34,CD1),(A3,R13,R22,R34,CD2),(A3,R13,R22,R34,C D3),(A3,R13,R22,R34,CD4),(A3,R13,R22,R34,CD5),(A3,R13,R22,R34,CD6),(A3,R13,R22,R 34,CD7),(A3,R13,R22,R35,CD1),(A3,R13,R22,R35,CD2),(A3,R13,R22,R35,CD3),(A3,R13,R 22,R35,CD4),(A3,R13,R22,R35,CD5),(A3,R13,R22,R35,CD6),(A3,R13,R22,R35,CD7),(A3,R 13,R23,R31,CD1),(A3,R13,R23,R31,CD2),(A3,R13,R23,R31,CD3),(A3,R13,R23,R31,CD4),( A3,R13,R23,R31,CD5),(A3,R13,R23,R31,CD6),(A3,R13,R23,R31,CD7),(A3,R13,R23,R32,C D1),(A3,R13,R23,R32,CD2),(A3,R13,R23,R32,CD3),(A3,R13,R23,R32,CD4),(A3,R13,R23,R 32,CD5),(A3,R13,R23,R32,CD6),(A3,R13,R23,R32,CD7),(A3,R13,R23,R33,CD1),(A3,R13,R 23,R33,CD2),(A3,R13,R23,R33,CD3),(A3,R13,R23,R33,CD4),(A3,R13,R23,R33,CD5),(A3,R 13,R23,R33,CD6),(A3,R13,R23,R33,CD7),(A3,R13,R23,R34,CD1),(A3,R13,R23,R34,CD2),( A3,R13,R23,R34,CD3),(A3,R13,R23,R34,CD4),(A3,R13,R23,R34,CD5),(A3,R13,R23,R34,C D6),(A3,R13,R23,R34,CD7),(A3,R13,R23,R35,CD1),(A3,R13,R23,R35,CD2),(A3,R13,R23,R 35,CD3),(A3,R13,R23,R35,CD4),(A3,R13,R23,R35,CD5),(A3,R13,R23,R35,CD6),(A3,R13,R 23,R35,CD7),(A3,R13,R24,R31,CD1),(A3,R13,R24,R31,CD2),(A3,R13,R24,R31,CD3),(A3,R 13,R24,R31,CD4),(A3,R13,R24,R31,CD5),(A3,R13,R24,R31,CD6),(A3,R13,R24,R31,CD7),( A3,R13,R24,R32,CD1),(A3,R13,R24,R32,CD2),(A3,R13,R24,R32,CD3),(A3,R13,R24,R32,C D4),(A3,R13,R24,R32,CD5),(A3,R13,R24,R32,CD6),(A3,R13,R24,R32,CD7),(A3,R13,R24,R 33,CD1),(A3,R13,R24,R33,CD2),(A3,R13,R24,R33,CD3),(A3,R13,R24,R33,CD4),(A3,R13,R 24,R33,CD5),(A3,R13,R24,R33,CD6),(A3,R13,R24,R33,CD7),(A3,R13,R24,R34,CD1),(A3,R 13,R24,R34,CD2),(A3,R13,R24,R34,CD3),(A3,R13,R24,R34,CD4),(A3,R13,R24,R34,CD5),( A3,R13,R24,R34,CD6),(A3,R13,R24,R34,CD7),(A3,R13,R24,R35,CD1),(A3,R13,R24,R35,C D2),(A3,R13,R24,R35,CD3),(A3,R13,R24,R35,CD4),(A3,R13,R24,R35,CD5),(A3,R13,R24,R 35,CD6),(A3,R13,R24,R35,CD7),(A3,R13,R25,R31,CD1),(A3,R13,R25,R31,CD2),(A3,R13,R 25,R31,CD3),(A3,R13,R25,R31,CD4),(A3,R13,R25,R31,CD5),(A3,R13,R25,R31,CD6),(A3,R 13,R25,R31,CD7),(A3,R13,R25,R32,CD1),(A3,R13,R25,R32,CD2),(A3,R13,R25,R32,CD3),( A3,R13,R25,R32,CD4),(A3,R13,R25,R32,CD5),(A3,R13,R25,R32,CD6),(A3,R13,R25,R32,C D7),(A3,R13,R25,R33,CD1),(A3,R13,R25,R33,CD2),(A3,R13,R25,R33,CD3),(A3,R13,R25,R 33,CD4),(A3,R13,R25,R33,CD5),(A3,R13,R25,R33,CD6),(A3,R13,R25,R33,CD7),(A3,R13,R 25,R34,CD1),(A3,R13,R25,R34,CD2),(A3,R13,R25,R34,CD3),(A3,R13,R25,R34,CD4),(A3,R 13,R25,R34,CD5),(A3,R13,R25,R34,CD6),(A3,R13,R25,R34,CD7),(A3,R13,R25,R35,CD1),( A3,R13,R25,R35,CD2),(A3,R13,R25,R35,CD3),(A3,R13,R25,R35,CD4),(A3,R13,R25,R35,C D5),(A3,R13,R25,R35,CD6),(A3,R13,R25,R35,CD7),(A3,R13,R26,R31,CD1),(A3,R13,R26,R 31,CD2),(A3,R13,R26,R31,CD3),(A3,R13,R26,R31,CD4),(A3,R13,R26,R31,CD5),(A3,R13,R 26,R31,CD6),(A3,R13,R26,R31,CD7),(A3,R13,R26,R32,CD1),(A3,R13,R26,R32,CD2),(A3,R 13,R26,R32,CD3),(A3,R13,R26,R32,CD4),(A3,R13,R26,R32,CD5),(A3,R13,R26,R32,CD6),( A3,R13,R26,R32,CD7),(A3,R13,R26,R33,CD1),(A3,R13,R26,R33,CD2),(A3,R13,R26,R33,C D3),(A3,R13,R26,R33,CD4),(A3,R13,R26,R33,CD5),(A3,R13,R26,R33,CD6),(A3,R13,R26,R 33,CD7),(A3,R13,R26,R34,CD1),(A3,R13,R26,R34,CD2),(A3,R13,R26,R34,CD3),(A3,R13,R 26,R34,CD4),(A3,R13,R26,R34,CD5),(A3,R13,R26,R34,CD6),(A3,R13,R26,R34,CD7),(A3,R 13,R26,R35,CD1),(A3,R13,R26,R35,CD2),(A3,R13,R26,R35,CD3),(A3,R13,R26,R35,CD4),( A3,R13,R26,R35,CD5),(A3,R13,R26,R35,CD6),(A3,R13,R26,R35,CD7),(A3,R14,R21,R31,C D1),(A3,R14,R21,R31,CD2),(A3,R14,R21,R31,CD3),(A3,R14,R21,R31,CD4),(A3,R14,R21,R 31,CD5),(A3,R14,R21,R31,CD6),(A3,R14,R21,R31,CD7),(A3,R14,R21,R32,CD1),(A3,R14,R 21,R32,CD2),(A3,R14,R21,R32,CD3),(A3,R14,R21,R32,CD4),(A3,R14,R21,R32,CD5),(A3,R 14,R21,R32,CD6),(A3,R14,R21,R32,CD7),(A3,R14,R21,R33,CD1),(A3,R14,R21,R33,CD2),( A3,R14,R21,R33,CD3),(A3,R14,R21,R33,CD4),(A3,R14,R21,R33,CD5),(A3,R14,R21,R33,C D6),(A3,R14,R21,R33,CD7),(A3,R14,R21,R34,CD1),(A3,R14,R21,R34,CD2),(A3,R14,R21,R 34,CD3),(A3,R14,R21,R34,CD4),(A3,R14,R21,R34,CD5),(A3,R14,R21,R34,CD6),(A3,R14,R 21,R34,CD7),(A3,R14,R21,R35,CD1),(A3,R14,R21,R35,CD2),(A3,R14,R21,R35,CD3),(A3,R 14,R21,R35,CD4),(A3,R14,R21,R35,CD5),(A3,R14,R21,R35,CD6),(A3,R14,R21,R35,CD7),( A3,R14,R22,R31,CD1),(A3,R14,R22,R31,CD2),(A3,R14,R22,R31,CD3),(A3,R14,R22,R31,C D4),(A3,R14,R22,R31,CD5),(A3,R14,R22,R31,CD6),(A3,R14,R22,R31,CD7),(A3,R14,R22,R 32,CD1),(A3,R14,R22,R32,CD2),(A3,R14,R22,R32,CD3),(A3,R14,R22,R32,CD4),(A3,R14,R 22,R32,CD5),(A3,R14,R22,R32,CD6),(A3,R14,R22,R32,CD7),(A3,R14,R22,R33,CD1),(A3,R 14,R22,R33,CD2),(A3,R14,R22,R33,CD3),(A3,R14,R22,R33,CD4),(A3,R14,R22,R33,CD5),( A3,R14,R22,R33,CD6),(A3,R14,R22,R33,CD7),(A3,R14,R22,R34,CD1),(A3,R14,R22,R34,C D2),(A3,R14,R22,R34,CD3),(A3,R14,R22,R34,CD4),(A3,R14,R22,R34,CD5),(A3,R14,R22,R 34,CD6),(A3,R14,R22,R34,CD7),(A3,R14,R22,R35,CD1),(A3,R14,R22,R35,CD2),(A3,R14,R 22,R35,CD3),(A3,R14,R22,R35,CD4),(A3,R14,R22,R35,CD5),(A3,R14,R22,R35,CD6),(A3,R 14,R22,R35,CD7),(A3,R14,R23,R31,CD1),(A3,R14,R23,R31,CD2),(A3,R14,R23,R31,CD3),( A3,R14,R23,R31,CD4),(A3,R14,R23,R31,CD5),(A3,R14,R23,R31,CD6),(A3,R14,R23,R31,C D7),(A3,R14,R23,R32,CD1),(A3,R14,R23,R32,CD2),(A3,R14,R23,R32,CD3),(A3,R14,R23,R 32,CD4),(A3,R14,R23,R32,CD5),(A3,R14,R23,R32,CD6),(A3,R14,R23,R32,CD7),(A3,R14,R 23,R33,CD1),(A3,R14,R23,R33,CD2),(A3,R14,R23,R33,CD3),(A3,R14,R23,R33,CD4),(A3,R 14,R23,R33,CD5),(A3,R14,R23,R33,CD6),(A3,R14,R23,R33,CD7),(A3,R14,R23,R34,CD1),( A3,R14,R23,R34,CD2),(A3,R14,R23,R34,CD3),(A3,R14,R23,R34,CD4),(A3,R14,R23,R34,C D5),(A3,R14,R23,R34,CD6),(A3,R14,R23,R34,CD7),(A3,R14,R23,R35,CD1),(A3,R14,R23,R 35,CD2),(A3,R14,R23,R35,CD3),(A3,R14,R23,R35,CD4),(A3,R14,R23,R35,CD5),(A3,R14,R 23,R35,CD6),(A3,R14,R23,R35,CD7),(A3,R14,R24,R31,CD1),(A3,R14,R24,R31,CD2),(A3,R 14,R24,R31,CD3),(A3,R14,R24,R31,CD4),(A3,R14,R24,R31,CD5),(A3,R14,R24,R31,CD6),( A3,R14,R24,R31,CD7),(A3,R14,R24,R32,CD1),(A3,R14,R24,R32,CD2),(A3,R14,R24,R32,C D3),(A3,R14,R24,R32,CD4),(A3,R14,R24,R32,CD5),(A3,R14,R24,R32,CD6),(A3,R14,R24,R 32,CD7),(A3,R14,R24,R33,CD1),(A3,R14,R24,R33,CD2),(A3,R14,R24,R33,CD3),(A3,R14,R 24,R33,CD4),(A3,R14,R24,R33,CD5),(A3,R14,R24,R33,CD6),(A3,R14,R24,R33,CD7),(A3,R 14,R24,R34,CD1),(A3,R14,R24,R34,CD2),(A3,R14,R24,R34,CD3),(A3,R14,R24,R34,CD4),( A3,R14,R24,R34,CD5),(A3,R14,R24,R34,CD6),(A3,R14,R24,R34,CD7),(A3,R14,R24,R35,C D1),(A3,R14,R24,R35,CD2),(A3,R14,R24,R35,CD3),(A3,R14,R24,R35,CD4),(A3,R14,R24,R 35,CD5),(A3,R14,R24,R35,CD6),(A3,R14,R24,R35,CD7),(A3,R14,R25,R31,CD1),(A3,R14,R 25,R31,CD2),(A3,R14,R25,R31,CD3),(A3,R14,R25,R31,CD4),(A3,R14,R25,R31,CD5),(A3,R 14,R25,R31,CD6),(A3,R14,R25,R31,CD7),(A3,R14,R25,R32,CD1),(A3,R14,R25,R32,CD2),( A3,R14,R25,R32,CD3),(A3,R14,R25,R32,CD4),(A3,R14,R25,R32,CD5),(A3,R14,R25,R32,C D6),(A3,R14,R25,R32,CD7),(A3,R14,R25,R33,CD1),(A3,R14,R25,R33,CD2),(A3,R14,R25,R 33,CD3),(A3,R14,R25,R33,CD4),(A3,R14,R25,R33,CD5),(A3,R14,R25,R33,CD6),(A3,R14,R 25,R33,CD7),(A3,R14,R25,R34,CD1),(A3,R14,R25,R34,CD2),(A3,R14,R25,R34,CD3),(A3,R 14,R25,R34,CD4),(A3,R14,R25,R34,CD5),(A3,R14,R25,R34,CD6),(A3,R14,R25,R34,CD7),( A3,R14,R25,R35,CD1),(A3,R14,R25,R35,CD2),(A3,R14,R25,R35,CD3),(A3,R14,R25,R35,C D4),(A3,R14,R25,R35,CD5),(A3,R14,R25,R35,CD6),(A3,R14,R25,R35,CD7),(A3,R14,R26,R 31,CD1),(A3,R14,R26,R31,CD2),(A3,R14,R26,R31,CD3),(A3,R14,R26,R31,CD4),(A3,R14,R 26,R31,CD5),(A3,R14,R26,R31,CD6),(A3,R14,R26,R31,CD7),(A3,R14,R26,R32,CD1),(A3,R 14,R26,R32,CD2),(A3,R14,R26,R32,CD3),(A3,R14,R26,R32,CD4),(A3,R14,R26,R32,CD5),( A3,R14,R26,R32,CD6),(A3,R14,R26,R32,CD7),(A3,R14,R26,R33,CD1),(A3,R14,R26,R33,C D2),(A3,R14,R26,R33,CD3),(A3,R14,R26,R33,CD4),(A3,R14,R26,R33,CD5),(A3,R14,R26,R 33,CD6),(A3,R14,R26,R33,CD7),(A3,R14,R26,R34,CD1),(A3,R14,R26,R34,CD2),(A3,R14,R 26,R34,CD3),(A3,R14,R26,R34,CD4),(A3,R14,R26,R34,CD5),(A3,R14,R26,R34,CD6),(A3,R 14,R26,R34,CD7),(A3,R14,R26,R35,CD1),(A3,R14,R26,R35,CD2),(A3,R14,R26,R35,CD3),( A3,R14,R26,R35,CD4),(A3,R14,R26,R35,CD5),(A3,R14,R26,R35,CD6),(A3,R14,R26,R35,C D7),(A3,R15,R21,R31,CD1),(A3,R15,R21,R31,CD2),(A3,R15,R21,R31,CD3),(A3,R15,R21,R 31,CD4),(A3,R15,R21,R31,CD5),(A3,R15,R21,R31,CD6),(A3,R15,R21,R31,CD7),(A3,R15,R 21,R32,CD1),(A3,R15,R21,R32,CD2),(A3,R15,R21,R32,CD3),(A3,R15,R21,R32,CD4),(A3,R 15,R21,R32,CD5),(A3,R15,R21,R32,CD6),(A3,R15,R21,R32,CD7),(A3,R15,R21,R33,CD1),( A3,R15,R21,R33,CD2),(A3,R15,R21,R33,CD3),(A3,R15,R21,R33,CD4),(A3,R15,R21,R33,C D5),(A3,R15,R21,R33,CD6),(A3,R15,R21,R33,CD7),(A3,R15,R21,R34,CD1),(A3,R15,R21,R 34,CD2),(A3,R15,R21,R34,CD3),(A3,R15,R21,R34,CD4),(A3,R15,R21,R34,CD5),(A3,R15,R 21,R34,CD6),(A3,R15,R21,R34,CD7),(A3,R15,R21,R35,CD1),(A3,R15,R21,R35,CD2),(A3,R 15,R21,R35,CD3),(A3,R15,R21,R35,CD4),(A3,R15,R21,R35,CD5),(A3,R15,R21,R35,CD6),( A3,R15,R21,R35,CD7),(A3,R15,R22,R31,CD1),(A3,R15,R22,R31,CD2),(A3,R15,R22,R31,C D3),(A3,R15,R22,R31,CD4),(A3,R15,R22,R31,CD5),(A3,R15,R22,R31,CD6),(A3,R15,R22,R 31,CD7),(A3,R15,R22,R32,CD1),(A3,R15,R22,R32,CD2),(A3,R15,R22,R32,CD3),(A3,R15,R 22,R32,CD4),(A3,R15,R22,R32,CD5),(A3,R15,R22,R32,CD6),(A3,R15,R22,R32,CD7),(A3,R 15,R22,R33,CD1),(A3,R15,R22,R33,CD2),(A3,R15,R22,R33,CD3),(A3,R15,R22,R33,CD4),( A3,R15,R22,R33,CD5),(A3,R15,R22,R33,CD6),(A3,R15,R22,R33,CD7),(A3,R15,R22,R34,C D1),(A3,R15,R22,R34,CD2),(A3,R15,R22,R34,CD3),(A3,R15,R22,R34,CD4),(A3,R15,R22,R 34,CD5),(A3,R15,R22,R34,CD6),(A3,R15,R22,R34,CD7),(A3,R15,R22,R35,CD1),(A3,R15,R 22,R35,CD2),(A3,R15,R22,R35,CD3),(A3,R15,R22,R35,CD4),(A3,R15,R22,R35,CD5),(A3,R 15,R22,R35,CD6),(A3,R15,R22,R35,CD7),(A3,R15,R23,R31,CD1),(A3,R15,R23,R31,CD2),( A3,R15,R23,R31,CD3),(A3,R15,R23,R31,CD4),(A3,R15,R23,R31,CD5),(A3,R15,R23,R31,C D6),(A3,R15,R23,R31,CD7),(A3,R15,R23,R32,CD1),(A3,R15,R23,R32,CD2),(A3,R15,R23,R 32,CD3),(A3,R15,R23,R32,CD4),(A3,R15,R23,R32,CD5),(A3,R15,R23,R32,CD6),(A3,R15,R 23,R32,CD7),(A3,R15,R23,R33,CD1),(A3,R15,R23,R33,CD2),(A3,R15,R23,R33,CD3),(A3,R 15,R23,R33,CD4),(A3,R15,R23,R33,CD5),(A3,R15,R23,R33,CD6),(A3,R15,R23,R33,CD7),( A3,R15,R23,R34,CD1),(A3,R15,R23,R34,CD2),(A3,R15,R23,R34,CD3),(A3,R15,R23,R34,C D4),(A3,R15,R23,R34,CD5),(A3,R15,R23,R34,CD6),(A3,R15,R23,R34,CD7),(A3,R15,R23,R 35,CD1),(A3,R15,R23,R35,CD2),(A3,R15,R23,R35,CD3),(A3,R15,R23,R35,CD4),(A3,R15,R 23,R35,CD5),(A3,R15,R23,R35,CD6),(A3,R15,R23,R35,CD7),(A3,R15,R24,R31,CD1),(A3,R 15,R24,R31,CD2),(A3,R15,R24,R31,CD3),(A3,R15,R24,R31,CD4),(A3,R15,R24,R31,CD5),( A3,R15,R24,R31,CD6),(A3,R15,R24,R31,CD7),(A3,R15,R24,R32,CD1),(A3,R15,R24,R32,C D2),(A3,R15,R24,R32,CD3),(A3,R15,R24,R32,CD4),(A3,R15,R24,R32,CD5),(A3,R15,R24,R 32,CD6),(A3,R15,R24,R32,CD7),(A3,R15,R24,R33,CD1),(A3,R15,R24,R33,CD2),(A3,R15,R 24,R33,CD3),(A3,R15,R24,R33,CD4),(A3,R15,R24,R33,CD5),(A3,R15,R24,R33,CD6),(A3,R 15,R24,R33,CD7),(A3,R15,R24,R34,CD1),(A3,R15,R24,R34,CD2),(A3,R15,R24,R34,CD3),( A3,R15,R24,R34,CD4),(A3,R15,R24,R34,CD5),(A3,R15,R24,R34,CD6),(A3,R15,R24,R34,C D7),(A3,R15,R24,R35,CD1),(A3,R15,R24,R35,CD2),(A3,R15,R24,R35,CD3),(A3,R15,R24,R 35,CD4),(A3,R15,R24,R35,CD5),(A3,R15,R24,R35,CD6),(A3,R15,R24,R35,CD7),(A3,R15,R 25,R31,CD1),(A3,R15,R25,R31,CD2),(A3,R15,R25,R31,CD3),(A3,R15,R25,R31,CD4),(A3,R 15,R25,R31,CD5),(A3,R15,R25,R31,CD6),(A3,R15,R25,R31,CD7),(A3,R15,R25,R32,CD1),( A3,R15,R25,R32,CD2),(A3,R15,R25,R32,CD3),(A3,R15,R25,R32,CD4),(A3,R15,R25,R32,C D5),(A3,R15,R25,R32,CD6),(A3,R15,R25,R32,CD7),(A3,R15,R25,R33,CD1),(A3,R15,R25,R 33,CD2),(A3,R15,R25,R33,CD3),(A3,R15,R25,R33,CD4),(A3,R15,R25,R33,CD5),(A3,R15,R 25,R33,CD6),(A3,R15,R25,R33,CD7),(A3,R15,R25,R34,CD1),(A3,R15,R25,R34,CD2),(A3,R 15,R25,R34,CD3),(A3,R15,R25,R34,CD4),(A3,R15,R25,R34,CD5),(A3,R15,R25,R34,CD6),( A3,R15,R25,R34,CD7),(A3,R15,R25,R35,CD1),(A3,R15,R25,R35,CD2),(A3,R15,R25,R35,C D3),(A3,R15,R25,R35,CD4),(A3,R15,R25,R35,CD5),(A3,R15,R25,R35,CD6),(A3,R15,R25,R 35,CD7),(A3,R15,R26,R31,CD1),(A3,R15,R26,R31,CD2),(A3,R15,R26,R31,CD3),(A3,R15,R 26,R31,CD4),(A3,R15,R26,R31,CD5),(A3,R15,R26,R31,CD6),(A3,R15,R26,R31,CD7),(A3,R 15,R26,R32,CD1),(A3,R15,R26,R32,CD2),(A3,R15,R26,R32,CD3),(A3,R15,R26,R32,CD4),( A3,R15,R26,R32,CD5),(A3,R15,R26,R32,CD6),(A3,R15,R26,R32,CD7),(A3,R15,R26,R33,C D1),(A3,R15,R26,R33,CD2),(A3,R15,R26,R33,CD3),(A3,R15,R26,R33,CD4),(A3,R15,R26,R 33,CD5),(A3,R15,R26,R33,CD6),(A3,R15,R26,R33,CD7),(A3,R15,R26,R34,CD1),(A3,R15,R 26,R34,CD2),(A3,R15,R26,R34,CD3),(A3,R15,R26,R34,CD4),(A3,R15,R26,R34,CD5),(A3,R 15,R26,R34,CD6),(A3,R15,R26,R34,CD7),(A3,R15,R26,R35,CD1),(A3,R15,R26,R35,CD2),( A3,R15,R26,R35,CD3),(A3,R15,R26,R35,CD4),(A3,R15,R26,R35,CD5),(A3,R15,R26,R35,C D6),(A3,R15,R26,R35,CD7),(A3,R16,R21,R31,CD1),(A3,R16,R21,R31,CD2),(A3,R16,R21,R 31,CD3),(A3,R16,R21,R31,CD4),(A3,R16,R21,R31,CD5),(A3,R16,R21,R31,CD6),(A3,R16,R 21,R31,CD7),(A3,R16,R21,R32,CD1),(A3,R16,R21,R32,CD2),(A3,R16,R21,R32,CD3),(A3,R 16,R21,R32,CD4),(A3,R16,R21,R32,CD5),(A3,R16,R21,R32,CD6),(A3,R16,R21,R32,CD7),( A3,R16,R21,R33,CD1),(A3,R16,R21,R33,CD2),(A3,R16,R21,R33,CD3),(A3,R16,R21,R33,C D4),(A3,R16,R21,R33,CD5),(A3,R16,R21,R33,CD6),(A3,R16,R21,R33,CD7),(A3,R16,R21,R 34,CD1),(A3,R16,R21,R34,CD2),(A3,R16,R21,R34,CD3),(A3,R16,R21,R34,CD4),(A3,R16,R 21,R34,CD5),(A3,R16,R21,R34,CD6),(A3,R16,R21,R34,CD7),(A3,R16,R21,R35,CD1),(A3,R 16,R21,R35,CD2),(A3,R16,R21,R35,CD3),(A3,R16,R21,R35,CD4),(A3,R16,R21,R35,CD5),( A3,R16,R21,R35,CD6),(A3,R16,R21,R35,CD7),(A3,R16,R22,R31,CD1),(A3,R16,R22,R31,C D2),(A3,R16,R22,R31,CD3),(A3,R16,R22,R31,CD4),(A3,R16,R22,R31,CD5),(A3,R16,R22,R 31,CD6),(A3,R16,R22,R31,CD7),(A3,R16,R22,R32,CD1),(A3,R16,R22,R32,CD2),(A3,R16,R 22,R32,CD3),(A3,R16,R22,R32,CD4),(A3,R16,R22,R32,CD5),(A3,R16,R22,R32,CD6),(A3,R 16,R22,R32,CD7),(A3,R16,R22,R33,CD1),(A3,R16,R22,R33,CD2),(A3,R16,R22,R33,CD3),( A3,R16,R22,R33,CD4),(A3,R16,R22,R33,CD5),(A3,R16,R22,R33,CD6),(A3,R16,R22,R33,C D7),(A3,R16,R22,R34,CD1),(A3,R16,R22,R34,CD2),(A3,R16,R22,R34,CD3),(A3,R16,R22,R 34,CD4),(A3,R16,R22,R34,CD5),(A3,R16,R22,R34,CD6),(A3,R16,R22,R34,CD7),(A3,R16,R 22,R35,CD1),(A3,R16,R22,R35,CD2),(A3,R16,R22,R35,CD3),(A3,R16,R22,R35,CD4),(A3,R 16,R22,R35,CD5),(A3,R16,R22,R35,CD6),(A3,R16,R22,R35,CD7),(A3,R16,R23,R31,CD1),( A3,R16,R23,R31,CD2),(A3,R16,R23,R31,CD3),(A3,R16,R23,R31,CD4),(A3,R16,R23,R31,C D5),(A3,R16,R23,R31,CD6),(A3,R16,R23,R31,CD7),(A3,R16,R23,R32,CD1),(A3,R16,R23,R 32,CD2),(A3,R16,R23,R32,CD3),(A3,R16,R23,R32,CD4),(A3,R16,R23,R32,CD5),(A3,R16,R 23,R32,CD6),(A3,R16,R23,R32,CD7),(A3,R16,R23,R33,CD1),(A3,R16,R23,R33,CD2),(A3,R 16,R23,R33,CD3),(A3,R16,R23,R33,CD4),(A3,R16,R23,R33,CD5),(A3,R16,R23,R33,CD6),( A3,R16,R23,R33,CD7),(A3,R16,R23,R34,CD1),(A3,R16,R23,R34,CD2),(A3,R16,R23,R34,C D3),(A3,R16,R23,R34,CD4),(A3,R16,R23,R34,CD5),(A3,R16,R23,R34,CD6),(A3,R16,R23,R 34,CD7),(A3,R16,R23,R35,CD1),(A3,R16,R23,R35,CD2),(A3,R16,R23,R35,CD3),(A3,R16,R 23,R35,CD4),(A3,R16,R23,R35,CD5),(A3,R16,R23,R35,CD6),(A3,R16,R23,R35,CD7),(A3,R 16,R24,R31,CD1),(A3,R16,R24,R31,CD2),(A3,R16,R24,R31,CD3),(A3,R16,R24,R31,CD4),( A3,R16,R24,R31,CD5),(A3,R16,R24,R31,CD6),(A3,R16,R24,R31,CD7),(A3,R16,R24,R32,C D1),(A3,R16,R24,R32,CD2),(A3,R16,R24,R32,CD3),(A3,R16,R24,R32,CD4),(A3,R16,R24,R 32,CD5),(A3,R16,R24,R32,CD6),(A3,R16,R24,R32,CD7),(A3,R16,R24,R33,CD1),(A3,R16,R 24,R33,CD2),(A3,R16,R24,R33,CD3),(A3,R16,R24,R33,CD4),(A3,R16,R24,R33,CD5),(A3,R 16,R24,R33,CD6),(A3,R16,R24,R33,CD7),(A3,R16,R24,R34,CD1),(A3,R16,R24,R34,CD2),( A3,R16,R24,R34,CD3),(A3,R16,R24,R34,CD4),(A3,R16,R24,R34,CD5),(A3,R16,R24,R34,C D6),(A3,R16,R24,R34,CD7),(A3,R16,R24,R35,CD1),(A3,R16,R24,R35,CD2),(A3,R16,R24,R 35,CD3),(A3,R16,R24,R35,CD4),(A3,R16,R24,R35,CD5),(A3,R16,R24,R35,CD6),(A3,R16,R 24,R35,CD7),(A3,R16,R25,R31,CD1),(A3,R16,R25,R31,CD2),(A3,R16,R25,R31,CD3),(A3,R 16,R25,R31,CD4),(A3,R16,R25,R31,CD5),(A3,R16,R25,R31,CD6),(A3,R16,R25,R31,CD7),( A3,R16,R25,R32,CD1),(A3,R16,R25,R32,CD2),(A3,R16,R25,R32,CD3),(A3,R16,R25,R32,C D4),(A3,R16,R25,R32,CD5),(A3,R16,R25,R32,CD6),(A3,R16,R25,R32,CD7),(A3,R16,R25,R 33,CD1),(A3,R16,R25,R33,CD2),(A3,R16,R25,R33,CD3),(A3,R16,R25,R33,CD4),(A3,R16,R 25,R33,CD5),(A3,R16,R25,R33,CD6),(A3,R16,R25,R33,CD7),(A3,R16,R25,R34,CD1),(A3,R 16,R25,R34,CD2),(A3,R16,R25,R34,CD3),(A3,R16,R25,R34,CD4),(A3,R16,R25,R34,CD5),( A3,R16,R25,R34,CD6),(A3,R16,R25,R3i4,CD7),(A3,R16,R25,R35,CD1),(A3,R16,R25,R35,C D2),(A3,R16,R25,R35,CD3),(A3,R16,R25,R35,CD4),(A3,R16,R25,R35,CD5),(A3,R16,R25,R 35,CD6),(A3,R16,R25,R35,CD7),(A3,R16,R26,R31,CD1),(A3,R16,R26,R31,CD2),(A3,R16,R 26,R31,CD3),(A3,R16,R26,R31,CD4),(A3,R16,R26,R31,CD5),(A3,R16,R26,R31,CD6),(A3,R 16,R26,R31,CD7),(A3,R16,R26,R32,CD1),(A3,R16,R26,R32,CD2),(A3,R16,R26,R32,CD3),( A3,R16,R26,R32,CD4),(A3,R16,R26,R32,CD5),(A3,R16,R26,R32,CD6),(A3,R16,R26,R32,C D7),(A3,R16,R26,R33,CD1),(A3,R16,R26,R33,CD2),(A3,R16,R26,R33,CD3),(A3,R16,R26,R 33,CD4),(A3,R16,R26,R33,CD5),(A3,R16,R26,R33,CD6),(A3,R16,R26,R33,CD7),(A3,R16,R 26,R34,CD1),(A3,R16,R26,R34,CD2),(A3,R16,R26,R34,CD3),(A3,R16,R26,R34,CD4),(A3,R 16,R26,R34,CD5),(A3,R16,R26,R34,CD6),(A3,R16,R26,R34,CD7),(A3,R16,R26,R35,CD1),( A3,R16,R26,R35,CD2),(A3,R16,R26,R35,CD3),(A3,R16,R26,R35,CD4),(A3,R16,R26,R35,C D5),(A3,R16,R26,R35,CD6),(A3,R16,R26,R35,CD7),(A3,R17,R21,R31,CD1),(A3,R17,R21,R 31,CD2),(A3,R17,R21,R31,CD3),(A3,R17,R21,R31,CD4),(A3,R17,R21,R31,CD5),(A3,R17,R 21,R31,CD6),(A3,R17,R21,R31,CD7),(A3,R17,R21,R32,CD1),(A3,R17,R21,R32,CD2),(A3,R 17,R21,R32,CD3),(A3,R17,R21,R32,CD4),(A3,R17,R21,R32,CD5),(A3,R17,R21,R32,CD6),( A3,R17,R21,R32,CD7),(A3,R17,R21,R33,CD1),(A3,R17,R21,R33,CD2),(A3,R17,R21,R33,C D3),(A3,R17,R21,R33,CD4),(A3,R17,R21,R33,CD5),(A3,R17,R21,R33,CD6),(A3,R17,R21,R 33,CD7),(A3,R17,R21,R34,CD1),(A3,R17,R21,R34,CD2),(A3,R17,R21,R34,CD3),(A3,R17,R 21,R34,CD4),(A3,R17,R21,R34,CD5),(A3,R17,R21,R34,CD6),(A3,R17,R21,R34,CD7),(A3,R 17,R21,R35,CD1),(A3,R17,R21,R35,CD2),(A3,R17,R21,R35,CD3),(A3,R17,R21,R35,CD4),( A3,R17,R21,R35,CD5),(A3,R17,R21,R35,CD6),(A3,R17,R21,R35,CD7),(A3,R17,R22,R31,C D1),(A3,R17,R22,R31,CD2),(A3,R17,R22,R31,CD3),(A3,R17,R22,R31,CD4),(A3,R17,R22,R 31,CD5),(A3,R17,R22,R31,CD6),(A3,R17,R22,R31,CD7),(A3,R17,R22,R32,CD1),(A3,R17,R 22,R32,CD2),(A3,R17,R22,R32,CD3),(A3,R17,R22,R32,CD4),(A3,R17,R22,R32,CD5),(A3,R 17,R22,R32,CD6),(A3,R17,R22,R32,CD7),(A3,R17,R22,R33,CD1),(A3,R17,R22,R33,CD2),( A3,R17,R22,R33,CD3),(A3,R17,R22,R33,CD4),(A3,R17,R22,R33,CD5),(A3,R17,R22,R33,C D6),(A3,R17,R22,R33,CD7),(A3,R17,R22,R34,CD1),(A3,R17,R22,R34,CD2),(A3,R17,R22,R 34,CD3),(A3,R17,R22,R34,CD4),(A3,R17,R22,R34,CD5),(A3,R17,R22,R34,CD6),(A3,R17,R 22,R34,CD7),(A3,R17,R22,R35,CD1),(A3,R17,R22,R35,CD2),(A3,R17,R22,R35,CD3),(A3,R 17,R22,R35,CD4),(A3,R17,R22,R35,CD5),(A3,R17,R22,R35,CD6),(A3,R17,R22,R35,CD7),( A3,R17,R23,R31,CD1),(A3,R17,R23,R31,CD2),(A3,R17,R23,R31,CD3),(A3,R17,R23,R31,C D4),(A3,R17,R23,R31,CD5),(A3,R17,R23,R31,CD6),(A3,R17,R23,R31,CD7),(A3,R17,R23,R 32,CD1),(A3,R17,R23,R32,CD2),(A3,R17,R23,R32,CD3),(A3,R17,R23,R32,CD4),(A3,R17,R 23,R32,CD5),(A3,R17,R23,R32,CD6),(A3,R17,R23,R32,CD7),(A3,R17,R23,R33,CD1),(A3,R 17,R23,R33,CD2),(A3,R17,R23,R33,CD3),(A3,R17,R23,R33,CD4),(A3,R17,R23,R33,CD5),( A3,R17,R23,R33,CD6),(A3,R17,R23,R33,CD7),(A3,R17,R23,R34,CD1),(A3,R17,R23,R34,C D2),(A3,R17,R23,R34,CD3),(A3,R17,R23,R34,CD4),(A3,R17,R23,R34,CD5),(A3,R17,R23,R 34,CD6),(A3,R17,R23,R34,CD7),(A3,R17,R23,R35,CD1),(A3,R17,R23,R35,CD2),(A3,R17,R 23,R35,CD3),(A3,R17,R23,R35,CD4),(A3,R17,R23,R35,CD5),(A3,R17,R23,R35,CD6),(A3,R 17,R23,R35,CD7),(A3,R17,R24,R31,CD1),(A3,R17,R24,R31,CD2),(A3,R17,R24,R31,CD3),( A3,R17,R24,R31,CD4),(A3,R17,R24,R31,CD5),(A3,R17,R24,R31,CD6),(A3,R17,R24,R31,C D7),(A3,R17,R24,R32,CD1),(A3,R17,R24,R32,CD2),(A3,R17,R24,R32,CD3),(A3,R17,R24,R 32,CD4),(A3,R17,R24,R32,CD5),(A3,R17,R24,R32,CD6),(A3,R17,R24,R32,CD7),(A3,R17,R 24,R33,CD1),(A3,R17,R24,R33,CD2),(A3,R17,R24,R33,CD3),(A3,R17,R24,R33,CD4),(A3,R 17,R24,R33,CD5),(A3,R17,R24,R33,CD6),(A3,R17,R24,R33,CD7),(A3,R17,R24,R34,CD1),( A3,R17,R24,R34,CD2),(A3,R17,R24,R34,CD3),(A3,R17,R24,R34,CD4),(A3,R17,R24,R34,C D5),(A3,R17,R24,R34,CD6),(A3,R17,R24,R34,CD7),(A3,R17,R24,R35,CD1),(A3,R17,R24,R 35,CD2),(A3,R17,R24,R35,CD3),(A3,R17,R24,R35,CD4),(A3,R17,R24,R35,CD5),(A3,R17,R 24,R35,CD6),(A3,R17,R24,R35,CD7),(A3,R17,R25,R31,CD1),(A3,R17,R25,R31,CD2),(A3,R 17,R25,R31,CD3),(A3,R17,R25,R31,CD4),(A3,R17,R25,R31,CD5),(A3,R17,R25,R31,CD6),( A3,R17,R25,R31,CD7),(A3,R17,R25,R32,CD1),(A3,R17,R25,R32,CD2),(A3,R17,R25,R32,C D3),(A3,R17,R25,R32,CD4),(A3,R17,R25,R32,CD5),(A3,R17,R25,R32,CD6),(A3,R17,R25,R 32,CD7),(A3,R17,R25,R33,CD1),(A3,R17,R25,R33,CD2),(A3,R17,R25,R33,CD3),(A3,R17,R 25,R33,CD4),(A3,R17,R25,R33,CD5),(A3,R17,R25,R33,CD6),(A3,R17,R25,R33,CD7),(A3,R 17,R25,R34,CD1),(A3,R17,R25,R34,CD2),(A3,R17,R25,R34,CD3),(A3,R17,R25,R34,CD4),( A3,R17,R25,R34,CD5),(A3,R17,R25,R34,CD6),(A3,R17,R25,R34,CD7),(A3,R17,R25,R35,C D1),(A3,R17,R25,R35,CD2),(A3,R17,R25,R35,CD3),(A3,R17,R25,R35,CD4),(A3,R17,R25,R 35,CD5),(A3,R17,R25,R35,CD6),(A3,R17,R25,R35,CD7),(A3,R17,R26,R31,CD1),(A3,R17,R 26,R31,CD2),(A3,R17,R26,R31,CD3),(A3,R17,R26,R31,CD4),(A3,R17,R26,R31,CD5),(A3,R 17,R26,R31,CD6),(A3,R17,R26,R31,CD7),(A3,R17,R26,R32,CD1),(A3,R17,R26,R32,CD2),( A3,R17,R26,R32,CD3),(A3,R17,R26,R32,CD4),(A3,R17,R26,R32,CD5),(A3,R17,R26,R32,C D6),(A3,R17,R26,R32,CD7),(A3,R17,R26,R33,CD1),(A3,R17,R26,R33,CD2),(A3,R17,R26,R 33,CD3),(A3,R17,R26,R33,CD4),(A3,R17,R26,R33,CD5),(A3,R17,R26,R33,CD6),(A3,R17,R 26,R33,CD7),(A3,R17,R26,R34,CD1),(A3,R17,R26,R34,CD2),(A3,R17,R26,R34,CD3),(A3,R 17,R26,R34,CD4),(A3,R17,R26,R34,CD5),(A3,R17,R26,R34,CD6),(A3,R17,R26,R34,CD7), A3,R17,R26,R35,CD1),(A3,R17,R26,R35,CD2),(A3,R17,R26,R35,CD3),(A3,R17,R26,R35,C D4),(A3,R17,R26,R35,CD5),(A3,R17,R26,R35,CD6),(A3,R17,R26,R35,CD7),(A3,R18,R21,R 31,CD1),(A3,R18,R21,R31,CD2),(A3,R18,R21,R31,CD3),(A3,R18,R21,R31,CD4),(A3,R18,R 21,R31,CD5),(A3,R18,R21,R31,CD6),(A3,R18,R21,R31,CD7),(A3,R18,R21,R32,CD1),(A3,R 18,R21,R32,CD2),(A3,R18,R21,R32,CD3),(A3,R18,R21,R32,CD4),(A3,R18,R21,R32,CD5),( A3,R18,R21,R32,CD6),(A3,R18,R21,R32,CD7),(A3,R18,R21,R33,CD1),(A3,R18,R21,R33,C D2),(A3,R18,R21,R33,CD3),(A3,R18,R21,R33,CD4),(A3,R18,R21,R33,CD5),(A3,R18,R21,R 33,CD6),(A3,R18,R21,R33,CD7),(A3,R18,R21,R34,CD1),(A3,R18,R21,R34,CD2),(A3,R18,R 21,R34,CD3),(A3,R18,R21,R34,CD4),(A3,R18,R21,R34,CD5),(A3,R18,R21,R34,CD6),(A3,R 18,R21,R34,CD7),(A3,R18,R21,R35,CD1),(A3,R18,R21,R35,CD2),(A3,R18,R21,R35,CD3),( A3,R18,R21,R35,CD4),(A3,R18,R21,R35,CD5),(A3,R18,R21,R35,CD6),(A3,R18,R21,R35,C D7),(A3,R18,R22,R31,CD1),(A3,R18,R22,R31,CD2),(A3,R18,R22,R31,CD3),(A3,R18,R22,R 31,CD4),(A3,R18,R22,R31,CD5),(A3,R18,R22,R31,CD6),(A3,R18,R22,R31,CD7),(A3,R18,R 22,R32,CD1),(A3,R18,R22,R32,CD2),(A3,R18,R22,R32,CD3),(A3,R18,R22,R32,CD4),(A3,R 18,R22,R32,CD5),(A3,R18,R22,R32,CD6),(A3,R18,R22,R32,CD7),(A3,R18,R22,R33,CD1),( A3,R18,R22,R33,CD2),(A3,R18,R22,R33,CD3),(A3,R18,R22,R33,CD4),(A3,R18,R22,R33,C D5),(A3,R18,R22,R33,CD6),(A3,R18,R22,R33,CD7),(A3,R18,R22,R34,CD1),(A3,R18,R22,R 34,CD2),(A3,R18,R22,R34,CD3),(A3,R18,R22,R34,CD4),(A3,R18,R22,R34,CD5),(A3,R18,R 22,R34,CD6),(A3,R18,R22,R34,CD7),(A3,R18,R22,R35,CD1),(A3,R18,R22,R35,CD2),(A3,R 18,R22,R35,CD3),(A3,R18,R22,R35,CD4),(A3,R18,R22,R35,CD5),(A3,R18,R22,R35,CD6),( A3,R18,R22,R35,CD7),(A3,R18,R23,R31,CD1),(A3,R18,R23,R31,CD2),(A3,R18,R23,R31,C D3),(A3,R18,R23,R31,CD4),(A3,R18,R23,R31,CD5),(A3,R18,R23,R31,CD6),(A3,R18,R23,R 31,CD7),(A3,R18,R23,R32,CD1),(A3,R18,R23,R32,CD2),(A3,R18,R23,R32,CD3),(A3,R18,R 23,R32,CD4),(A3,R18,R23,R32,CD5),(A3,R18,R23,R32,CD6),(A3,R18,R23,R32,CD7),(A3,R 18,R23,R33,CD1),(A3,R18,R23,R33,CD2),(A3,R18,R23,R33,CD3),(A3,R18,R23,R33,CD4),( A3,R18,R23,R33,CD5),(A3,R18,R23,R33,CD6),(A3,R18,R23,R33,CD7),(A3,R18,R23,R34,C D1),(A3,R18,R23,R34,CD2),(A3,R18,R23,R34,CD3),(A3,R18,R23,R34,CD4),(A3,R18,R23,R 34,CD5),(A3,R18,R23,R34,CD6),(A3,R18,R23,R34,CD7),(A3,R18,R23,R35,CD1),(A3,R18,R 23,R35,CD2),(A3,R18,R23,R35,CD3),(A3,R18,R23,R35,CD4),(A3,R18,R23,R35,CD5),(A3,R 18,R23,R35,CD6),(A3,R18,R23,R35,CD7),(A3,R18,R24,R31,CD1),(A3,R18,R24,R31,CD2),( A3,R18,R24,R31,CD3),(A3,R18,R24,R31,CD4),(A3,R18,R24,R31,CD5),(A3,R18,R24,R31,C D6),(A3,R18,R24,R31,CD7),(A3,R18,R24,R32,CD1),(A3,R18,R24,R32,CD2),(A3,R18,R24,R 32,CD3),(A3,R18,R24,R32,CD4),(A3,R18,R24,R32,CD5),(A3,R18,R24,R32,CD6),(A3,R18,R 24,R32,CD7),(A3,R18,R24,R33,CD1),(A3,R18,R24,R33,CD2),(A3,R18,R24,R33,CD3),(A3,R 18,R24,R33,CD4),(A3,R18,R24,R33,CD5),(A3,R18,R24,R33,CD6),(A3,R18,R24,R33,CD7),( A3,R18,R24,R34,CD1),(A3,R18,R24,R34,CD2),(A3,R18,R24,R34,CD3),(A3,R18,R24,R34,C D4),(A3,R18,R24,R34,CD5),(A3,R18,R24,R34,CD6),(A3,R18,R24,R34,CD7),(A3,R18,R24,R 35,CD1),(A3,R18,R24,R35,CD2),(A3,R18,R24,R35,CD3),(A3,R18,R24,R35,CD4),(A3,R18,R 24,R35,CD5),(A3,R18,R24,R35,CD6),(A3,R18,R24,R35,CD7),(A3,R18,R25,R31,CD1),(A3,R 18,R25,R31,CD2),(A3,R18,R25,R31,CD3),(A3,R18,R25,R31,CD4),(A3,R18,R25,R31,CD5),( A3,R18,R25,R31,CD6),(A3,R18,R25,R31,CD7),(A3,R18,R25,R32,CD1),(A3,R18,R25,R32,C D2),(A3,R18,R25,R32,CD3),(A3,R18,R25,R32,CD4),(A3,R18,R25,R32,CD5),(A3,R18,R25,R 32,CD6),(A3,R18,R25,R32,CD7),(A3,R18,R25,R33,CD1),(A3,R18,R25,R33,CD2),(A3,R18,R 25,R33,CD3),(A3,R18,R25,R33,CD4),(A3,R18,R25,R33,CD5),(A3,R18,R25,R33,CD6),(A3,R 18,R25,R33,CD7),(A3,R18,R25,R34,CD1),(A3,R18,R25,R34,CD2),(A3,R18,R25,R34,CD3),( A3,R18,R25,R34,CD4),(A3,R18,R25,R34,CD5),(A3,R18,R25,R34,CD6),(A3,R18,R25,R34,C D7),(A3,R18,R25,R35,CD1),(A3,R18,R25,R35,CD2),(A3,R18,R25,R35,CD3),(A3,R18,R25,R 35,CD4),(A3,R18,R25,R35,CD5),(A3,R18,R25,R35,CD6),(A3,R18,R25,R35,CD7),(A3,R18,R 26,R31,CD1),(A3,R18,R26,R31,CD2),(A3,R18,R26,R31,CD3),(A3,R18,R26,R31,CD4),(A3,R 18,R26,R31,CD5),(A3,R18,R26,R31,CD6),(A3,R18,R26,R31,CD7),(A3,R18,R26,R32,CD1),( A3,R18,R26,R32,CD2),(A3,R18,R26,R32,CD3),(A3,R18,R26,R32,CD4),(A3,R18,R26,R32,C D5),(A3,R18,R26,R32,CD6),(A3,R18,R26,R32,CD7),(A3,R18,R26,R33,CD1),(A3,R18,R26,R 33,CD2),(A3,R18,R26,R33,CD3),(A3,R18,R26,R33,CD4),(A3,R18,R26,R33,CD5),(A3,R18,R 26,R33,CD6),(A3,R18,R26,R33,CD7),(A3,R18,R26,R34,CD1),(A3,R18,R26,R34,CD2),(A3,R 18,R26,R34,CD3),(A3,R18,R26,R34,CD4),(A3,R18,R26,R34,CD5),(A3,R18,R26,R34,CD6),( A3,R18,R26,R34,CD7),(A3,R18,R26,R35,CD1),(A3,R18,R26,R35,CD2),(A3,R18,R26,R35,C D3),(A3,R18,R26,R35,CD4),(A3,R18,R26,R35,CD5),(A3,R18,R26,R35,CD6),(A3,R18,R26,R 35,CD7),(A3,R19,R21,R31,CD1),(A3,R19,R21,R31,CD2),(A3,R19,R21,R31,CD3),(A3,R19,R 21,R31,CD4),(A3,R19,R21,R31,CD5),(A3,R19,R21,R31,CD6),(A3,R19,R21,R31,CD7),(A3,R 19,R21,R32,CD1),(A3,R19,R21,R32,CD2),(A3,R19,R21,R32,CD3),(A3,R19,R21,R32,CD4),( A3,R19,R21,R32,CD5),(A3,R19,R21,R32,CD6),(A3,R19,R21,R32,CD7),(A3,R19,R21,R33,C D1),(A3,R19,R21,R33,CD2),(A3,R19,R21,R33,CD3),(A3,R19,R21,R33,CD4),(A3,R19,R21,R 33,CD5),(A3,R19,R21,R33,CD6),(A3,R19,R21,R33,CD7),(A3,R19,R21,R34,CD1),(A3,R19,R 21,R34,CD2),(A3,R19,R21,R34,CD3),(A3,R19,R21,R34,CD4),(A3,R19,R21,R34,CD5),(A3,R 19,R21,R34,CD6),(A3,R19,R21,R34,CD7),(A3,R19,R21,R35,CD1),(A3,R19,R21,R35,CD2),( A3,R19,R21,R35,CD3),(A3,R19,R21,R35,CD4),(A3,R19,R21,R35,CD5),(A3,R19,R21,R35,C D6),(A3,R19,R21,R35,CD7),(A3,R19,R22,R31,CD1),(A3,R19,R22,R31,CD2),(A3,R19,R22,R 31,CD3),(A3,R19,R22,R31,CD4),(A3,R19,R22,R31,CD5),(A3,R19,R22,R31,CD6),(A3,R19,R 22,R31,CD7),(A3,R19,R22,R32,CD1),(A3,R19,R22,R32,CD2),(A3,R19,R22,R32,CD3),(A3,R 19,R22,R32,CD4),(A3,R19,R22,R32,CD5),(A3,R19,R22,R32,CD6),(A3,R19,R22,R32,CD7),( A3,R19,R22,R33,CD1),(A3,R19,R22,R33,CD2),(A3,R19,R22,R33,CD3),(A3,R19,R22,R33,C D4),(A3,R19,R22,R33,CD5),(A3,R19,R22,R33,CD6),(A3,R19,R22,R33,CD7),(A3,R19,R22,R 34,CD1),(A3,R19,R22,R34,CD2),(A3,R19,R22,R34,CD3),(A3,R19,R22,R34,CD4),(A3,R19,R 22,R34,CD5),(A3,R19,R22,R34,CD6),(A3,R19,R22,R34,CD7),(A3,R19,R22,R35,CD1),(A3,R 19,R22,R35,CD2),(A3,R19,R22,R35,CD3),(A3,R19,R22,R35,CD4),(A3,R19,R22,R35,CD5),( A3,R19,R22,R35,CD6),(A3,R19,R22,R35,CD7),(A3,R19,R23,R31,CD1),(A3,R19,R23,R31,C D2),(A3,R19,R23,R31,CD3),(A3,R19,R23,R31,CD4),(A3,R19,R23,R31,CD5),(A3,R19,R23,R 31,CD6),(A3,R19,R23,R31,CD7),(A3,R19,R23,R32,CD1),(A3,R19,R23,R32,CD2),(A3,R19,R 23,R32,CD3),(A3,R19,R23,R32,CD4),(A3,R19,R23,R32,CD5),(A3,R19,R23,R32,CD6),(A3,R 19,R23,R32,CD7),(A3,R19,R23,R33,CD1),(A3,R19,R23,R33,CD2),(A3,R19,R23,R33,CD3),( A3,R19,R23,R33,CD4),(A3,R19,R23,R33,CD5),(A3,R19,R23,R33,CD6),(A3,R19,R23,R33,C D7),(A3,R19,R23,R34,CD1),(A3,R19,R23,R34,CD2),(A3,R19,R23,R34,CD3),(A3,R19,R23,R 34,CD4),(A3,R19,R23,R34,CD5),(A3,R19,R23,R34,CD6),(A3,R19,R23,R34,CD7),(A3,R19,R 23,R35,CD1),(A3,R19,R23,R35,CD2),(A3,R19,R23,R35,CD3),(A3,R19,R23,R35,CD4),(A3,R 19,R23,R35,CD5),(A3,R19,R23,R35,CD6),(A3,R19,R23,R35,CD7),(A3,R19,R24,R31,CD1),( A3,R19,R24,R31,CD2),(A3,R19,R24,R31,CD3),(A3,R19,R24,R31,CD4),(A3,R19,R24,R31,C D5),(A3,R19,R24,R31,CD6),(A3,R19,R24,R31,CD7),(A3,R19,R24,R32,CD1),(A3,R19,R24,R 32,CD2),(A3,R19,R24,R32,CD3),(A3,R19,R24,R32,CD4),(A3,R19,R24,R32,CD5),(A3,R19,R 24,R32,CD6),(A3,R19,R24,R32,CD7),(A3,R19,R24,R33,CD1),(A3,R19,R24,R33,CD2),(A3,R 19,R24,R33,CD3),(A3,R19,R24,R33,CD4),(A3,R19,R24,R33,CD5),(A3,R19,R24,R33,CD6),( A3,R19,R24,R33,CD7),(A3,R19,R24,R34,CD1),(A3,R19,R24,R34,CD2),(A3,R19,R24,R34,C D3),(A3,R19,R24,R34,CD4),(A3,R19,R24,R34,CD5),(A3,R19,R24,R34,CD6),(A3,R19,R24,R 34,CD7),(A3,R19,R24,R35,CD1),(A3,R19,R24,R35,CD2),(A3,R19,R24,R35,CD3),(A3,R19,R 24,R35,CD4),(A3,R19,R24,R35,CD5),(A3,R19,R24,R35,CD6),(A3,R19,R24,R35,CD7),(A3,R 19,R25,R31,CD1),(A3,R19,R25,R31,CD2),(A3,R19,R25,R31,CD3),(A3,R19,R25,R31,CD4),( A3,R19,R25,R31,CD5),(A3,R19,R25,R31,CD6),(A3,R19,R25,R31,CD7),(A3,R19,R25,R32,C D1),(A3,R19,R25,R32,CD2),(A3,R19,R25,R32,CD3),(A3,R19,R25,R32,CD4),(A3,R19,R25,R 32,CD5),(A3,R19,R25,R32,CD6),(A3,R19,R25,R32,CD7),(A3,R19,R25,R33,CD1),(A3,R19,R 25,R33,CD2),(A3,R19,R25,R33,CD3),(A3,R19,R25,R33,CD4),(A3,R19,R25,R33,CD5),(A3,R 19,R25,R33,CD6),(A3,R19,R25,R33,CD7),(A3,R19,R25,R34,CD1),(A3,R19,R25,R34,CD2),( A3,R19,R25,R34,CD3),(A3,R19,R25,R34,CD4),(A3,R19,R25,R34,CD5),(A3,R19,R25,R34,C D6),(A3,R19,R25,R34,CD7),(A3,R19,R25,R35,CD1),(A3,R19,R25,R35,CD2),(A3,R19,R25,R 35,CD3),(A3,R19,R25,R35,CD4),(A3,R19,R25,R35,CD5),(A3,R19,R25,R35,CD6),(A3,R19,R 25,R35,CD7),(A3,R19,R26,R31,CD1),(A3,R19,R26,R31,CD2),(A3,R19,R26,R31,CD3),(A3,R 19,R26,R31,CD4),(A3,R19,R26,R31,CD5),(A3,R19,R26,R31,CD6),(A3,R19,R26,R31,CD7),( A3,R19,R26,R32,CD1),(A3,R19,R26,R32,CD2),(A3,R19,R26,R32,CD3),(A3,R19,R26,R32,C D4),(A3,R19,R26,R32,CD5),(A3,R19,R26,R32,CD6),(A3,R19,R26,R32,CD7),(A3,R19,R26,R 33,CD1),(A3,R19,R26,R33,CD2),(A3,R19,R26,R33,CD3),(A3,R19,R26,R33,CD4),(A3,R19,R 26,R33,CD5),(A3,R19,R26,R33,CD6),(A3,R19,R26,R33,CD7),(A3,R19,R26,R34,CD1),(A3,R 19,R26,R34,CD2),(A3,R19,R26,R34,CD3),(A3,R19,R26,R34,CD4),(A3,R19,R26,R34,CD5),( A3,R19,R26,R34,CD6),(A3,R19,R26,R34,CD7),(A3,R19,R26,R35,CD 1),(A3,R19,R26,R35,C D2),(A3,R19,R26,R35,CD3),(A3,R19,R26,R35,CD4),(A3,R19,R26,R35,CD5),(A3,R19,R26,R 35,CD6) or (A3,R19,R26,R35,CD7).

The compound of the present invention is useful against diseases induced by production, secretion or deposition of amyloid-β proteins. For example, the compound is effective for treating and/or preventing, and ameliorating symptoms of diseases such as dementia of the Alzheimer's type (e.g. Alzheimer's disease and senile dementia of the Alzheimer's type), Down syndrome, memory disorder, prion diseases (e.g. Creutzfeldt-Jakob disease), mild cognitive impairment (MCI), hereditary cerebral hemorrhage with amyloidosis-Dutch type, cerebral amyloid angiopathy, other degenerative dementia, vascular and degenerative mixed dementia, dementia associated with Parkinson's disease, dementia associated with progressive supranuclear palsy, dementia associated with corticobasal degeneration, Alzheimer's disease with diffuse Lewy bodies, age-related macular degeneration, Parkinson's disease and amyloid angiopathy.

The compound of the present invention has a high inhibitory activity on BACE1 and/or high selectivity against other enzymes. Thus, the compound can be used as pharmaceuticals with reduced side effects. In addition, the compound of the present invention has various advantages such as high metabolic stability, high solubility, high oral absorbability, high bioavailability, good clearance, high brain transferability, a long half life, high protein unbinding ratio, low hERG channel inhibition, low CYP inhibition and /or negative in Ames test.

When the compound according to the present invention is administered, other medicaments (e.g. other therapeutic agents for Alzheimer's disease such as acetylcholine esterase) may be used in combination. For example, anti-dementia drugs such as donepezil hydrochloride, tacrine, galantamine, rivastigmine, zanapezil, memantine and vinpocetine may be used in combination.
When the compound according to the present invention is administered to humans, it may be orally administered as powders, granules, tablets, capsules, pills, liquids and the like, or may be parenterally administered as injections, suppositories, transdermal systems, inhalant and the like. In addition, if needed, an effective amount of the present compound may be mixed with pharmaceutical additives such as diluents, binders, humectants, disintegrants and lubricants suitable for its dosage form, and thereby the compound may be formed into a pharmaceutical preparation.
The dosage depends on disease conditions, the route of administration, the age or the weight of a patient. In the case of oral administration to adults, the dosage is generally 0.1µg to 1 g/day, and preferably 0.01 to 500 mg/day. In the case of parenteral administration, the dosage is generally 1 µg to 10 g/day, and preferably 0.1 to 5 g/day.

### [Examples]

The present invention will be described in more detail with reference to, but not limited to, the following examples and test examples.
In the examples, meaning of each abbreviation is as follows.
Me: methyl
Et: ethyl
Ph: phenyl
Bz:benzoyl
Bn: benzyl
Boc: t-butoxycarbonyl
Ms: methanesulfonyl
HATU: O-(7-azabenzotriazole-1-yl)-N,N,N',N'-tetramethyluronium hexafluorophosphate
WSCD: water soluble carbodiimide (1-ethyl -3-(3-dimethyl amino propyl)carbodiimide)
THF: tetrahydrofuran
DMF: dimethyl formamide

### Example 1

### Synthesis of Compound 3

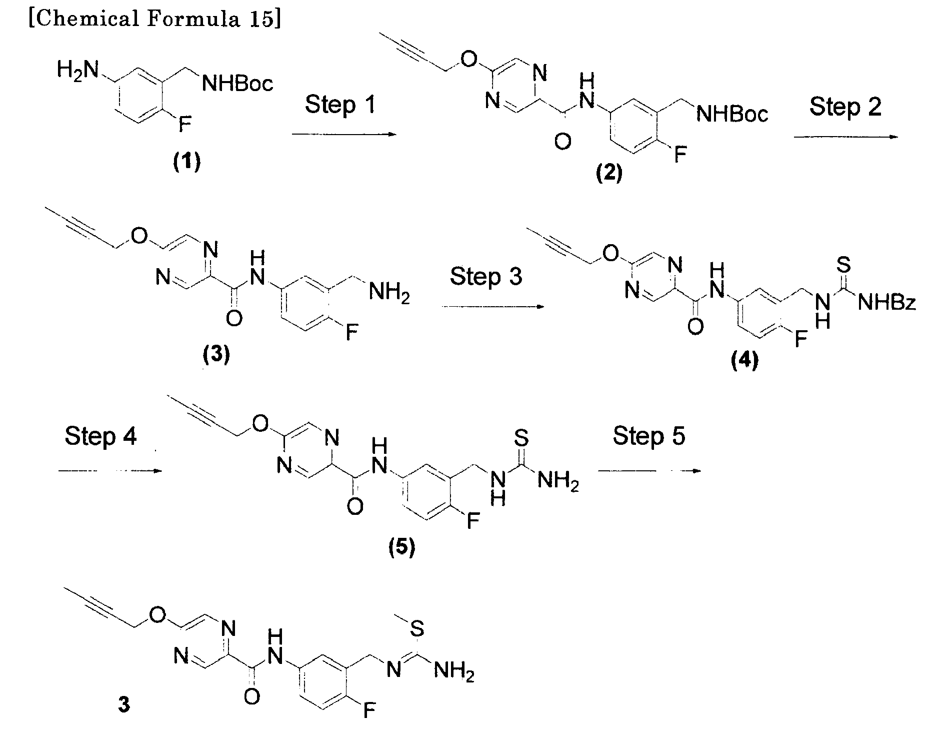

### Step 1

Compound (1) (667 mg), HATU (1369 mg) and 5-(2-butynyl)pyrazine-2-carboxylic acid (749 mg) were dissolved in N,N-dimethylformamide(10 ml) and triethylamine(628 µl) and stirred at room temperature for 2 hours. Ethyl acetate was added to the solution, washed with potassium carbonate and brine, and dried over magnesium sulfate. The solvent was removed under reduced pressure and the residue was washed with methanol to afford Compound (2)(563 mg). ¹ H - NMR (DMSO-d₆) δ: 1.40 (9H, s), 1.85 (3H, s), 4.13 (2H, d, J = 5.56 Hz), 5.09 (2H, d, J = 2.02 Hz), 7.00 (1H, d, J = 7.58 Hz), 7.30 (1H, t, J = 7.83 Hz), 7.37-7.40 (1H, m), 7.69 (1H, d, J = 7.58 Hz), 7.79 (1H, s), 8.44 (1H, s), 8.89 (1H, s), 10.44 (1H, s).

### Step 2

Compound (2) (382 mg) was dissolved in chloroform (16 ml), trifluoroacetic acid (2 ml) was added at room temperature, and the mixture was stirred at room temperature for 2 hours. The solvent was removed under reduced pressure, ethyl acetate was added and the mixture was washed with potassium carbonate and brine. After being removed the solvent, the solid was collected by filtration to afford Compound (3)(207 mg). ¹ H - NMR (DMSO-d₆) δ: 1.85 (3H, s), 3.71 (2H, s), 5.09 (2H, d, J = 2.02 Hz), 7.10 (1H, d, J = 7.58 Hz), 7.28 (1H, t, J = 7.58 Hz), 7.69 (1H, d, J = 8.08 Hz), 7.81 (1H, s), 8.44 (1H, s), 8.89 (1H, s), 10.37 (1H, s).

### Step 3

Compound (3)(200 mg) was suspended in acetone(4 ml), benzoylisothiocyanate(100 µl) was addled at room temperature and the mixture was stirred for 90 minutes. The precipitated solid was collected by filtration to afford Compound (4)(180 mg).
¹ H - NMR (DMSO-d₆) δ: 1.85 (3H, s), 4.90 (2H, d, J = 5.56 Hz), 5.09 (2H, d, J = 2.02 Hz), 7.15 (1H, d, J = 7.58 Hz), 7.36 (1H, t, J = 7.83 Hz), 7.52 (2H, t, J = 7.83 Hz), 7.64 (1H, t, J = 7.33 Hz), 7.80 (1H, d, J = 9.09 Hz), 7.94 (3H, t, J = 8.08 Hz), 8.44 (1H, s), 8.89 (1H, s), 10.51 (1H, s), 11.25-11.28 (1H, m), 11.44 (1H, s).

### Step 4

Compound (4)(171 mg) was suspended in methanol (5 ml), an aqueous solution of sodium hydroxide (2 mol/1, 205 µl) was added at room temperature and the mixture was stirred for 3.5 hours. The precipitated solid was collected by filtration to afford Compound (5)(114 mg). ¹ H - NMR (DMSO-d₆) δ: 1.86 (3H, s), 4.63 (2H, br s), 5.09 (2H, d, J = 2.02 Hz), 7.05 (1H, d, J = 7.58 Hz), 7.09 (1H, br s), 7.32 (1H, t, J = 8.08 Hz), 7.72 (1H, d, J = 7.07 Hz), 7.83 (1H, s), 8.00 (1H, br s), 8.44 (1H, s), 8.90 (1H, s), 10.46 (1H, s).

### Step 5

A solution (2 ml) of methane iodide (23.9 mg) in acetonitorile was added to Compound (5)(50 mg) at room temperature and the mixture was heated at 60°C for about 5 hours. The solvent was removed under reduced pressure and ethyl acetate was added. The mixture was washed with potassium carbonate and brine, dried over magnesium sulfate and the solvent was removed under reduces pressure. The resulting residue was purified by chromatography to afford Compound 3 (37.6 mg). ¹ H - NMR (DMSO-d₆) δ: 1.86 (3H, br s), 2.28 (3H, s), 4.30 (2H, br s), 5.09 (2H, d, J = 2.03 Hz), 7.07-7.09 (1H, m), 7.28 (1H, t, J = 7.86 Hz), 7.68 (1H, d, J = 8.11 Hz), 7.84 (1H, s), 8.44-8.45 (1H, m), 8.89-8.90 (1H, m), 10.41 (1H, s).

### Example 2

### Synthesis of Compound 15

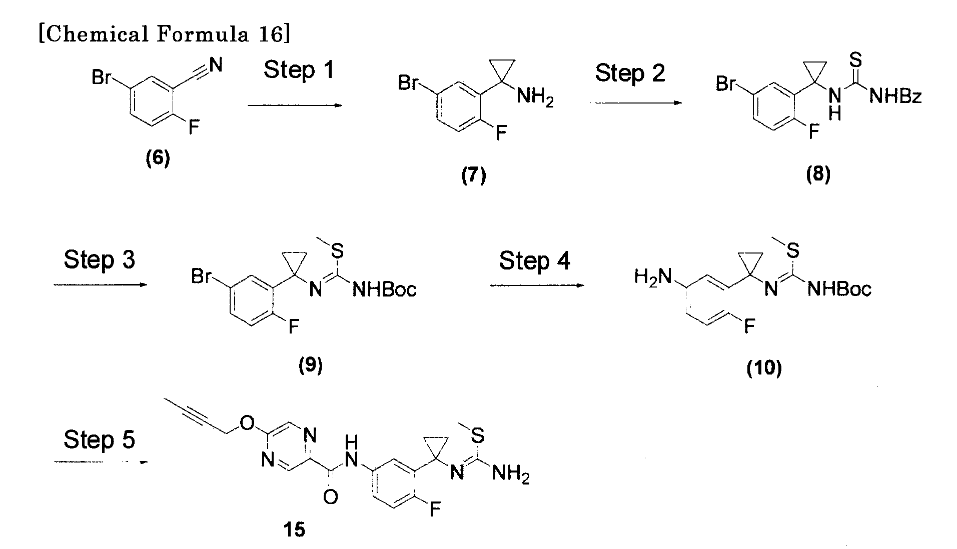

### Step 1

Compound (6)(800 mg) was dissolved in diethyl ether (20 ml). Tetraisopropoxytitanium (1.32 ml) and a solution of ethylmagnesium bromide in tetrahydrofuran (1 mol/l, 8.80 ml) to the mixture at -78°C under nitrogen atmosphere. The mixture was stirred for 10 minutes, allowed to warm to room temperature and stirred for 50 minutes. The mixture was cooled with ice and diethyl ether complex of trifluoroborane (1.00 ml) was added. The mixture was allowed to warm to room temperature and stirred for 60 minutes. The mixture was cooled with ice, an aqueous solution of hydrochloric acid and extracted with diethyl ether. The aqueous layer was neutralized with an aqueous solution of sodium hydroxide and extracted with diethyl ether. The organic layer was washed with brine, dried over magnesium sulfate and the solvent was removed under reduced pressure to afford Compound (7)(389 mg). ¹ H - NMR (CDCl₃) δ: 0.88-0.91 (2H, m), 1.00-1.02 (2H, m), 1.97 (2H, br s), 6.90-6.95 (0H, m), 7.30-7.34 (1H, m), 7.42 (1H, dd, J = 6.59, 2.53 Hz).

### Step 2

Compound (7)(280 mg) was dissolved in acetone (6 ml). Benzoyl isothiocyanate (66µl) was added to the mixture at room temperature and the mixture was stirred for 90 minutes. The solvent was removed under reduced pressure and the residue was purified by chromatography to afford Compound (8) (402 mg). ¹ H - NMR (DMSO-d₆) δ: 1.39 (4H, br s), 7.19 (1H, t, J = 9.09 Hz), 7.48-7.52 (3H, m), 7.61-7.65 (1H, m), 7.76-7.77 (1H, m), 7.90 (2H, d, J = 7.58 Hz), 11.36 (1H, br s), 11.50 (1H, s).

### Step 3

Compound (8) (200 mg) was dissolved in methanol (4 ml). An aqueous solution of sodium hydroxide (2 mol/l, 280 µl) was added to the mixture at room temperature and the mixture was stirred for 60 minutes. The solvent was removed under reduced pressure and the residue was extracted with ethyl acetate. The organic layer was washed with brine, dried over magnesium sulfate and the solvent was removed under reduced pressure. The resulting residue was dissolved in acetonitrile (4 ml) and methane iodide (108 mg) was added to the mixture at room temperature. The mixture was allowed to warm to 60°C and stirred for 90 minutes. The solvent was removed under reduced pressure and the resulting residue was dissolved in methanol (4 ml). Triethylamine (106 µl) and di-tert-butyl dicarbonate (Boc₂ O) (177 µl) were added to the mixture at room temperature and the mixture was stirred for 50 minutes. The solvent was removed under reduced pressure, ethyl acetate was added and insoluble residue was filtered off. The solvent was removed again under reduced pressure and the resulting residue was purified by chromatography to afford Compound (9) (184 mg).
¹ H - NMR (DMSO-d₆) δ: 1.32-1.41 (4H, m), 1.40 (9H, s), 2.29 (3H, s), 7.18-7.22 (1H, m), 7.50-7.54 (1H, m), 7.61-7.65 (1H, m).

### Step 4

Compound (9) (180 mg), tris(dibenzylidene acetone)di palladium (Pd₂(dba)₃ (61 mg) and butyl di-1-adamantyl phosphine(50 mg) were dissolved in toluene (3 ml), and stirred at room temperature under nitrogen atmosphere for 10 minutes. Then a solution of lithium hexamethyl disilazide (LHMDS) in tetrahydrofuran (1 mol/l, 1.33 ml) was added to the mixture and stirred at 80°C for about 3 hours. Then the mixture was cooled with ice, diethyl ether (5 ml) and an aqueous solution of hydrochloric acid (1 mol/1, 2.67 ml) were added to the mixture and stirred for 15 minutes. The mixture was neutralized with an aqueous solution of potassium carbonate and extracted with ethyl acetate. The organic layer was wahed with brine and dried over magnesium sulfate. The solvent was removed under reduced pressure and the residue was purified with chromatography to afford Compound (10) (41 mg).
¹ H - NMR (DMSO-d₆) δ: 1.25 (4H, d, J = 23.24 Hz), 1.40 (9H, s), 2.28 (3H, s), 4.96 (2H, br s), 6.42-6.46 (1H, m), 6.58-6.62 (1H, m), 6.79-6.85 (1H, m), 10.22 (1H, br s).

### Step 5

Compound (10) (40 mg), HATU (30.8 mg) and 5-(2-butynyl)pyrazine -2-carboxylic acid (15.5 mg) were dissolved in N,N-dimethylformamide (1 ml) and triethylamine (12 µl) and the mixture was stirred at room temperature for 2 hours. Ethyl acetate was poured to the mixture, the organic layyer was washed with potassium carbonate and brine, and dried over magnesium sulfate. The solvent was removed under reduced pressure, and the resulting residue was purified with chromatography. The obtained material was dissolved in dichloromethane (1 ml), trifluoroacetic acid (0.2 ml) was added to the mixture at room temperature. The mixture was stirred at room temperature for about 3 hours. Then the solvent was removed under reduced pressure and ethyl acetate was added. The organic layer was washed with aq.potassium carbonate and brine.and the residue was purified with chromatography to afford Compound 15 (14.9 mg).
¹ H - NMR (DMSO-d₆) δ: 1.04 (2H, br s), 1.14 (2H, s), 1.86 (3H, s), 2.19 (3H, s), 5.10 (2H, s), 7.09-7.13 (1H, m), 7.74-7.79 (1H, m), 7.98-8.04 (1H, m), 8.45 (1H, s), 8.90 (1H, s), 10.49 (1H, s).

### Example 3

### Synthesis of Compound 20

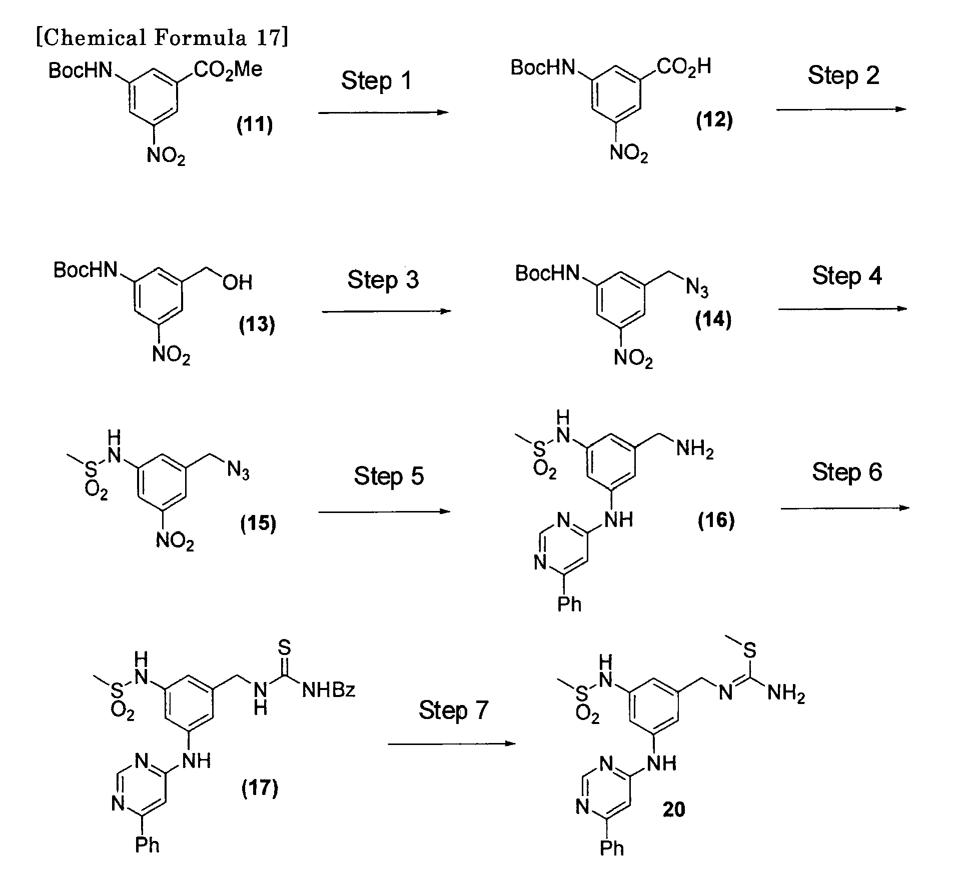

### Step 1

Compound (11) (2000 mg) was suspended in methanol (10 ml) and tetrahydrofuran (10 ml). 2 mol/l aqueous solution of sodiumu hydroxide (4.05 ml) was added the mixture and the mixture was stirred at room temperature for 2 hours. 2 mol/l aqueous solution of hydrochloric acid (4.1 ml) was added to the mixture and the solvent was removed under reduced pressure. The precipitated solid was collected by filtration to afford Compound (12) (2200 mg).
¹ H - NMR (DMSO-d₆) δ: 1.51 (9H, s), 8.21-8.22 (1H, m), 8.42 (1H, s), 8.63 (1H, s), 10.12 (1H, s), 13.66 (1H, br s).

### Step 2

Compound (12) (900 mg) was dissolved in tetrahydrofuran (9 ml). A solution of borane dimethyl sulfide complex in tetrahydrofuran (1.9 mol/l, 2.5 ml) was added to the mixture and the mixture was heated with stirring at 50°C for about 3 hours. Then a solution of borane dimethyl sulfide complex in tetrahydrofuran (1.9 mol/l, 2.0 ml) was added to the mixture again and the mixture was heated with stirring at 50°C for about additional 3 hours.
Methanol (3 ml) was added to the mixture and the solvent was removed under reduced pressure. After addition of ethyl acetate, the organic layer was wahed with aq.sodium carbonate and brine, dried over magnesium sulfate and the solvent was removed under reduced pressure. The resulting residue was purified by chromatography to afford Compound (13) (506.3 mg).
¹ H - NMR (DMSO-d₆) δ: 1.50 (9H, s), 4.56 (2H, d, J = 5.58 Hz), 5.49 (1H, t, J = 5.83 Hz), 7.79 (2H, d, J = 9.12 Hz), 8.30 (1H, s), 9.89 (1H, s).

### Step 3

Compound (13)(663 mg) and triphenyl phosphine (778 mg) were dissolved in N,N-dimethyl formamide (3.3 ml). Carbon tetrabromide (984 mg) was added to the mixture and the mixture was stirred under nitrogen atmosphere at room temperature for about 2 hours. Then sodium azide (321 mg) was added to the mixture and the mixture was stirred at room temperature for about 5 hours. Ethyl acetate was added to the reaction mixture and the organic layer was washed with aq.sodium carbonate and brine. The organic layer was dried over magnesium sulfate and the solvent was removed under reduced pressure. The resulting residue was purified by chromatography to afford Compound (14) (579 mg).
¹ H - NMR (DMSO-d₆) δ: 1.50 (9H, s), 4.62 (2H, s), 7.84 (2H, s), 8.38 (1H, s), 9.99 (1H, s).

### Step 4

Compound (14) (300 mg) was dissolved in dichloromethane (4 ml). trifluoroacetic acid(1 ml) was added to the mixture and the mixture was stirred at room temperature for about 2 hours. Then the solvent was removed under reduced pressure, ethyl acetate was added to the mixture. The organic layer was washed with potassium carbonate and brine. The resulting residue was dissolved in dichloromethane (2 ml) and pyridine (0.7 ml). Methanesulfonyl chloride (218 mg) was added to the mixture under cooling with ice and the mixture was stirred at room temperature for about 4 hours. Ethyl acetate was added to the mixture and the organic layer was washed with 1 mol/l hydrochloride and brine. The organic layer was dried over magnesium sulfate and the solvent was removed under reduced pressure. The resulting residue was purified by chromatography to afford Compound (15) (195 mg).
¹ H - NMR (CDCl₃) δ: 3.14 (3H, s), 4.52 (2H, s), 5.30 (1H, s), 7.54 (1H, s), 8.00 (2H, s), 8.02 (2H, t, J = 1.77 Hz).

### Step 5

Compound (15)(195 mg) was dissolved in methanol (5 ml). 10% palladium carbon powders (containing 51.84% H₂O, 100 mg) was added, to the mixture and the mixture was stirred at room temperature under hydrogen atmosphere overnight. The reaction mixture was filtrated and the solvent was removed under reduced pressure. The residue was suspended in isopropanol (3.24 ml) and conc. sulfuric acid (0.195 ml), and reacted in a microwave reactor (100°C, 15 minutes). Ethyl acetate was added to the mixture and an insoluble material was collected by filtration to afford sulfate of Compound (16) (270.5 mg). Ethyl acetate was added to a portion of the material (166 mg) and the organic layer was washed with aq.sodium carbonate and brine, and dried over magnesium sulfate. The solvent was removed under reduced pressure to afford Compound (16) (105 mg).
¹ H - NMR (DMSO-d₆) δ: 3.01 (3H, s), 3.70 (2H, s), 6.86 (1H, s), 7.28 (1H, s), 7.44 (2H, d, J = 8.62 Hz), 7.52-7.54 (3H, m), 8.01-8.04 (2H, m), 8.71 (1H, s), 9.71 (1H, s).

### Step 6

Compound (16) (105 mg) was dissolved in acetone (5 ml). Benzoylisothiocyanate (56.8µl) was added to the mixture at room temperature and the mixture was stirred for about 4 hours. The solvent was removed under reduced pressure and the resulting residue was purified by chromatography to afford Compound (17) (62.1 mg).
¹ H - NMR (DMSO-d₆) δ: 3.07 (3H, s), 4.87-4.90 (2H, m), 6.91 (1H, s), 7.30 (1H, s), 7.49-7.65 (7H, m), 7.95 (2H, d, J = 7.07 Hz), 8.02-8.05 (2H, m), 8.71 (1H, s), 9.82 (1H, s), 9.88 (1H, s), 11.29 (1H, br s), 11.47 (1H, s).

### Step 7

Compound (17) (62 mg) was suspended in methanol (2 ml). An aqueous solution of sodium hydroxide (2 mol/l, 70 µl) was added to the mixture at room temperature and the mixture was stirred for about 3.5 hours. Then the solvent was removed under reduced pressure and ethyl acetate was added. The organic layer was washed with aq.potassium carbonate and brine, dried over magnesium sulfate, and the solvent was removed under reduced pressure. a solution (2 ml) of iodomethane (19.8 mg) in acetonitrile was poured to the residue at room temperature and the mixture was heated at 60°C for about 5 hours. Then the solvent was removed under reduced pressure and ethyl acetate was added. The orgainc layer was washed with aq.potassium carbonate and brine, dried over magnesium sulfate and the solvent was removed under reduced pressure to afford Compound 20 (45.1 mg).
¹ H - NMR (DMSO-d₆) δ: 2.30 (3H, s), 3.02 (3H, s), 4.26 (2H, s), 6.89 (1H, br s), 7.28-7.29 (1H, m), 7.42-7.57 (5H, m), 8.01-8.04 (2H, m), 8.70 (1H, s), 9.72 (1H, s).

### Example 4

### Synthesis of Compound 24

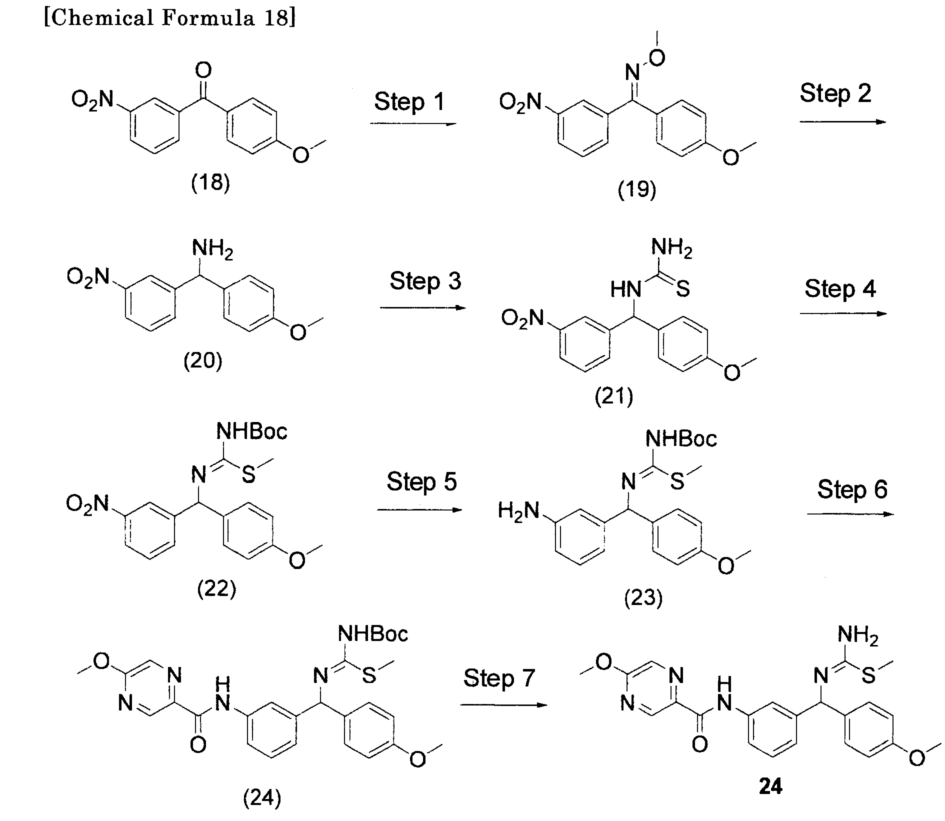

### Step 1

A known Compound (18) (10.32 g) was dissolved in pyridine (100 mL). O-methyl hydroxyamine hydrochloride(5.36 g) was added to the mixture at room temperature and the mixture was stirred at 60°C for about 3 hours. Then the solvent was removed under reduced pressure, diethyl ether was added. The organic layer was washed with water and brine, and dried over sodium sulfate. The solvent was removed under reduced pressure to afford crude Compound (19) (12 g).

### Step 2

The crude Compound (19)(556 mg) was dissolved in tetrahydrofuran (5.5 mL). Borane/tetrahydrofuran solution (1 mol/L:4.66 mL) was added to the mixture at room temperature and the mixture was stirred at 70°C for 7 hours. After cooling to 0°C, water (5 mL) and 2 mol/l aqueous solution of sodium hydroxide (10 mL) were added to the mixture and the mixture was stirred at 70°C overnight. After cooling to room temperature, the mixture was extracted with hexane The organic layer was washed with brine and dried over sodium sulfate. The resulting residue was purified by chromatography to afford Compound (20) (330 mg).
¹ H - NMR (DMSO-d₆) δ: 8.30 (1H, s), 8.04 (1H, d, J = 7.83 Hz), 7.82 (1H, d, J = 7.83 Hz), 7.57 (1H, t, J = 7.83 Hz), 7.33 (2H, d, J = 8.34 Hz), 6.87 (2H, d, J = 8.34 Hz), 5.21 (1H, s), 3.71 (3H, s).

### Step 3

Compound (20)(215 mg) was dissolved in tetrahydrofuran (2 mL). Benzoylisothiocyanate (0.118 mL) was added to the mixture at 0°C and the mixture was stirred for 2.5 hours. Then the mixture was allwed to warm to room temperature. Methanol (1 mL) and 1 mol/l aqueous solution of sodium hydroxide (0.916 mL) was added to the mixture and the mixture was stirred at 50°C for 1 hour. Tetrahydrofuran was removed under reduced pressure, the precipitated solid was collected by filtration and washed with water to afford Compound (21) (233 mg).
¹ H - NMR (DMSO-d₆) δ: 8.64 (1H, d, J = 8.34 Hz), 8.13 (1H, d, J = 8.34 Hz), 8.08 (1H, s), 7.75-7.60 (2H, m), 7.20 (2H, d, J = 7.58 Hz), 6.94 (2H, d, J = 7.58 Hz), 6.67 (1H, br s), 3.74 (3H, s).

### Step 4

Compound (21) (339 mg) was dissolved in acetonitrile (6 mL). Methane iodide (0.08 mL) was added to the mixture at room temperature, and the mixture was stirred at 50°C for 3.5 hours. The reaction mixture was allowed to cool to room temperature, methane iodide (0.02 mL) was added and the mixture was stirred at 50°C for 1 hour. The solvent was removed under reduced pressure. Methanol (3 mL), triethylamine (0.222 mL), and di-tert-butyldicarbonate (Boc₂ O) (0.297 mL) were added to resulting residue at room temperature and the mixture was stirred for 3.5 hours. The solvent was removed under reduced pressure and the residue was purified by chromatography to afford Compound (22) (406 mg).
¹ H - NMR (DMSO-d₆) δ: 8.19 (1H, s), 8.15 (1H, d, J = 7.07 Hz), 7.80 (1H, d, J = 7.07 Hz), 7.70-7.66 (1H, m), 7.28 (2H, d, J = 7.33 Hz), 6.95 (2H, d, J = 7.33 Hz), 6.33 (1H, br s), 3.74 (3H, s), 2.42 (3H, s), 1.39 (9H, s).

### Step 5

Compound (22) (100 mg) was dissolved in methanol (1 mL). Tetrahydrofuran (1 mL) and water (0.5 mL), ammonium chloride (49.6 mg) and reduced iron (51.8 mg) were added to the mixture at room temperature, and the mixture was stirred at 70°C for 2.5 hours. The mixture was allowed to cool to room temperature, ethyl acetate was added and the precipitated insoluble material was filtered off. The filtrate was washed with brine to afford crude Compound (23) (88 mg).

### Step 6

Compound (23) (88.4 mg), HATU (100 mg) and 5-methoxypyrazine-2-caboxylic acid (37.3 mg) were dissolved in N,N-dimethylformamide (2 mL). Triethylamine (0.046 mL) was added to the mixture at room temperature, and the mixture was stirred for 2 hours. An aqueous solution of ammonium chloride was added and the mixture was extracted with ethyl acetate. The organic layer was washed with water and brine, and dried over sodium sulfate. The solvent was removed under reduced pressure and the residue was purified by chromatography to afford Compound (24) (84 mg).
¹ H - NMR (CDCl₃) δ: 10.47 (1H, s), 9.51 (1H, s), 9.01 (1H, s), 8.14 (1H, s), 7.83 (1H, d, J = 7.07 Hz), 7.52 (1H, s), 7.37 (1H, t, J = 7.07 Hz), 7.20 (2H, d, J = 8.34 Hz), 7.06 (1H, d, J = 7.07 Hz), 6.88 (2H, d, J = 8.34 Hz), 5.93 (1H, s), 4.06 (3H, s), 3.79 (3H, s), 2.44 (3H, s), 1.51 (9H, s).

### Step 7

Compound (24) (84 mg) was dissolved in chloroform (1 mL). Trifluoroacetic acid (1 mL) was added to the mixture at room temperatur, and the mixture was stirred for 1.5 hours. The solvent was removed under reduced pressure, then an aqueous solution of sodium carbonate was added. The aqueous layer was extracted with ethyl acetate. The organic layer was washed with brine and dried over sodium sulfate. The solvent was removed under reduced pressure and the precipitated solid was washed with diisopropyl ether to afford Compound 24 (44 mg).
¹ H - NMR (CDCl₃) δ: 9.48 (1H, s), 9.00 (1H, s), 8.14 (1H, s), 7.75 (1H, d, J = 7.83 Hz), 7.60 (1H, s), 7.34 (1H, t, J = 7.83 Hz), 7.25-7.22 (2H, m), 7.12-7.09 (1H, m), 6.85 (2H, d, J = 7.33 Hz), 6.02 (1H, s), 4.06 (3H, s), 3.78 ( 3H, s), 2.37 (3H, s).

### Example 5

### Synthesis of Compound 26

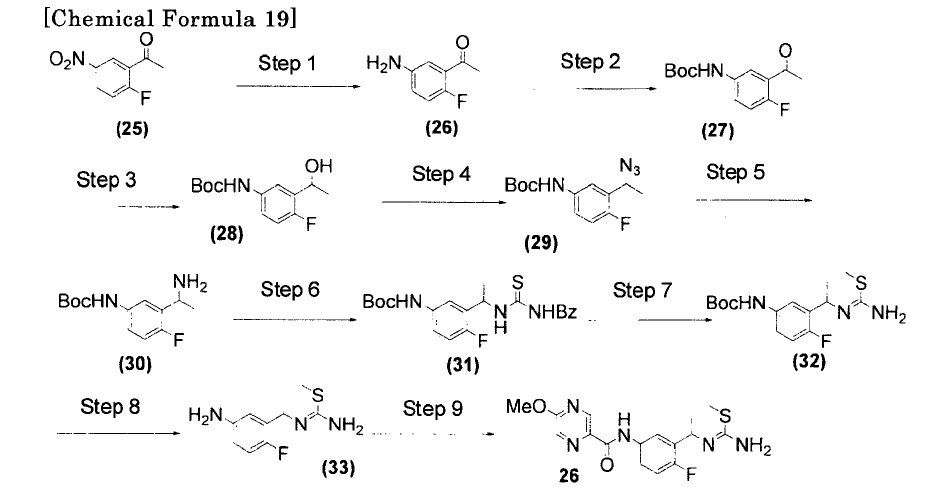

### Step 1

Compound (25) (3.02 g) was dissolved in ethanol (40 ml), water (14 ml) and tetrahydrofuran (8.4 ml). Ammonium chloride (0.88 g) was added to the mixture at room temperature and the mixture was stirred at 70°C for 10 minutes. After iron (3.68 g) was added, the mixture was stirred for 30 minutes at the same temperature, then allowed to warm to room temperature and stirred for additional 1.5 hours. The solvent was concentrated under reduced pressure, ethyl acetate was added and the organic layer was washed with an aqueous solution of sodium carbonate and brine. The solvent was removed and the resulting solid was purified by recrystallization to afford Compound (26) (1.82 g).
¹ H - NMR (CDCl₃) δ: 2.61 (dd, J = 5.1, 0.4 Hz, 3H), 3.65 (br s, 2H), 6.80 (dt, J = 8.6, 3.5 Hz, 1H), 6.93 (dd, J = 10.4, 8.8 Hz, 1H), 7.12 (dd, J = 5.9, 3.1 Hz, 1H).

### Step 2

Compound (26) (1.82g) was dissolved in dichloromethane (35 ml). Triethylamine (1.8 ml). t-Butyl carbonate (2.85g) and 4-dimethyl amino pyridine (145 mg) were added to the mixture at room temperature and the mixture was stirred at room temperature overnight. A saturated aqueous solution of ammonium chloride was poured to the mixture, followed by ethyl acetate.. The organic layer was washed with water and brine, dried over sodium sulfate and the solvent was removed under reduced pressure. The resulting residue was purified by chromatography to afford Compound (27)(0.51g).
¹ H - NMR (CDCl₃) δ: 1.51 (s, 9H), 2.63 (d, J = 4.9 Hz, 3H), 6.59 (s, 1H), 7.08 (dd, J = 10.1, 9.1 Hz, 1H), 7.63 (dd, J = 6.2, 3.0 Hz, 1H), 7.76 (m, 1H).

### Step 3

Compound (27) (203 mg) was dissolved in methanol(1.5 ml) and dichloromethane (1.5 ml). Sodium borohydride (33.4 mg) was added to the mixture under cooling with ice and the mixture was stirred for 30 minutes. A saturated aqueous solution of ammonium chloride was poured to the mixture, followed by ethyl acetate. The organic layer was washed with water and brine, and dried over sodium sulfate. The solvent was removed under reduced pressure to afford Compound (28)(203 mg).
¹ H - NMR (CDCl₃) δ: 1.43-1.58 (m, 12H), 1.95 (d, J = 4.5 Hz, 1H), 5.15 (m, 1H), 6.44 (s, 1H), 6.94 (t, J = 9.4 Hz, 1H), 7.42 (dd, J = 6.4, 2.6 Hz, 1H).

### Step 4

Compound (28) (103.3 mg) was dissolved in dichloromethane (1 ml). Triethylamine(84µl) and methanesulfonyl chloride (38 µl) were added to the mixture at room temperature and the mixture was stirred for 1.5 hours. Ethyl acetate was added and the organic layer was washed with water and brine, dried over sodium sulfate and the solvent was removed under reduced pressure.
The resulting residue was dissolved in dimethylformamide (1 ml).
Sodium azide (52.6 mg) was added to the mixture and the mixture was stirred at 90°C for 1 hour. Water was poured to the mixture, followed by ethyl acetate. The organic layer was washed with water and brine, dried over sodium sulfate and the solvent was removed under reduced pressure. The resulting residue was purified by chromatography to afford Compound (29)(81.5 mg).
¹ H - NMR (CDCl₃) δ: 1.52-1.53 (m, 12H), 4.93 (q, J = 6.8 Hz, 1H), 6.50 (s, 1H), 6.99 (t, J = 9.2 Hz, 1H), 7.28 (dd, J = 8.5, 5.0 Hz, 1H), 7.37 (dd, J = 6.3, 2.8 Hz, 1H).

### Step 5

Compound (29) (115 mg) was dissolved in tetrahydrofuran (1.5 ml) and water (0.15 ml). Triphenylphosphine (113 mg) was added to the mixture at room temperature, and the mixture was stirred at 50°C for 3 hours and at 70°C for additional 1 hour. 10% aqueous solution of citric acid was poured to the mixture, followed by and ethyl acetate.. 10% Aqueous solution of sodium hydrogencarbonate was added to an aqueous layer, followed by ethyl acetate. The organic layer was washed with water, dried over sodium sulfate and the solvent was removed under reduced pressure to afford Compound (30)(85.2 mg).
¹ H - NMR (CDCl₃) δ: 1.41 (d, J = 6.6 Hz, 3H), 1.51 (s, 9H), 1.81 (br s, 3H), 4.35 (q, J = 6.6 Hz, 1H), 6.47 (br s, 1H), 6.93 (t, J = 9.5 Hz, 1H), 7.19 (m, 1H), 7.40 (dd, J = 6.3, 2.7 Hz, 1H).

### Step 6

Compound (30)(85.2 mg) was dissolved in tetrahydrofuran (1 ml). Benzoylisothiocyanate (51 µl) was added to the mixture at room temperature, and the mixture was stirred for 30 minutes. The solvent was removed under reduced pressure and the resulting residue was purified by chromatography to afford Compound (31) (132 mg).
¹ H-NMR (CDCl₃) δ: 1.50 (d, J = 0.3 Hz, 9H), 1.66 (d, J = 7.0 Hz, 3H), 1.72 (s, 1H), 5.65-5.75 (m, 1H), 6.51 (s, 1H), 7.02 (t, J = 9.5 Hz, 1H), 7.24 (dd, J = 6.4, 2.7 Hz, 1H), 7.36 (s, 1H), 7.51 (t, J = 7.6 Hz, 2H), 7.60-7.65 (m, 1H), 7.81-7.86 (m, 2H), 8.99 (s, 1H), 11.23 (d, J = 7.7 Hz, 1H).

### Step 7

Compound (31) (131 mg) was dissolved in methanol (1.5 ml). An aqueous solution of sodium hydroxide (1 mol/l 347 µl) was added to the mixture and the mixture was stirred at 50°C for 25 minutes. Ethyl acetate was added to the mixture and the organic layer was washed with water and brine, dried over sodium sulfate and the solvent was removed under reduced pressure.
The resulting residue was dissolved in acetonitrile (1.5 ml), methane iodide (24 µl) was added at room temperature, and the mixture was stirred at 60°C for 3 hours. The solvent was removed under reduced pressure, and the resulting residue was purified by chromatography to afford Compound (32) (130 mg).
¹ H - NMR (CDCl₃) δ: 1.51 (s, 10H), 1.67 (d, J = 6.7 Hz, 3H), 2.70 (s, 3H), 5.05 (q, J = 6.6 Hz, 1H), 6.70 (s, 1H), 7.01 (t, J = 9.2 Hz, 1H), 7.18 (dd, J = 5.7, 2.3 Hz, 1H), 7.61 (s, 1H).

### Step 8

Compound (32) (103 mg) was dissolved in chloroform (1.5 ml). Trifluoroacetic acid (1.5 ml) was added to the mixture at room temperature, and the mixture was stirred for 80 minutes. Water, 10% aqueous solution of sodium hydrogen carbonate and ethyl acetate were added to the mixture and the organic layer was washed with water and brine, and dried over sodium sulfate. The solvent was removed under reduced pressure to afford Compound (33) (63.1 mg).
¹ H - NMR (CD₃ OD) δ: 1.43 (d, J = 6.7 Hz, 3H), 2.38 (s, 3H), 4.89 (s, 6H), 5.03 (q, J = 6.8 Hz, 1H), 6.58 (ddd, J = 8.6,4.2,2.9 Hz, 1H), 6.70 (dd, J = 6.4, 2.9 Hz, 1H), 6.80 (dd, J = 10.3, 8.6 Hz, 1H).

### Step 9

Compound (33)(42.9 mg) and 5-methoxypyrazine-2-carboxylic acid (30.5 mg) were dissolved in methanol (1 ml). Hydrochloric acid (2 mol/l, 94 µl) and WSCD HCl (38.0 mg) were added to the mixture at room temperature and the mixture was stirred for 1 hour. Water, 10%sodium hydrogen carbonate and ethyl acetate were added to the mixture, and the organic layer was washed with water and brine, and dried over sodium sulfate. The solvent was removed under reduced pressure and the resulting residue was purified by chromatography, followed by TLC to afford Compound 26 (22.0 mg).
¹ H - NMR (CDCl₃) δ: 1.58 (d, J = 6.7 Hz, 3H), 2.46 (s, 3H), 4.06 (s, 3H), 5.12-5.14 (m, 1H), 7.06 (t, J = 9.5 Hz, 1H), 7.55 (dd, J = 6.3, 2.7 Hz, 1H), 7.81 (dt, J = 10.3, 2.9 Hz, 1H), 8.14 (d, J = 1.2 Hz, 1H), 8.99 (d, J = 1.2 Hz, 1H), 9.51 (s, 1H).

### Example 6

### Synthesis of Compound 28

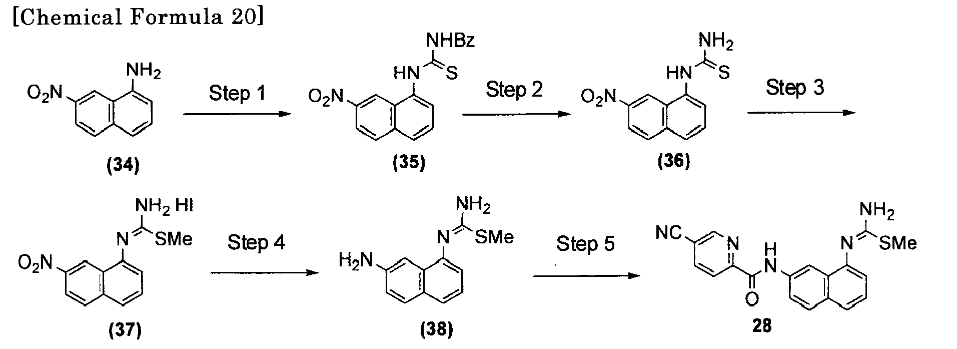

### Step 1

To a solution of Compound (34) (200 mg) in tetrahydrofuran (0.6 ml) was added benzoylisothiocyanate (151µl) at 0°C. The mixture was stirred for 10 minutes. Diisopropyl ether was added and the precipitated solid was collected by filtration to afford Compound (35) (277 mg).

### Step 2

To a solution of Compound (35) (277 mg) in tetrahydrofuran (0.83 ml) and methanol (0.83 ml) was added 1 mol/l aqueous solution of sodium hydroxide(0.83 ml). The mixture was stirred at room temperature for 1 hour. Tetrahydrofuran was removed under reduced pressure and the precipitated solid was collected by filtration. The solid was washed with water and ethyl acetate to afford Compound (36) (81 mg).

### Step 3

To a suspension of Compound (36) (81 mg) in methanol(2 ml) was added methane iodide (31 µl). The mixture was stirred at 40°C for 1 hour. After addition of methane iodide (31µl), the mixture was stirred at 60°C for 2 hours. Moreover addition of methane iodide (62µl) and stirring for 3 hours. The solvent was removed under reduced pressure to afford Compound (37) (127 mg).

### Step 4

To a solution of Compound (37) in acetic acid (1 ml) and methanol (2 ml) was added iron (91 mg) at 60°C and the mixture was stirred for 1 hour. Ethyl acetate was added and the mixture was made alkaline with saturated sodium hydrogen carbonate and insoluble material was filtered off. The organic layer of the filtrate was washed with saturated brine and dried over magnesium sulfate. The solvent was removed under reduced pressure to afford crude Compound (38).

### Step 5

To a solution of the crude Compound (38) in methanol (2 ml), 2 mol/l hydrochloric acid (146 µl) were added 4-cyano pyridine-2-carboxylic acid monohydrate (54 mg) and 1-ethyl-3-(3-dimethylaminopropyl)carbodiimide hydrochloride (62 mg). The mixture was stirred at room temperature for 1 hour. 1 mol/l aqueous solution of sodium hydroxide (400µl) was added. The precipitated solid was collected by filtration and washed with water. The solid was recrystallized from acetone to afford Compound 28 (31 mg).
¹ H - NMR, (DMSO-d6) δ: 2.44(3H,s), 6.40(2H,s), 6.83(1H,d, J = 8.0Hz), 7.30(1H,t, J = 8.0Hz), 7.45(1H,d, J = 8.0Hz), 7.82(1H,d J = 8.0Hz), 7.92(1H,d J =8.0 Hz), 8.31(1H,d J = 8.0Hz), 8.57(2H,m), 9.20(1H,s), 10.93(1H,s).

### Example 7

### Synthesis of Compound 31

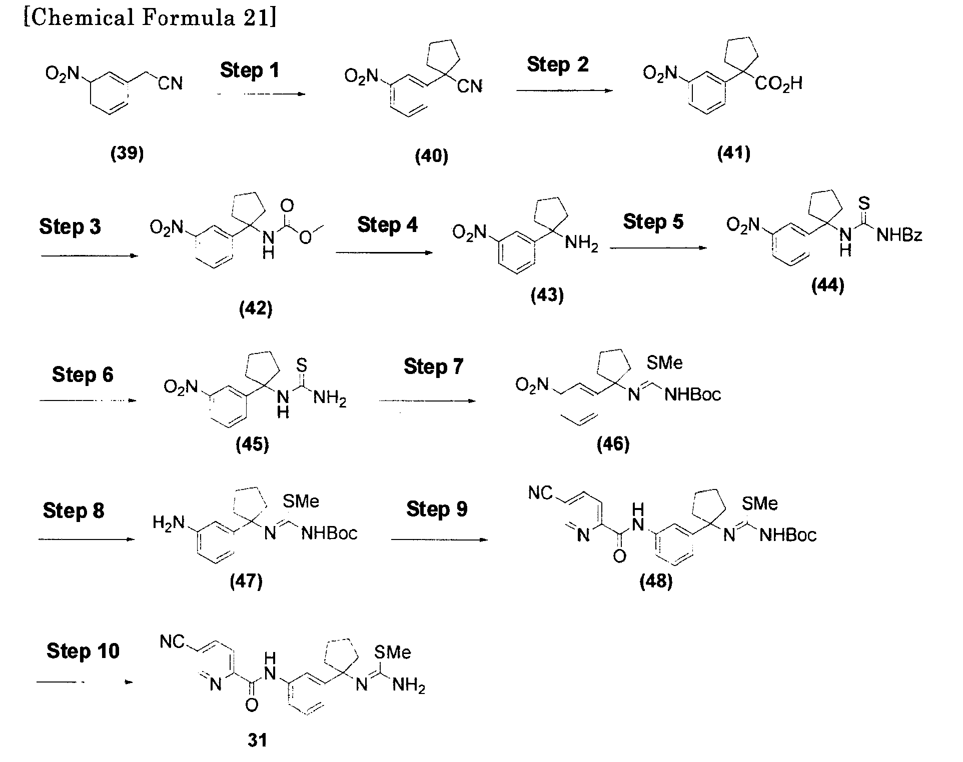

### Step 1

Sodium hydride(2.85 g, 35.7 mmol) was suspended in dimethylsulfoxide (50 ml) and a solution of 2-(3-nitrophenyl)acetonitrile (Compound (39), 5.26 g, 32.4 mmol) and 1,4-dibromobutane (4.23 ml, 35.7 mmol) in dimethylsulfoxide/ethyl ether (50 ml/25 ml) was added dropwise below 35°C over 25 minutes. Aftrer addition, the mixture was stirred at room temperature for 1.5 hours. The mixture was poured into ice water-2 mol/1-hydrochloric acid and extracted with ethyl acetate. The organic layer was washed with an aqueous solution of sodium hydrogen carbonate and water and dried over anhydrous sodium sulfate. The solvent was concentrated under reduced pressure and the residue was chromatographed on silica gel with ethyl acetate /n-hexane (1 : 5) as eluent. The eluent was concentrated under reduced pressure to afford a pale brown Compound (40) (6.42 g, 92%).
¹ H-NMR (CDCl₃) δ: 2.06-2.18 (6H, m), 2.15-2.62 (2H, m), 7.59 (1H, m), 7.85 (1H, m), 8.19 (1H, m), 8.29 (1H, m)

### Step 2

Compound (40) (3.5 g, 16.19 mmol) was suspended in 35 ml of sulfuric acid-water (1 : 1) and heated under reflux for 8 hours. The reaction mixture was poured into ice water, the precipitated solid was collected by filtration, and air-dried to afford Compound (41)(3.59 g, 94%) as a pale brown solid.
¹ H-NMR (CDCl₃) δ: 1.72-2.03 (6H, m), 2.66-2.78 (2H, m), 7.49 (1H, m), 7.72 (1H, m), 8.11 (1H, m), 8.25 (1H, m)

### Step 3

Compound (41) (2.0g, 8.50mmol) was suspended in toluene (20 ml) and triethylamine (1.77 ml, 12.75 mmol) and diphenylphosphoryl azide (2.02 ml, 9.35 mmol) were added, followed by stirring at room temperature for 35 minutes. After confirming disappearance of the starting material, the reaction mixture was stirred with heat at 100°C for 35 minutes, followed by addition of methanol (5 ml), and the mixture was heated with stirring at 100°C for 3 hours. The reaction mixture was poured into ice-water and extracted with ethyl acetate. The organic layer was washed with water, dried over anhydrous sodium sulfate and the solvent was concentrated under reduced pressure. n-Hexane was added to the resulting residue to yield Compound (42)(2.12 g, 94%) as a white solid.
¹ H-NMR (CDCl₃) δ: 1.80-1.96 (4H, m), 1.98-2.34 (2H, m), 2.26-2.40 (2H, m), 3.57 (3H, s), 5.12 (1H, s), 7.48 (1H, m), 7.76 (1H, d, J = 7.5 Hz), 8.08 (1H, m), 8.26 (1H, m)

### Step 4

Compound (42) (1.0g, 3.78 mmol) was dissolved in dimethylsulfoxide (5 ml) and 5 ml of 20% aqueous solution of lithium hydroxide was added. The reaction mixture was heated with stirring at 110°C for 2 hours. The mixture was poured into ice-water and extracted with ethyl acetate. The organic layer was washed with water and dried over sodium sulfate. The solvent was concentrated under reduced pressure to afford Compound (43) (0.78 g, ∼100%) as a pale brown oil. Compound (21) was used in the following reaction without purification.
¹ H-NMR (CDCl₃) δ: 1.55 (2H, s), 1.80-2.10 (8H, m), 7.49 (1H, m), 7.82 (1H, m), 8.07 (1H, m), 8.35 (1H, m)

### Step 5

Compound (43) (0.78 g, 3.78 mmol) was dissolved in acetone (16 ml) and benzoylisothiocyanate (0.61 ml, 4.54 mmol) was added at room temperature and the mixture was stirred for 1 hour. The reaction mixture was concentrated under reduced pressure. After being removed an insoluble material with chloroform, the residue was chromatographed on column with ethyl acetate /n-hexane (1 : 5) as eluent. The eluent was concentrated under reduced pressure to afford Compound (44) (1.3 g, 93%) as a pale yellow oil.
¹ H-NMR (CDCl₃) δ: 1.86-2.04 (4H, m), 2.20-2.35 (2H, m), 2.60-2.73 (2H, m), 7.46-7.60 (3H, m), 7.65 (1H, m), 7.75 (1H, m), 7.82-7.88 (2H, m), 8.11 (1H, m), 8.26 (1H, m), 8.83 (1H, s), 11.39 (1H, s).

### Step 6

Compound (44)(1.3 g, 3.52 mmol) was dissolved in methanol (13 ml) and 1 mol/l aqueous solution of sodium hydroxide(4.22 ml, 4.22 mmol) was added and heated with stirring at 50°C for 10 minutes. The reaction mixture was poured into ice-water and extracted with ethyl acetate. The organic layer was washed with water, dried over sodium sulfate and concentrated under reduced pressure. The resulting residue was recrystallized from ethyl acetate /n-hexane (1 : 5) to afford Compound (45) (0.82 g, 88%) as a pale yellow solid.
¹ H-NMR (CDCl₃) δ: 1.85-2.35 (8H, m), 5.54 (2H, br s), 6.92 (1H, s), 7.60 (1H, m), 7.83 (1H, m), 8.18 (1H, m), 8.26 (1H, m)

### Step 7

Compound (45) (0.5 g, 1.88 mmol) was dissolved in acetonitrile (10 ml) and dimethyl sulfate (0.18 ml, 1.88 mmol) was added and heated with stirring at 50°C for 2 hours. The reaction mixture was allowed to cool to room temperature, di-tert-butyl dicarbonate (0.41 g, 1.88 mmol) and triethylamine (0.26 ml, 1.88 mmol) were added successively, and stirred at room temperature for 2.5 hours. The reaction mixture was poured into ice-water and extracted with ethyl acetate. The organic layer was washed with water, dried over sodium sulfate and concentrated under reduced pressure. The residue was chromatographed on column using ethyl acetate /n-hexane (1 : 5) as eluent, concentrated under reduced pressure to afford Compound (46) (0.7 g, 98%) as a white solid.
¹ H-NMR (CDCl₃) δ: 1.54 (9H, s), 1.80-2.04 (4H, m), 2.14-2.26 (2H, m), 2.28 (3H, s), 2.30-2.42 (2H, m), 7.50 (1H, m), 7.72 (1H, m), 8.12 (1H, m), 8.23 (1H, m), 10.65 (1H, s).

### Step 8

Compound (46) (0.3 g, 0.79 mmol) was dissolved in tetrahydrofuran (4 ml) and methanol (2 ml) and water (1 ml), ammonium chloride (106 mg, 1.98 mmol), then iron powders (221 mg, 3.95 mmol) were added and the mixture was heated to reflux for 3.5 hours. Insoluble material was filtered off and the filtrate was concentrated under reduced pressure to afford Compound (47) (300 mg, ∼100%) as a pale yellow foam. Compound (25) was used in the following reaction without purification. ¹ H-NMR (CDCl₃) δ: 1.53 (9H, s), 1.74-1.94 (4H, m), 2.10-2.30 (4H, m), 2.29 (3H, s), 3.66 (2H, s), 6.57 (1H, m), 6.69 (1H, m), 6.75 (1H, m), 7.10 (1H, m), 10.56 (1H, s).

### Step 9

Compound (47)(105 mg, 0.3 mmol) was dissolved in ethyl acetate (2 ml) and 5-cyano picoline acid (monohydrate)(49.9 mg, 0.3 mmol), triethylamine(0.063 ml, 0.45 mmol) and diphenylphosphoryl chloride (0.062 ml, 0.3 mmol) were added successively, and the mixture was stirred at room temperature for 50 minutes. The reaction mixture was poured into ice-water and extracted with ethyl acetate. The organic layer was washed with an aqueous solution of sodium hydrogen carbonate and water successively, and dried over sodium sulfate. The solvent was concentrated under reduced pressure, chromatographed on column using ethyl acetate /n-hexane (1 : 5) as eluent and concentrated under reduced pressure. The crude residue was purified by preparative TLC with ethyl acetate /n-hexane (1 : 1) to ethyl acetate to afford Compound (48)(87 mg, 60%) as a white oil.
¹ H-NMR (CDCl₃) δ: 1.55 (9H, s), 1.80-2.00 (4H, m), 2.16-2.38 (4H, m), 2.27 (3H, s), 7.21 (1H, d, J = 6.9 Hz), 7.38 (1H, m), 7.65 (1H, m), 7.84 (1H, d, J = 7.2 Hz), 8.21 (1H, dd, J = 2.1, 8.4 Hz), 8.44 (1H, d, J = 8.4 Hz), 8.92 (1H, d, J = 1.8 Hz), 9.88 (1H, s), 10.63 (1H, s).

### Step 10

Compound (48) (87 mg, 0.18 mmol) was dissolved in formic acid (0.5 ml) and stirred at room temperature for 18 hours. The reaction mixture was poured into ice-water and neutralized with an aqueous solution of sodium hydrogen carbonate, then extracted with ethyl acetate. The organic layer was washed with water, dried over sodium sulfate and concentrated under reduced pressure. The residue was recrystallized from ethyl acetate /n-hexane (1 : 1) to afford Compound 31(19 mg, 28%) as a white powder.
¹ H-NMR (DMSO-d₆) δ: 1.66-1.86 (4H, m), 1.19-2.06 (2H, m), 2.24-2.36 (2H, m), 2.28 (3H, s), 7.13 (1H, d, J = 8.4 Hz), 7.30 (1H, m), 7.77 (1H, d, J = 8.7 Hz), 7.93 (1H, s), 8.28 (1H, dd, J = 0.9, 8.1 Hz), 8.58 (1H, dd, J = 2.1, 8.1 Hz), 9.19 (1H, m), 10.75 (1H, s).

### Example 8

### Synthesis of Compound 29

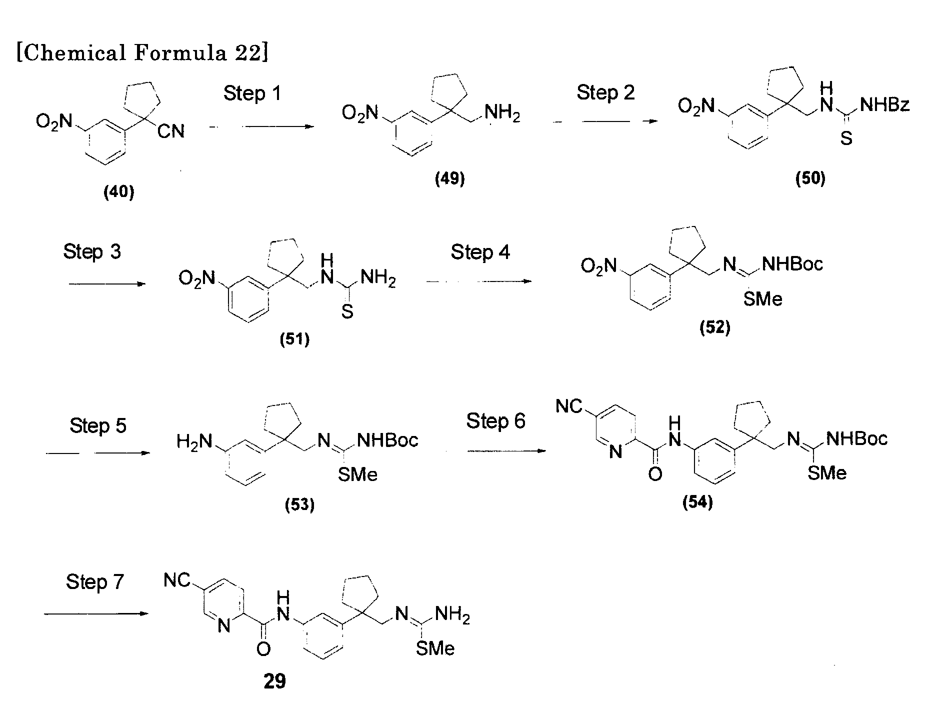

### Step 1

To 1 mol/l solution of borane-tetrahydrofuran complex (3.70 ml, 3.70 mmol) was added a solution of 1-(3-nitro phenyl)cyclopentane carbonitrile (0.4g, 1.85 mmol) (Compound (40): synthesized in Step 1) in tetrahydrofuran (2 ml) and heated to reflux for 2.5 hours. After quenching with methanol, the mixture was partitioned into ethyl acetate and an aqueous solution of sodium hydrogen carbonate. The organic layer was washed with water and dried over sodium sulfate. The solvent was concentrated under reduced pressure and the residue was chromatographed on column with ethyl acetate /n-hexane (1 : 3) as eluent. The eluent was concentrated under reduced pressure to afford Compound (49) (128 mg, 31%) as a pale yellow oil.
¹ H-NMR (CDCl₃) δ: 1.47 (2H, s), 1.68-2.08 (8H, m), 2.81 (2H, s), 7.49 (1H, m), 7.62 (1H, m), 8.07 (1H, m), 8.14 (1H, m)

### Step 2

Compound (49)(128 mg, 0.58 mmol) was dissolved in acetone (1 ml) and benzoylisothiocyanate(0.094 ml, 0.70 mmol) was added at room temperature, and stirred for 30 minutes. The reaction mixture was poured into ice-water and extracted with ethyl acetate. The organic layer was wahed with water and dried over sodium sulfate. The solvent was concentrated under reduced pressure and the residue was chromatographed on column with ethyl acetate /n-hexane (1 : 3) as eluent. The eluent was concentrated under reduced pressure to afford Compound (50) (110 mg, 49%) as a white solid.
¹ H-NMR (CDCl₃) δ: 1.75-2.22 (8H, m), 7.44-7.66 (4H, m), 7.70-7.80 (3H, m), 8.13 (1H, m), 8.24 (1H, m), 8.87 (1H, s), 10.61 (1H, s).

### Step 3

Compound (50)(100 mg, 0.26 mmol) was dissolved in methanol (1 ml) and 1 mol/l aqueous solution of sodium hydroxide(0.31 ml, 0.31 mmol) was added, and heated with stirring at 50°C for 20 minutes. The reaction mixture was poured into ice-water and extracted with ethyl acetate. The organic layer was washed with water and dried over sodium sulfate. The solvent was concentrated under reduced pressure and the resulting residue was recrystallized from ethyl acetate /n-hexane (1 : 5) to afford Compound (51) (75 mg, ∼100%) as a pale yellow solid.
¹ H-NMR (CDCl₃) δ: 1.70-2.16 (8H, m), 3.80 (2H, br s), 5.55-5.90 (3H, m), 7.54 (1H, m), 7.68 (1H, d, J=8.1 Hz), 8.09 (1H, d, J=8.4 Hz), 7.14 (1H, s)

### Step 4

Compound (51)(72.9 mg, 0.26 mmol) was dissolved in acetonitrile (2 ml) and dimethyl sulfate (0.025 ml, 0.26 mmol) was added, and heated with stirring at 50°C for 3 hours. The reaction mixture was allowed to cool to room temperature and di-tert-butyl dicarbonate (57 mg, 0.26 mmol) and triethylamine(0.036 ml, 0.26 mmol) were added successively, and the mixture was stirred at room temperature for 2 hours. The reaction mixture was poured into ice-water and extracted with ethyl acetate. The organic layer was washed with 2 mol/l hydrochloric acid, an aqueous solution of sodium hydrogen carbonate and water successively, and dried over sodium sulfate. The solvent was concentrated under reduced pressure to afford Compound (52) (102 mg, 99%) as a colorless oil. Compound (30) was used in the following reaction without purification.
¹ H-NMR (CDCl₃) δ: 1.45 (9H, s), 1.75-2.20 (8H, m), 2.36 (3H, s), 2.44 (2H, s), 7.50 (1H, m), 7.63 (1H, m), 8.12 (1H, m), 9.85 (1H, m)

### Step 5

Compound (52) (102 mg, 0.26 mmol) was dissolved in tetrahydrofuran (2 ml), methanol (1 ml) and water (0.5 ml) and ammonium chloride(34.7 mg, 0.65 mmol) and iron powders (72.4 mg, 1.30 mmol) were added, and heated to reflux for 2.5 hours. Insoluble material was filtered off and the filtrate was concentrated under reduced pressure to afford Compound (53) (96 mg, ∼100%) as a pale yellow oil. Compound (31) was used in the following reaction withoug purification.
¹ H-NMR (CDCl₃) δ: 1.47 (9H, s), 1.66-2.20 (8H, m), 2.38 (3H, s), 3.34 (2H, s), 3.66 (1H, br s), 6.55 (1H, dd, J=2.1, 7.5 Hz), 6.64 (1H, m), 6.68 (1H, d, J= 7.5 Hz), 7.10 (1H, m), 9.68 (1H, br s).

### Step 6

Compound (53)(94 mg, 0.26 mmol) was dissolved in ethyl acetate (2 ml) and 5-cyano picolic acid (monohydrate) (64.5 mg, 0.39 mmol), triethylamine(0.072 ml, 0.52 mmol) and diphenylphospholic chloride (0.054 ml, 0.26 mmol) were added successively, and the mixture was stirred at room temperature for 15 minutes. The reaction mixture was poured into ice-water and extracted with ethyl acetate. The organic layer was washed with an aqueous solution of sodium hydrogen carbonate and water successively, and dried over sodium sulfate. The solvent was concentrated under reduced pressure and chromatographed on column with ethyl acetate /n-hexane (1 : 3) as eluent. The eluent was concentrated under reduced pressure to afford Compound (54) (71 mg, 56%) as colorless oil.
¹ H-NMR (CDCl₃) δ: 1.40 (9H, s), 1.90-2.14 (8H, m), 2.38 (3H, s), 3.40 (2H, d, J = 3.6 Hz), 7.13 (1H, d, J = 7.8 Hz), 7.37 (1H, m), 7.60 (1H, s), 7.77 (1H, d, J = 8.1 Hz), 8.20 (1H, dd, J = 1.8, 8.1 Hz), 8.43 (1H, d, J = 8.1 Hz), 8.88 (1H, m), 9.72 (1H, br s), 9.86 (1H, s).

### Step 7

Compound (54)(71 mg, 0.14 mmol) was dissolved in formic acid (0.5 ml) and stirred at room temperature for 18 hours. The reaction mixture was poured into ice-water and neutralized with an aqueous solution of sodium hydrogen carbonate, then extracted with ethyl acetate. The organic layer was washed with water, dried over sodium sulfate and concentrated under reduced pressure. The residue was recrystallized from ethyl acetate /n-hexane (1 : 3) to afford Compound 29 (15.2 mg, 27%) as a white powders.
¹ H-NMR (CDCl₃) δ: 1.68-2.12 (8H, m), 2.31 (3H, s), 3.47 (2H, s), 7.15 (1H, d, J = 7.5 Hz), 7.38 (1H, m), 7.64-7.74 (2H, m), 8.21 (1H, dd, J = 1.8, 8.1 Hz), 8.44 (1H, d, J = 8.1 Hz), 8.91 (1H, d, J = 1.8 Hz), 9.89 (1H, s).

### Example9

### Synthesis of Compound 32

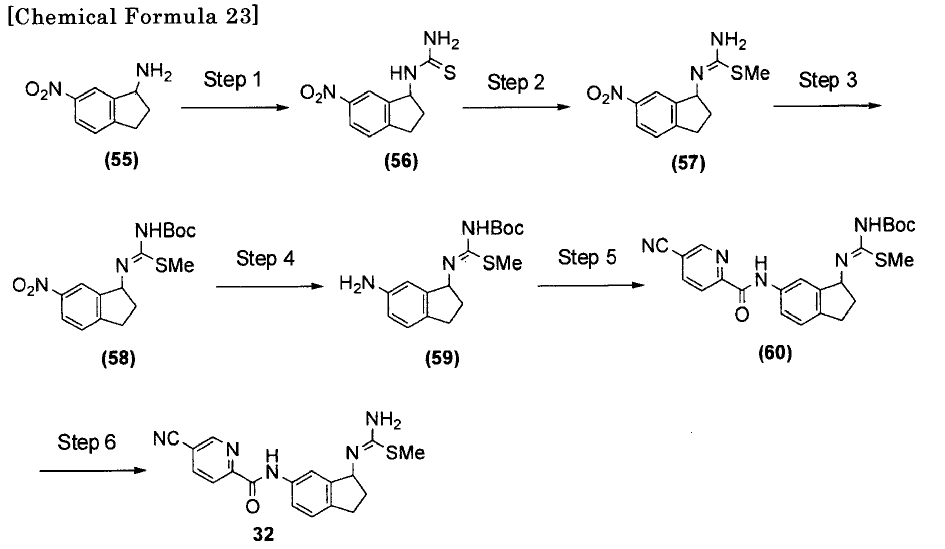

### Step 1

To a solution of Compound (55) (82 mg) in tetrahydrofuran (1 ml) was added benzoylisothiocyanate(62 µl) at 0°C and stirred for 10 minutes. 1 mol/l aqueous solution of sodium hydroxide (460 µl) was added thereto and the mixture was stirred at room temperature for 40 minutes, followed by at 50°C for 50 minutes. Methanol (0.5 ml) was added and the mixture was stirred for 1 hour. After cooling to room temperature, the mixture was extracted with ethyl acetate, washed with saturated brine and dried over magnesium sulfate. The solvent was removed under reduced pressure to afford Compound (56) (109 mg).

### Step 2

To a solution of Compound (56) (109 mg) in acetonitrile (2 ml) was added dimethyl sulfate (44 µl) and the mixture was stirred at 50°C for 2 hours. Ethyl acetate and a saturated solution of sodium bicarbonate were added thereto and the organic layer was washed with a saturated solution of sodium bicarbonate and a saturated brine and dried over magnesium sulfate. The solvent was removed under reduced pressure to afford Compound (57) (122 mg).

### Step 3

To a solution of Compound (57) (122 mg) in methanol(1 ml) was added di-tert-butyl dicarbonate (115 mg)and the mixture was stirred at room temperature overnight. The solvent was removed under reduced pressure and the resulting residue was chromatographed on column to afford Compound (58) (64 mg). ¹ H-NMR (CDCl₃) δ: 1.50(3H, s), 2.01-2.14(1H,m)2.57(3H,s) 2.72-3.18(4H,m), 5.35(1H,m), 7.39(1H,d, J = 8.4Hz), 8.15(1H,dd, J = 8.4,2.4Hz),8.22(1H,s), 10.12(1H, br s).

### Step 4

To a solution of Compound (58) in methanol (0.6 ml), tetrahydrofuran (0.2 ml) and water (0.13 ml) were added ammonium chloride (39 mg) and iron (41 mg) and the mixture was stirred at 70°C for 1.5 hours. Methanol (0.5 ml), tetrahydrofuran (0.5 ml) and iron (41 mg) were added and the mixture was stirred for additional 20 minutes. Ethyl acetate was added and insoluble matereial was filtered off. The organic layer was washed with saturated brine and dried over magnesium sulfate. The solvent was removed under reduced pressure to afford Compound (59) (55 mg).

### Step 5

To a solution of Compound (59) in tetrahydrofuran (1 ml) were added4-cyano pyridine-2-carboxylic acid monohydrate (31 mg) and triethylamine (60 µl). Diphenyl phospholic chloride (43 µl) was added at 0°C and the mixture was stirred for 5 minutes. Ethyl acetate was added and the mixture was washed with saturated brine and dried over magnesium sulfate. The solvent was removed under reduced pressure and the resulting residue was chromatographed on column to afford Compound (60) (49 mg).

### Step 5

Formic acid (165 µl) was added to Compound (60) (49 mg) and stirred at room temperature for 4.5 hours. Ethyl acetate was added thereto and the mixture was made alkaline with 2 mol/l sodium carbonate. The organic layer was washed with saturated brine and dried over magnesium sulfate. The solvent was removed under reduced pressure and the resulting residue was washed with ether to afford Compound 32.
¹ H-NMR (CDCl₃) δ: 1.86-1.98(1H,m) 2.40(3H,s), 2.60-3.04(3H,m), 5.48(1H,m), 7.26(1H,m), 7.70(2H,m), 8.19(1H,dd, J = 8.1,2.0Hz), 8.43(1H,d, J = 8.1Hz), 8.89(1H,d, J = 2.0Hz), 9.84(1H, s).

### Example 10

### Synthesis of Compound 33

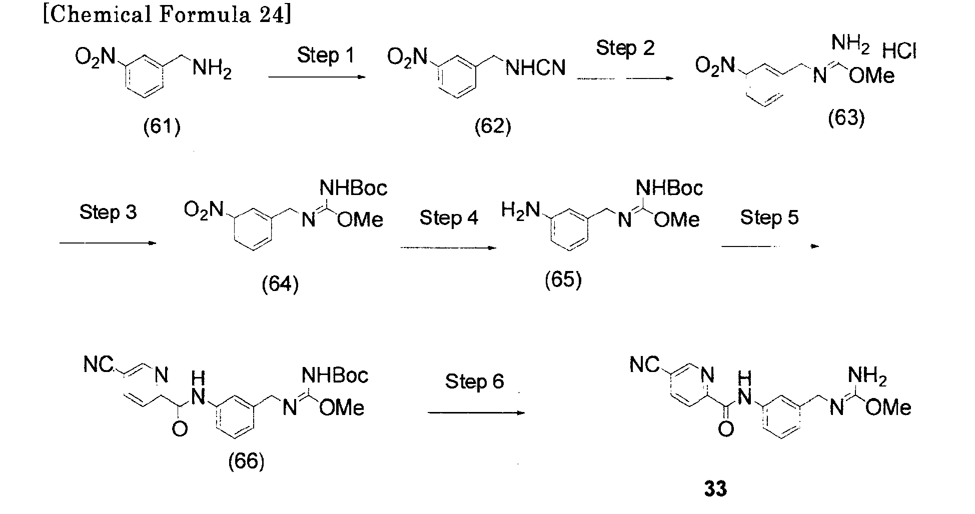

### Step 1

Compound (61) (0.92 g) was dissolved in dichloromethane (3.2 ml) and 3 mol/l cyanogen bromide/dichloromethane solution (1.01 ml) was added dropwise thereto with stirring under ice-cooling. The mixture was stirred at the same temperature for 20 minutes, followed by stirring at room temperature for 2 hours. The precipitated insoluble material was filtered off and the filtrate was concentrated under reduced pressure to afford Compound (62) as a crude product.

### Step 2

Compound (62) obtained in Step 1 was dissolved in methanol (7.7 ml) and 1.37 mol/l hydrogen chloride/methanol solution (2.65 ml) was added with stirring at room temperature. After stirring for 2.5 hours, the solvent was removed under reduced pressure to afford Compound (63) as a crude product.

### Step 3

Compound (63) obtained in Step 2 was dissolved in tetrahydrofuran (3.5 ml) and di-t-butyl dicarbonate (1.98 g) and triethylamine(1.53 g) were added with stirring at room temperature and stirred overnight. The precipitated insoluble material was filtered off, the filtrate was concentrated under reduced pressure and the residue was chromatographed on silica gel to afford Compound (64) (0.25 g).
¹ H-NMR (CDCl₃) δ: 1.52 (9H, s), 3.88 (3H, s), 4.54 (2H, d, J = 6.1 Hz), 7.53 (1H, t, J = 7.6 Hz), 7.61 (1H, d, J = 7.6 Hz), 8.14-8.15 (2H, m), 9.28 (1H, br s).

### Step 4

Tetrahydrofuran (2 ml), methanol (1 ml) and water (0.5 ml) were added to Compound (64)(0.25 mg). Reduced iron (0.23 g) and ammonium chloride (0.11 g) were added to the mixture at room temperature. The mixture was heated to reflux for 40 minutes and the solvent was removed under reduced pressure. Dichloromethane was added thereto, insoluble material was filtered off and the filtrate was concentrated under reduced pressure. The residue was chromatographed on silica gel to afford Compound (65) (0.15 g).
¹ H-NMR (CDCl₃) δ: 1.50 (9H, s), 3.68 (2H, br), 3.87 (3H, s), 4.34 (2H, d, J = 5.9 Hz), 6.58-6.64 (3H, m), 7.11 (1H, t, J = 7.5 Hz), 9.04 (1H, br s).

### Step 5

To 5-cyano picolic acid monohydrate (0.13 g) were added tetrahydrofuran (1.5 ml), diphenyl chlorophosphate (0.14 g) and triethylamine (0.14 g) with stirring under ice-cooling. The mixture was stirred at the same temperature for 20 minutes and a solution of Compound (65) (0.15 g) in tetrahydrofuran (3 ml) and triethylamine (0.11 g) were added thereto. The mixture was stirred at room temperature for 30 minutes, water and sodium hydrogen carbonate were added thereto and the reaction mixture was extracted with ethyl acetate. The organic layer was washed with water and saturated brine, dried over anhydrous sodium sulfate and the solvent was removed under reduced pressure to afford Compound (66) as a crude product.

### Step 6

Formic acid (1.1 ml) was added to Compound (66) obtained in Step 5 and the mixture was stirred at room temperature overnight. Ice and a saturated aqueous solution of sodium hydrogen carbonate were added to the reaction mixture. After the mixture was extracted with chloroform, the organic layer was washed with water and saturated brine and dried over anhydrous sodium sulfate. The solvent was removed under reduced pressure and crystallized from ethyl acetate/ether to afford Compound 33 (0.10 g) as a white solid.
¹ H-NMR (CDCl₃) δ: 3.76 (3H, s), 4.32 (2H, s), 7.15 (1H, d, J = 7.9 Hz), 7.37 (1H,t, J=7.9 Hz), 7.70 (2H, t, J = 8.2 Hz), 8.20 (1H, dd, J = 8.2, 2.1 Hz), 8.43 (1H, dd, J = 8.2, 0.8 Hz), 8.89 (1H, dd, J = 2.1, 0.8 Hz), 9.86 (1H, s).

### Reference Example 1 Synthesis of MeO-pyrazine carboxylic acid-d3 (69)

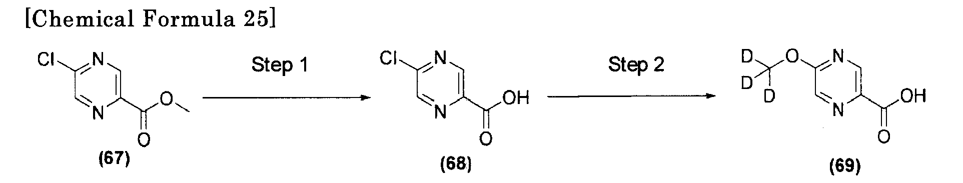

### Step 1

Compound (67)(10 g) was suspended in water (35 ml) and 2 mol/L-aqueous solution of sodium hydroxide (34.8 ml) was added and the mixture was stirred at room temperature for 4.5 hours. 2 mol/L-hydrochloric acid (34.8 ml) was added to neutralize and stirred at room temperature overnight. The precipitated insoluble material was collected by filtration, washed with water and dried under reduced pressure to afford Compound (68) (3.47 g).
¹ H-NMR (DMSO-d₆) δ: 8.93 (1H, s), 9.03 (1H, s).

### Step 2

Compound (68) (2.5 g) was dissolved in N,N-dimethylformamide (20 ml) and methanol-D4 (12.8 ml) was added and the mixture was cooled to 0°C. Potassium t-butoxide (7.08 g) was added and the mixture was stirred at 50°C for 3 hours 45 minutes. The mixture was cooled to room temperature and water was added to dissolve insoluble material. 2 mol/l-hydrochloric acid (27.6 ml) was added and the mixture was extracted with ethyl acetate. The organic layer was dried over anhydrous magnesium sulfate and the solvent was removed under reduced pressure to afford Compound (69) (1.56 g).
¹ H-NMR (DMSO-d₆) δ: 8.39 (1H, s), 8.81 (1H, s), 13.28 (1H, br s).

### Example 11

### Optical resolution of Compound 88

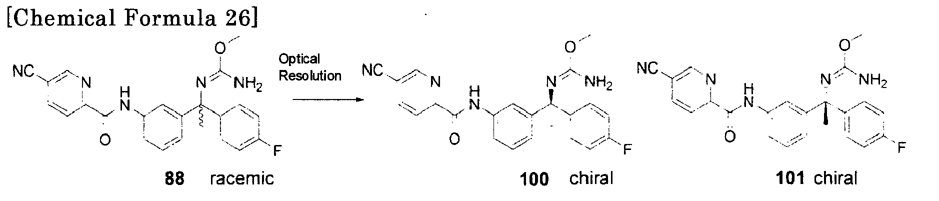

### Step 3

Compound (88) (about 240 mg) was optically resolved under the follwing condisitons to afford Compound (100) (110 mg) and Compound (101) (110 mg).
Chiral column: CHIRALCEL OD-H, size: 5 cm I.D. x 25 cmL, mobile phase: n-hexane/ethanol/diethylamine=50/50/0.1, temperature: 40°C, detection wave length: 313 nM.
Compound 100: m/z 418 [M+1]
Compound 101: m/z 418 [M+1]

### Example 12

### Synthesis of Compound 97

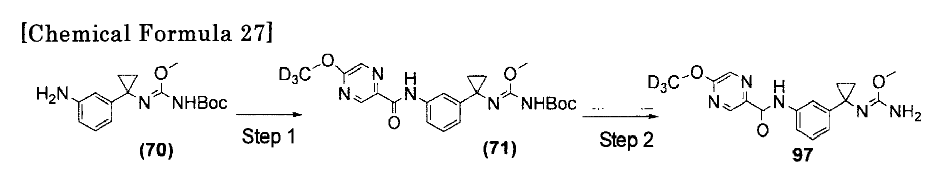

### Step 1

Compound (70) (84.1 mg) was dissolved in ethyl acetate (1.6 ml) and 5-CD₃O-pyrazine-2-carboxylic acid(47.6 mg), triethylamine(191 µl), and diphenyl phosphorochloridate (69 µl) were added at 0°C and the mixture was stirred for 45 minutes. Water and ethyl acetate were added successively, and the organic layer was washed with water and brine and dried over sodium sulfate. The solvent was removed under reduced pressure and the resulting residue was purified by chromatography to afford Compound (71) (106 mg).
¹ H-NMR (CDCl₃) δ: 1.32 (4H, s), 1.53 (9H, s), 3.82 (3H, s), 6.94-6.98 (1H, m), 7.30 (1H, t, J = 7.9 Hz), 7.42 (1H, s), 7.66-7.69 (1H, m), 8.16 (1H, s), 9.01 (1H, s), 9.33 (1H, s), 9.48 (1H, s).

### Step 2

Compound (71) (102 mg) was dissolved in dichloromethane (2.0 ml) and trifluoroacetic acid (353 µl) was added at room temperature, and the mixture was stirred for 2 hours. Water, a saturated aqueous solution of sodium hydrogen carbonate, chloroform and methanol were added and the organic layer was washed with water and brine and dried over sodium sulfate. The solvent was removed under reduced pressure and the obtained residue was purified by chromatography to afford Compound 97 (71.9 mg).
¹ H-NMR (CDCl₃) δ: 1.17-1.40 (4H, m), 3.76 (3H, s), 4.21 (1H, br s), 5.00 (1H, br s), 6.95 (1H, d, J = 8.1 Hz), 7.31 (1H, t, J = 7.9 Hz), 7.55-7.58 (2H, m), 8.15 (1H, d, J = 1.2 Hz), 9.01 (1H, d, J = 1.2 Hz), 9.48 (1H, s).

### Example 13

### Synthesis of Compound 98

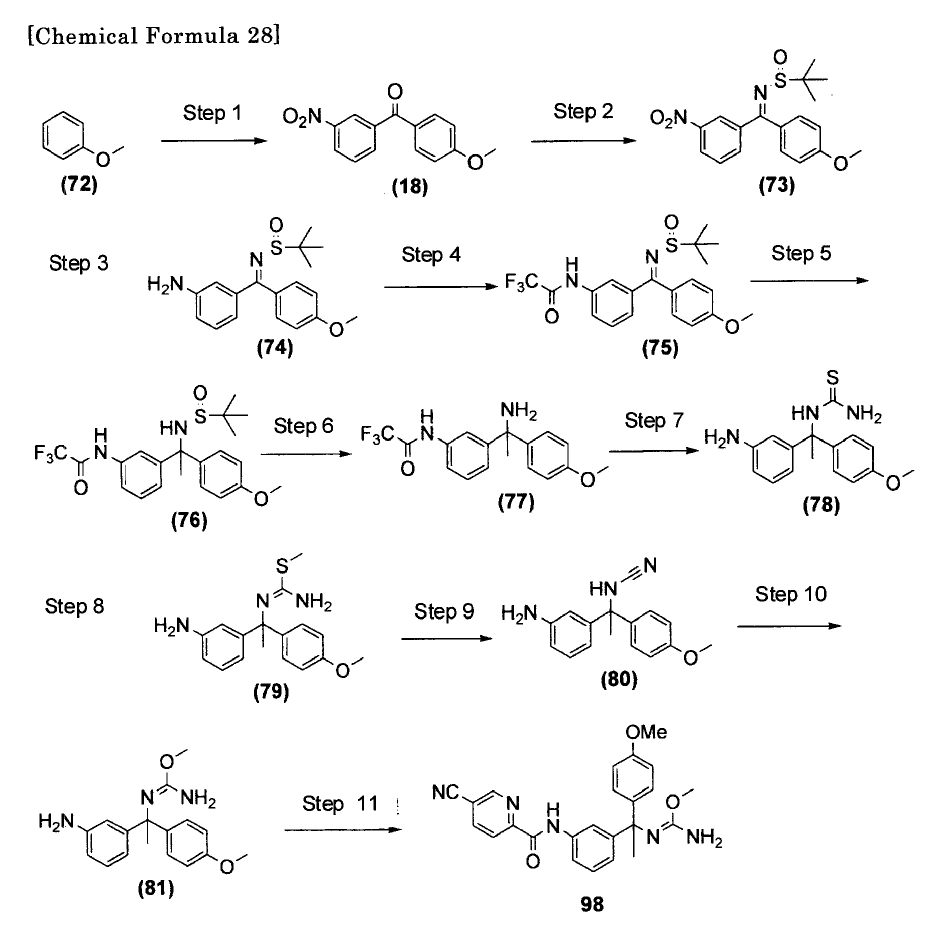

### Step 1

3-nitro benzoyl chloride (13.5 g) was dissolved in dichloromethane (130 ml) and anisole (1) (7.22 ml) and aluminium chloride (11.5 g) were added at room temperature, and the mixture was stirred for 2 hours. Water and dichloromethane were added thereto and the organic layer was washed with brine and dried over sodium sulfate. The solvent was removed under reduced pressure and diisopropyl ether was added to the resulting residue. The precipitated solid was collected and purified to afford Compound (18) (12.1 g).
¹ H - NMR (CDCl₃) δ: 3.91 (3H, s), 7.00 (2H, dt, J = 9.4, 2.4 Hz), 7.69 (1H, t, J=7.9Hz), 7.81 (2H, dt, J = 9.4, 2.4 Hz), 8.07-8.11 (1H, m), 8.40-8.44 (1H, m), 8.57 (1H, t, J = 1.9 Hz).

### Step 2

Compound (18)(5.01 g) was dissolved in tetrahydrofuran (50 ml) and 2-methylpropane-2-sulfine amide (3.07 g) and tetraethyl orthotitanate (7.22 ml) were added at room temperature, and the mixture was heated to reflux for 4 hours. Additional 2-methylpropane-2-sulfineamide (1.18 g) and tetraethyl orthotitanate (4.69 ml) were added and the mixture was heated to reflux for 1.5 hours. Brine and ethyl acetate were added thereto and the mixture was filtered. The filtrated organic layer was washed with water and brine, dried over magnesium sulfate and the solvent was removed under reduced pressure. The resulting residue was purified by chromatography to afford Compound (73) (5.50 g).
¹H - N M R (CDCl₃) δ: 1.32 (9H, s), 3.87 (3H, s), 6.93 (2H, d, J = 7.7 Hz), 7.57-7.67 (4H, m), 8.18 (1H, br s), 8.34 (1H, dd, J = 9.2, 2.1 Hz).

### Step 3

Compound (73) (5.46 g) was dissolved in methanol (55 ml), water (22 ml) and tetrahydrofuran (82 ml) and iron (3.68 g) and ammonium chloride(0.88 g) were added at room temperature and the mixture was heated to reflux for 1.5 hours. The reaction mixture was filtered and the solvent was removed under reduced pressure. Water and ethyl acetate were added, the organic layer was washed with water and brine, dried over sodium sulfate and the solvent was removed under reduced pressure. The resulting residue was purified by chromatography to afford Compound (74)(4.39 g).
¹ H - NMR (CDCl₃) δ: 1.27 (9H, s), 3.78 (2H, s), 3.85 (3H, s), 6.67-6.89 (5H, m), 7.21 (1H, t, J = 7.5 Hz), 7.71 (2H, br s).

### Step 4

Compound (74)(4.32 g) was dissolved in tetrahydrofuran (22 ml) and triethylamine (2.72 ml) and trifluoroacetic acid anhydride(2.21 ml) were added at 0°C, and the mixture was stirred for 45 minutes. Water and ethyl acetate were added successively, the organic layer was washed with water and brine and dried over magnesium sulfate. The solvent was removed under reduced pressure and the resulting residue was purified by chromatography to afford Compound (75)(4.69 g).
¹ H - NMR (CDCl₃) δ: 1.31 (9H, s), 3.85 (3H, s), 6.89 (2H, d, J=8.5Hz), 7.15 (1H, br s), 7.39 (1H, t, J = 7.8 Hz), 7.64 (4H, br s), 9.17 (1H, br s).

### Step 5

Methyl magnesium bromide (18.2 ml, 3.0 mol/l solution in ether) was dissolved in tetrahydrofuran (76 ml) and a solution of Compound (5) (3.87 g) in tetrahydrofuran (38 ml) was added at 0°C. The mixture was stirred at room temperature for 8 hours. A saturated aqueous solution of ammonium chloride and ethyl acetate were added successively, and the organic layer was washed with water and brine and dried over sodium sulfate. The solvent was removed under reduced pressure and the resulting residue was purified by chromatography to afford Compound (76) (1.58 g).
¹ H - NMR (CDCl₃) δ: 1.23 (s) and 1.25 (s) (9H), 2.05 (s) and 2.07 (s) (3H), 3.79 (3H, s), 3.84 (1H, s), 6.80-6.87 (2H, m), 7.15-7.24 (4H, m), 7.30-7.35 (1H, m), 7.54-7.61 (2H, m), 8.14 (br s) and 8.40 (br s) (1H).

### Step 6

Compound (76) (1.50 g) was dissolved in 4 mol/l solution of hydrochloric acid in dioxane (7.0 ml). Methanol (192 µl) was added to the mixture at room temperature. After stirring for 1 hour, water and ether were added successively and the aqueous layer was separated. 2 mol/l Aqueous solution of potassium carbonate and ethyl acetate were added to the aqueous layer and the organic layer was washed with water and brine, and dried over sodium sulfate to afford Compound (77) (1.16 g).
¹ H - NMR (CDCl₃) δ: 1.84 (3H, s), 3.71-3.81 (5H, m), 6.81-6.87 (2H, m), 7.23-7.36 (4H, m), 7.46 (1H, t, J = 2.0 Hz), 7.58-7.60 (1H, m), 7.82 (1H, br s).

### Step 7

Compound (77) (1.16 g) was dissolved in tetrahydrofuran (12 ml) and benzoylisothiocyanate (484 µl) was added at 0°C, and the mixture was stirred at room temperature for 15 minutes. Benzoylisothiocyanate (46 µl) was added at 0°C and the mixture was stirred at room temperature for 20 minutes. Methanol (12 ml) and 1 mol/l aqueous solution of sodium hydroxide(10.3 ml) were added at room temperature and the mixture was heated to reflux for 1.5 hours. Water and ethyl acetate were added successively, the organic layer was washed with water and brine and dried over sodium sulfate. The solvent was removed under reduced pressure and the resulting residue was purified by chromatography to afford Compound (78) (806 mg).
¹ H - NMR (CDCl₃) δ: 2.02 (3H, s), 3.74 (2H, s), 3.81 (3H, s), 6.60-6.63 (2H, m), 6.68-6.71 (1H, m), 6.86-6.91 (2H, m), 6.94 (1H, s), 7.11-7.17 (1H, m), 7.25-7.30 (4H, m).

### Step 8

Compound (78)(794 mg) was dissolved in dimethylformamide (22 ml) and methyl iodide(181 µl) was added at room temperature, and the mixture was stirred for 1 hour. A saturated aqueous solution of sodium hydrogen carbonate and ethyl acetate were added successively, the organic layer was washed with water and brine and dried over sodium sulfate. The solvent was removed under reduced pressure to afford crude mixture of Compound (79)(1.09 g).
¹ H - NMR (CDCl₃) δ: 1.61-1.80 (2H, m), 2.12-2.51 (5H, m), 3.63 (2H, s), 3.78 (3H, s), 3.95 (1H, br s), 6.54-6.84 (5H, m), 7.09 (1H, t, J = 7.7 Hz), 7.25-7.39 (2H, m).

### Step 9

The crude mixture of Compound (79) (4.32 g) was dissolved in methanol (10 ml) and 1 mol/l solution of sodium methoxide in methanol (3.95 ml) was added at room temperature and the mixture was stirred for 1 hour. Water and ethyl acetate were added successively, and the organic layer was washed with water and brine and dried over sodium sulfate. The solvent was removed under reduced pressure and the resulting residue was purified by chromatography to afford Compound (80) (559 mg).
¹ H - NMR (CDCl₃) δ: 2.01 (3H, s), 3.68 (2H, s), 3.80 (3H, s), 4.06 (1H, br s), 6.59-6.62 (2H, m), 6.67-6.70 (1H, m), 6.83-6.88 (2H, m), 7.09-7.15 (1H, m), 7.21-7.26 (2H, m).

### Step 10

Compound (80) (542 mg) was dissolved in 2 mol/l solution of hydrochloric acid in methanol (20 ml) and the mixture was stirred at room temperature for 1 hour 15 minutes. A saturated aqueous solution of sodium hydrogen carbonate and ethyl acetate were added successively, the organic layer was washed with brine, and dried over sodium sulfate. The solvent was removed under reduced pressure and the resulting residue was purified by chromatography to afford Compound (81) (526 mg).
¹ H - NMR (DMSO-d₆) δ: 1.63 (s) and 1.79 (s) (3H), 3.43-3.72 (6H, m), 4.50 (2H, s), 4.92 (s) and 5.01 (s) (2H), 6.31-6.60 (3H, m), 6.77-6.95 (3H, m), 7.22 (d, J = 8.8 Hz) and 7.36 (2d, J = 8.8 Hz) (2H).

### Step 11

Compound (81) (29.9 mg) was dissolved in methanol (0.6 ml) and 2 mol/l aqueous solution of hydrochloric acid (50 µl) was added at room temperature, and the mixture was stirred for 10 minutes. 5-cyanopyridine-2-carboxylic acid monohydrate (29.9 mg) and WSCD HCl (23.0 mg) were added and the mixture was stirred for 1 hour. A saturated aqueous solution of sodium hydrogen carbonate and ethyl acetate were added successively and the organic layer was washed with brine and dried over sodium sulfate. The solvent was removed under reduced pressure and the resulting residue was purified by chromatography to afford Compound 98 (38.7 mg).
¹ H - NMR (DMSO-d₆) δ: 1.74 (s) and 1.89 (s) (3H), 3.44 (s) and 4.59 (s) (2H), 3.70-3.73 (6H, m), 6.80-6.89 (2H, m), 7.06-7.38 (4H, m), 7.71 (d, J = 6.5 Hz) and 7.81 (d, J = 6.5 Hz) (1H), 7.95 (s) and 8.01 (s) (1H), 8.26 (1H, d, J = 7.9 Hz), 8.56 (1H, dd, J = 8.2, 2.1 Hz), 9.17 (1H, s), 10.68 (s) and 10.78 (s) (1H).

### Example 14

### Synthesis of Compound 99

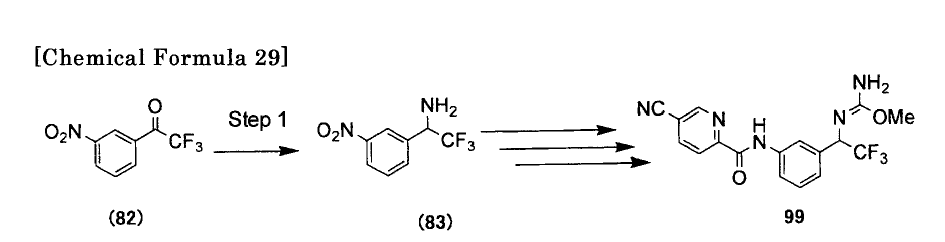

### Step 1

Compound (83) was synthesized from Compound (82) in one step according to the method disclosed in a literature (Org. Lett. 2005. 7. 355.).
¹ H - NMR (CDCl₃) δ: 1.86 (br s, 2H), 4.55 (br s, 1H), 7.59 (t, J = 8.0 Hz, 1H), 7.80 (d, J = 7.7 Hz, 1H), 8.25 (dq, J = 8.2, 1.0 Hz, 1H), 8.35 (s, 1H).

Compound 99 was synthesized from Compound (83) according to the aforementioned procedure for synthesis of Compound g.
¹ H - NMR (CDCl₃)δ: 1.55 (d, J = 18.8 Hz, 4H), 3.80 (s, 3H), 3.89 (s, 2H), 4.58 (q, J = 7.3 Hz, 1H), 7.30 (d, J = 7.9 Hz, 1H), 7.41 (t, J = 7.9 Hz, 1H), 7.81 (s, 1H), 7.85 (m, 1H), 8.21 (dd, J = 8.1, 2.0 Hz, 1H), 8.43 (dd, J = 8.2, 0.8 Hz, 1H), 8.90 (dd, J = 2.0, 0.9 Hz, 1H), 9.89 (s, 1H).

### Example 15

### Synthesis of Compound 102

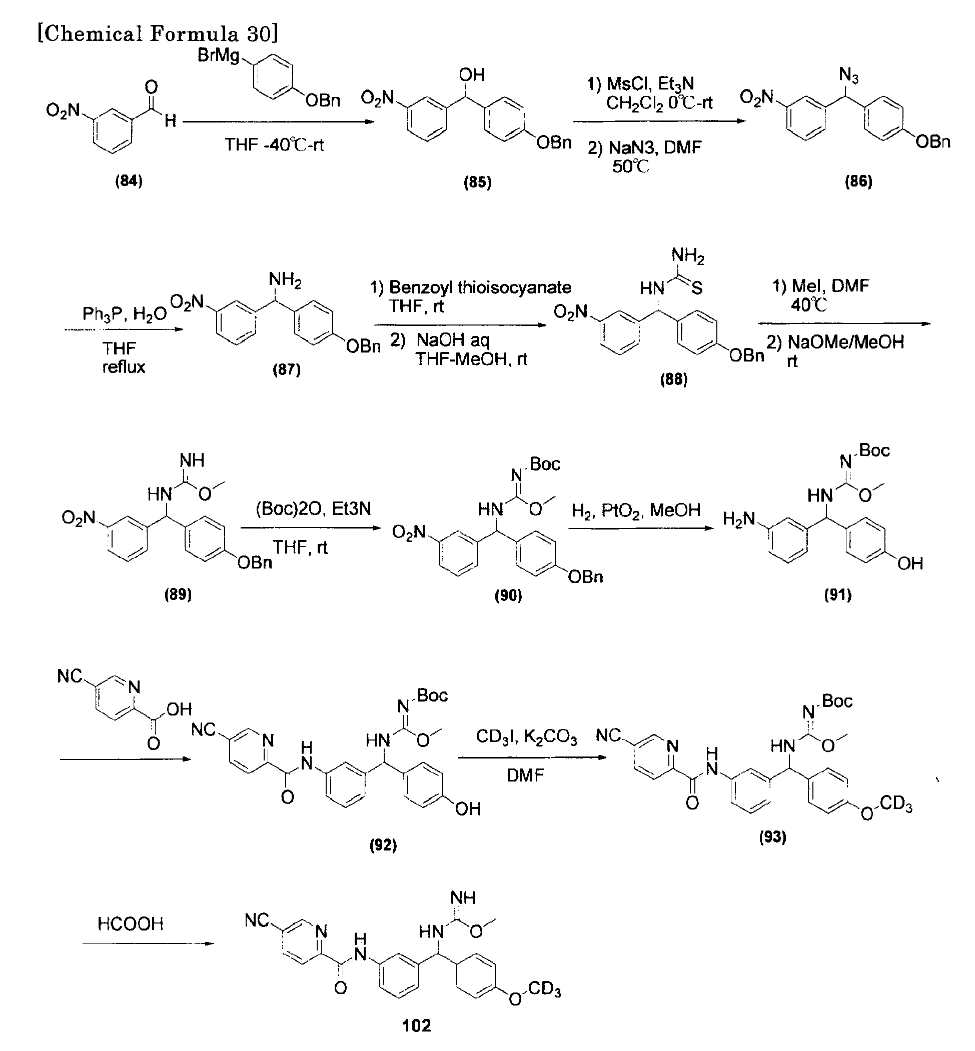

### Step 1

3-nitro benzoaldehyde(7.56 g) was dissolved in tetrahydrofuran (100 ml) and a solution of (4-(benzyloxy)phenyl)magnesium bromide in tetrahydrofuran (1.0 mol/l, 55 ml) was added at -40°C over 30 minutes. After warmed to 0°C, the mixture was stirred for 20 minutes. 10% Aqueous solution of citric acid (50 ml) was added to the reaction mixture and the mixture was extracted with ethyl acetate. The organic layer was washed with brine and dried over magnesium sulfate. The solvent was removed under reduced pressure and the resulting residue was purified by chromatography to afford Compound (85) (16.77g).
¹ H - NMR (CDCl₃) δ: 2.42 (d, J = 3.1 Hz, 1H), 5.06 (s, 3H), 5.88 (br s, 1H), 6.92-7.00 (m, 3H), 7.24-7.53 (m, 7H), 7.70 (d, J = 7.2 Hz, 1H), 8.11 (d, J = 7.5 Hz, 1H), 8.28 (s, 1H).

### Step 2

Compound (85) (14.15 g) was dissolved in dichloromethane (100 ml) and triethylamine (8.8 ml) was added at 0°C ,then methane sulfonyl chloride(5.80 g) was added over 10 minutes. The mixture was warmed to room temperature and stirred for 30 minutes. The reaction mixture was concentrated under reduced pressure, water and ethyl acetate were added to the residue and the layers were separated. The organic layer was washed with a saturated aqueous solution of ammonium chloride, a saturated aqueous solution of sodium hydrogen carbonate and brine and dried over magnesium sulfate. The solvent was removed under reduced pressure, the resulting residue was dissolved in dimethyl formamide (50 ml) and sodium azide (3.57 g) was added at room temperature. The reaction mixture was warmed to 50°C and stirred for 30 minutes. Water was added to the reaction mixture and extracted with ethyl acetate. The organic layer was washed with water and saturated brine and dried over magnesium sulfate. The solvent was removed under reduced pressure and the resulting residue was purified by chromatography to afford Compound (86) (15.21g).
¹ H - NMR (CDCl₃) δ: 5.07 (s, 2H), 5.77 (s, 1H), 6.96-7.02 (m, 2H), 7.18-7.24 (m, 2H), 7.30-7.45 (m, 5H), 7.53 (t, J = 7.9 Hz, 1H), 7.65 (d, J = 7.8 Hz, 1H), 8.13-8.18 (m, 1H), 8.19-8.22 (m, 1H).

### Step 3

Compound (86) (9.69 g) was dissolved in tetrahydrofuran (50 ml) and triphenylphosphine (7.76 g) and water (4.84 g) were added at room temperature. The reaction mixture was warmed to 60°C and stirred for 8 hours. The reaction mixture was concentrated under reduced pressure and ethyl acetate (100 ml) and 1 mol/l hydrochloric acid (100 ml) were added to the residue. The resulting precipitate was collected by filtration and washed with ethyl acetate and water. The obtained solid was dissolved in a mixture of ethyl acetate (30 ml)-methanol (20 ml)- a saturated aqueous solution of sodium hydrogen carbonate (50 ml) and the layers were separated. The aqueous layer was extracted with ethyl acetate and the organic layers were combined. The combined organic layer was washed with saturated brine and dried over magnesium sulfate. The solvent was removed under reduced pressure to afford Compound (87) (crude yield: 8.99 g).

### Step 4

Compound (87) (7.57 g) was dissolved in tetrahydrofuran (50 ml) and benzoyl isothiocyanate(4.15 g) was added at room temperature, and the mixture was stirred for 30 minutes. Methanol(50 ml) and 1 mol/l aqueous solution of sodium hydroxide(25 ml) were added to the reaction mixture and the reaction mixture was stirred at room temperature overnight. The reaction mixture was concentrated under reduced pressure and water was added to the residue. The resulting solid was collected by filtration, washed with water and washed under reduced pressure to afford Compound (88) (8.46 g).
¹ H - NMR (CDCl₃) δ: 5.10 (s, 2H), 5.82 (s, 2H), 6.67 (d, 2H), 6.97-7.03 (m, 2H), 7.17-7.22 (m, 2H), 7.35-7.46 (m, 5H), 7.59 (t, J = 7.9 Hz, 1H), 7.69 (d, J = 7.7 Hz, 1H), 8.15-8.17 (m, 1H), 8.17-8.22 (m, 1H).

### Step 5

Compound (88) (2.00 g) was dissolved in dimethylformamide (10 ml) and methane iodide (0.35 ml) was added at room temperature. The reaction mixture was warmed to 40°C and stirred for 1.5 hours. A saturated aqueous solution of sodium hydrogen carbonate was added and the mixture was extracted with ethyl acetate. The organic layer was washed with saturated brine, dried over magnesium sulfate and the solvent was removed under reduced pressure. To the resulting residue was added a solution of sodium methoxide in methanol (1 mol/l, 16.8 ml) at room temperature and the mixture was stirred at the same temperature for 3 days. Water was added thereto and the mixture was extracted with ethyl acetate, the organic layer was washed with saturated brine and dried over magnesium sulfate. The solvent was removed under reduced pressure and the resulting residue was partially purified by chromatography to afford Compound (89) (crude yield: 1.99 g).

### Step 6

Compound (89) (1.35 g) was dissolved in tetrahydrofuran (10 ml) and di-tert-butyl dicarbonate (2.00 ml) and triethylamine (1.43 ml) were added at room temperature, and the mixture was stirred at the same temperature for 1 hour. The reaction mixture was concentrated and the residue was purified by chromatography to afford Compound (90) (1.99g).
¹ H - NMR (CDCl₃) δ: 1.51 (s, 9H), 3.81 (s, 3H), 5.05 (s, 2H), 6.02 (d, J = 7.5 Hz, 1H), 6.92-6.98 (m, 2H), 7.11-7.17 (m, 2H), 7.28-7.43 (m, 5H), 7.51 (t, J = 7.9 Hz, 1H), 7.61 (d, J = 7.8 Hz, 1H), 8.07-8.15 (m, 2H), 9.49 (d, J = 7.2 Hz, 1H).

### Step 7

Compound (90) (1.18 g) was dissolved in methanol (10 ml) and PtO₂ (500 mg) was added. The mixture was stirred under hydrogen atmosphere at room temperature for 8 hours. Insoluble material was filtered off through celite, the filtrate was concentrated under reduced pressure and the residue was purified by chromatography to afford Compound (91) (760 mg).
¹H - N M R (CDCl₃) δ: 1.49 (s, 9H), 3.79 (s, 3H), 5.81 (d, J = 7.5 Hz, 1H), 6.52-6.72 (m, 5H), 6.99-7.13 (m, 3H), 9.30 (d, J = 7.6 Hz, 1H).

### Step 8

Compound (91) (502 mg) was dissolved in methanol (3 ml), 5-cyano picoline acid (220 mg) and 4-(4,6-dimethoxy-1,3,5-triazine-2-yl)-4-methyl morpholinium chloride (660 mg) were added at room temperature and the mixture was stirred at the same temperature for 2 hours. Water was added to the reaction mixture and the mixture was extracted with ethyl acetate. The organic layer was washed with saturated brine, dried over magnesium sulfate and the solvent was removed under reduced pressure. The resulting residue was purified by chromatography to afford Compound (92) (502 mg).
¹H - N M R (CDCl₃) δ: 1.52 (s, 9H), 3.83 (s, 3H), 5.98 (d, J = 6.9 Hz, 1H), 6.66 (br s, 1H), 6.77-6.84 (m, 2H), 7.07-7.16 (m, 3H), 7.40 (t, J = 7.9 Hz, 1H), 7.59 (br s, 1H), 7.81 (d, J = 8.2 Hz, 1H), 8.22 (dd, J = 8.1, 1.9 Hz, 1H), 8.43 (dd, J = 8.1, 0.9 Hz, 1H), 8.90-8.93 (m, 1H), 9.41 (d, J = 7.6 Hz, 1H), 9.91 (s, 1H).

### Step 9

Compound (92) (50 mg) was dissolved in dimethylformamide(1 ml), potassium carbonate (21 mg) and methane iodide-D3 (17 mg) were added at room temperature and the mixture was stirred at the same temperature for 90 minutes. Water was added to the reaction mixture and the mixture was extracted with ethyl acetate. The organic layer was washed with saturated brine, dried over magnesium sulfate and the solvent was removed under reduced pressure. The resulting residue was purified by chromatography to afford Compound (93) (40 mg).
¹H - N M R (CDCl₃) δ: 1.52 (s, 9H), 3.82 (s, 3H), 5.98 (d, J = 7.5 Hz, 1H), 6.84-6.88 (m, 2H), 7.08 (d, J = 7.3 Hz, 1H), 7.16-7.20 (m, 2H), 7.38 (t, J = 7.9 Hz, 1H), 7.51 (br s, 1H), 7.84 (d, J = 7.6 Hz, 1H), 8.20 (dd, J = 8.2, 2.1 Hz, 1H), 8.39-8.45 (m, 1H), 8.86-8.91 (m, 1H), 9.42 (d, J = 7.0 Hz, 1H), 9.84 (s, 1H).

### Step 10

Compound (93) (40 mg) was dissolved in formic acid (1 ml) and stirred at 40°C for 90 minutes. The reaction mixture was concentrated under reduced pressure, a saturated aqueous solution of sodium hydrogen carbonate was added to the resulting residue and the mixture was extracted with ethyl acetate. The organic layer was washed with saturated brine, dried over magnesium sulfate and the solvent was removed under reduced pressure. The resulting residue was purified by chromatography to afford Compound 102 (4 mg). ¹ H - NMR (CDCl₃) δ: 3.71 (s, 3H), 5.51 (br s, 1H), 6.81-6.86 (m, 2H), 7.15 (d, J = 8.2 Hz,
1H), 7.22-7.32 (m, 3H), 7.68 (d, J = 8.5 Hz, 1H), 7.94 (br s, 1H), 8.18 (s, 1H), 8.26 (d, J = 7.5 Hz, 1H), 8.55-8.60 (m, 1H), 9.18 (s, 1H), 10.71 (s, 1H).

The following compounds are synthesized in a similar manner to the above Examples.
With respect to LC-MS in the table, the unit of retention time is "minute", and the measurement conditions were as follows:

### (Method A)

column: Xbridge C18 (5 µm, i.d. 4.6 x 50 mm)(Waters)
Flow rate: 3 mL/min.
UV detection wavelength: 254 nm
Mobile phase: [A] 0.1% formic acid-containing aqueous solution; [B] 0.1% formic aci d-containing acetonitrile solution
Gradient: performing linear gradient of 10% to 100% solvent [B] for 3 minutes, and keeping 100% solvent [B] for 1 minute

### (MethodB)

column: Shim-pack XR-ODS (2.2 µm, i.d. 50 x 3.0 mm) (Shimadzu)
Flow rate: 1.6 mL/min.
UV detection wavelength: 254 nm
Mobile phase: [A] 0.1% formic acid-containing aqueous solution; [B] 0.1% formic aci d-containing acetonitrile solution
Gradient: performing linear gradient of 10% to 100% solvent [B] for 3 minutes, and keeping 100% solvent [B] for 1 minute

### (Method C)

column: Gemini-NX (5 µm, i.d. 4.6 x 50 mm) (Phenomenex)
Flow rate: 3 mL/min.
UV detection wavelength: 254nm
Mobile phase: [A] 0.1% formic acid-containing aqueous solution; [B] 0.1% formic aci d-containing methanol solution
Gradient: performing linear gradient of 5% to 100% solvent [B] for 3.5 minutes, and keeping 100% solvent [B] for 0.5 minute

**[Table 1]**

| Compound No. | Structure | LC/MS m/z | LC/MS Retention time | LC/MS Measurement Method |
|---|---|---|---|---|
| 1 | 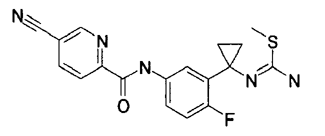 | 370 | 1.10 | Method B |
| 2 | 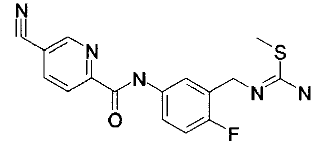 | 344 | 1.01 | Method B |
| 3 | 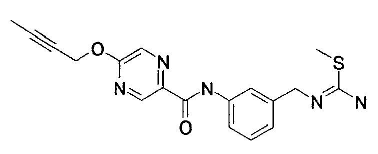 | 370 | 1.30 | Method B |
| 4 | 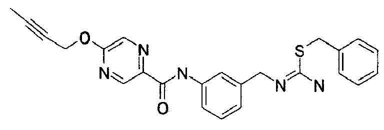 | 446 | 1.57 | Method B |
| 5 | 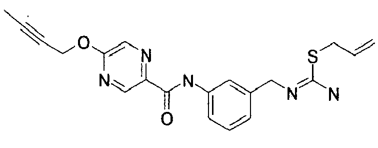 | 396 | 1.40 | Method B |
| 6 | 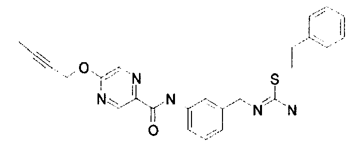 | 460 | 1.65 | Method B |
| 7 | 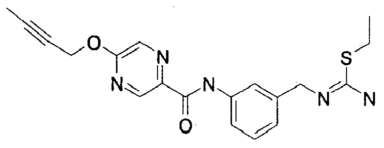 | 384 | 1.45 | Method B |

**[Table 2]**

| Compound No. | Structure | LC/MS m/z | LC/MS Retention time | LC/MS Measurement Method |
|---|---|---|---|---|
| 8 | 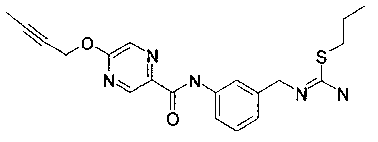 | 398 | 1.49 | Method B |
| 9 | 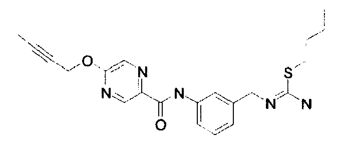 | 412 | 1.58 | Method B |
| 10 | 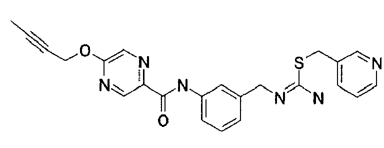 | 447 | 1.16 | Method B |
| 11 | 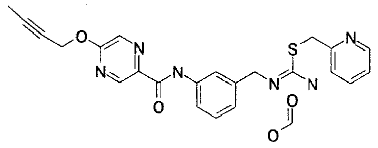 | 447 | 1.46 | Method B |
| 12 | 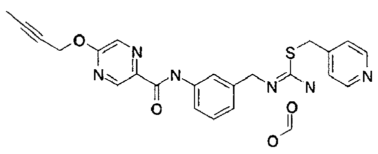 | 447 | 1.09 | Method B |
| 13 | 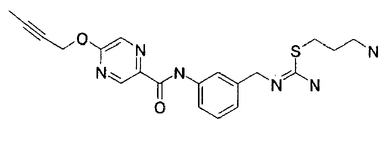 | 413 | 0.90 | Method B |
| 14 | 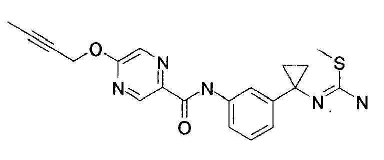 | 396 | 1.33 | Method B |

**[Table 3]**

| Compound No. | Structure | LC/MS m/z | LC/MS Retention time | LC/MS Measurement Method |
|---|---|---|---|---|
| 15 | 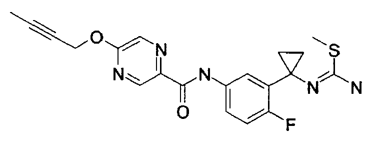 | 414 | 1.37 | Method B |
| 16 | 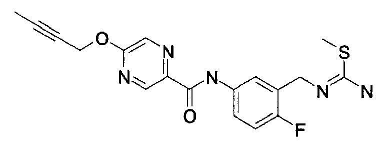 | 388 | 1.26 | Method B |
| 17 | 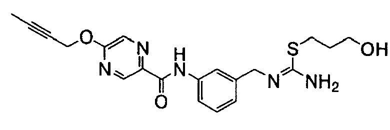 | 414 | 1.23 | Method B |
| 18 | 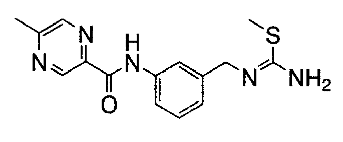 | 316 | 0.88 | Method B |
| 19 | 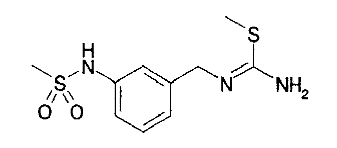 | 274 | 0.50 | Method B |
| 20 | 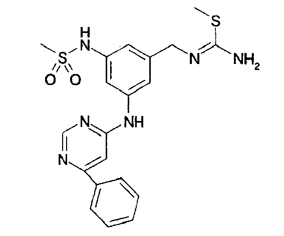 | 443 | 0.93 | Method B |

**[Table 4]**

| Compound No. | Structure | LC/MS m/z | LC/MS Retention time | LC/MS Measurement Method |
|---|---|---|---|---|
| 21 | 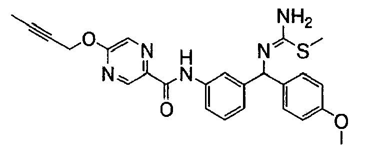 | 476 | 1.52 | Method B |
| 22 | 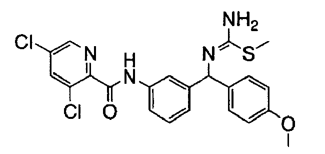 | 475 | 1.50 | Method B |
| 23 | 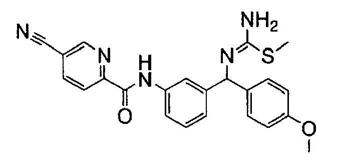 | 432 | 1.27 | Method B |
| 24 | 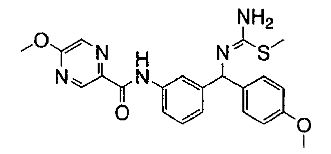 | 438 | 1.27 | Method B |
| 25 | 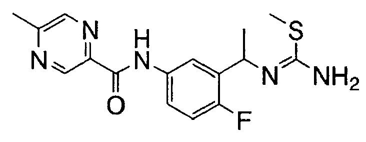 | 348 | 0.98 | Method B |
| 26 | 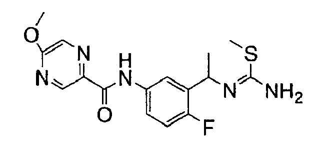 | 364 | 1.16 | Method B |
| 27 | 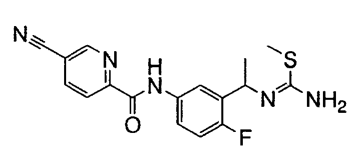 | 358 | 1.12 | Method B |

**[Table 5]**

| Compound No. | Structure | LC/MS m/z | LC/MS Retention time | LC/MS Measurement Method |
|---|---|---|---|---|
| 28 | 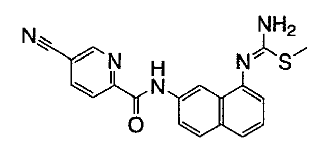 | 362 | 1.16 | Method B |
| 29 | 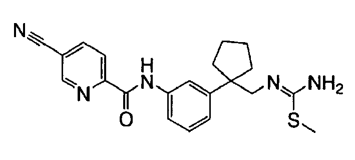 | 394 | 1.44 | Method B |
| 30 | 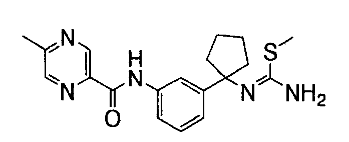 | 370 | 1.17 | Method B |
| 31 | 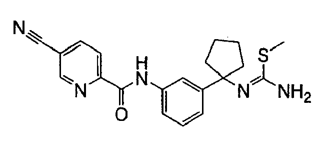 | 380 | 1.27 | Method B |
| 32 | 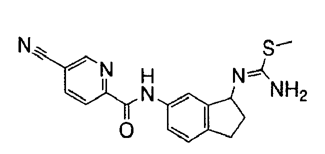 | 352 | 1.18 | Method B |
| 33 | 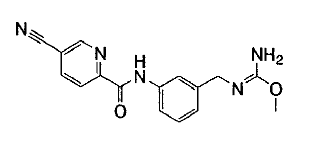 | | | |

**[Table 6]**

| Compound No. | Structure | LC/MS m/z | LC/MS Retention time | LC/MS Measurement Method |
|---|---|---|---|---|
| 34 | 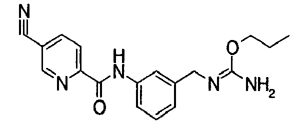 | 338 | 1.12 | Method A |
| 35 | 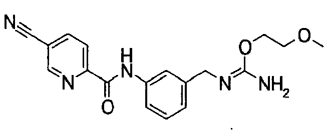 | 354 | 0.95 | Method A |
| 36 | 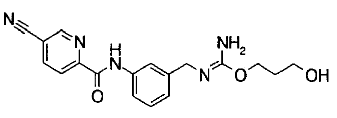 | 354 | 0.81 | Method A |
| 37 | 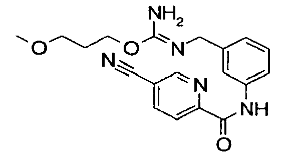 | 368 | 0.99 | Method A |
| 38 | 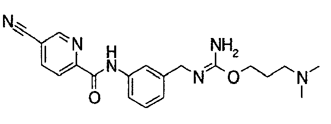 | 381 | 0.48 | Method A |
| 39 | 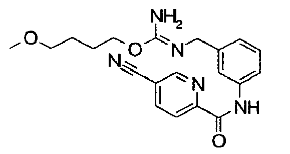 | 382 | 1.05 | Method A |
| 40 | 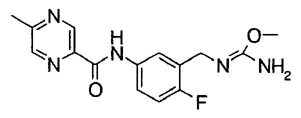 | 318 | 0.95 | Method B |
| 41 | 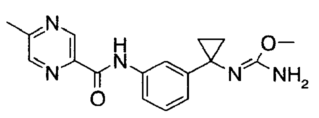 | 326 | 0.92 | Method B |

**[Table 7]**

| Compound No. | Structure | LC/MS m/z | LC/MS Retention time | LC/MS Measurement Method |
|---|---|---|---|---|
| 42 | 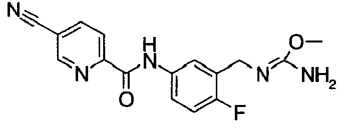 | 328 | 1.02 | Method B |
| 43 | 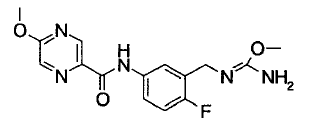 | 334 | 1.08 | Method B |
| 44 | 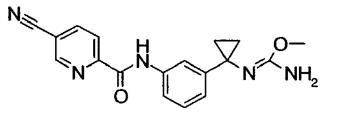 | 336 | 1.02 | Method B |
| 45 | 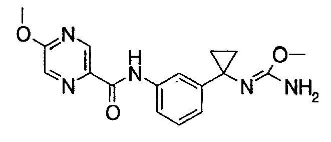 | | | |
| 46 | 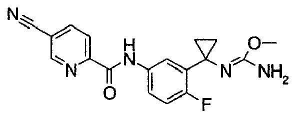 | 354 | 1.08 | Method B |
| 47 | 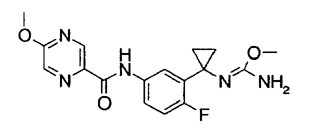 | 360 | 1.14 | Method B |
| 48 | 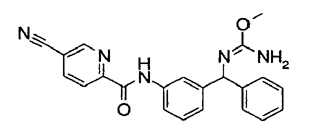 | 386 | 1.34 | Method B |
| 49 | 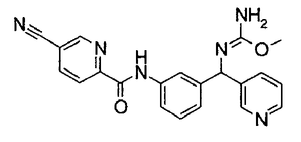 | 388 | 0.96 | Method B |

**[Table 8]**

| Compound No. | Structure | LC/MS m/z | LC/MS Retention time | LC/MS Measurement Method |
|---|---|---|---|---|
| 50 | 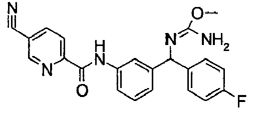 | 404 | 1.40 | Method B |
| 51 | 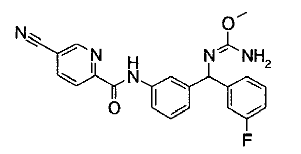 | 404 | 1.44 | Method B |
| 52 | 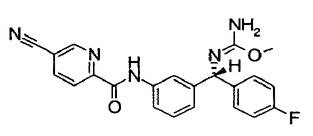 | | | |
| 53 | 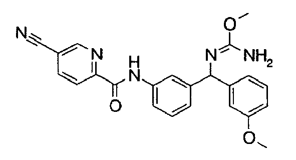 | 416 | 1.34 | Method B |
| 54 | 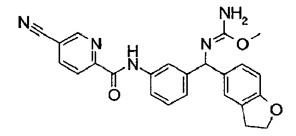 | 428 | 1.33 | Method A |
| 55 | 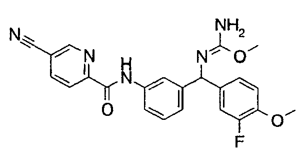 | 434 | 1.32 | Method B |
| 56 | 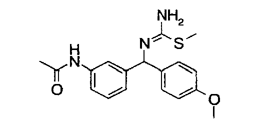 | 344 | 0.92 | Method A |
| 57 | 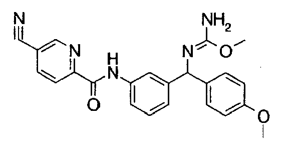 | 416 | 1.36 | Method B |

**[Table 9]**

| Compound No. | Structure | LC/MS m/z | LC/MS Retention time | LC/MS Measurement Method |
|---|---|---|---|---|
| 58 | 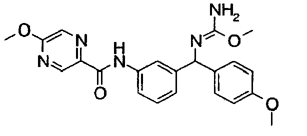 | 422 | 1.39 | Method B |
| 59 | 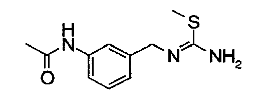 | 238 | 0.39 | Method B |
| 60 | 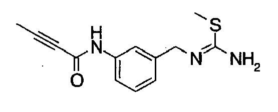 | 262 | 0.72 | Method B |
| 61 | 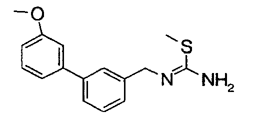 | 287 | 1.28 | Method B |
| 62 | 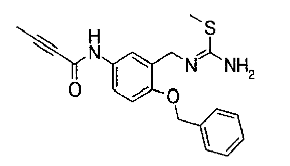 | 368 | 1.26 | Method B |
| 63 | 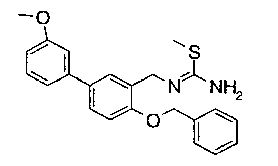 | 393 | 1.61 | Method B |
| 64 | 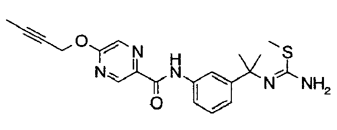 | 398 | 1.33 | Method B |
| 65 | 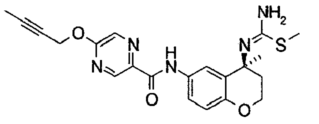 | 426 | 1.33 | Method B |

**[Table 10]**

| Compound No. | Structure | LC/MS m/z | LC/MS Retention time | LC/MS Measurement Method |
|---|---|---|---|---|
| 66 | 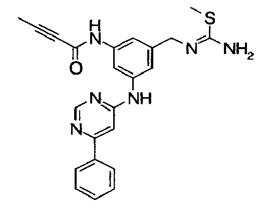 | 431 | 1.10 | Method B |
| 67 | 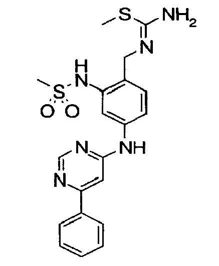 | 443 | 0.94 | Method B |
| 68 | 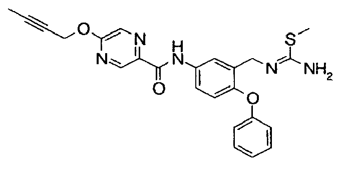 | 462 | 1.55 | Method B |
| 69 | 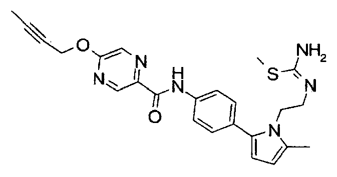 | 463 | 1.55 | Method B |
| 70 | 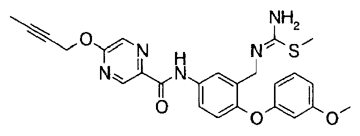 | 492 | 1.47 | Method B |
| 71 | 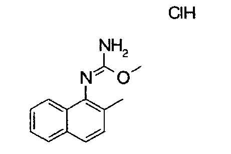 | | | |

**[Table 11]**

| Compound No. | Structure | LC/MS m/z | LC/MS Retention time | LC/MS Measurement Method |
|---|---|---|---|---|
| 72 | 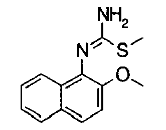 | | | |
| 73 | 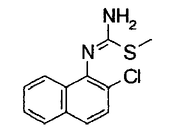 | | | |
| 74 | 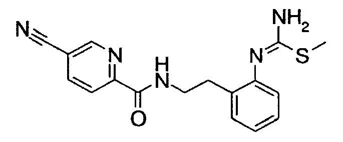 | | | |
| 75 | 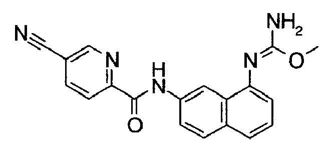 | | | |
| 76 | 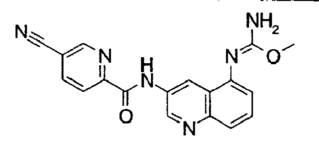 | | | |
| 77 | 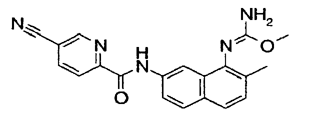 | | | |
| 78 | 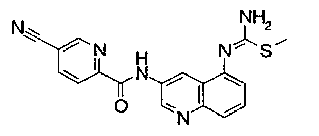 | | | |
| 79 | 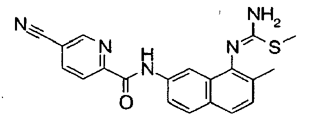 | | | |

**[Table 12]**

| Compound No. | Structure | LC/MS m/z | LC/MS Retention time | LC/MS Measurement Method |
|---|---|---|---|---|
| 80 | 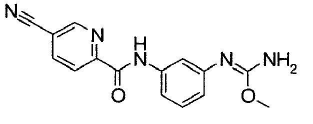 | 296 | 0.90 | Method B |
| 81 | 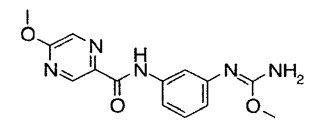 | 302 | 0.94 | Method B |
| 82 | 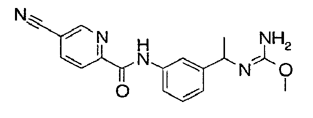 | 324 | 1.09 | Method B |
| 83 | 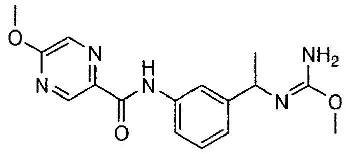 | 330 | 1.11 | Method B |
| 84 | 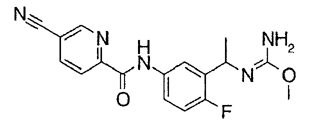 | 342 | 0.94 | Method A |
| 85 | 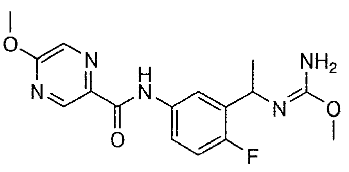 | 348 | 0.99 | Method A |
| 86 | 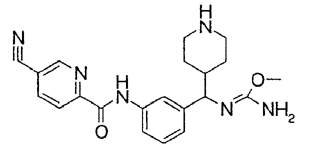 | 393 | 0.40 | Method B |
| 87 | 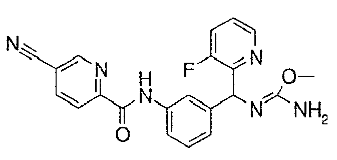 | 405 | 1.23 | Method B |

**[Table 13]**

| Compound No. | Structure | LC/MS m/z | LC/MS Retention time | LC/MS Measurement Method |
|---|---|---|---|---|
| 88 | 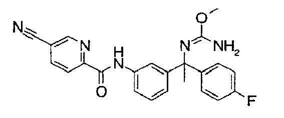 | 418 | 1.47 | Method B, |
| 89 | 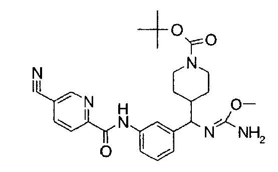 | 493 | 1.53 | Method B |
| 90 | 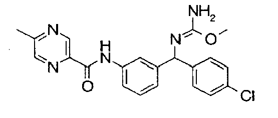 | 410 | 1.38 | Method B |
| 91 | 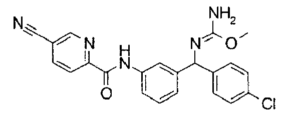 | 420 | 1.48 | Method B |
| 92 | 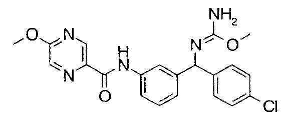 | 426 | 1.54 | Method B |
| 93 | 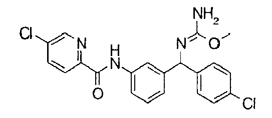 | 429 | 1.64 | Method B |
| 94 | 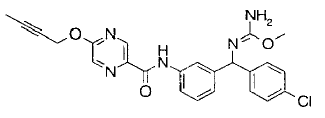 | 464 | 1.72 | Method B |
| 95 | 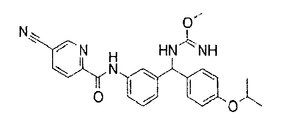 | 444 | 1.06 | Method A |
| 96 | 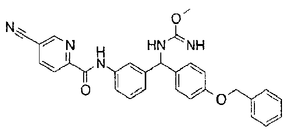 | 492 | 2.30 | Method A |

**[Table 14]**

| Compound No. | Structure | LC/MS m/z | LC/MS Retention time | LC/MS Measurement Method |
|---|---|---|---|---|
| 97 | 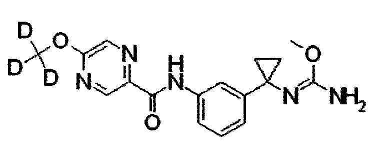 | 345 | 0.97 | Method A |
| 98 | 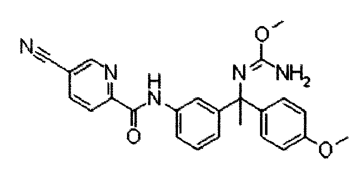 | 430 | 1.33 | Method A |
| 99 | 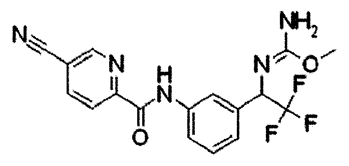 | 378 | 1.12 | Method B |
| 100 | 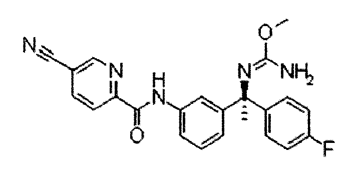 | 418 | 1.36 | Method B |
| 101 | 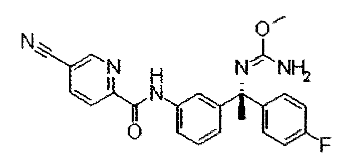 | 418 | 1.32 | Method B |
| 102 | 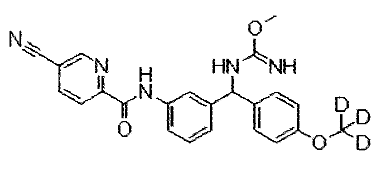 | 419 | 1.89 | Method C |

**[Table 15]**

| Compound No. | Structure |
|---|---|
| 103 | 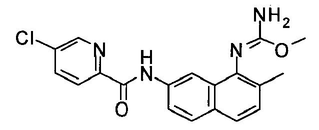 |
| 104 | 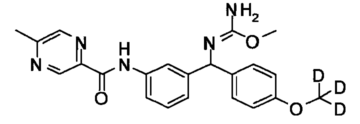 |
| 105 | 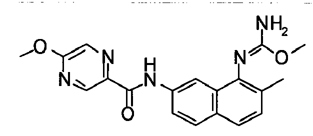 |
| 106 | 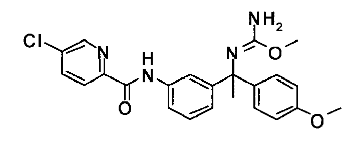 |
| 107 | 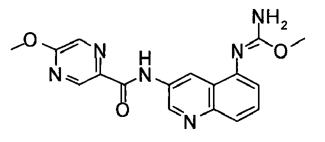 |
| 108 | 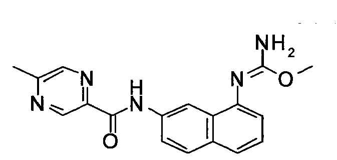 |
| 109 | 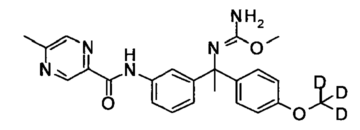 |
| 110 | 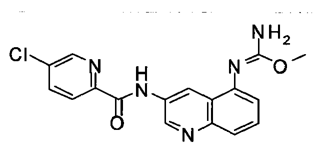 |
| 111 | 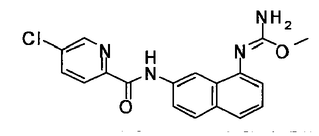 |
| 112 | 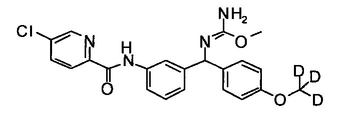 |

**[Table 16]**

| Compound No. | Structure |
|---|---|
| 113 | 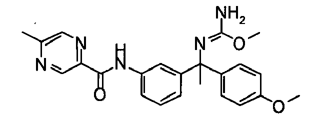 |
| 114 | 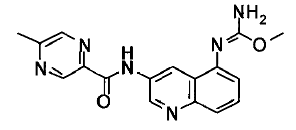 |
| 115 | 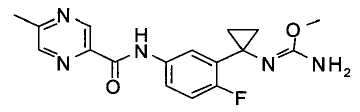 |
| 116 | 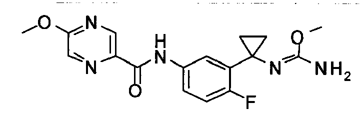 |
| 117 | 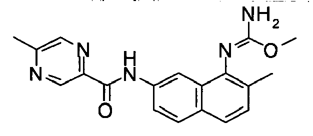 |
| 118 | 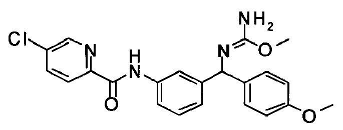 |
| 119 | 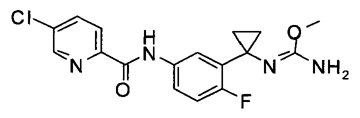 |
| 120 | 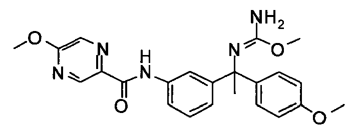 |
| 121 | 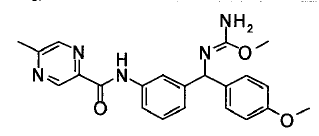 |
| 122 | 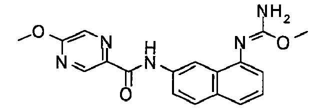 |

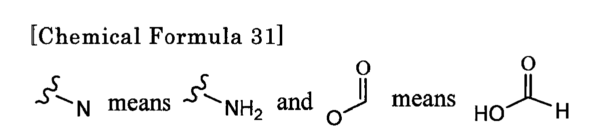

in the tables.

NMR data of the compounds other than the above Examples 1 to 10 are as follows:

### Compound 14

¹ H - NMR (CDCl₃) δ: 1.35-1.39 (4H, m), 1.88-1.90 (3H, m), 2.40 (3H, s), 5.04-5.05 (2H, m), 6.99 (1H, d, J = 8.11 Hz), 7.29-7.34 (1H, m), 7.51 (1H, s), 7.62 (1H, d, J = 8.11 Hz), 8.21 (1H, s), 9.02 (1H, s), 9.48 (1H, s).

### Compound 27

¹ H - NMR (CD₃OD) δ: 1.66 (d, J = 6.9 Hz, 3H), 2.67 (s, 3H), 5.23 (d, J = 6.4 Hz, 1H), 7.21 (dd, J = 10.2, 8.9 Hz, 1H), 7.75 (ddd, J = 8.8, 4.5, 2.8 Hz, 1H), 7.98 (dd, J = 6.8, 2.6 Hz, 1H), 8.35 (dd, J = 8.1, 0.9 Hz, 1H), 8.42 (dd, J = 8.2, 2.0 Hz, 1H), 9.05 (dd, J = 2.0, 0.8 Hz, 1H).

### Compound 34

¹H-NMR (DMSO-d₆) δ: 0.88 (3H, t, J = 7.4 Hz), 1.60-1.65 (2H, m), 4.05 (2H, t, J = 6.6 Hz), 4.22 (2H, s), 7.08 (1H, d, J = 7.6 Hz), 7.31 (1H, t, J = 7.9 Hz), 7.73-7.74 (1H, m), 7.87 (1H, s), 8.27 (1H, dd, J = 8.1, 0.9 Hz), 8.35 (1H, s), 8.57 (1H, dd, J = 8.2, 2.1 Hz), 9.19 (1H, dd, J = 2.1, 0.9 Hz), 10.77 (1H, s).

### Compound 35

¹H-NMR (CDCl₃) δ: 3.42 (3H, s), 3.65 (2H, t, J = 4.5 Hz), 4.30 (4H, s), 7.14-7.17 (1H, m), 7.35-7.39 (1H, m), 7.70-7.71 (2H, m), 8.20 (1H, dd, J = 8.2, 2.1 Hz), 8.43 (1H, dd, J = 8.2, 0.8 Hz), 8.90 (1H, dd, J = 2.0, 0.9 Hz), 9.86 (1H, s).

### Compound 36

¹H-NMR (CDCl₃) δ: 1.81-1.83 (2H, m), 3.61 (2H, t, J = 5.6 Hz), 4.29 (2H, s), 4.39 (2H, t, J = 5.6 Hz), 7.13 (1H, d, J = 7.6 Hz), 7.37 (1H, t, J = 7.9 Hz), 7.61 (1H, d, J = 7.9 Hz), 7.80 (1H, s), 8.20 (1H, dd, J = 8.1, 2.0 Hz), 8.42 (1H, dd, J = 8.1, 0.6 Hz), 8.89 (1H, dd, J = 1.8, 0.8 Hz), 9.86 (1H, s).

### Compound 37

¹H-NMR (CDCl₃) δ: 1.94-1.96 (2H, m), 3.33 (3H, s), 3.47 (2H, t, J = 6.1 Hz), 4.16 (2H, s), 4.31 (2H, s), 7.13-7.15 (1H, m), 7.37 (1H, t, J = 7.9 Hz), 7.69-7.71 (3H, m), 8.20 (1H, dd, J = 8.2, 2.1 Hz), 8.43 (1H, dd, J = 8.1, 0.8 Hz), 8.89 (1H, dd, J = 2.0, 0.6 Hz), 9.85 (1H, s).

### Compound 38

¹H-NMR (CDCl₃) δ: 1.84-1.86 (2H, m), 2.19 (6H, s), 2.31 (2H, t, J = 7.0 Hz), 4.26 (2H, t, J = 5.8 Hz), 4.31 (2H, s), 7.09 (1H, d, J = 7.6 Hz), 7.32 (1H, t, J = 7.9 Hz), 7.65 (1H, s), 7.71 (1H, d, J = 7.9 Hz), 8.16 (1H, dd, J = 8.2, 2.0 Hz), 8.38 (1H, d, J = 8.1 Hz), 8.85 (1H, d, J = 1.4 Hz), 9.93 (1H, s).

### Compound 39

¹H-NMR (CDCl₃) δ: 1.71-1.76 (4H, m), 3.29 (3H, s), 3.36 (2H, t, J = 6.1 Hz), 4.20 (2H, t, J = 6.2 Hz), 4.30 (2H, s), 7.10 (1H, d, J = 7.8 Hz), 7.33 (1H, t, J = 7.8 Hz), 7.67-7.70 (2H, m), 8.18 (1H, dd, J = 8.1, 2.0 Hz), 8.39 (1H, d, J = 8.1 Hz), 8.86 (1H, d, J = 1.2 Hz), 9.88 (1H, s).

### Compound 45

¹H-NMR (CDCl₃) δ: 1.31 (s, 4H), 3.95 (s, 3H), 4.07 (s, 3H), 7.01 (d, J = 8.5 Hz, 1H), 7.30 (t, J = 7.9 Hz, 1H), 7.55 (t, J = 1.8 Hz, 1H), 7.62 (d, J = 8.1 Hz, 1H), 8.16 (d, J = 1.3 Hz, 1H), 9.00 (d, J = 1.3 Hz, 1H), 9.50 (s, 1H).

### Compound 52

¹H-NMR (DMSO-d₆) δ: 3.67 (s, 3H), 5.43 (s, 1H), 5.69 (s, 2H), 7.07 (t, J = 8.9 Hz, 2H), 7.18-7.27 (m, 2H), 7.44 (dd, J = 8.6, 5.9 Hz, 2H),7.66 (d, J = 7.7 Hz, 1H), 7.97 (s, 1H), 8.26 (d, J = 8.2 Hz, 1H), 8.56 (dd, J = 8.2, 2.0 Hz, 1H), 9.17 (d, J = 2.0 Hz, 1H), 0.68 (s, 1H).

### Compound 71

¹H-NMR (DMSO-d₆) δ: 2.38 (3H, s), 3.93 (0.5H, s), 4.19 (2.5H, s), 7.51-8.25 (6H, m), 8.25 (0.5H, br), 8.99 (0.5H, br), 10.89 (1H, s)

### Compound 72

¹H-NMR (CDCl₃) δ: 2.67 (3H, br s), 3.93 (3H, s), 4.46 (2H, br s), 7.28 (1H, d, J = 8.9 Hz), 7.34 (1H, ddd, J = 8.1, 6.6, 1.2 Hz), 7.41 (1H, ddd, J = 8.1, 6.6, 1.2 Hz), 7.57 (1H, d, J = 8.9 Hz), 7.75 (1H, dd, J = 8.1, 1.2 Hz), 7.90 (1H, d, J = 8.1, 1.2 Hz)

### Compound 73

¹H-NMR (CDCl₃) δ: 2.63 (3H, s), 4.48 (2H, br s), 7.42-7.51 (4H, m), 7.75-7.89 (2H, m)

### Compound 74

¹H-NMR (CDCl₃) δ: 2.53 (3H, s), 2.86 (2H, t, J = 6.5 Hz), 3.70 (2H, q, J = 6.5 Hz), 4.59 (2H, br s), 6.85-7.21 (4H, m), 8.10 (1H, dd, J = 8.1, 2.0 Hz), 8.21-8.29 (2H, m), 8.80 (1H, dd, J = 2.0, 0.8 Hz)

### Compound 75

¹H-NMR (CDCl₃) δ: 4.06 (3H, s), 4.13 (2H, s), 7.03 (1H, dd, J = 7.2, 1.0 Hz),
7.37 (1H, dd, J = 8.2, 7.2 Hz), 7.52 (1H, dd, J = 8.2, 1.0 Hz), 7.86 (1H, d, J = 8.8 Hz), 7.95 (1H, dd, J = 8.8, 2.2 Hz), 8.21 (1H, dd, J = 8.1, 2.0 Hz), 8.37 (1H, d, J = 2.2 Hz), 8.47 (1H, dd, J = 8.1, 0.9 Hz), 8.92 (1H, dd, J = 2.0, 0.9 Hz), 10.02 (1H, s)

### Compound 76

¹H-NMR (CDCl₃) δ: 4.05 (3H, s), 4.20 (2H, s), 7.10 (1H, dd, J = 7.3, 1.1 Hz),
7.59 (1H, dd, J = 8.5, 7.3 Hz), 7.78 (1H, dd, J = 8.5, 1.1 Hz), 8.23 (1H, dd, J = 8.2, 2.0 Hz), 8.47 (1H, dd, J = 8.2, 0.8 Hz), 8.94 (1H, dd, J = 2.0, 0.8 Hz), 8.98 (1H, d, J = 2.8 Hz), 9.12 (1H, d, J = 2.8 Hz),10.08 (1H, s)

### Compound 77

¹H-NMR (CDCl₃) δ: 2.33 (3H, s), 3.92 (2H, s), 4.09 (3H, s), 7.29 (1H, d, J = 8.4 Hz), 7.46 (1H, d, J = 8.4 Hz), 7.81 (1H, d, J = 8.9 Hz), 7.89 (1H, dd, J = 8.9, 2.0 Hz), 8.20 (1H, d, J = 2.0 Hz), 8.21 (1H, J = 8.2, 2.0 Hz), 8.47 (1H, dd, J = 8.2, 0.9 Hz), 8.92 (1H, dd, J = 2.0, 0.9 Hz), 9.99 (1H, s)

### Compound 78

¹H-NMR (CDCl₃) δ: 2.65 (3H, br s), 4.66 (2H, br s), 7.10 (1H, dd, J = 7.4, 1.0 Hz), 7.60 (1H, dd, J = 8.4, 7.4 Hz), 7.80 (1H, dd, J = 8.4, 1.0 Hz), 8.23 (1H, dd, J = 8.1, 2.0 Hz), 8.47 (1H, dd, J = 8.1, 1.0 Hz), 8.87 (1H, d, J = 2.7 Hz), 8.94 (1H, d, J = 2.0, 1.0 Hz), 9.14 (1H, d, J = 2.7 Hz), 10.08 (1H, s)

### Compound 79

¹H-NMR (CDCl₃) δ: 2.31 (3H, s), 2.72 (3H, s), 4.38 (2H, s), 7.29 (1H, d, J = 8.3 Hz), 7.49 (1H, d, J = 8.3 Hz), 7.83 (1H, d, J = 8.9 Hz), 7.94 (1H, dd, J = 8.9, 2.2 Hz), 8.08 (1H, J = 2.2 Hz), 8.21 (1H, J = 8.1, 2.0 Hz), 8.48 (1H, dd, J = 8.1, 0.9 Hz), 8.94 (1H, dd, J = 2.0, 0.9 Hz), 10.00 (1H, s)

### Compound 97

¹H-NMR (CDCl₃) δ: 1.17-1.40 (4H, m), 3.76 (3H, s), 4.21 (1H, br s), 5.00 (1H, br s), 6.95 (1H, d, J = 8.1 Hz), 7.31 (1H, t, J = 7.9 Hz), 7.55-7.58 (2H, m), 8.15 (1H, d, J = 1.2 Hz), 9.01 (1H, d, J = 1.2 Hz), 9.48 (1H, s).

### Compound 98

¹H-NMR (DMSO-d6) δ: 1.74 (s) and 1.89 (s) (3H), 3.44 (s) and 4.59 (s) (2H), 3.70-3.73 (6H, m), 6.80-6.89 (2H, m), 7.06-7.38 (4H, m), 7.71 (d, J = 6.5 Hz) and 7.81 (d, J = 6.5 Hz) (1H), 7.95 (s) and 8.01 (s) (1H), 8.26 (1H, d, J = 7.9 Hz), 8.56 (1H, dd, J = 8.2, 2.1 Hz), 9.17 (1H, s), 10.68 (s) and 10.78 (s) (1H).

### Compound 102

¹H-NMR (CDCl₃) δ: 3.71 (s, 3H), 5.51 (br s, 1H), 6.81-6.86 (m, 2H), 7.15 (d, J = 8.2 Hz, 1H), 7.22-7.32 (m, 3H), 7.68 (d, J = 8.5 Hz, 1H), 7.94 (br s, 1H), 8.18 (s, 1H), 8.26 (d, J = 7.5 Hz, 1H), 8.55-8.60 (m, 1H), 9.18 (s, 1H), 10.71 (s, 1H).

The effect of the present invention was confirmed by the following Test Examples.

### Test Example 1 Assay of BACE1 inhibiting activity

48.5 µL of substrate peptide solution (Biotin-XSEVNLDAEFRHDSGC-Eu: X=e-amino-n-capronic acid, Eu=Europium cryptate) was added to each well of 96-hole half-area plate (a black plate: Costar Corporation), and after addition of 0.5 µl of the test compound (dissolved in N,N'-dimethylformaldehyde) and 1 µl of Recombinant human BACE1(R&D Systems), the reaction mixture was incubated at 30°C for 3 hours. The substrate peptide was synthesized by reacting Cryptate TBPCOOH mono SMP (CIS bio international) with Biotin-XSEVNLDAEFRHDSGC (Peptide Institute, Inc.). The final concentrations of the substrate peptide and Recombinant human BACE1 were adjusted to 18 nM and 7.4 nM respectively, and the reaction was performed in sodium acetate buffer (50 mM sodium acetate, pH 5.0, 0.008% Triton X-100). After the completion of reaction, 50 µl of 8.0 µg/ml Streptavidin-XL665(CIS bio international) dissolved in phosphate buffer (150 mM K₂HPO₄-KH₂PO₄, pH 7.0, 0.008 % Triton X-100, 0.8 M KF) was added to each well and left stand at 30°C for an hour. Then, fluorescence intensity was measured (excitation wavelength: 320 nm, measuring wavelength: 620 nm and 665 nm) using Wallac 1420 multilabel counter (Perkin Elmer life sciences). Enzymatic activity was determined from counting ratio of each wavelength (10,000 x Count 665/Count 620) and 50% inhibitory concentration against the enzymatic activity was calculated.
IC₅₀ values of the test compounds are indicated in Table 17.

**[Table 17]**

| Compound No. | IC50 (µM) |
|---|---|
| 1 | 3.67 |
| 3 | 0.13 |
| 7 | 1.21 |
| 11 | 0.29 |
| 12 | 0.27 |
| 15 | 0.14 |
| 28 | 0.79 |
| 50 | 0.188 |
| 94 | 0.032 |

The following compounds showed the IC₅₀ value of 1 µM or less in the similar test.
Compounds 4, 5, 6, 8, 9, 10, 14, 16, 17, 21, 44, 48, 51, 52, 53, 54, 55, 57, 58, 68, 70, 75, 77, 79, 88, 91, 92, 93, 95, 96, 97, 98, 101 and 102.

### Test Example 2: Measurement of β-amyloid (Aβ) production inhibitory effect in cell

Neuroblastoma SH-SY5Y cells (SH/APPwt) with human wild-type β-APP excessively expressed therein were prepared at 8 X 10⁵ cells/mL, and 150 µl portions thereof were inoculated into each well of a 96-well culture plate (Falcon). The cells were cultured for 2 hours at 37°C in a 5% gaseous carbon dioxide incubator. Then, a solution which had been preliminarily prepared by adding and suspending the test compound (DMSO (dimethyl sulfoxide) solution) so as to be 2 µl/50 µl medium was added to the cell sap. Namely, the final DMSO concentration was 1%, and the amount of the cell culture was 200 µl. After the incubation was performed for 24 hours from the addition of the test compound, 100 µl of the culture supernatant was collected from each fraction. The amount of the Aβ in each fraction was measured.
The Aβ amount was measured as follows. 10 µl of a homogeneous time resolved fluorescence (HTRF) measurement reagent (Amyloid β 1-40 peptide; IBA Molecular Holding, S.A.) and 10 µl of the culture supernatant were put into a 384-well half area microplate (black microplate, Costar) and mixed with each other, and then left standing overnight at 4°C while the light was shielded. Then, the fluorescence intensity (excitation wavelength: 337 nm, measurement wavelength: 620 nm and 665 nm) was measured with a Wallac 1420 multilabel counter (Perkin Elmer life sciences). The Aβ amount was determined from the count rate at each measurement wavelength (10000 X Count 665/Count 620), and the amount needed to inhibit Aβ production by 50 % (IC₅₀) was calculated from at least six different dosages. Table 18 shows the IC₅₀ value of each test compound.

**[Table 18]**

| Compound No. | IC₅₀ (µM) |
|---|---|
| 1 | 0.055 |
| 2 | 0.100 |
| 3 | 0.005 |
| 4 | 0.119 |
| 5 | 0.160 |
| 6 | 0.172 |
| 7 | 0.082 |
| 8 | 0.040 |
| 9 | 0.061 |
| 11 | 0.524 |
| 12 | 0.036 |
| 15 | 0.004 |
| 28 | 0.193 |
| 32 | 0.260 |
| 45 | 0.028 |
| 58 | 0.018 |
| 88 | 0.024 |

The following compounds showed the IC₅₀ value of 1 µM or less in the similar test.
Compounds 10, 14, 16, 17, 21, 22, 23, 24, 26, 27, 31, 33, 34, 35, 36, 37, 38, 40, 41, 42, 43, 44, 46, 47, 48, 49, 50, 51, 52, 53, 54, 55, 57, 64, 68, 70, 75, 77, 79, 82, 83, 84, 85, 90, 91, 92, 93, 94, 95, 96, 97, 98, 99, 100, 101 and 102.

### Test Example 3 Lowering effect on brain β amyloid in rats

A test compound is suspended in 0.5% methylcellulose, the final concentration is adjusted to 2 mg/mL, and this is orally administered to male Crj:SD rat (7 to 9 week old) at 10 mg/kg. In a vehicle control group, only 0.5% methylcellulose is administered, and an administration test is performed at 3 to 8 animals per group. A brain is isolated 3 hours after administration, a cerebral hemisphere is isolated, a weight thereof is measured, the hemisphere is rapidly frozen in liquid nitrogen, and stored at - 80°C until extraction date. The frozen cerebral hemisphere is transferred to a homogenizer made of Teflon (registered trade mark) under ice cooling, a 5-fold volume of a weight of an extraction buffer (containing 1% CHAPS ({3-[(3-chloroamidopropyl)dimethylammonio]-1-propanesulfonate}), 20 mM Tris-HCl (pH 8.0), 150 mM NaCl, Complete (Roche) protease inhibitor) is added, up and down movement is repeated, and this is homogenized to solubilize for 2 minutes. The suspension is transferred to a centrifugation tube, allowed to stand on an ice for 3 hours or more and, thereafter centrifuged at 100,000 x g and 4°C for 20 minutes. After centrifugation, the supernatant is transferred to an ELISA plate (Wako, product No. 294-62501) for measuring β amyloid 40. ELISA measurement is performed according to the attached instructions. The lowering effect is calculated as a ratio compared to the brain β amyloid 40 level of vehicle control group of each test.

### Test Example 4 CYP3A4 fluorescent MBI test

The CYP3A4 fluorescent MBI test is a test to examine enhancement of CYP3A4 inhibition by metabolic reaction of the compound. Here, E. coli-expressing CYP3A4 was used as an enzyme, and a reaction wherein 7-benzyloxy trifluoromethylcumarin (BFC) was debenzylated by the CYP3A4 enzyme into a fluorescent metabolite 7-hydroxytrifluoromethylcumarin (HFC) was employed as an indicator reaction. The reaction conditions were as follows.
Substrate: 5.6 µM/L 7-BFC
Pre-reaction time: 0 or 30 minutes
Reaction time: 15 minutes
Reaction temperature: 25°C (room temperature)
CYP3A4 content (E. coli-expressing enzyme): 62.5 pmol/mL at pre-reaction, 6.25 pmol/mL at reaction (10-fold diluted)
Test compound concentration: 0.625, 1.25, 2.5, 5, 10, and 20 µM (six different concentrations).
The enzyme and a test compound solution were added to a K-Pi buffer (pH 7.4) in the aforementioned amounts for the pre-reaction so that a pre-reaction solution was prepared, and this solution was put into a 96-well microplate. Part of this solution was transferred to another 96-well microplate such that it was diluted with the substrate and the K-Pi buffer to get a 10-fold diluted solution. NADPH, which is a coenzyme, was added so as to trigger the indicator reaction (without pre-reaction). As the reaction proceeded for a predetermined time period, acetonitrile/0.5 M Tris (tris hydroxyaminomethane) = 4/1 was added to terminate the reaction. NADPH was also added to the residual pre-reaction solution so as to trigger the pre-reaction (with pre-reaction). As the pre-reaction proceeded for a predetermined time period, part of the solution was transferred to another microplate so that the solution was diluted with the substrate and the K-Pi buffer to get a 10-fold diluted solution, and then the indicator reaction was triggered. As the reaction proceeded for a predetermined time period, acetonitrile/0.5 M Tris (tris hydroxyaminomethane) = 4/1 was added so as to terminate the reaction. With respect to each of the microplates on which the indicator reaction was performed, the fluorescence value of the metabolite 7-HFC was measured with a fluorescence plate reader (Ex = 420 nm, Em = 535 nm).
Control (100%) was prepared by adding only DMSO, which was used as a solvent to dissolve the drug, to the reaction system. The remaining activity (%) at each concentration after adding the test compound solution was calculated, and the IC₅₀ values of without pre reaction and with pre reaction were calculated by the formula: y=A+((B-A)/(1+((C/x)^{D}))) using XLfit. The IC₅₀ shift value was calculated by the formula: (the IC₅₀ value of without pre reaction)-(the IC₅₀ value of with pre reaction). The IC₅₀ shift values of the test compounds are shown in Table 19.

**[Table 19]**

| Compound No. | IC50 shift (µM) |
|---|---|
| 1 | 0 |
| 2 | 0 |
| 3 | 0 |
| 4 | 1 |
| 5 | -2 |
| 6 | -2 |
| 7 | <-2 |
| 8 | -4 |
| 9 | -5 |
| 11 | -2 |
| 12 | <-2 |
| 15 | <-3 |
| 18 | 0 |
| 28 | 1 |
| 30 | 0 |
| 32 | 0 |

### Test Example 5 hERG test

As risk assessment for QT prolongation in vitro, the action on delayed rectifier potassium current was studied using a HEK293 cell with a human delayed potassium ether-a-go-go related gene (hERG) channel expressed therein.
A cell was maintained at a membrane potential of -80 mV, then +50 mV of a depolarizing stimulus (2 seconds) was applied to the cell, further -50 mV of a repolarizing stimulus (2 seconds) was applied thereto and the induced peak tail current was measured by a whole-cell patch clamp technique. 5 µM of the test compounds was applied to the cells for 7 minutes, the inhibition rate before application of the test compound was calculated, and it was compared to the rate of vehicle group (0.5% dimethylsulfoxide solution). The inhibition rate of the test compounds are shown in Table 20.

**[Table 20]**

| Compound No. | Inhibition Rate (%) |
|---|---|
| 1 | 63.8 |
| 3 | 67.6 |
| 15 | 31.5 |
| 18 | 10.8 |
| 28 | 60.4 |
| 30 | 14.1 |
| 38 | 8.4 |
| 45 | 2.5 |

### Test Example 6 Ames test

Ames test was conducted by using TA1537 as strain without metabolic activation conditions in the pre-incubation method. The test showed the following compounds were detected as negative.

### Compounds 1, 2, 3 and 18.

### Test Example 7 CYP inhibition test

Using commercially available pooled human hepatic microsome, and employing, as markers, 7-ethoxyresorufin O-deethylation (CYP1A2), tolbutamide methyl-hydroxylation (CYP2C9), mephenytoin 4'-hydroxylation (CYP2C19), dextromethorphan O-demethylation (CYP2D6), and terfenedine hydroxylation as typical substrate metabolism reactions of human main five CYP enzyme forms (CYP1A2, 2C9, 2C19, 2D6, 3A4), an inhibitory degree of each metabolite production amount by a test compound was assessed.
The reaction conditions were as follows:
Substrate, 0.5 µmol/L ethoxyresorufin (CYP1A2), 100 µmol/L tolbutamide (CYP2C9), 50 µmol/L S-mephenitoin (CYP2C19), 5 µmol/L dextromethorphan (CYP2D6), 1 µmol/L terfenedine (CYP3A4); Reaction time, 15 minutes; Reaction temperature, 37°C; Enzyme, pooled human hepatic microsome 0.2 mg protein/mL; Test drug concentration, 1, 5, 10, 20 umol/L (four points).
Each five kinds of substrates, human hepatic microsome, or a test drug in 50 mM Hepes buffer as a reaction solution was added to a 96-well plate at the composition as described above, NADPH,a cofactor, was added to initiate metabolism reactions as markers and, after the incubation at 37°C for 15 minutes, a methanol/acetonitrile = 1/1 (v/v) solution was added to stop the reaction. After the centrifugation at 3000 rpm for 15 minutes, resorufin (CYP1A2 metabolite) in the supernatant was quantified by a fluorescent multilabel counter and tributamide hydroxide (CYP2CP metabolite), mephenytoin 4' hydroxide (CYP2C19 metabolite), dextromethorphan (CYP2D6 metabolite), and terfenadine alcohol (CYP3A4 metabolite) were quantified by LC/MS/MS.
Addition of only DMSO being a solvent dissolving a drug to a reaction system was adopted as a control (100%), remaining activity (%) was calculated at each concentration of a test drug added as the solution and IC₅₀ was calculated by reverse presumption by a logistic model using a concentration and an inhibition rate.

### (Result)

Compound 36: five kinds >20µM
Compound 41: five kinds >20µM

### Test Example 7 FAT Test

Each 20 µL of freeze-stored *Salmonella typhimurium* (TA98 and TA100 strain) was inoculated in 10 mL of liquid nutrient medium (2.5% Oxoid nutrient broth No.2), and the cultures were incubated at 37°C under shaking for 10 hours. 9 mL of TA98 culture was centrifuged (2000 x g, 10 minutes) to remove medium, and the bacteria was suspended in 9 mL of Micro F buffer (K₂HPO₄: 3.5 g/L, KH₂PO₄: 1 g/L, (NH₄)₂SO₄: 1 g/L, trisodium citrate dehydrate: 0.25 g/L, MgSO₄ · 7H₂O: 0.1 g/L), and the suspension was added to 110 mL of Exposure medium (Micro F buffer containing Biotin: 8 µg/mL, histidine: 0.2 µg/mL, glucose: 8 mg/mL). 3.16 mL of TA100 culture was added to 120 mL of Exposure medium to prepare the test bacterial solution. 588 µL of the test bacterial solution (or mixed solution of 498 ml of the test bacterial solution and 90 µL of the S9 mix in the case with metabolic activation system) were mixed with each 12 µL of the following solution: DMSO solution of the test substance (eight dose levels from maximum dose 50 mg/mL at 2-fold ratio); DMSO as negative control; 50 µg/mL of 4-nitroquinoline-1-oxide DMSO solution as positive control for TA98 without metabolic activation system; 0.25 µg/mL of 2-(furyl)-3-(5-nitro-2-furyl)acrylamide DMSO solution as positive control for TA100 without metabolic activation system; 40 µg/mL of 2-aminoanthracene DMSO solution as positive control for TA98 with metabolic activation system; or 20 µg/mL of 2-aminoanthracene DMSO solution as positive control for TA100 with metabolic activation system. 12 µL of the solution and 588 µL of the test bacterial solution (a mixed solution of 498 µl of the test bacterial solution and 90 µL of S9 mix with metabolic activation condition) were mixed and incubated at 37°C under shaking for 90 minutes. 460 µL of the bacterial solution exposed to the test substance was mixed with 2300 µL of Indicator medium (Micro F buffer containing biotin: 8 µg/mL, histidine: 0.2 µg/mL, glucose: 8 mg/mL, Bromo Cresol Purple: 37.5 µg/mL), each 50 µL was dispensed into 48 wells per dose in the microwell plates, and was subjected to stationary cultivation at 37°C for 3 days. A well containing the bacteria, which has obtained the ability of proliferation by mutation in the gene coding amino acid (histidine) synthetase, turns the color from purple to yellow due to pH change. The number of the yellow wells among the 48 total wells per dose was counted, and evaluated the mutagenicity by comparing with the negative control group. (-) means that mutagenicity is negative and (+) means positive.

### (Result)

Compound 44 (-)

### Test Example 8 Solubility Test

A 2-fold dilution series (12 points) of a 10 mM solution of a test compound in DMSO was added to a medium (JP-I, JP-II) (2%), and solubility was assessed by 3 stages (High; > 40 µM, Medium; 3-40 µM, Low; < 3 µM) from a turbidity after 4 hours (crystallization information).

### (Result)

Compound 28 High(JP-I)
Compound 31 High(JP-I)
Compound 40 High(JP-I)
Compound 45 High(JP-I)
Compound 76 High(JP-I)

### Test Example 8(2) Solubility test

The solubility of each compound is determined under 1% DMSO addition conditions. A 10 mM solution of the compound is prepared with DMSO, and 6 µL of the compound solution is added to 594 µL of an artificial intestinal juice (water and 118 mL of 0.2 mol/L NaOH reagent are added to 250 mL of 0.2 mol/L potassium dihydrogen phosphate reagent to reach 1000 mL) with a pH of 6.8. The mixture is left standing for 16 hours at 25°C, and the mixture is vacuum-filtered. The filtrate is two-fold diluted with methanol/water = 1/1, and the compound concentration in the filtrate is measured with HPLC or LC/MS/MS by the absolute calibration method.

### Test Example 9 Metabolic stability test

The target compound was reacted with commercially available pooled human liver microsomes for a predetermined time period. The residual rate was calculated by comparing the reacted sample and unreacted sample, and thus the degree of metabolism in liver was evaluated.
In 0.2 mL of a buffer (50 mmol/L tris-HCl pH 7.4, 150 mmol/L potassium chloride, 10 mmol/L magnesium chloride) containing human liver microsomes 0.5 mg protein/mL, reaction was carried out in the presence of 1 mmol/L NADPH for 0 minute or 30 minutes at 37°C (oxidative reaction). After the reaction, 50 µL of the reaction solution was added to and mixed with 100 µL of a solution of methanol/acetonitrile = 1/1 (v/v), and the mixture was centrifuged for 15 minutes at 3000 rpm. The test compound in the centrifuged supernatant was quantified by LC/MS/MS, and the residual amount of the test compound after the reaction was calculated based on the compound amount at 0-minute reaction set as 100%.

### (Results) .

Compound 50 100%
Compound 76 98.9%

### Test Example 9 Powder solubility test

Appropriate amounts of the test substances are put into appropriate containers. To the respective containers are added 200 µL of JP-1 fluid (sodium chloride 2.0 g, hydrochloric acid 7.0 mL and water to reach 1000 mL), 200 µL of JP-2 fluid (phosphate buffer (pH 6.8) 500 mL and water 500 mL), and 200 µL of 20 mmol/L TCA (sodium taurocholate)/JP-2 fluid (TCA 1.08 g and water to reach 100 mL). In the case that the test compound is dissolved after the addition of the test fluid, the bulk powder is added as appropriate. The containers are sealed, and shaken for 1 hour at 37°C. The mixtures are filtered, and 100 µL of methanol is added to each of the filtrate (100 µL) so that the filtrates are two-fold diluted. The dilution ratio may be changed if necessary. The dilutions are observed for bubbles and precipitates, and then the containers are sealed and shaken. Quantification is performed by HPLC with an absolute calibration method.

### Test Example 11 BA test

Materials and methods for studies on oral absorption
(1) Animal: SD rats
(2) Breeding conditions: rats were allowed to freely take solid feed and sterilized tap water
(3) Dose and grouping: orally or intravenously administered at a predetermined dose; grouping was as follows (Dose depends on the compound)
   Oral administration: 1 to 30 mg/kg (n=2 to 3)
   Intravenous administration: 0.5 to 10 mg/kg (n=2 to 3)
(4) Preparation of dosing solution: for oral administration, in a solution or a suspension state; for intravenous administration, in a solubilized state
(5) Administration method: in oral administration, forcedly administer into ventriculus with oral probe; in intravenous administration, administer from caudal vein with a needle-equipped syringe
(6) Evaluation items: blood was collected over time, and the plasma concentration of drug was measured by LC/MS/MS
(7) Statistical analysis: regarding the transition of the plasma concentration, the area under the plasma concentration-time curve (AUC) was calculated by non-linear least squares program WinNonlin (Registered trademark), and the bioavailability (BA) was calculated from the AUCs of the oral administration group and intravenous administration group

### (Result)

Compound 48: 87.2%
Compound 77: 75.0%

### Preparation Example 1

A granule containing the following ingredients is produced.

| | | |
|---|---|---|
| Ingredient | Compound represented by the formula (I) | 10 mg |
| | Lactose | 700 mg |
| | Corn starch | 274 mg |
| | HPC-L | 16 mg |
| | | 1000 mg |

The compound represented by the formula (I) and lactose are passed through a 60 mesh sieve. Corn starch is passed through a 120 mesh sieve. These are mixed with a V-type mixer. To the mixed powder is added a HPC-L (low viscosity hydroxypropylcellulose) aqueous solution, this is kneaded, granulated (extrusion granulation, pore diameter 0.5 to 1 mm), and dried. The resulting dry granule is passed through a vibration sieve (12/60 mesh) to obtain a granule.

### Preparation Example 2

A granule for filling a capsule containing the following ingredients is produced.

| | | |
|---|---|---|
| Ingredient | Compound represented by the formula (I) | 15 mg |
| | Lactose | 90 mg |
| | Corn starch | 42 mg |
| | HPC-L | 3 mg |
| | | 150 mg |

The compound represented by the formula (I), and lactose are passed through a 60 mesh sieve. Corn starch is passed through a 120 mesh sieve. These are mixed, a HPC-L solution is added to the mixed powder, this is kneaded, granulated, and dried. The resulting dry granule is adjusted in a size, and 150 mg of it is filled into a No.4 hard gelatin capsule.

### Preparation Example 3

A tablet containing the following ingredients is produced.

| | | |
|---|---|---|
| Ingredient | Compound represented by the formula (I) | 10 mg |
| | Lactose | 90 mg |
| | Microcrystalline cellulose | 30 mg |
| | CMC-Na | 15 mg |
| | Magnesium stearate | 5 mg |
| | | 150 mg |

The compound represented by the formula (I), lactose, microcrystalline cellulose, and CMC-Na (carboxymethylcellulose sodium salt) are passed through a 60 mesh sieve, and mixed. Magnesium stearate is mixed into the mixed powder to obtain a mixed powder for tabletting. The present mixed powder is directly compressed to obtain a 150 mg of a tablet.

### Preparation Example 4

The following ingredients are warmed, mixed, and sterilized to obtain an injectable.

| | | |
|---|---|---|
| Ingredient | Compound represented by the formula (I) | 3 mg |
| | Nonionic surfactant | 15 mg |
| | Purified water for injection | 1 ml |

### [Industrial Applicability]

The compound of the present appliaccation can be a useful therapeutic agent for diseases induced by production, secretion, and/or deposition of amyloid-β proteins.

## Claims

1. A compound of formula (I): 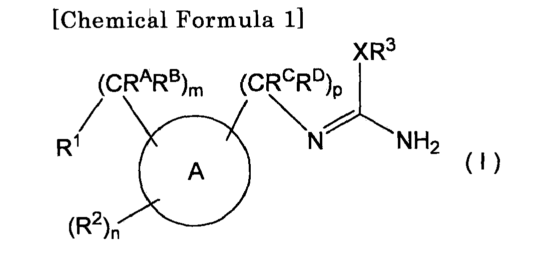 wherein X is an oxygen atom or a sulfur atom,
ring A is a carbocycle or a heterocycle,
(1) when X is an oxygen atom,
R¹ is substituted amino, provided that substituted amino is not imino(phenoxy)methylamino or imino(lower alkoxy)methylamino; substituted or unsubstituted mercapto; substituted or unsubstituted carbocyclyl; or substituted or unsubstituted heterocyclyl;
and R¹ may be hydrogen when ring A is naphthalene,
(2) when X is a sulfur atom,
R¹ is substituted or unsubstituted acylamino, provided that substituted or unsubstituted acylamino is not unsubstituted acetylamino or unsubstituted benzoylamino; substituted or unsubstituted halogenosulfonylamino; substituted or unsubstituted lower alkylsulfonylamino; substituted or unsubstituted lower alkenylsulfonylamino; substituted or unsubstituted lower alkynylsulfonylamino; substituted or unsubstituted acylsulfonylamino; substituted or unsubstituted carbocyclylsulfonylamino; substituted or unsubstituted heterocyclylsulfonylamino; substituted or unsubstituted mercapto; substituted or unsubstituted carbocyclyl; or substituted or unsubstituted heterocyclyl, provided that substituted or unsubstituted heterocyclyl is not thiazolyl substituted with unsubstituted acetylamino;
and R¹ may be hydrogen when ring A is naphthalene,
R² is halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted acyl, substituted or unsubstituted carbocyclyl,
or substituted or unsubstituted heterocyclyl,
R³ is substituted or unsubstituted lower alkyl, substituted or unsubstituted lower alkenyl, substituted or unsubstituted lower alkynyl, substituted or unsubstituted carbocyclyl, or substituted or unsubstituted heterocyclyl,
R^{A} and R^{B} are each independently hydrogen, halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted acyl, substituted or unsubstituted carbocyclyl, or substituted or unsubstituted heterocyclyl, or
R^{A} and R^{B} taken together may form oxo, or R^{A} and R^{B} together with the carbon atom to which they are attached may form a substituted or unsubstituted carbocycle or a substituted or unsubstituted heterocycle,
R^{C} and R^{D} are each independently hydrogen, halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted acyl, substituted or unsubstituted carbocyclyl, or substituted or unsubstituted heterocyclyl, or
R^{C} and R^{D} together with the carbon atom to which they are attached may form a substituted or unsubstituted carbocycle or a substituted or unsubstituted heterocycle, m is an integer of 0 to 2,
n is an integer of 0 to 4, and
p is an integer of 0 to 2,
its pharmaceutically acceptable salt, or a solvate thereof.

2. The compound according to claim 1 wherein
(1) when X is an oxygen atom, R¹ is substituted amino, provided that substituted amino is not imino(phenoxy)methylamino or imino(lower alkoxy)methylamino; substituted or unsubstituted mercapto; substituted or unsubstituted cycloalkyl; substituted or unsubstituted aryl; or substituted or unsubstituted heterocyclyl; and
(2) when X is a sulfur atom, R¹ is substituted or unsubstituted acylamino, provided that substituted or unsubstituted acylamino is not unsubstituted acetylamino or unsubstituted benzoylamino; substituted or unsubstituted halogenosulfonylamino; substituted or unsubstituted lower alkylsulfonylamino; substituted or unsubstituted lower alkenylsulfonylamino; substituted or unsubstituted lower alkynylsulfonylamino; substituted or unsubstituted acylsulfonylamino; substituted or unsubstituted carbocyclylsulfonylamino; substituted or unsubstituted heterocyclylsulfonylamino; substituted or unsubstituted mercapto; substituted or unsubstituted cycloalkyl; substituted or unsubstituted aryl; or substituted or unsubstituted heterocyclyl, provided that substituted or unsubstituted heterocyclyl is not thiazolyl substituted with unsubstituted acetylamino;
its pharmaceutically acceptable salt, or a solvate thereof.

3. The compound according to claim 1 or 2 wherein
ring A is an aromatic carbocycle or an aromatic heterocycle,
m is an integer of 0,
R¹ is substituted or unsubstituted acylamino, provided that when X is a sulfur atom, then substituted or unsubstituted acylamino is not unsubstituted acetylamino or unsubstituted benzoylamino;, and
R² is halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted acyl, substituted or unsubstituted carbocyclyl, or substituted or unsubstituted heterocyclyl,
its pharmaceutically acceptable salt or a solvate thereof

4. The compound according to any one of claims 1 to 3 wherein X is an oxygen atom, its pharmaceutically acceptable salt or a solvate thereof.

5. The compound according to any one of claims 1 to 3 wherein X is a sulfur atom, its pharmaceutically acceptable salt or a solvate thereof.

6. The compound according to any one of claims 1 to 5 wherein (i) ring A is a monocycle and p is 1 or 2, or (ii) ring A is a fused ring and p is 0, its pharmaceutically acceptable salt or a solvate thereof.

7. The compound according to any one of claims 1 to 6 wherein ring A is a benzene ring or a benzene ring fused with another ring, its pharmaceutically acceptable salt or a solvate thereof.

8. The compound according to any one of claims 1 to 7 wherein R¹ is substituted or unsubstituted acylamino, provided that when X is a sulfur atom, then substituted or unsubstituted acylamino is not unsubstituted acetylamino or unsubstituted benzoyl amino; substituted or unsubstituted halogenosulfonylamino; substituted or unsubstituted lower alkylsulfonylamino; substituted or unsubstituted lower alkenylsulfonylamino; substituted or unsubstituted lower alkynylsulfonylamino; substituted or unsubstituted acylsulfonylamino; substituted or unsubstituted carbocyclylsulfonylamino; substituted or unsubstituted heterocyclylsulfonylamino; or substituted or unsubstituted aryl; its pharmaceutically acceptable salt or a solvate thereof.

9. The compound according to any one of claims 1 to 8 wherein R¹ is substituted or unsubstituted acylamino, provided that when X is a sulfur atom, then "substituted or unsubstituted acylamino" is not unsubstituted acetylamino or unsubstituted benzoylamino; or substituted or unsubstituted lower alkylsulfonylamino; its pharmaceutically acceptable salt or a solvate thereof.

10. The compound according to any one of claims 1 to 9 wherein m is an integer of 0, its pharmaceutically acceptable salt or a solvate thereof.

11. The compound according to any one of claims 1 to 10 wherein R² is halogen and n is an integer of 0 or 1, its pharmaceutically acceptable salt or a solvate thereof.

12. The compound according to any one of claims 1 to 11 wherein R³ is substituted or unsubstituted lower alkyl or substituted or unsubstituted lower alkenyl, its pharmaceutically acceptable salt or a solvate thereof.

13. The compound according to any one of claims 1 to 12 wherein R^{C} and R^{D} are each independently hydrogen, substituted or unsubstituted lower alkyl, substituted or unsubstituted aryl or substituted or unsubstituted heterocyclyl, or R^{C} and R^{D} together with the carbon atom to which they are attached may form a substituted or unsubstituted carbocycle, and p is an integer of 1, its pharmaceutically acceptable salt or a solvate thereof.

14. A compound of formula (II): 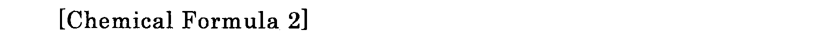 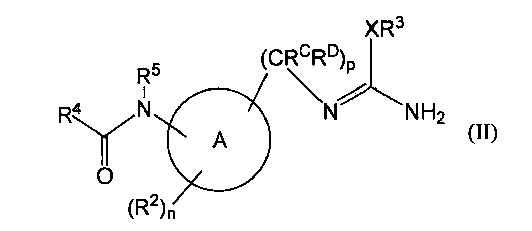 wherein ring A is a carbocycle or a heterocycle,
R⁵ is hydrogen, substituted or unsubstituted lower alkyl, substituted or unsubstituted lower alkenyl, substituted or unsubstituted lower alkynyl or substituted or unsubstituted acyl,
R⁴ is substituted or unsubstituted lower alkyl, provided that when X is a sulfur atom, then substituted or unsubstituted lower alkyl is not unsubstituted methyl; substituted or unsubstituted lower alkenyl; a substituted or unsubstituted carbocycle, provided that when X is a sulfur atom, then a substituted or unsubstituted carbocycle is not unsubstituted phenyl; or a substituted or unsubstituted heterocycle;
R² is halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted acyl, substituted or unsubstituted carbocyclyl or substituted or unsubstituted heterocyclyl,
R³ is substituted or unsubstituted lower alkyl, substituted or unsubstituted lower alkenyl, substituted or unsubstituted lower alkynyl, substituted or unsubstituted carbocyclyl or substituted or unsubstituted heterocyclyl,
R^{C} and R^{D} are each independently hydrogen, halogen, substituted or unsubstituted hydroxy, substituted or unsubstituted mercapto, substituted or unsubstituted amino, substituted or unsubstituted lower alkyl, substituted or unsubstituted lower alkenyl, substituted or unsubstituted lower alkynyl, substituted or unsubstituted acyl, substituted or unsubstituted carbocyclyl, or substituted or unsubstituted heterocyclyl, or R^{C} and R^{D} together with the carbon atom to which they are attached may form a substituted or unsubstituted carbocycle or a substituted or unsubstituted heterocycle,
n is an integer of 0 to 4, and
p is an integer of 0 to 2,
its pharmaceutically acceptable salt or a solvate thereof.

15. The compound according to claim 14 wherein R⁴ is a substituted or unsubstituted aromatic heterocycle or a substituted or unsubstituted aromatic carbocycle, its pharmaceutically acceptable salt or a solvate thereof.

16. The compound according to claim 14 or 15 wherein ring A is a monocycle, and R⁴ is a substituted or unsubstituted nitrogen-containing heterocycle, its pharmaceutically acceptable salt or a solvate thereof.

17. A pharmaceutical composition comprising the compound according to any one of claims 1 to 16, its pharmaceutically acceptable salt or a solvate thereof.

18. A pharmaceutical composition having BACE1 inhibitory activity comprising the compound according to any one of claims 1 to 16, its pharmaceutically acceptable salt or a solvate thereof.

19. A pharmaceutical composition having amyloid β production inhibitory activity comprising the compound according to any one of claims 1 to 16, its pharmaceutically acceptable salt or a solvate thereof.

20. A method for inhibiting BACE1 activity comprising administering the compound according to any one of claims 1 to 16, its pharmaceutically acceptable salt or a solvate thereof.

21. A compound according to any one of claims 1 to 16, its pharmaceutically acceptable salt or a solvate thereof for use in a method for inhibiting BACE1 activity.

22. A method for inhibiting amyloid β production comprising administering the compound according to any one of claims 1 to 16, its pharmaceutically acceptable salt or a solvate thereof.

23. A compound according to any one of claims 1 to 16, its pharmaceutically acceptable salt or a solvate thereof for use in a method for inhibiting amyloid β production.
